# EUROPEAN PATENT SPECIFICATION

(11) **EP 2 063 887 B1**
(45) Date of publication and mention of the grant of the patent: **29.05.2013**
(21) Application number: 07837579.7
(22) Date of filing: 30.08.2007
(51) Int. Cl.: A61K 31/407, A61K 31/497, C07D 209/96, C07D 211/06, A61K 31/5377, A61P 35/00

(54) **NEW SMALL MOLECULE INHIBITORS OF MDM2 AND THE USES THEREOF**
NEUE KLEINMOLEKÜLIGE MDM2-HEMMER UND VERWENDUNGEN DAVON
NOUVELLES PETITES MOLÉCULES INHIBITRICES DE MDM2 ET LEURS UTILISATIONS

(30) Priority: 30.08.2006 US 841150 P
(43) Date of publication of application: 03.06.2009
(73) Proprietor: The Regents of the University of Michigan, Ann Arbor, MI 48109-1280 (US)
(72) Inventor: WANG, Shaomeng, Saline, MI 48176 (US); QIN, Dongguang, Ann Arbor, MI 48105 (US); CHEN, Jianyong, Ann Arbor, MI 48105 (US); YU, Shanghai, Ann Arbor, MI 48105 (US)
(74) Representative: Glawe, Delfs, Moll
(86) International application number: PCT/US2007/019128
(87) International publication number: WO 2008/036168

(56) References cited:
- WO-A2-2006/091646
- DING KE ET AL: "Structure-Based Design of Spiro-oxindoles as Potent, Specific Small-Molecule Inhibitors of the MDM2-p53 Interaction" JOURNAL OF MEDICINAL CHEMISTRY, AMERICAN CHEMICAL SOCIETY, WASHINGTON, US LNKD- DOI:10.1021/JM051122A, vol. 49, no. 12, 1 January 2006 (2006-01-01), pages 3432-3435, XP002496820 ISSN: 0022-2623 [retrieved on 2006-05-13]
- DING KE ET AL: "Structure-based design of potent non-peptide MDM2 inhibitors" JOURNAL OF THE AMERICAN CHEMICAL SOCIETY, AMERICAN CHEMICAL SOCIETY, NEW YORK, US LNKD- DOI:10.1021/JA051147Z, vol. 127, no. 29, 27 July 2005 (2005-07-27), pages 10130-10131, XP002527584 ISSN: 0002-7863 [retrieved on 2005-06-30]
- DING K ET AL: "Synthesis of spirooxindoles via asymmetric 1,3-dipolar cycloaddition" TETRAHEDRON LETTERS, ELSEVIER, AMSTERDAM, NL LNKD- DOI:10.1016/J.TETLET.2005.06.114, vol. 46, no. 35, 29 August 2005 (2005-08-29), pages 5949-5951, XP004996267 ISSN: 0040-4039
- PELLEGRINI C., ET AL.: 'Total synthesisof (+)-elacomine and (-)-isoelacomine, two hitherto unnamed oxindole alkaloids from Elaeagnus commutata' HELVETICA CHIMICA ACTA vol. 79, no. 1, 1996, pages 151 - 168, XP003024151

## Description

The present application claims priority to U.S. Provisional Patent Application Serial No. 60/841,150, filed 08/30/2006,

### BACKGROUND OF THE INVENTION

### Field of the Invention

This invention is defined in the claims and is in the field of medicinal chemistry. In particular, the invention relates to small molecules which function as antagonists of the interaction between p53 and MDM2 and their use therapeutics for the treatment of cancer and other diseases.

### Related Art

The aggressive cancer cell phenotype is the result of a variety of genetic and epigenetic alterations leading to deregulation of intracellular signaling pathways (Ponder, Nature 411:336 (2001)). The commonality for all cancer cells, however, is their failure to execute an apoptotic program, and lack of appropriate apoptosis due to defects in the normal apoptosis machinery is a hallmark of cancer (Lowe et al., Carcinogenesis 21:485 (2000)). The inability of cancer cells to execute an apoptotic program due to defects in the normal apoptotic machinery is thus often associated with an increase in resistance to chemotherapy, radiation, or immunotherapy-induced apoptosis. Primary or acquired resistance of human cancer of different origins to current treatment protocols due to apoptosis defects is a major problem in current cancer therapy (Lowe et al., Carcinogenesis 21:485 (2000); Nicholson, Nature 407:810 (2000)). Accordingly, current and future efforts towards designing and developing new molecular target-specific anticancer therapies to improve survival and quality of life of cancer patients must include strategies that specifically target cancer cell resistance to apoptosis. In this regard, targeting crucial negative regulators that play a central role in directly inhibiting apoptosis in cancer cells represents a highly promising therapeutic strategy for new anticancer drug design.

The p53 tumor suppressor plays a central role in controlling cell cycle progression and apoptosis (Vogelstein et al., Nature 408:307 (2000)). It is an attractive therapeutic target for anticancer drug design because its tumor suppressor activity can be stimulated to eradicate tumor cells (Vogelstein et al., Nature 408:307 (2000); Chene, Nat. Rev. Cancer 3:102 (2003)). A new approach to stimulating the activity of p53 is through inhibition of its interaction with the protein MDM2 using non-peptide small molecule inhibitors (Chene, Nat. Rev. Cancer 3:102 (2003); Vassilev et al., Science 303:844 (2004)). MDM2 and p53 are part of an auto-regulatory feed-back loop (Wu et al., Genes Dev. 7:1126 (1993)). MDM2 is transcriptionally activated by p53 and MDM2, in turn, inhibits p53 activity by at least three mechanisms (Wu et al., Genes Dev. 7:1126 (1993). First, MDM2 protein directly binds to the p53 transactivation domain and thereby inhibits p53-mediated transactivation. Second, MDM2 protein contains a nuclear export signal sequence, and upon binding to p53, induces the nuclear export of p53, preventing p53 from binding to the targeted DNAs. Third, MDM2 protein is an E3 ubiquitin ligase and upon binding to p53 is able to promote p53 degradation. Hence, by functioning as a potent endogenous cellular inhibitor of p53 activity, MDM2 effectively inhibits p53-mediated apoptosis, cell cycle arrest and DNA repair. Therefore, small-molecule inhibitors that bind to MDM2 and block the interaction between MDM2 and p53 can promote the activity of p53 in cells with a functional p53 and stimulate p53-mediated cellular effects such as cell cycle arrest, apoptosis, or DNA repair (Chene, Nat. Rev. Cancer 3:102 (2003); Vassilev et al.. Science 303:844 (2004))

Although high-affinity peptide-based inhibitors have been successfully designed in the past (Garcia-Echeverria et al., Med. Chem 43:3205 (2000)), these inhibitors are not drug-like molecules because of their poor cell permeability and *in vivo* bioavailability. Despite intensive efforts by the pharmaceutical industry, high throughput screening strategies have had very limited success in identifying potent, non-peptide small molecule inhibitors. Accordingly, there is a need for non-peptide, drug-like, small molecule inhibitors of the p53-MDM2 interaction.

The design of non-peptide small-molecule inhibitors that target the p53-MDM2 interaction is currently being pursued as an attractive strategy for anti-cancer drug design (Chene, Nat. Rev. Cancer 3:102 (2003); Vassilev et al. Science 303:844 (2004); Diug et al., J.Med. chem. 49: 3432-34-35 (2006); Diug et al., J.An.Cliene. Soc. 127: 10130-10131 (2005); Diug et al., Tetrahedron Wetters 46: 5959-5951 (2005)). structural basis of this interaction has been established by x-ray crystallography (Kussie et al., Science 274:948 (1996)). The crystal structure shows that the interaction between p53 and MDM2 is primarily mediated by three hydrophobic residues (Phe19, Trp23 and Leu26) from p53 and a small, deep hydrophobic cleft in MDM2. This hydrophobic cleft is an ideal site for designing small-molecule inhibitors that can disrupt the p53-MDM2 interaction (Chene, Nat. Rev. Cancer 3:102 (2003)).

### SUMMARY OF THE INVENTION

It is generally accepted that the inability of cancer cells or their supporting cells to undergo apoptosis in response to genetic lesions or exposure to inducers of apoptosis (such as anticancer agents and radiation) is a major factor in the onset and progression of cancer. The induction of apoptosis in cancer cells or their supporting cells (*e.g*., neovascular cells in the tumor vasculature) is thought to be a universal mechanism of action for virtually all of the effective cancer therapeutic drugs or radiation therapies on the market or in practice today. One reason for the inability of a cell to undergo apoptosis is a decrease in the tumor suppressor activity of p53, which in many instances is due to the inhibitory actions of MDM2 on p53 in tumor cells containing functional p53. The inhibition of p53 activity results in alterations in apoptosis pathways as well as cell cycle regulation.

The present invention is defined in the claims and contemplates that exposure of animals suffering from cancer to therapeutically effective amounts of drug(s) (*e.g.*, small molecules) that increase the function(s) of p53 and p53-related proteins (*e.g.*, p63, p73) by inhibiting the interaction between p53 or p53-related proteins and MDM2 or MDM2-related proteins (*e.g.*, MDMX) will inhibit the growth of cancer cells or supporting cells outright and/or render such cells as a population more susceptible to the cell death-inducing activity of cancer therapeutic drugs or radiation therapies. In particular, the inhibitors of the invention may prolong the half-life of p53 by interfering with the p53-MDM2 interaction that would normally promote degradation of p53. The present invention contemplates that inhibitors of the interaction between p53 or p53-related proteins and MDM2 and MDM2-related proteins satisfy an unmet need for the treatment of multiple cancer types, either when administered as monotherapy to induce cell growth inhibition, apoptosis and/or cell cycle arrest in cancer cells, or when administered in a temporal relationship with additional agent(s), such as other cell death-inducing or cell cycle disrupting cancer therapeutic drugs or radiation therapies (combination therapies), so as to render a greater proportion of the cancer cells or supportive cells susceptible to executing the apoptosis program compared to the corresponding proportion of cells in an animal treated only with the cancer therapeutic drug or radiation therapy alone.

In certain examples relating to the invention, combination treatment of animals with a therapeutically effective amount of a compound of the present invention and a course of an anticancer agent or radiation produces a greater tumor response and clinical benefit in such animals compared to those treated with the compound or anticancer drugs/radiation alone. Put another way, because the compounds will lower the apbptotic threshold of all cells, the proportion of cells that will successfully execute the apoptosis program in response to the apoptosis inducing activity of anticancer drugs/radiation is increased. Alternatively, the compounds of the present invention will be used to allow administration of a lower, and therefore less toxic and more tolerable, dose of an anticancer agent and/or radiation to produce the same tumor response/clinical benefit as the conventional dose of the anticancer agent/radiation alone. Since the doses for all approved anticancer drugs and radiation treatments are known, the present invention contemplates the various combinations of them with the present compounds. Also, since the compounds of the present invention may act at least in part by stimulating the pro-apoptotic and/or cell cycle-inhibiting activities of p53 and p53-related proteins, the exposure of cancer cells and supporting cells to therapeutically effective amounts of the compounds should be temporally linked to coincide with the attempts of cells to execute the apoptosis program in response to the anticancer agent or radiation therapy. Thus, in some examples administering the compositions of the present invention in connection with certain temporal relationships, relates to especially efficacious therapeutic practices.

In other examples relating to the invention, inhibitors of the interaction between p53 or p53-related proteins and MDM2 and MDM2-related proteins may protect normal (*e.g.*, non-hyperproliferative) cells from the toxic effects of certain chemotherapeutic agents and radiation, possibly through the ability of the inhibitors to induce cell cycle arrest. In particular, the inhibitors of the invention may cause cell cycle arrest in cells comprising wild-type p53 while having no effect on cancer cells comprising mutated or deleted p53. This differential protective effect may allow for more effective treatment of cancer by allowing the use of higher doses or longer treatments of chemotherapeutic agents or treatments without increasing the toxic side effects of such treatment.

The present invention is defined in the claims and relates to compounds that are useful for inhibiting the interaction between p53 or p53-related proteins and MDM2 or MDM2-related proteins and increasing the sensitivity of cells to inducers of apoptosis and/or cell cycle arrest. Examples which are not covered by the claims are used as illustrative examples which are useful for understanding the invention but are not embodiment of the invention. In one particular examples the compounds have Formula I: 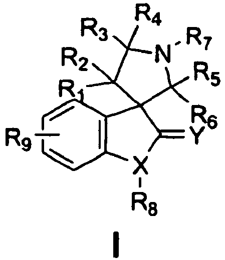 or a pharmaceutically acceptable salt or prodrug thereof, wherein:
X is CH, O, N, or S, wherein R₈ is absent if X is O or S;
Y is O, S, or NR';
R₁, R₂, R₃, R₄, R₅, R₆, and R₇ are independently H or optionally substituted alkyl, cycloalkyl, alkenyl, cycloalkenyl, alkynyl, aryl, heterocyclic, CO₂R', OCOR', CONR'R", NR"COR', NR'SO₂R", SO₂NR'R", (C=NR')NR"R"', or NR'R"; or
R₇ forms an aryl, cycloalkyl, or heterocyclic group with one of R₅ or R₆;
R₈ is H or optionally substituted alkyl, cycloalkyl, alkenyl, cycloalkenyl, alkynyl, aryl, heterocyclic, CO₂R', OCOR', CONR'R", SO₂NR'R", or (C=NR')NR"R"';
R₉ represents a 6-chloro and a 5-fluoro group; and
each R', R" and R'" is independently H or optionally substituted alkyl, cycloalkyl, alkenyl, cycloalkenyl, alkynyl, aryl, or heterocyclic; or
R' and R", or R" and R"', form a ring;
wherein one of R₃ and R₄ is CONRR', and one of R and R' is an optionally substituted cycloalkyl-alkyl or monocyclo-heterocycloalkyl group or a dihydroxyalkyl amino group not containing a hydroxyl group at the 3-position of the alkyl group.

The invention is defined in the claims and relates to compounds represented by Formula I, which are inhibitors of the interaction between p53 or p53-related proteins and MDM2 or MDM2-related proteins. The invention relates to the use of the compounds of the invention to induce cell cycle arrest and/or apoptosis in cells containing functional p53 or p53-related proteins. The invention also relates to the use of the compounds of the invention for sensitizing cells to additional agent(s), such as inducers of apoptosis and/or cell cycle arrest, and chemoprotection of normal cells through the induction of cell cycle arrest prior to treatment with chemotherapeutic agents. In one example the invention relates to methods of rendering a normal cell resistant to chemotherapeutic agents or treatments, comprising contacting the cell with a compound of the invention. In one example the invention relates to methods of protecting normal cells in an animal with a hyperproliferative disease from the toxic side effects of chemotherapeutic agents or treatments, comprising administering to said animal a compound of the invention. In a particular example, the of invention is related to the treatment, amelioration, or prevention of disorders, side effects, or conditions caused by the administration of chemotherapeutic agents to normal noncancerous cells by administering to an animal undergoing chemotherapy a compound of the present invention. Examples of such disorders and conditions caused by chemotherapy include, without limitation, mucositis, stomatitis, xerostomia, gastrointestinal disorders, and alopecia.

The compounds of the invention are useful for usein the treatment, amelioration, or prevention of disorders, such as those responsive to induction of apoptotic cell death, *e.g.,* disorders characterized by dysregulation of apoptosis, including hyperproliferative diseases such as cancer. In certain examples the compounds can be used to treat, ameliorate, or prevent cancer that is characterized by resistance to cancer therapies (*e.g.,* those cancer cells which are chemoresistant, radiation resistant, hormone resistant, and the like). In other examples, the compounds can be used to treat hyperproliferative diseases characterized by expression of functional p53 or p53-related proteins. In other examples the invention relates to the use of the compounds of the invention to protect normal (*e.g.,* non-hyperproliferative) cells from the toxic side effects of chemotherapeutic agents and treatments by the induction of cell cycle arrest in those cells.

The present invention is defined in the claims and provides pharmaceutical compositions comprising a compound of Formula I in a therapeutically effective amount to induce apoptosis in cells or to sensitize cells to inducers of apoptosis.

The invention further related to kits comprising a compound of Formula I and instructions for administering the compound to an animal. The kits may optionally contain other therapeutic agents, *e.g.,* anticancer agents or apoptosis-modulating agents.

The invention also relates to methods of making compounds of Formula I.

### DETAILED DESCRIPTION OF THE INVENTION

The present invention relates to compounds represented by Formula I, which function as inhibitors of the interaction between p53 or p53-related proteins and MDM2 or MDM2-related proteins. By inhibiting the negative effect of MDM2 or MDM2-related proteins on p53 or p53-related proteins, these compounds sensitize cells to inducers of apoptosis and/or cell cycle arrest and, in some instances, themselves induce apoptosis and/or cell cycle arrest. Therefore, the invention relates to methods of sensitizing cells to inducers of apoptosis and/or cell cycle arrest and to methods of inducing apoptosis and/or cell cycle arrest in cells, comprising contacting the cells with a compound of Formula I alone or in combination with additional agent(s), *e.g.,* an inducer of apoptosis or a cell cycle disrupter. The invention further relates to methods of treating, ameliorating, or preventing disorders in an animal, such as those that are responsive to induction of apoptosis, comprising administering to the animal a compound of Formula I and additional agent(s), *e.g.,* an inducer of apoptosis. Such disorders include those characterized by a dysregulation of apoptosis and those characterized by the proliferation of cells expressing functional p53 or p53-related proteins. In other examples, the invention relates to methods of protecting normal (*e.g.,* non-hyperproliferative) cells in an animal from the toxic side effects of chemotherapeutic agents and treatments comprising administering to the animal a compound of Formula I.

The terms "anticancer agent" and "anticancer drug," as used herein, refer to any therapeutic agents (*e.g.,* chemotherapeutic compounds and/or molecular therapeutic compounds), antisense therapies, radiation therapies, or surgical interventions, used in the treatment of hyperproliferative diseases such as cancer (*e.g.,* in mammals).

The term "prodrug," as used herein, refers to a pharmacologically inactive derivative of a parent "drug" molecule that requires biotransformation (*e.g.,* either spontaneous or enzymatic) within the target physiological system to release, or to convert (*e.g.,* enzymatically, physiologically, mechanically, electromagnetically) the prodrug into the active drug. Prodrugs are designed to overcome problems associated with stability, toxicity, lack of specificity, or limited bioavailability. Exemplary prodrugs comprise an active drug molecule itself and a chemical masking group (*e.g.,* a group that reversibly suppresses the activity of the drug). Some prodrugs are variations or derivatives of compounds that have groups cleavable under metabolic conditions. Exemplary prodrugs become pharmaceutically active *in vivo* or *in vitro* when they undergo solvolysis under physiological conditions or undergo enzymatic degradation or other biochemical transformation (*e.g.,* phosphorylation, hydrogenation, dehydrogenation, glycosylation). Prodrugs often offer advantages of solubility, tissue compatibility, or delayed release in the mammalian organism. *(See e.g.,* Bundgard, Design of Prodrugs, pp. 7-9, 21-24, Elsevier, Amsterdam (1985); and Silverman, The Organic Chemistry of Drug Design and Drug Action, pp. 352-401, Academic Press, San Diego, CA (1992)). Common prodrugs include acid derivatives such as esters prepared by reaction of parent acids with a suitable alcohol (*e.g.,* a lower alkanol), amides prepared by reaction of the parent acid compound with an amine, or basic groups reacted to form an acylated base derivative (*e.g.,* a lower alkylamide).

The term "pharmaceutically acceptable salt," as used herein, refers to any salt (*e.g.,* obtained by reaction with an acid or a base) of a compound of the present invention that is physiologically tolerated in the target animal (*e.g.,* a mammal). Salts of the compounds of the present invention may be derived from inorganic or organic acids and bases. Examples of acids include, but are not limited to, hydrochloric, hydrobromic, sulfuric, nitric, perchloric, fumaric, maleic, phosphoric, glycolic, lactic, salicylic, succinic, toluene-p-sulfonic, tartaric, acetic, citric, methanesulfonic, ethanesulfonic, formic, benzoic, malonic, sulfonic, naphthalene-2-sulfonic, benzenesulfonic acid, and the like. Other acids, such as oxalic, while not in themselves pharmaceutically acceptable, may be employed in the preparation of salts useful as intermediates in obtaining the compounds of the invention and their Pharmaceutically acceptable acid addition salts.

Examples of bases include, but are not limited to, alkali metal (*e.g.,* sodium) hydroxides, alkaline earth metal (*e.g.,* magnesium) hydroxides, ammonia, and compounds of formula NW₄⁺, wherein W is C₁₋₄ alkyl, and the like.

Examples of salts include, but are not limited to: acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, fumarate, flucoheptanoate, glycerophosphate, hemisulfate, heptanoate, hexanoate, chloride, bromide, iodide, 2-hydroxyethanesulfonate, lactate, maleate, mesylate, methanesulfonate, 2-naphthalenesulfonate, nicotinate, oxalate, palmoate, pectinate, persulfate, phenylpropionate, picrate, pivalate, propionate, succinate, tartrate, thiocyanate, tosylate, undecanoate, and the like. Other examples of salts include anions of the compounds of the present invention compounded with a suitable cation such as Na⁺, NH₄⁺, and NW₄⁺ (wherein W is a C₁₋₄ alkyl group), and the like. For therapeutic use, salts of the compounds of the present invention are contemplated as being pharmaceutically acceptable. However, salts of acids and bases that are non-pharmaceutically acceptable may also find use, for example, in the preparation or purification of a pharmaceutically acceptable compound.

The term "therapeutically effective amount," as used herein, refers to that amount of the therapeutic agent sufficient to result in amelioration of one or more symptoms of a disorder, or prevent advancement of a disorder, or cause regression of the disorder. For example, with respect to the treatment of cancer, in one example, a therapeutically effective amount will refer to the amount of a therapeutic agent that decreases the rate of tumor growth, decreases tumor mass, decreases the number of metastases, increases time to tumor progression, or increases survival time by at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or at least 100%.

The terms "sensitize" and "sensitizing," as used herein, refer to making, through the administration of a first agent (*e.g.,* a compound of Formula I), an animal or a cell within an animal more susceptible, or more responsive, to the biological effects (*e.g.,* promotion or retardation of an aspect of cellular function including, but not limited to, cell division, cell growth, proliferation, invasion, angiogenesis, necrosis, or apoptosis) of a second agent. The sensitizing effect of a first agent on a target cell can be measured as the difference in the intended biological effect (*e.g.*, promotion or retardation of an aspect of cellular function including, but not limited to, cell growth, proliferation, invasion, angiogenesis, or apoptosis) observed upon the administration of a second agent with and without administration of the first agent. The response of the sensitized cell can be increased by at least 10%, at least 20%, at least 30%, at least 40%, at least 50%, at least 60%, at least 70%, at least 80%, at least 90%, at least 100%, at least 150%, at least 200%, at least 350%, at least 300%, at least 350%, at least 400%, at least 450%, or at least 500% over the response in the absence of the first agent.

The term "dysregulation of apoptosis," as used herein, refers to any aberration in the ability of (*e.g.*, predisposition) a cell to undergo cell death via apoptosis. Dysregulation of apoptosis is associated with or induced by a variety of conditions, non-limiting examples of which include, autoimmune disorders (*e.g.,* systemic lupus erythematosus, rheumatoid arthritis, graft-versus-host disease, myasthenia gravis, or Sjögren's syndrome), chronic inflammatory conditions (*e.g.,* psoriasis, asthma or Crohn's disease), hyperproliferative disorders (*e.g.,* tumors, B cell lymphomas, or T cell lymphomas), viral infections (*e.g.,* herpes, papilloma, or HIV), and other conditions such as osteoarthritis and atherosclerosis. It should be noted that when the dysregulation is induced by or associated with a viral infection, the viral infection may or may not be detectable at the time dysregulation occurs or is observed. That is, viral-induced dysregulation can occur even after the disappearance of symptoms of viral infection.

The term "functional p53," as used herein, refers to wild-type p53 expressed at normal, high, or low levels and mutant p53 that retains at least 5% of the activity of wild-type p53, e.g., at least 10%, 20%, 30%, 40%, 50%, or more of wild-type activity.

The term "p53-related protein," as used herein, refers to proteins that have at least 25% sequence homology with p53, have tumor suppressor activity, and are inhibited by interaction with MDM2 or MDM2-related proteins. Examples of p53-related proteins include, but are not limited to, p63 and p73.

The term "MDM2-related protein," as used herein, refers to proteins that have at least 25% sequence homology with MDM2, and interact with and inhibit p53 or p53-related proteins. Examples of MDM2-related proteins include, but are not limited to, MDMX and HDM2.

The term "hyperproliferative disease," as used herein, refers to any condition in which a localized population of proliferating cells in an animal is not governed by the usual limitations of normal growth. Examples of hyperproliferative disorders include tumors, neoplasms, lymphomas and the like. A neoplasm is said to be benign if it does not undergo invasion or metastasis and malignant if it does either of these. A "metastatic" cell means that the cell can invade and destroy neighboring body structures. Hyperplasia is a form of cell proliferation involving an increase in cell number in a tissue or organ without significant alteration in structure or function. Metaplasia is a form of controlled cell growth in which one type of fully differentiated cell substitutes for another type of differentiated cell.

The pathological growth of activated lymphoid cells often results in an autoimmune disorder or a chronic inflammatory condition. As used herein, the term "autoimmune disorder" refers to any condition in which an organism produces antibodies or immune cells which recognize the organism's own molecules, cells or tissues. Non-limiting examples of autoimmune disorders include autoimmune hemolytic anemia, autoimmune hepatitis, Berger's disease or IgA nephropathy, celiac sprue, chronic fatigue syndrome, Crohn's disease, dermatomyositis, fibromyalgia, graft versus host disease, Grave's disease, Hashimoto's thyroiditis, idiopathic thrombocytopenia purpura, lichen planus, multiple sclerosis, myasthenia gravis, psoriasis, rheumatic fever, rheumatic arthritis, scleroderma, Sjögren's syndrome, systemic lupus erythematosus, type I diabetes, ulcerative colitis, vitiligo, and the like.

The term "neoplastic disease," as used herein, refers to any abnormal growth of cells being either benign (non-cancerous) or malignant (cancerous).

The term "normal cell," as used herein, refers to a cell that is not undergoing abnormal growth or division. Normal cells are non-cancerous and are not part of any hyperproliferative disease or disorder.

The term "anti-neoplastic agent," as used herein, refers to any compound that retards the proliferation, growth, or spread of a targeted (*e.g.,* malignant) neoplasm.

The terms "prevent," "preventing," and "prevention," as used herein, refer to a decrease in the occurrence of pathological cells (*e.g.,* hyperproliferative or neoplastic cells) in an animal. The prevention may be complete, *e.g.,* the total absence of pathological cells in a subject. The prevention may also be partial, such that the occurrence of pathological cells in a subject is less than that which would have occurred without the present invention.

The term "apoptosis-modulating agents," as used herein, refers to agents which are involved in modulating (*e.g.,* inhibiting, decreasing, increasing, promoting) apoptosis. Examples of apoptosis-modulating agents include proteins which comprise a death domain such as, but not limited to, Fas/CD95, TRAMP, TNF RI, DR1, DR2, DR3, DR4, DR5, DR6, FADD, and RIP. Other examples of apoptosis-modulating agents include, but are not limited to, TNFα, Fas ligand, antibodies to Fas/CD95 and other TNF family receptors, TRAIL (also known as Apo2 Ligand or Apo2L/TRAIL), antibodies to TRAIL-R1 or TRAIL-R2, Bcl-2, p53, BAX, BAD, Akt, CAD, PI3 kinase, PP1, and caspase proteins. Modulating agents broadly include agonists and antagonists of TNF family receptors and TNF family ligands. Apoptosis-modulating agents may be soluble or membrane bound (e.g. ligand or receptor). Apoptosis-modulating agents include those which are inducers of apoptosis, such as TNF or a TNF-related ligand, particularly a TRAMP ligand, a Fas/CD95 ligand, a TNFR-1 ligand, or TRAIL.

Examples which are not covered by the claims are used as illustrative examples which are useful for understanding the invention but are not embodiments of the invention. In one aspect of the invention, the inhibitors of the interaction between p53 and MDM2 are compounds of Formula I: 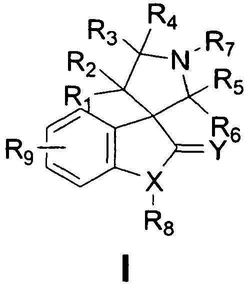 or a pharmaceutically acceptable salt or prodrug thereof, wherein:
X is CH, O, N, or S, wherein R₈ is absent if X is O or S;
Y is O, S, or NR';
R₁, R₂, R₃, R₄, R₅, R₆, and R₇ are independently H or optionally substituted alkyl, cycloalkyl, alkenyl, cycloalkenyl, alkynyl, aryl, heterocyclic, CO₂R', OCOR', CONR'R", NR"COR', NR'SO₂R", SO₂NR'R", (C=NR')NR"R"', or NR'R"; or
R₇ forms an aryl, cycloalkyl, or heterocyclic group with one of R₅ or R₆;
R₈ is H or optionally substituted alkyl, cycloalkyl, alkenyl, cycloalkenyl, alkynyl, aryl, heterocyclic, CO₂R', OCOR', CONR'R", SO₂NR'R", or (C=NR')NR"R"';
R₉ represents a 6-chloro and a 5-fluoro group; and
each R', R" and R"' is independently H or optionally substituted alkyl, cycloalkyl, alkenyl, cycloalkenyl, alkynyl, aryl, or heterocyclic; or
R' and R", or R" and R"', form a ring;
wherein one of R₃ and R₄ is CONRR', and one of R and R' is an optionally substituted cycloalkyl-alkyl or monocyclo-heterocycloalkyl group or a dihydroxyalkyl amino group not containing a hydroxyl group at the 3-position of the alkyl group.

In a more particular embodiment, one of R₁ and R₂ of Formula I is a substituted or unsubstituted aryl (e.g., phenyl), substituted or unsubstituted heteroaryl, cycloalkyl, straight or branched alkyl, amide or ester.

In another embodiment, one of R₅ and R₆ is a C₃₋₁₈ alkyl group, *e.g*., propyl, isopropyl, *sec*-butyl, *tert*-butyl, isopentyl, cyclopentyl, norbornyl, or adamantyl, or a 5- or 6-membered aryl or heteroaryl group.

In another embodiment, the compounds of Formula I have a stereochemical structure as shown in Formula II or Formula III: 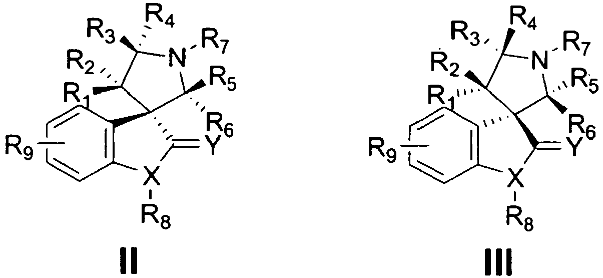 or a pharmaceutically acceptable salt or prodrug thereof.

In one embodiment, the compounds of Formula I have Formula IV: 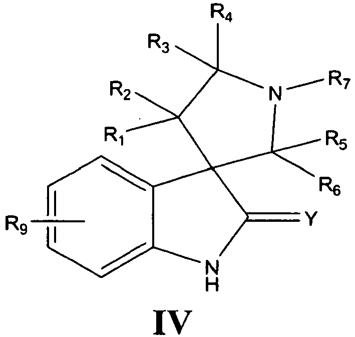 wherein R₁-R₉ and Y are as defined above.

In another embodiment, the compounds of Formula IV have a stereochemical structure as shown in Formula V or Formula VI: 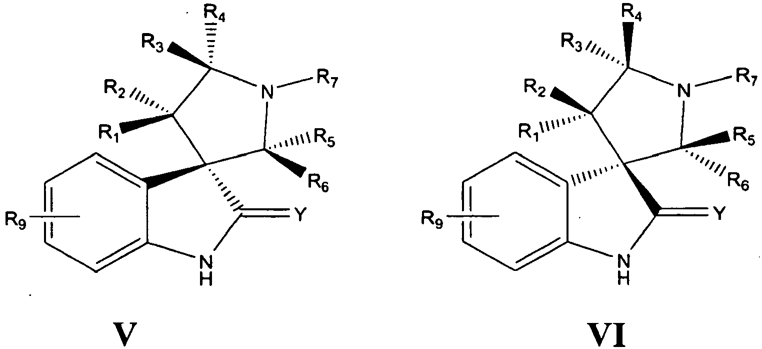 or a pharmaceutically acceptable salt or prodrug thereof.

In one embodiment, the compounds of Formula I have Formula VII: 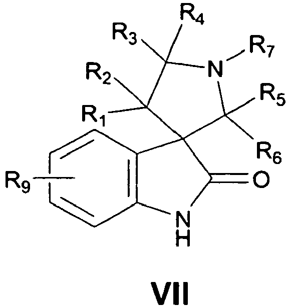 wherein R₁-R₉ are as defined above.

In another embodiment, the compounds of Formula VII have a stereochemical structure as shown in Formula VIII or Formula IX: 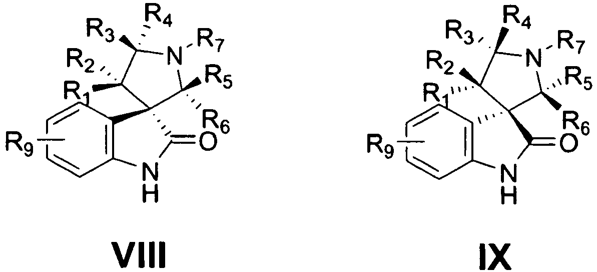 or a pharmaceutically acceptable salt or prodrug thereof.

In one embodiment, the compounds of Formula I have Formula X: 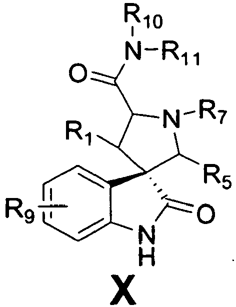 wherein:
R₁, R₅, R₇, and R₉ are as defined above;
R₁₀ is H; and
R₁₁ is an optionally substituted cycloalkyl-alkyl or monocyclo-heterocycloalkyl group or a dihydroxyalkyl amino group not containing a hydroxyl group at the 3-position of the alkyl group;
or R₁₀ and R₁₁ together form an optionally substituted monocyclo-heterocycloalkyl group.

Examples of substituted cycloalkyl-alkyl groups include C₁₋₆ alkyl substituted by a cycloalkyl group as described herein below which is substituted further by one or more hydroxyl groups. Examples of heterocycloalkyl groups include C₁₋₆ alkyl substituted by a monocyclo-heterocycloalkyl group as described herein below.

Particular examples of cycloalkyl-alkyl groups include 2-(3-hydroxycyclopentyl)ethylamino; and 3-(3-hydroxycyclopentyl)propylamino groups. The respective hydroxyl groups may have either the R or S configurations.

Particular examples of substituted heterocycloalkyl groups include 2-(1-morpholinyl)ethylamino and 3-(1-morpholinyl)propylamino groups.

Particular examples of dihydroxyalkylamino groups include R and S 4,5-dihydroxypentylamino, and 4-hydroxy-3-(methylhydroxy)butylamino groups.

It is expected that when a hydroxyl group is not present at the 3-position of the alkyl group, the compound may exhibit improved stability during its preparation. Without being limited to any particular theory, the improved stability may result from the absence of a nucleophilic 3-hydroxyl group which may displace the amino group of the amide thus forming a 6-membered ester containing ring. It is believed that dihydroxyalkyl groups not containing a 3-hydroxy group will not participate in such a reaction and will be more stable and able to be made in higher yield.

In a further embodiment, the compounds of Formula I have one of Formulae XI-XXVI: 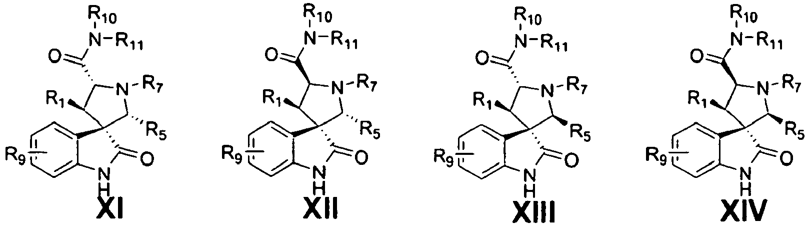 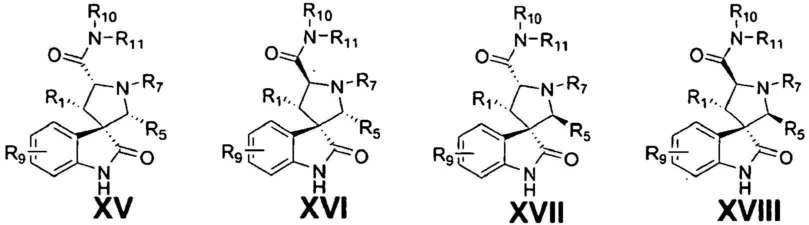 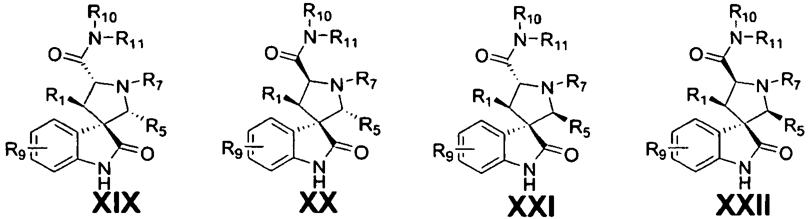 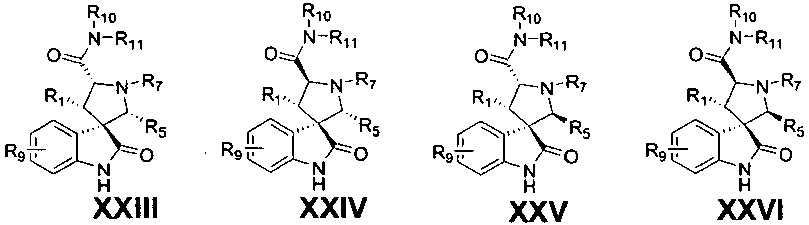 wherein R₁, R₅, R₇, R₉, R₁₀, and R₁₁ are as defined above.

In another embodiment, the compounds of Formula I have one of Formulae XXVII and XXVIII: 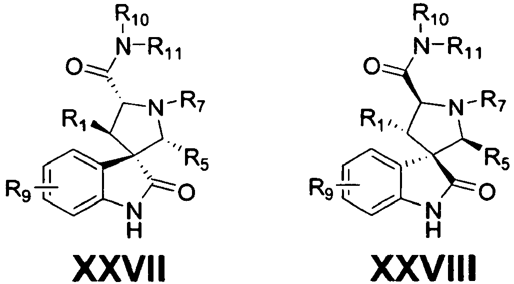 wherein R₁, R₅, R₇, R₉, R₁₀, and R₁₁ are as defined above.

In another embodiment, the compounds of Formula I have Formula XLVIII: 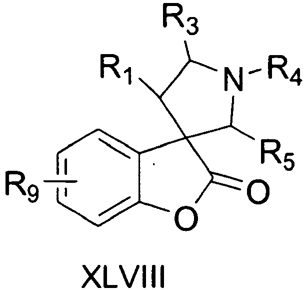 wherein:
R₁, R₃, R₄, R₅ and R₉ are as defined above.

In a further embodiment, the compounds of Formula I have one of Formulae XLIX-LXIV: 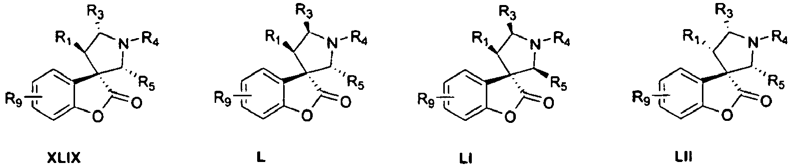 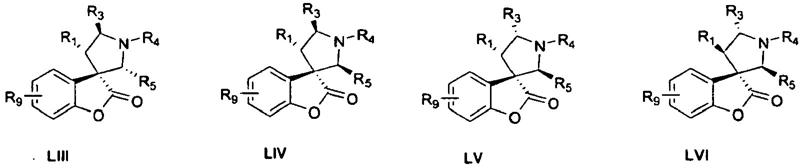 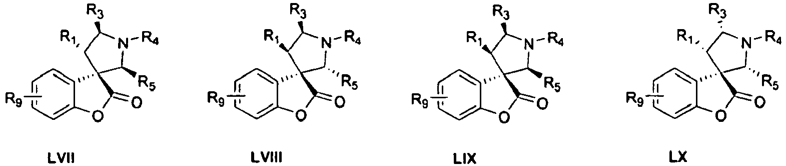 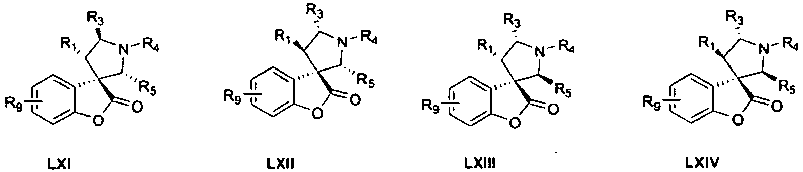 wherein:
R₁, R₃, R₄, R₅ and R₉ are as defined above.

In a further embodiment, the compounds of Formula I have Formula LXV: 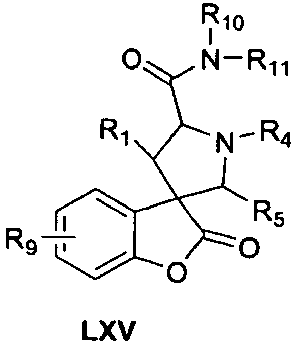 wherein:
R₁, R₄, R₅, R₉, R₁₀, and R₁₁ are as defined above.

In a further embodiment, the compounds of Formula I have one of Formulae LXVI and LXVII: 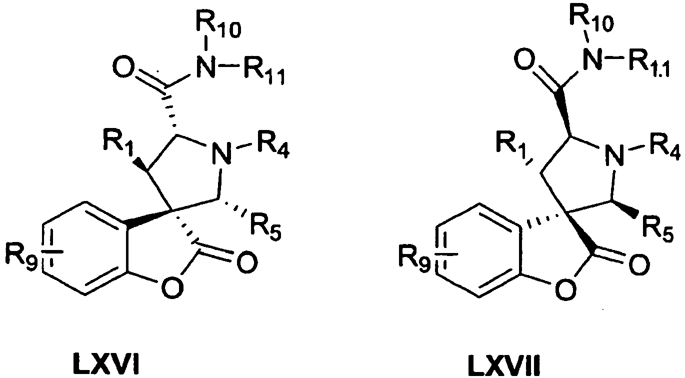 wherein:
R₁, R₄, R₅, R₉, R₁₀, and R₁₁ are as defined above.

Particular examples relating to the present invention include, any one of the following compounds:

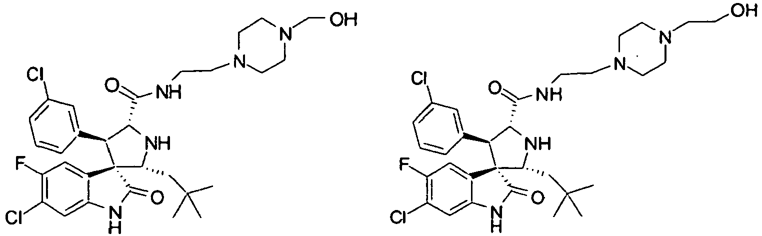

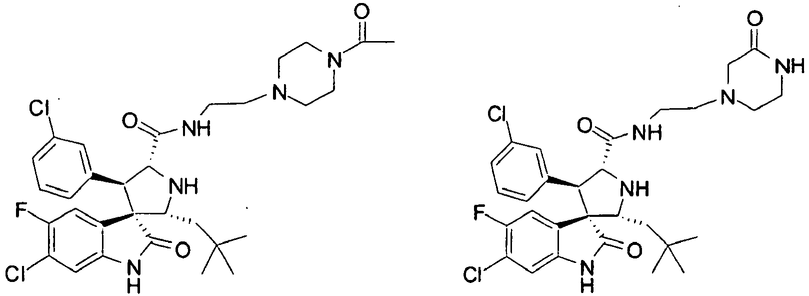

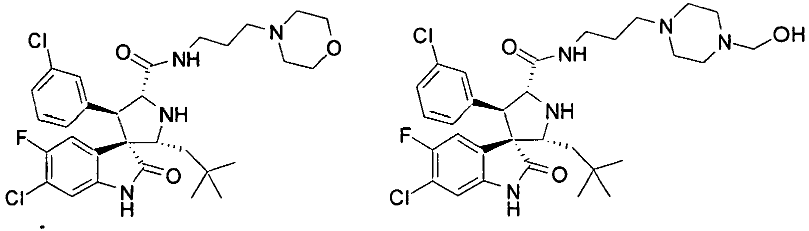

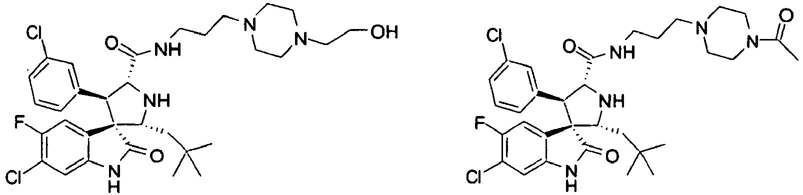

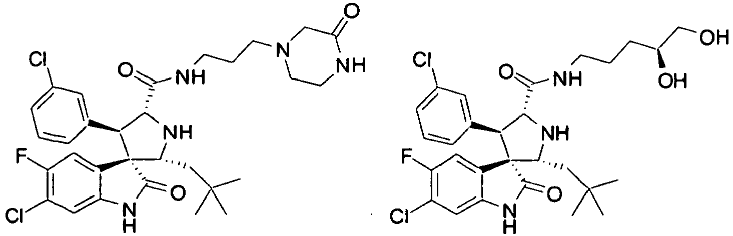

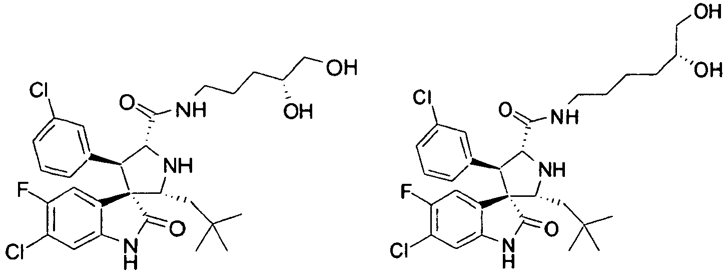

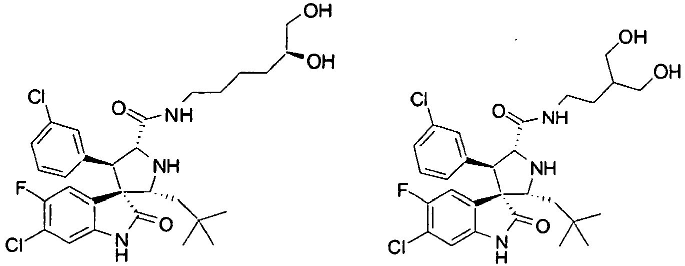

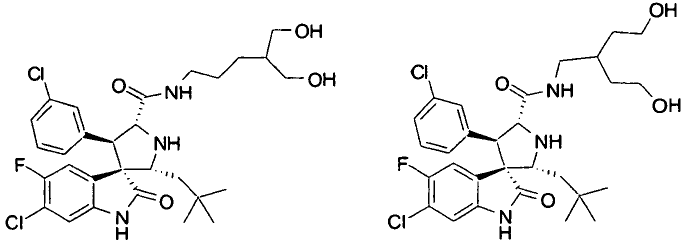

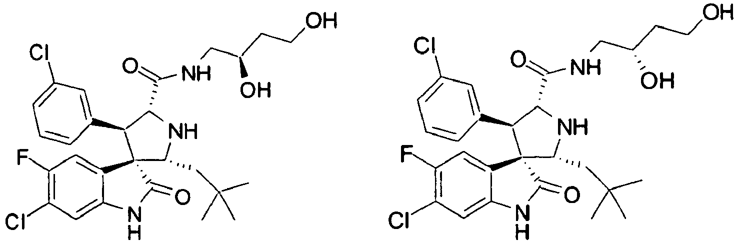

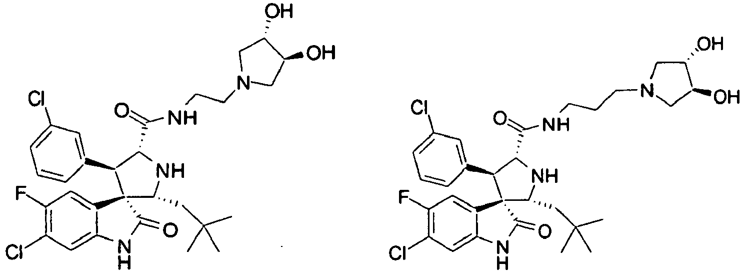

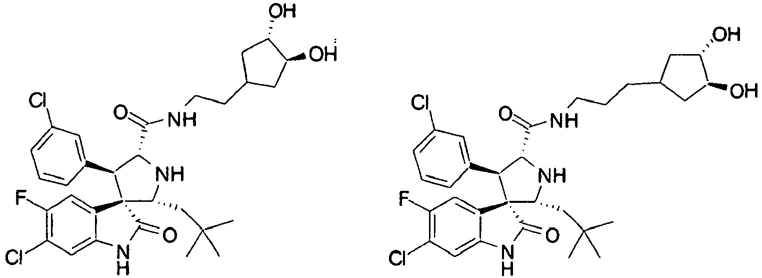

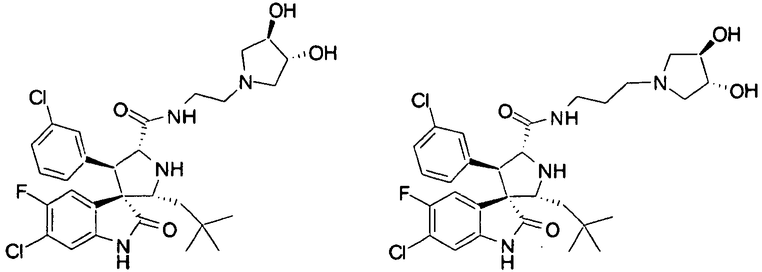

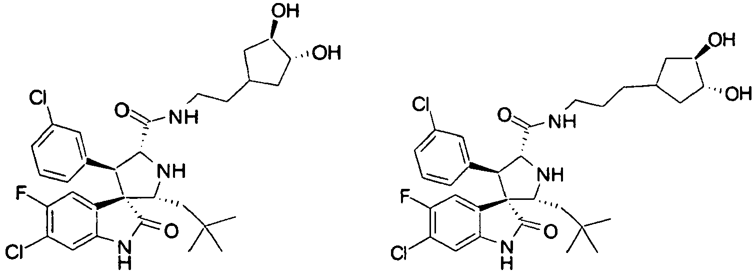

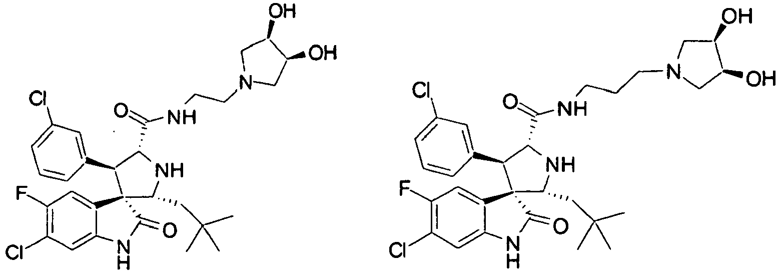

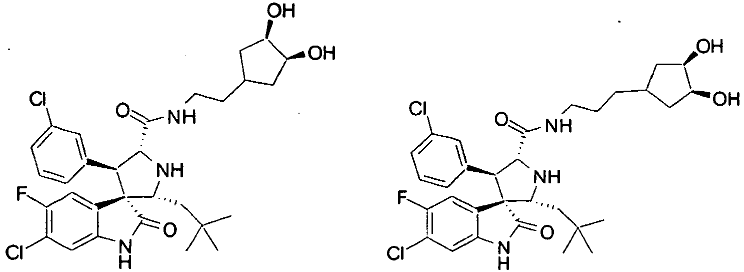

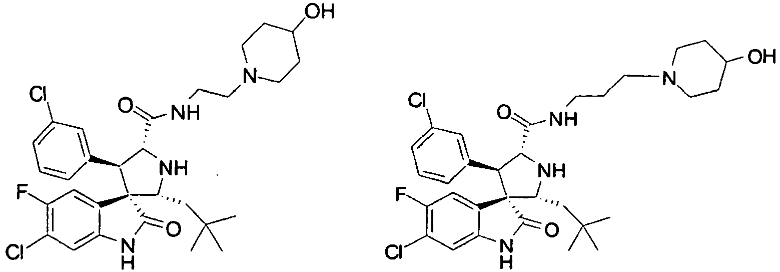

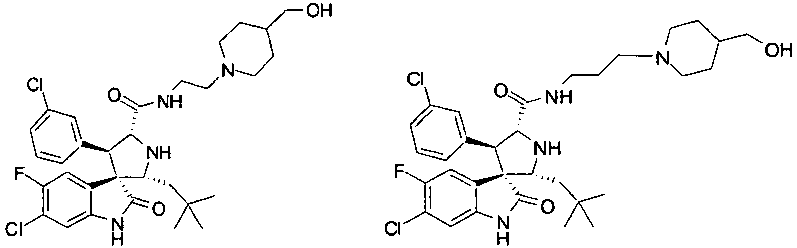

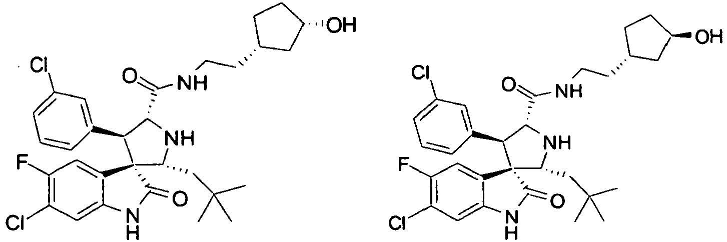

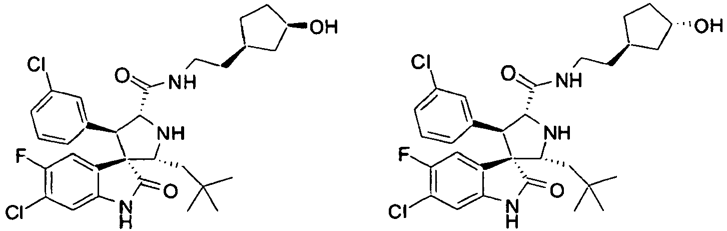

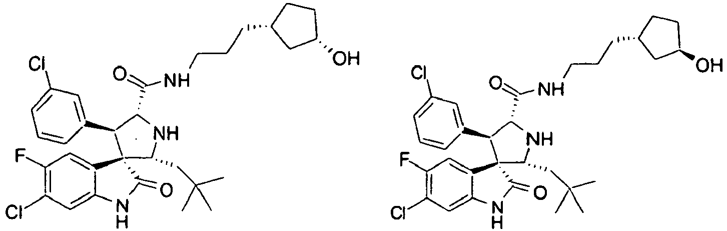

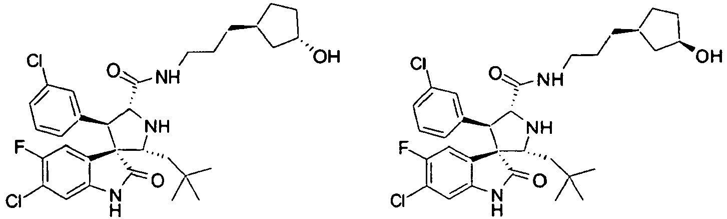

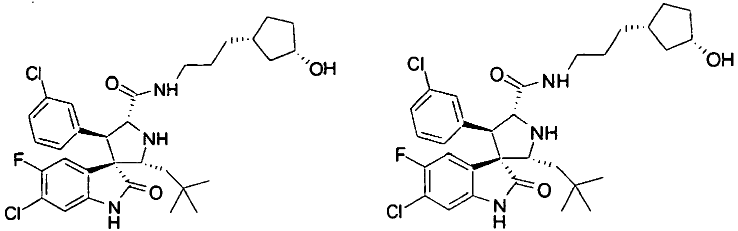

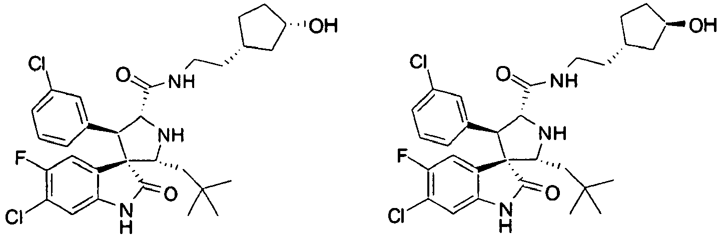

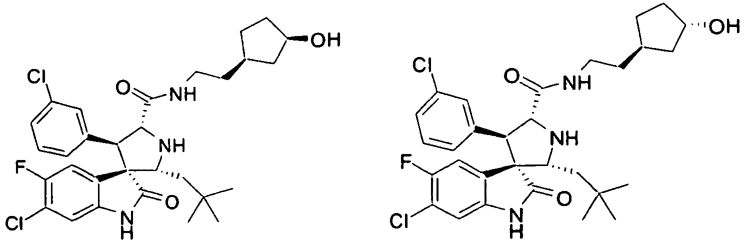

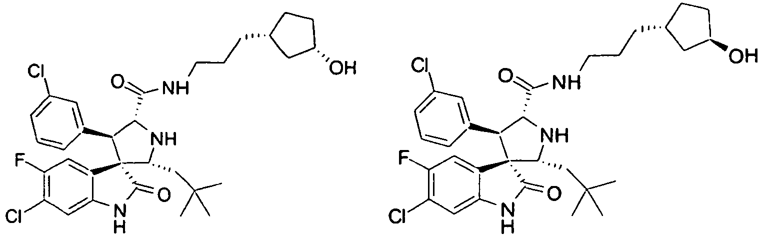

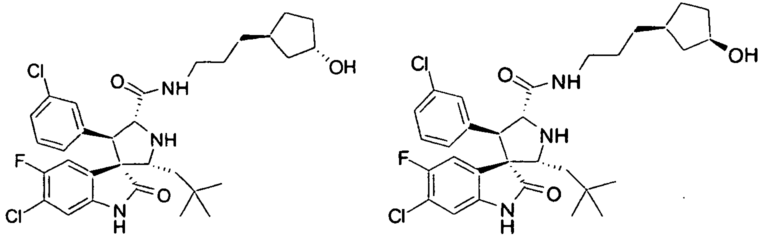

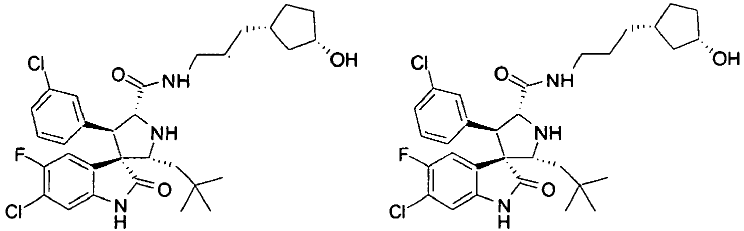

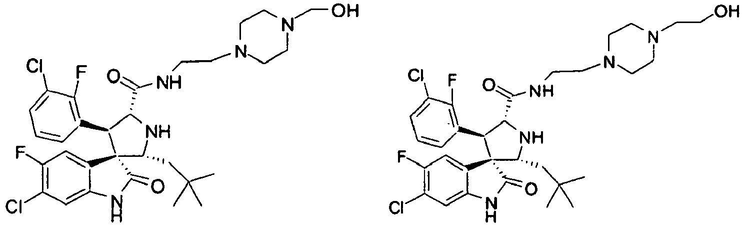

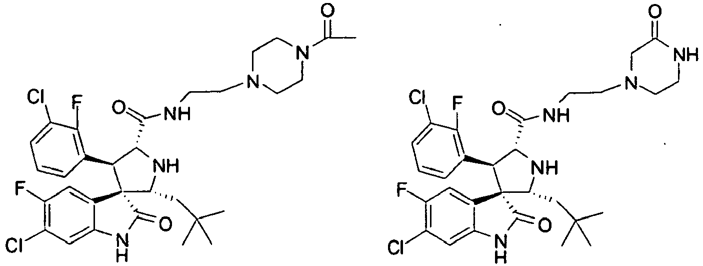

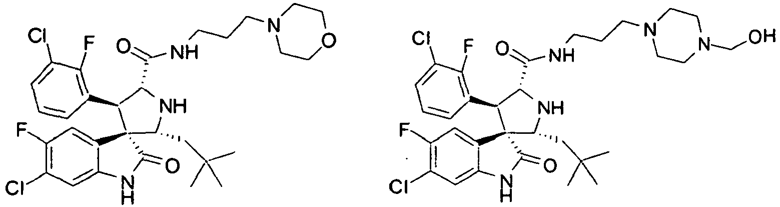

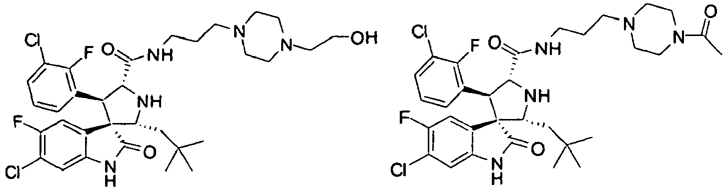

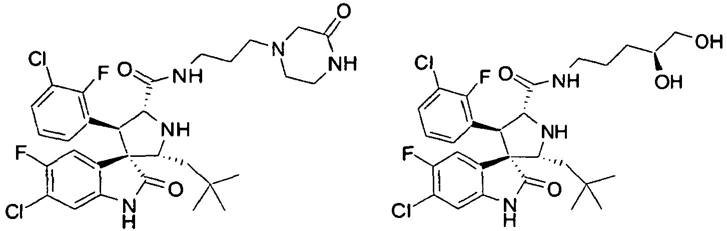

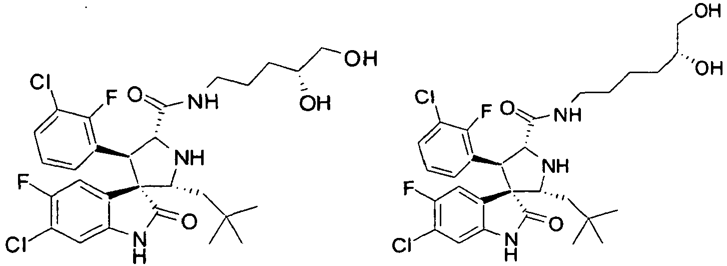

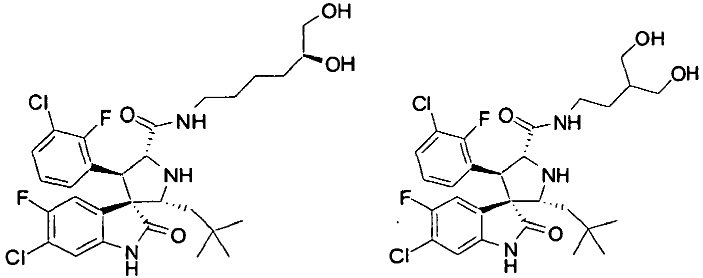

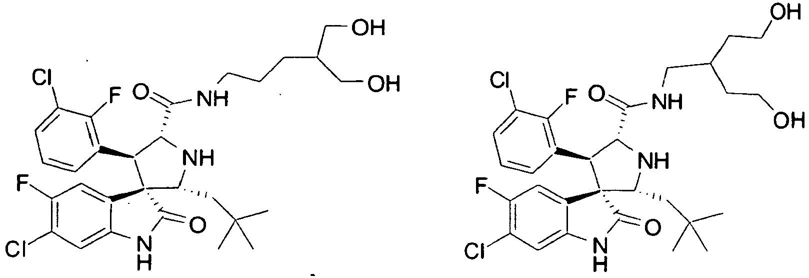

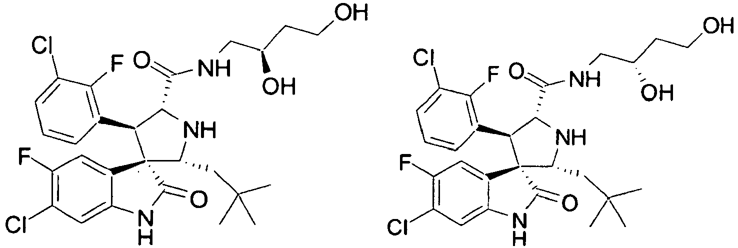

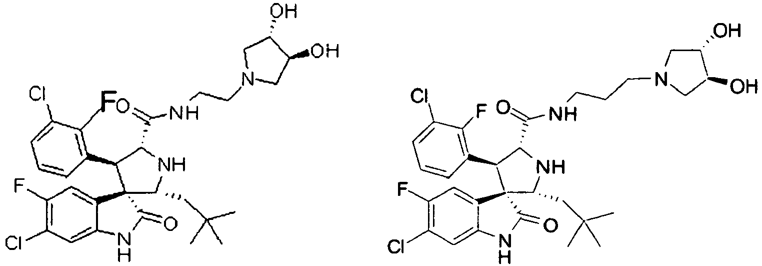

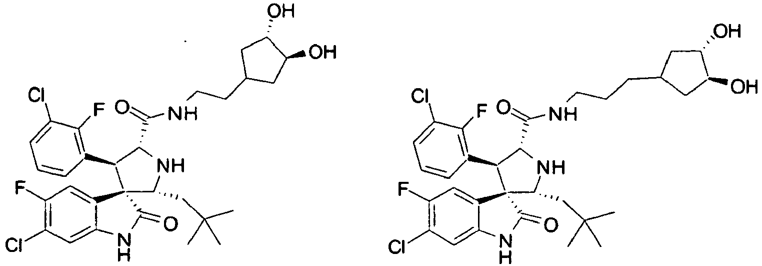

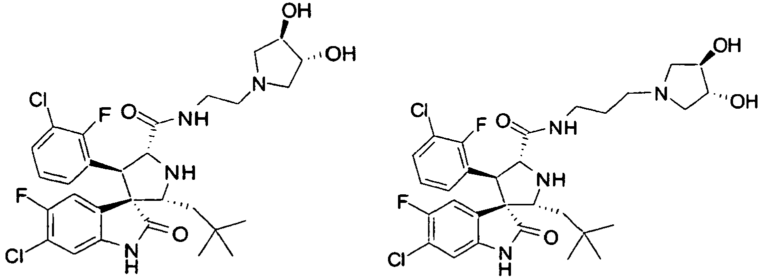

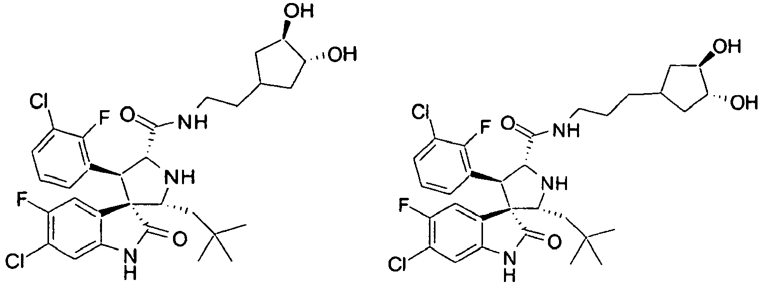

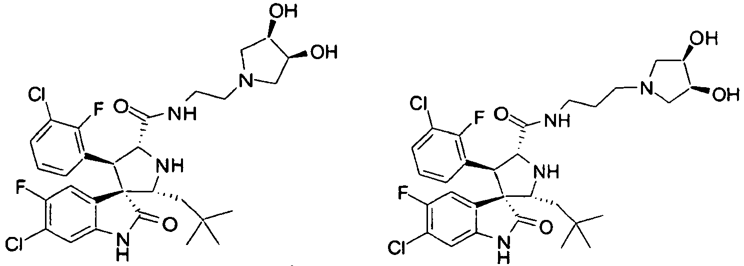

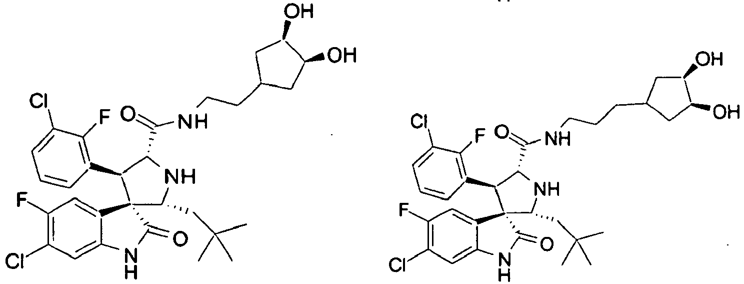

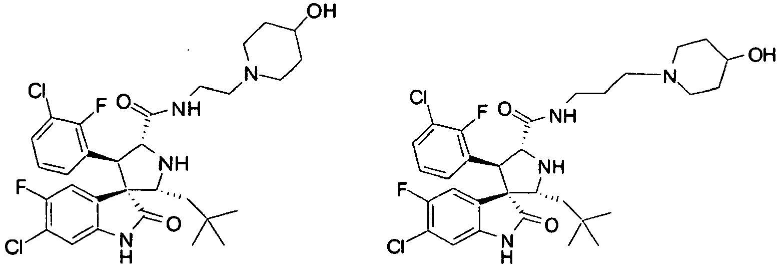

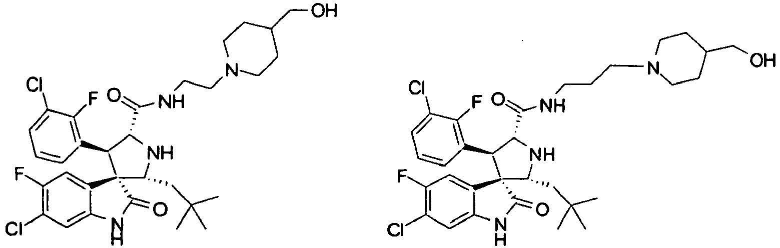

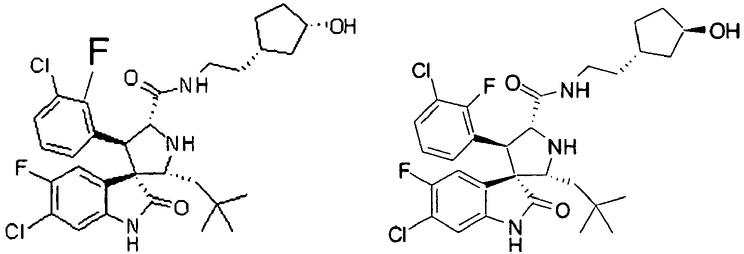

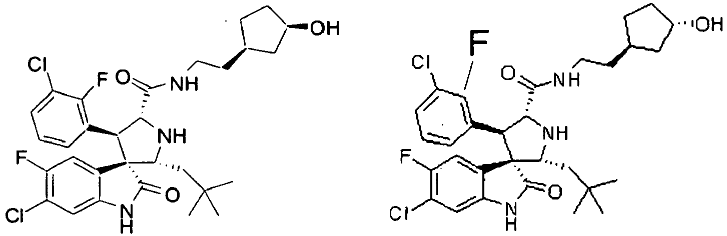

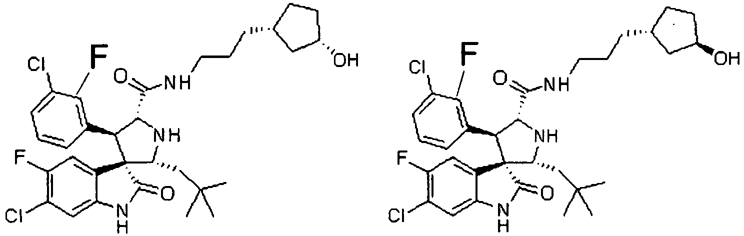

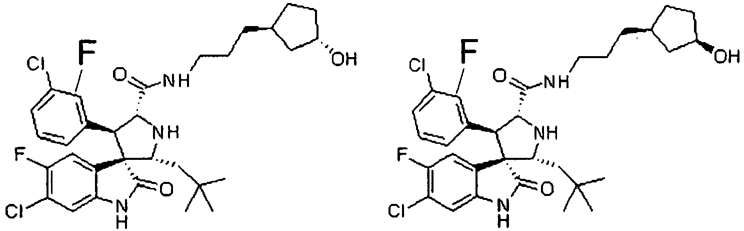

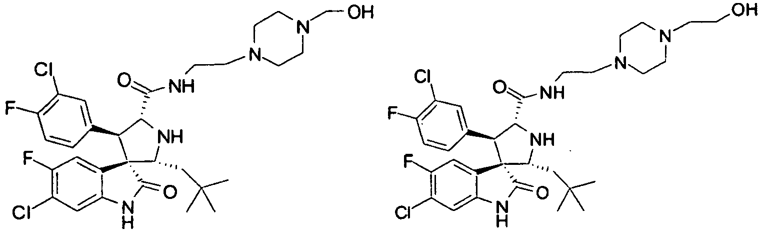

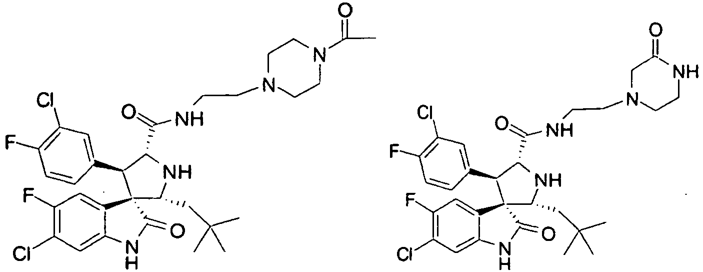

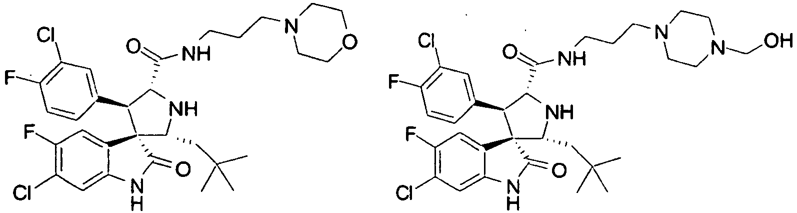

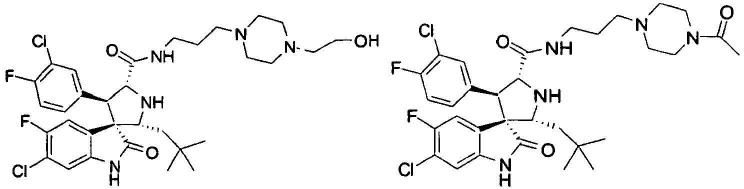

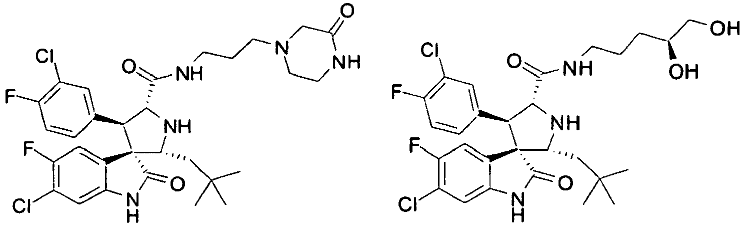

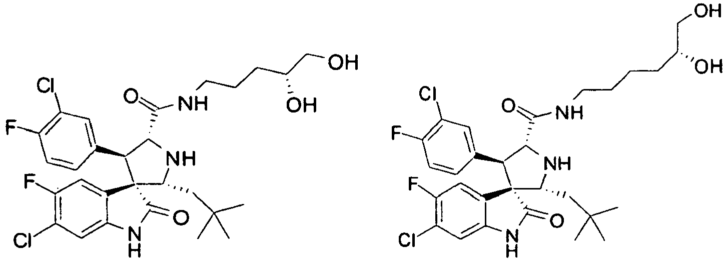

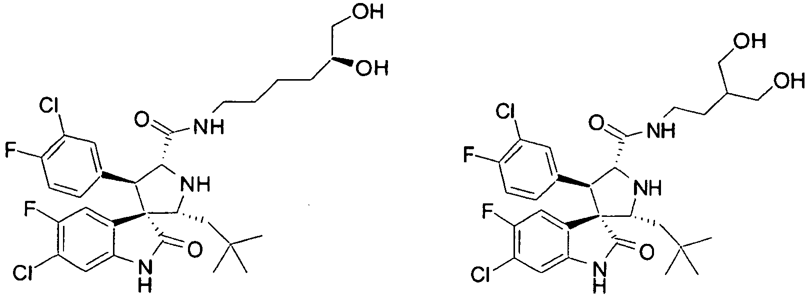

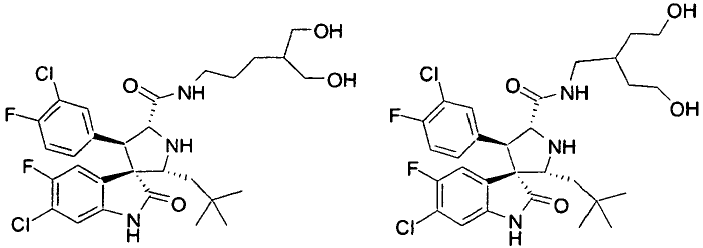

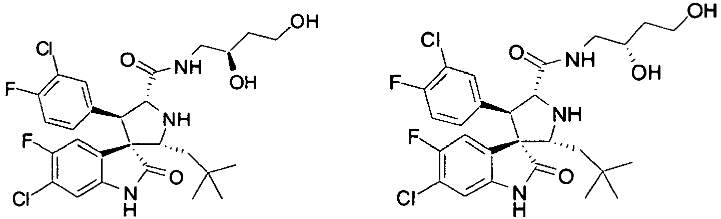

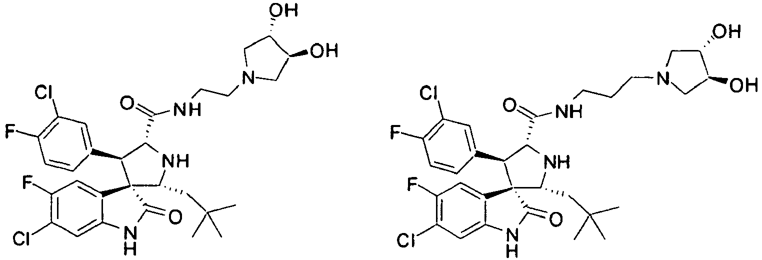

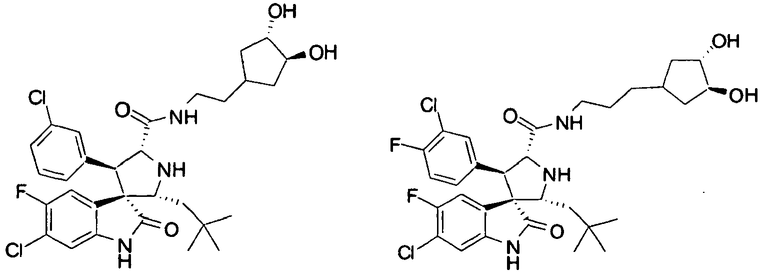

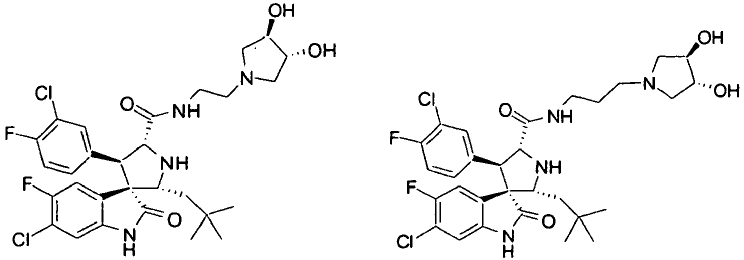

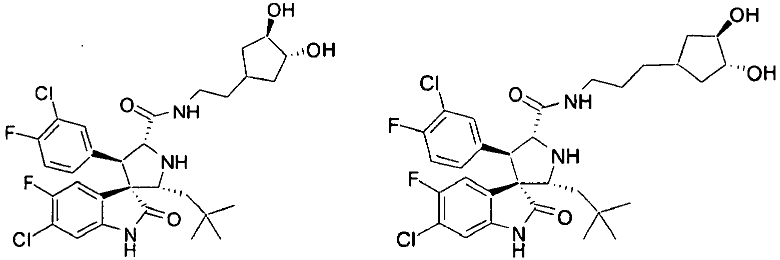

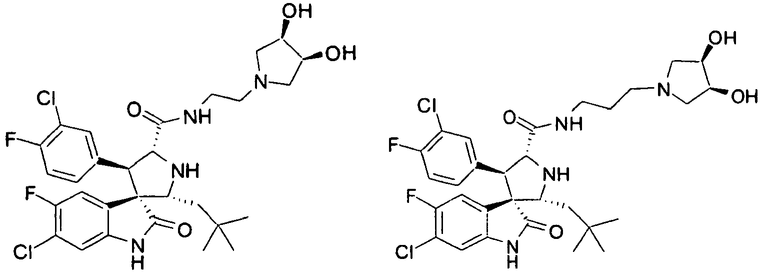

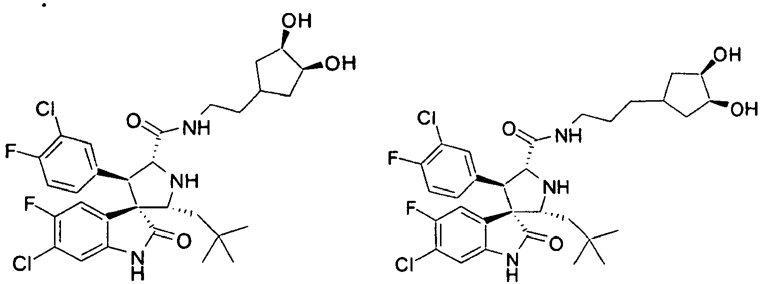

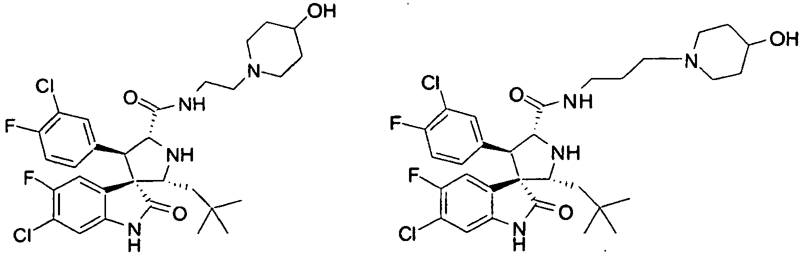

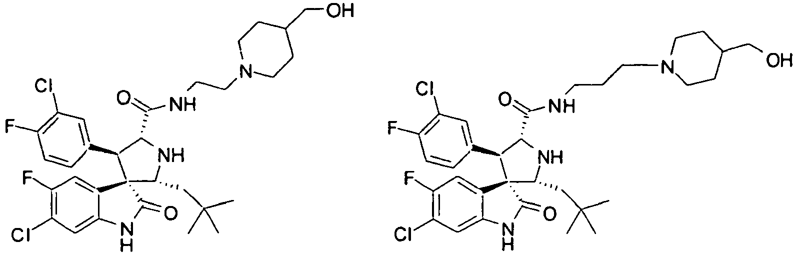

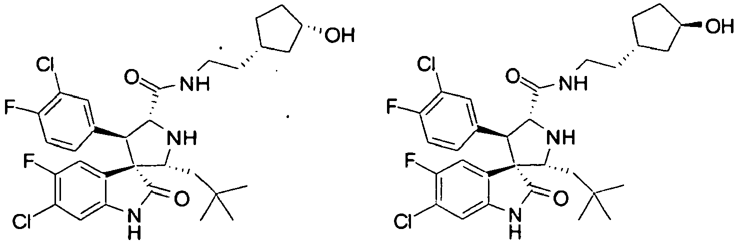

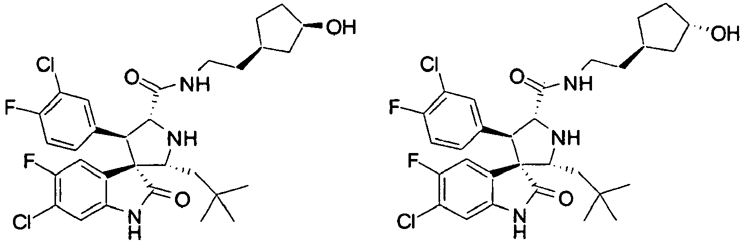

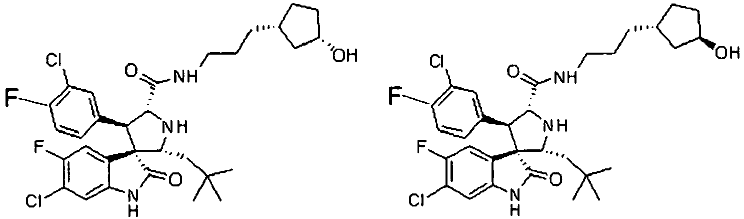

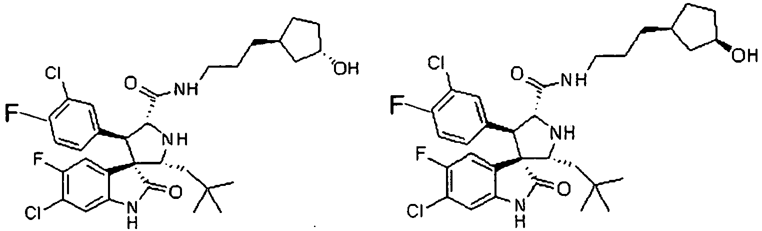

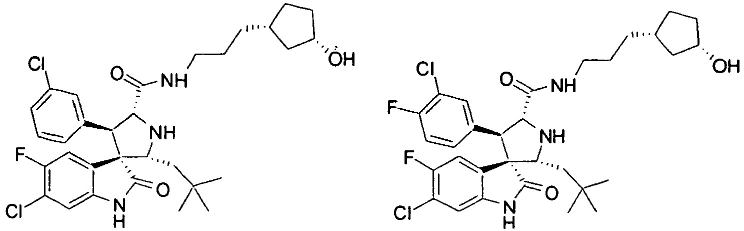

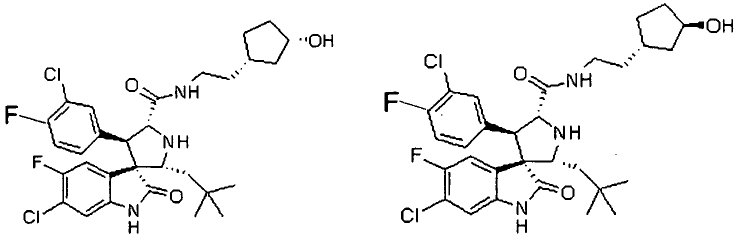

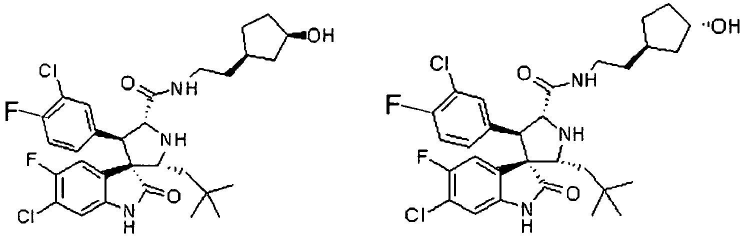

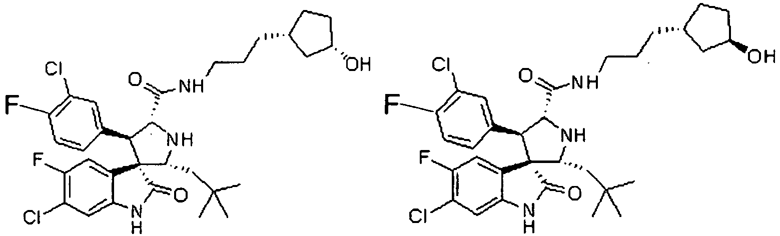

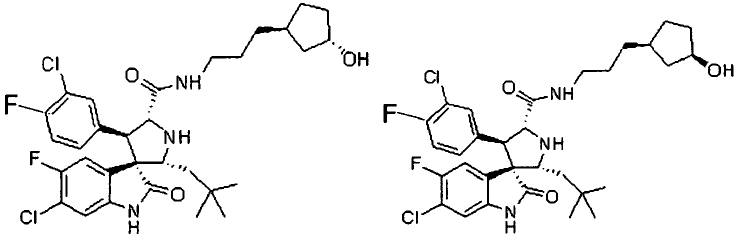

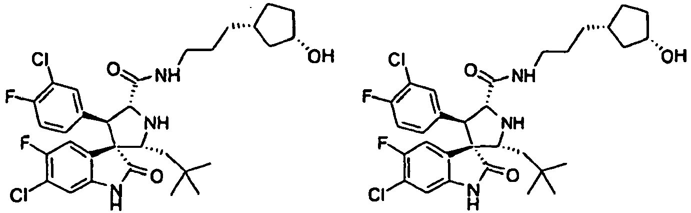

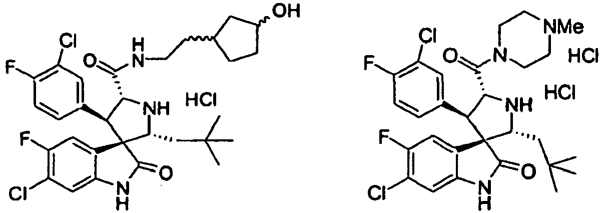

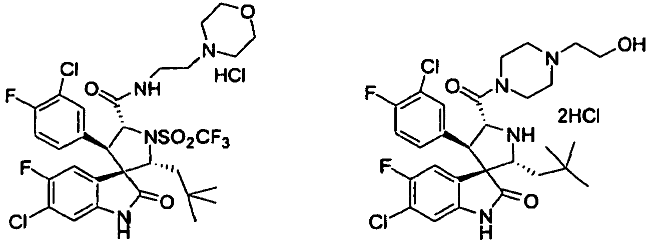

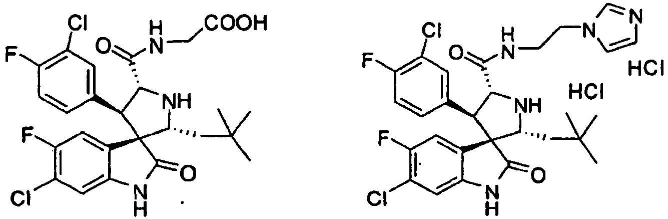

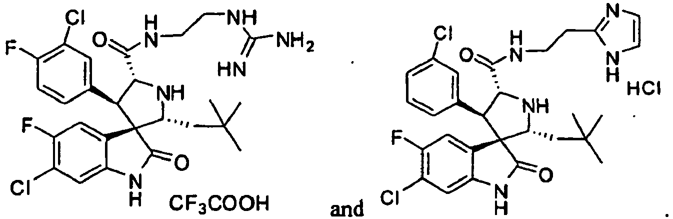

Another particular example relating to the present invention includes, any one of the following compounds: 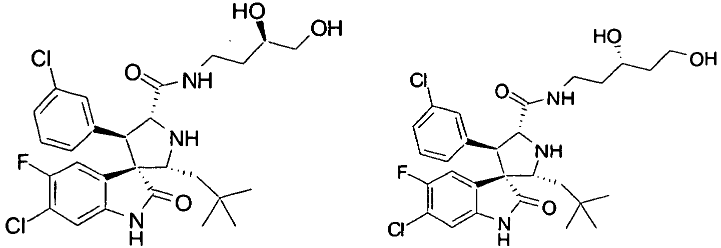 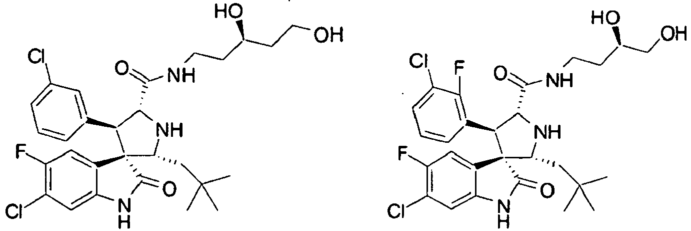 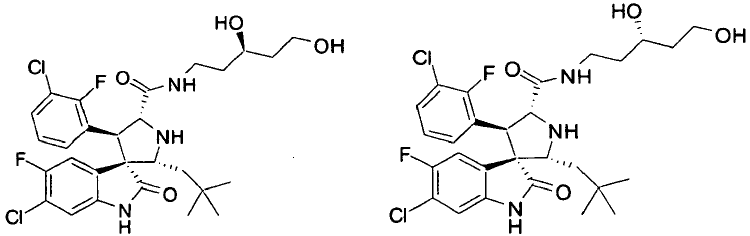 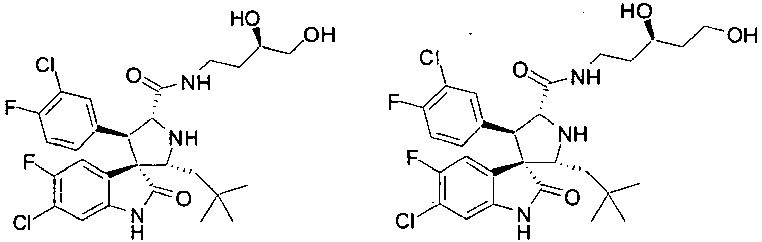 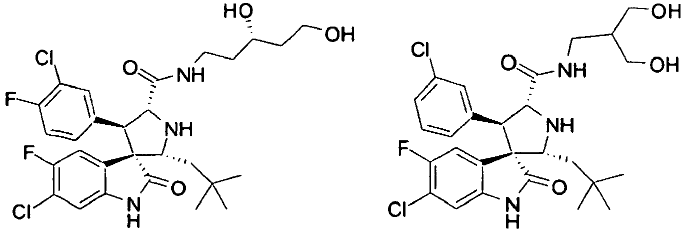 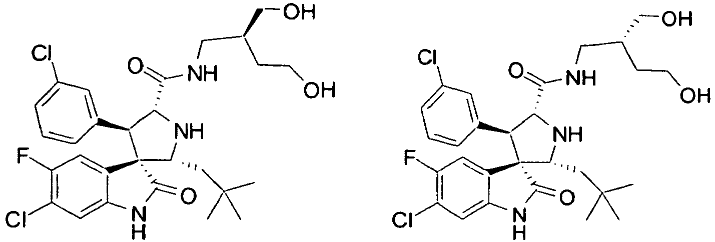 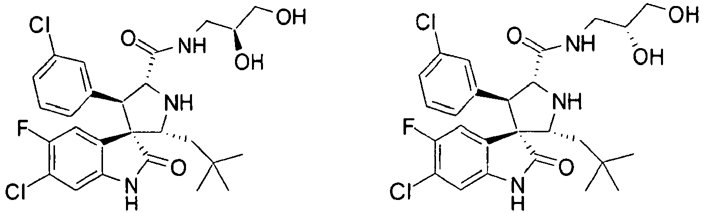 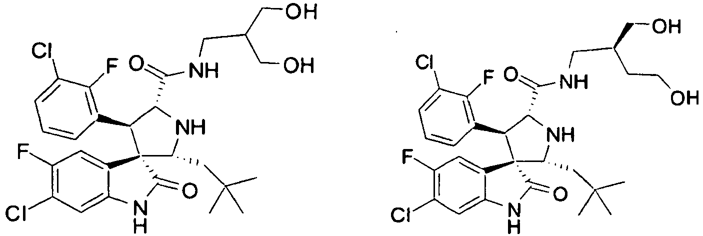 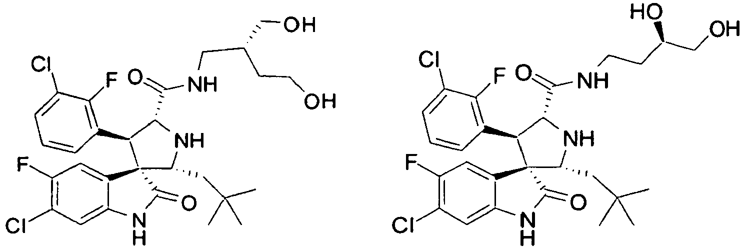 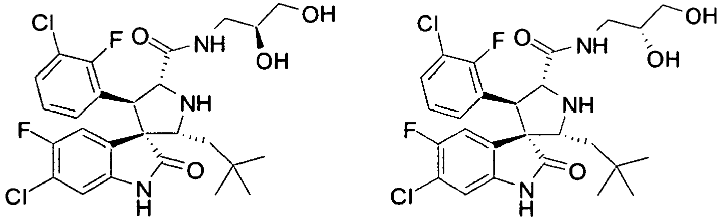 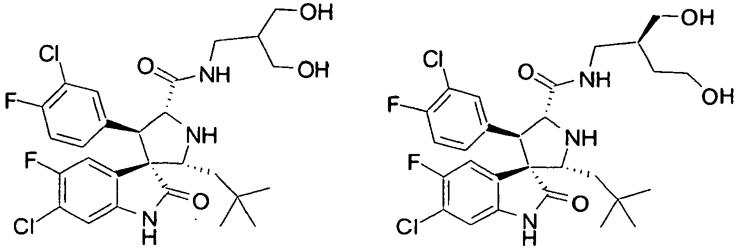 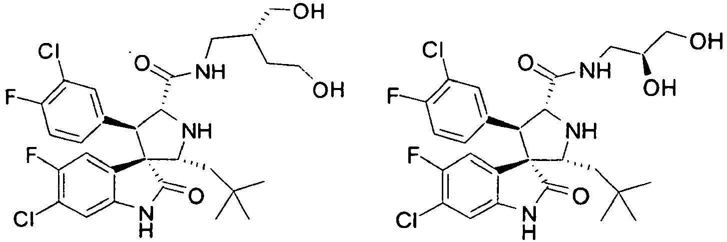 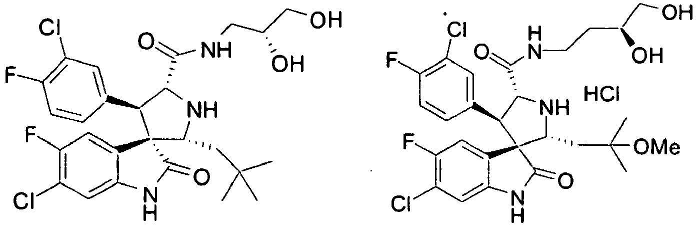 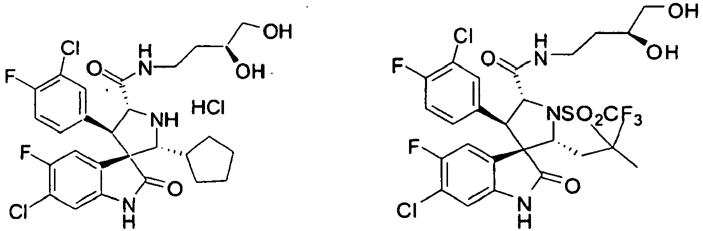 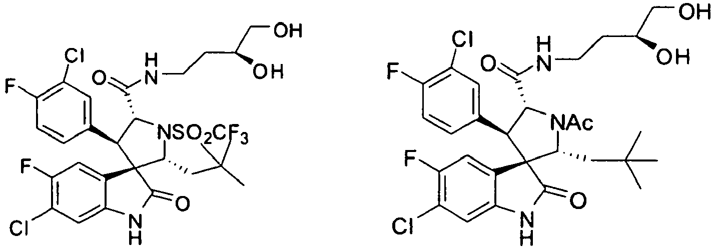 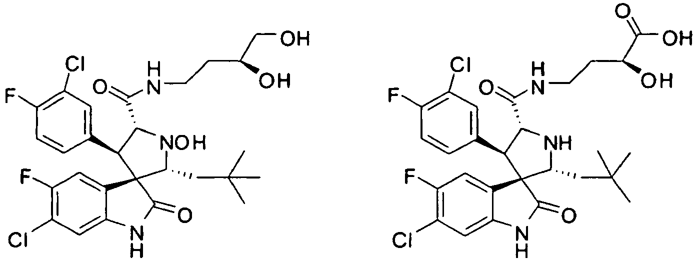 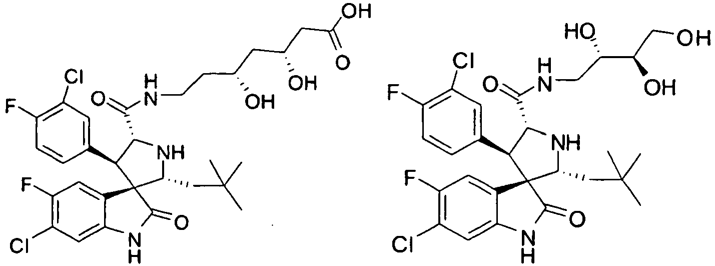 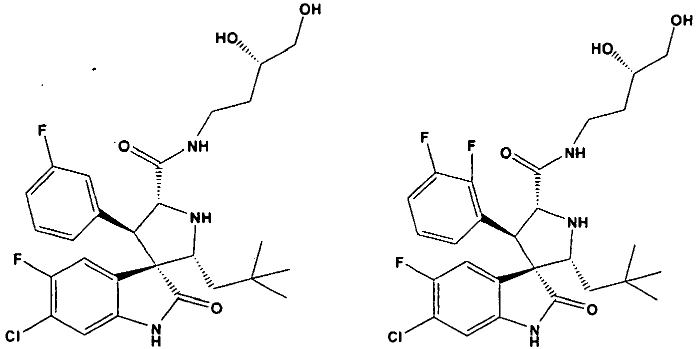 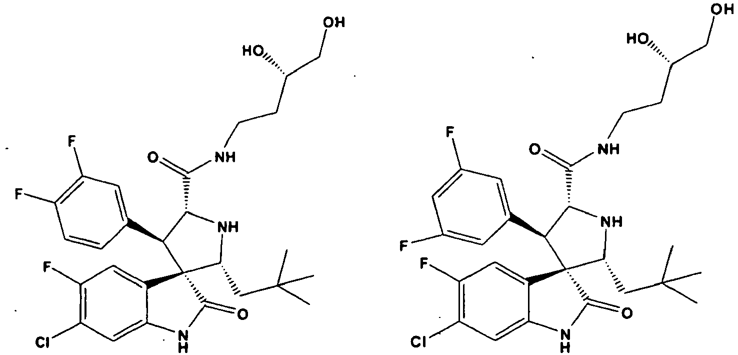 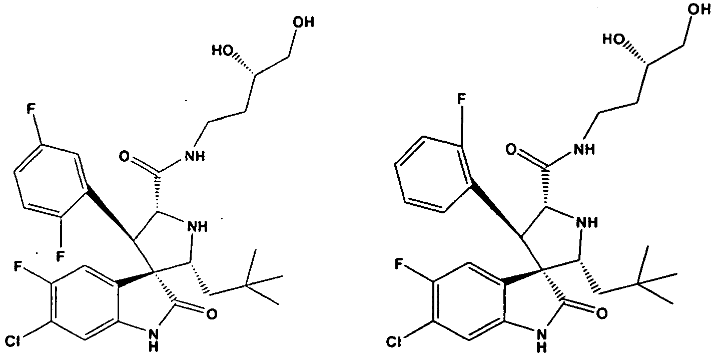 and

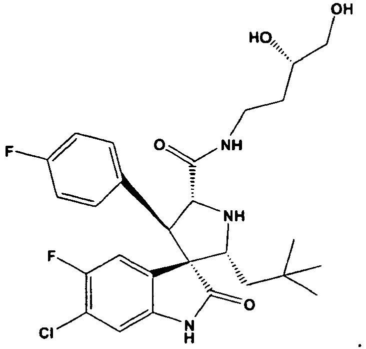

Useful alkyl groups include straight-chained or branched C₁₋₁₈ alkyl groups, especially methyl, ethyl, propyl, isopropyl, t-butyl, sec-butyl, 3-pentyl, adamantyl, norbornyl, and 3-hexyl groups.

Useful alkenyl groups include straight-chained or branched C₂₋₁₈ alkyl groups, especially ethenyl, propenyl, isopropenyl, butenyl, isobutenyl, and hexenyl.

Useful alkynyl groups are C₂₋₁₈ alkynyl groups, especially ethynyl, propynyl, butynyl, and 2-butynyl groups

Useful cycloalkyl groups are C₃₋₈ cycloalkyl. Typical cycloalkyl groups include cyclopropyl, cyclobutyl, cyclopentyl, cyclohexyl and cycloheptyl.

Useful aryl groups include C₆₋₁₄ aryl, especially phenyl, naphthyl, phenanthrenyl, anthracenyl, indenyl, azulenyl, biphenyl, biphenylenyl, and fluorenyl groups.

Useful heteroaryl groups include thienyl, benzo[b]thienyl, naphtho[2,3-b]thienyl, thianthrenyl, furyl, pyranyl, isobenzofuranyl, chromenyl, xanthenyl, phenoxanthenyl, 2H-pyrrolyl, pyrrolyl, imidazolyl, pyrazolyl, pyridyl, pyrazinyl, pyrimidinyl, pyridazinyl, indolizinyl, isoindolyl, 3H-indolyl, indolyl, indazolyl, purinyl, 4H-quinolizinyl, isoquinolyl, quinolyl, phthalzinyl, naphthyridinyl, quinozalinyl, cinnolinyl, pteridinyl, carbazolyl, β-carbolinyl, phenanthridinyl, acridinyl, perimidinyl, phenanthrolinyl, phenazinyl, isothiazolyl, phenothiazinyl, isoxazolyl, furazanyl, phenoxazinyl, 1,4-dihydroquinoxaline-2,3-dione, 7-aminoisocoumarin, pyrido[1,2-a]pyrimidin-4-one, 1,2-benzoisoxazol-3-yl, benzimidazolyl, 2-oxindolyl, and 2-oxobenzimidazolyl. Where the heteroaryl group contains a nitrogen atom in a ring, such nitrogen atom may be in the form of an N-oxide, e.g., a pyridyl N-oxide, pyrazinyl N-oxide, pyrimidinyl N-oxide, and the like.

Useful heterocyclic groups include monocyclic heterocyclic groups such as tetrahydrofuranyl, pyranyl, piperidinyl, piperizinyl, pyrrolidinyl, imidazolidinyl, imidazolinyl, morpholinyl, pyrazolidinyl, pyrazolinyl, and the like. Multi-cyclo heterocyclic groups include indolinyl, isoindolinyl, quinuclidinyl, chromanyl, isochromanyl, tetronoyl, tetrahydroisoquinolinyl groups, as well as heterocyclic groups fused with a heteroaryl ring, e.g. optionally substituted 5,6-dihydro-8H-[1,2,4]triazolo[4,3-A]pyrazinyl groups.

Optional substituents include one or more alkyl; halo; haloalkyl; cycloalkyl; aryl optionally substituted with one or more lower alkyl, lower alkoxy, methylenedioxy, halo, haloalkyl, aminosulfonyl, aryl, or heteroaryl groups; aryloxy optionally substituted with one or more lower alkyl, lower alkoxy, methylenedioxy, halo, haloalkyl, aminosulfonyl, aryl, or heteroaryl groups; aralkyl; heteroaryl optionally substituted with one or more lower alkyl, lower alkoxy, methylenedioxy, halo, haloalkyl, aminosulfonyl, aryl, or heteroaryl groups; heteroaryloxy optionally substituted with one or more lower alkyl, lower alkoxy, methylenedioxy, halo, haloalkyl, aminosulfonyl, aryl, or heteroaryl groups; alkoxy; alkylthio; arylthio; amido; amino; aminoalkyl, alkylamino, acyloxy; arylacyloxy optionally substituted with one or more lower alkyl, lower alkoxy, methylenedioxy, halo, haloalkyl, aminosulfonyl, aryl, or heteroaryl groups; diphenylphosphinyloxy optionally substituted with one or more lower alkyl, lower alkoxy, methylenedioxy, halo, haloalkyl, aminosulfonyl, aryl, or heteroaryl groups; heterocyclo optionally substituted with one or more lower alkyl, lower alkoxy, methylenedioxy, halo, haloalkyl, aminosulfonyl, aryl, heteroaryl, amino acid substituted sulfonyl, or amino acid derivative substituted sulfonyl groups; heterocycloacyl optionally substituted with one or more lower alkyl, lower alkoxy, methylenedioxy, halo, haloalkyl, aminosulfonyl, aryl, or heteroaryl groups; heterocycloalkoxy optionally substituted with one or more lower alkyl, lower alkoxy, methylenedioxy, halo, haloalkyl, aminosulfonyl, aryl, or heteroaryl groups; partially unsaturated heterocycloalkyl optionally substituted with one or more lower alkyl, lower alkoxy, methylenedioxy, halo, haloalkyl, aminosulfonyl, aryl, or heteroaryl groups; or partially unsaturated heterocycloalkyloxy optionally substituted with one or more lower alkyl, lower alkoxy, methylenedioxy, halo, haloalkyl, aminosulfonyl, aryl, or heteroaryl groups.

Certain of the compounds of the present invention may exist as stereoisomers including optical isomers. The invention includes all stereoisomers, both as pure individual stereoisomer preparations and enriched preparations of each, and both the racemic mixtures of such stereoisomers as well as the individual enantiomers that may be separated according to methods that are well known to those of skill in the art.

The compounds and processes of the present invention will be better understood in connection with the following synthetic schemes which illustrate the methods by which the compounds of the invention may be prepared. Starting materials can be obtained from commercial sources or prepared by well-established literature methods known to those of ordinary skill in the art. It will be readily apparent to one of ordinary skill in the art that the compounds defined above can be synthesized by substitution of the appropriate reagents and agents in the syntheses shown below.

Compounds have the general structure of formula X are synthesized by using a asymmetric 1, 3- dipolar cycloaddition as the key step (Scheme 1).

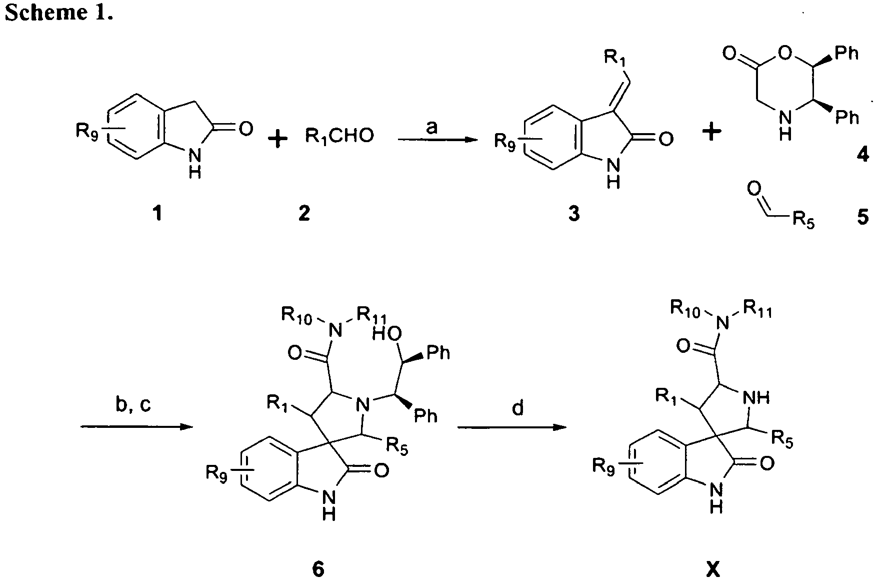

Reagents and conditions: a) CH₂Cl₂-CH₃CN, KF-Al₂O₃, microwave, or methanol, piperidine reflux; b) 4Å molecular sieves, toluene, 70°C; c) amine, r.t.; d) Pb(OAc)₄, CH₂Cl₂-MeOH (1:1), 0°C, or ammonium cerium(IV) nitrate (CAN), CH₃CN, K₂CO₃, r.t.

Compounds having Formula LXV are prepared by a similar method as the preparation of Formula X (Scheme 5).

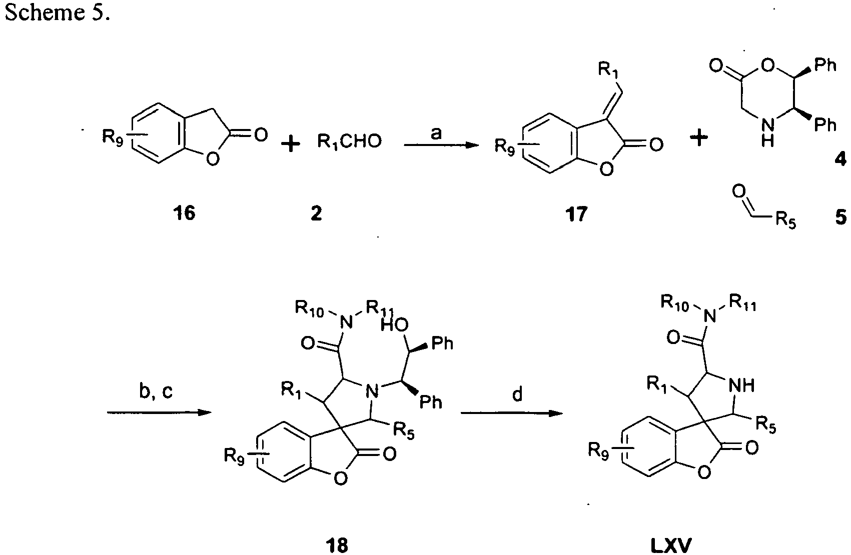

Reagents and conditions: a) CH₂Cl₂-CH₃CN, KF-Al₂O₃, microwave, or methanol, piperidine reflux; b) 4Å molecular sieves, toluene, 70°C; c) amine, r.t.; d) Pb(OAc)₄, CH₂Cl₂-MeOH (1:1), 0°C, or ammonium cerium(IV) nitrate (CAN), CH₃CN, K₂CO₃, r.t.

One aspect of the invention related to methods of preparing MDM2 inhibitor compounds. In one example the invention relates to a method of preparing a compound having formula X, comprising
a) condensing a compound of Formula 1 with a compound of Formula 2, *e.g.,* in a solvent or a mixture of solvents (*e.g.,* CH₂Cl₂ and CH₃CN) under microwave in the presence of a catalyst (*e.g.*, KF-Al₂O₃) or in the presence of a base in a suitable solvent to form a compound of Formula 3; 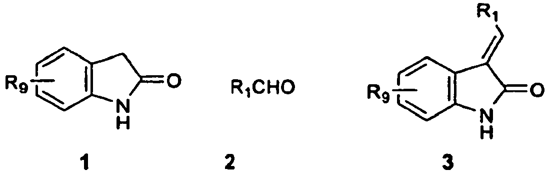
b) condensing the compound, of Formula 3 with a compound of Formula 4 and a compound of Formula 5, *e.g.,* in a non-polar solvent (*e.g.,* toluene) in the presence of a dehydrating agent (*e.g.,* 4A molecular sieve) at elevated temperature (*e.g.,* about 70°C) to form a compound of Formula 6; and 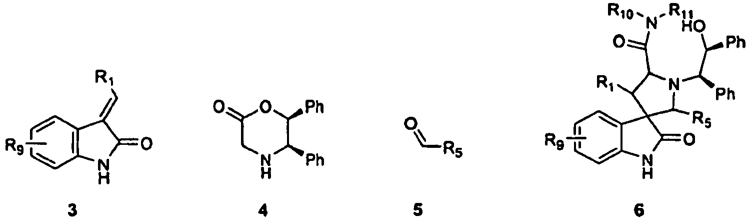
c) treating the compound of Formula 6 with an oxidizing agent (*e.g.,* Pb(OAc)₄, Ammonium cerium nitrate) in a solvent or mixture of solvents (*e.g.,* CH₂Cl₂ and MeOH, CH₃CN) at a suitable temperature (*e.g.,* about 0°C or room temperature) to form a compound of Formula X.

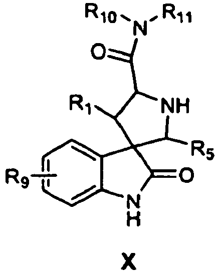

In another example, the invention relates to a method of preparing a compound having formula LXV, comprising
a) condensing a compound of Formula 16 with a compound of Formula 2, e.g., in a solvent or a mixture of solvents (e.g., CH₂Cl₂ and CH₃CN) under microwave in the presence of a catalyst (e.g., KF-Al₂O₃) or in the presence of a base in a suitable solvent to form a compound of Formula 17; 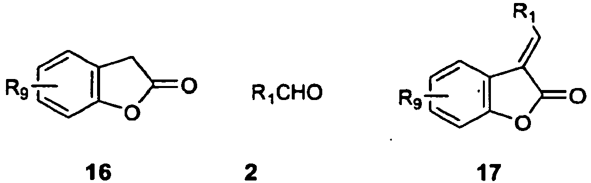
b) condensing the compound of Formula 17 with a compound of Formula 4 and a compound of Formula 5, *e.g.,* in a non-polar solvent (*e.g.*, toluene) in the presence of a dehydrating agent (e.g., 4Å molecular sieve) at elevated temperature (e.g., about 70°C) to form a compound of Formula 18; and 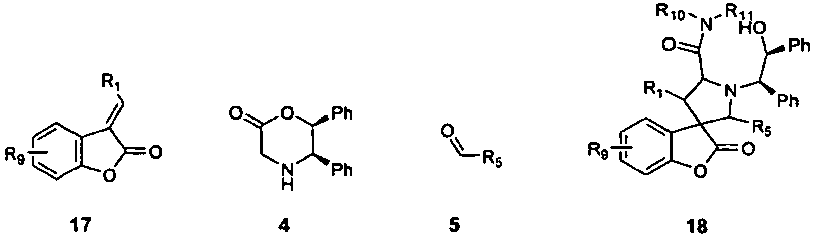
c) treating the compound of Formula 18 with an oxidizing agent (e.g., Pb(OAc)₄, Ammonium cerium nitrate) in a solvent or mixture of solvents (*e.g.*, CH₂Cl₂ and MeOH, CH₃CN) at a suitable temperature (e.g., about 0°C or room temperature) to form a compound of Formula LXV; 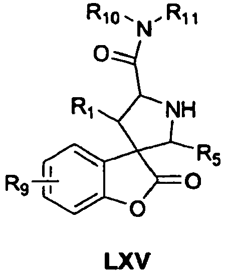 wherein:
   R₁, R₅, R₉, R₁₀ and R₁₁ are as defined above.

An important aspect of the present invention is that compounds of Formula I induce cell cycle arrest and/or apoptosis and also potentiate the induction of cell cycle arrest and/or apoptosis either alone or in response to additional apoptosis induction signals. Therefore, it is contemplated that these compounds sensitize cells to induction of cell cycle arrest and/or apoptosis, including cells that are resistant to such inducing stimuli. The inhibitors of the interaction between p53 or p53-related proteins and MDM2 or MDM2-realted proteins of the present invention can be used to induce apoptosis in any disorder that can be treated, ameliorated, or prevented by the induction of apoptosis. In one example, the inhibitors can be used to induce apoptosis in cells comprising functional p53 or p53-related proteins.

In another example the invention pertains to modulating an apoptosis associated state which is associated with one or more apoptosis-modulating agents. Examples of apoptosis-modulating agents include, but are not limited to, Fas/CD95, TRAMP, TNF RI, DR1, DR2, DR3, DR4, DR5, DR6, FADD, RIP, TNFα, Fas ligand, TRAIL, antibodies to TRAIL-R1 or TRAIL-R2, Bcl-2, p53, BAX, BAD, Akt, CAD, PI3 kinase, PP1, and caspase proteins. Other agents involved in the initiation, decision and degradation phase of apoptosis are also included. Examples of apoptosis-modulating agents include agents, the activity, presence, or change in concentration of which, can modulate apoptosis in a subject. Apoptosis-modulating agents include those which are inducers of apoptosis, such as TNF or a TNF-related ligand, particularly a TRAMP ligand, a Fas/CD95 ligand, a TNFR-1 ligand, or TRAIL.

In some examples, the compositions and methods relating to the present invention are used to treat diseased cells, tissues, organs, or pathological conditions and/or disease states in an animal (e.g., a mammalian subject including, but not limited to, humans and veterinary animals). In this regard, various diseases and pathologies are amenable to treatment or prophylaxis using the methods and compositions relating to the present invention. A non-limiting exemplary list of these diseases and conditions includes, but is not limited to, breast cancer, prostate cancer, lymphoma, skin cancer, pancreatic cancer, colon cancer, melanoma, malignant melanoma, ovarian cancer, brain cancer, primary brain carcinoma, head-neck cancer, glioma, glioblastoma, liver cancer, bladder cancer, non-small cell lung cancer, head or neck carcinoma, breast carcinoma, ovarian carcinoma, lung carcinoma, small-cell lung carcinoma, Wilms' tumor, cervical carcinoma, testicular carcinoma, bladder carcinoma, pancreatic carcinoma, stomach carcinoma, colon carcinoma, prostatic carcinoma, genitourinary carcinoma, thyroid carcinoma, esophageal carcinoma, myeloma, multiple myeloma, adrenal carcinoma, renal cell carcinoma, endometrial carcinoma, adrenal cortex carcinoma, malignant pancreatic insulinoma, malignant carcinoid carcinoma, choriocarcinoma, mycosis fungoides, malignant hypercalcemia, cervical hyperplasia, leukemia, acute lymphocytic leukemia, chronic lymphocytic leukemia, acute myelogenous leukemia, chronic myelogenous leukemia, chronic granulocytic leukemia, acute granulocytic leukemia, hairy cell leukemia, neuroblastoma, rhabdomyosarcoma, Kaposi's sarcoma, polycythemia vera, essential thrombocytosis, Hodgkin's disease, non-Hodgkin's lymphoma, soft-tissue sarcoma, osteogenic sarcoma, primary macroglobulinemia, and retinoblastoma, and the like, T and B cell mediated autoimmune diseases; inflammatory diseases; infections; hyperproliferative diseases; AIDS; degenerative conditions, vascular diseases, and the like. In some examples, the cancer cells being treated are metastatic. In other examples, the cancer cells being treated are resistant to anticancer agents.

In some examples, infections suitable for treatment with the compositions and methods relating to the present invention include, but are not limited to, infections caused by viruses, bacteria, fungi, mycoplasma, prions, and the like.

Some examples relating to the present invention relate to methods for administering an effective amount of a compound of Formula I and at least one additional therapeutic agent (including, but not limited to, chemotherapeutic antineoplastics, apoptosis-modulating agents, antimicrobials, antivirals, antifungals, and anti-inflammatory agents) and/or therapeutic technique (*e*.*g*., surgical intervention, and/or radiotherapies).

A number of suitable anticancer agents are contemplated for use in the methods relating to the present invention. Indeed, the present invention contemplates, but is not limited to, administration of numerous anticancer agents such as: agents that induce apoptosis; polynucleotides (e.g., anti-sense, ribozymes, siRNA); polypeptides (*e.g.*, enzymes and antibodies); biological mimetics (e.g., gossypol or BH3 mimetics); agents that bind (*e.g.*, oligomerize or complex) with a Bcl-2 family protein such as Bax; alkaloids; alkylating agents; antitumor antibiotics; antimetabolites; hormones; platinum compounds; monoclonal or polyclonal antibodies (e.g., antibodies conjugated with anticancer drugs, toxins, defensins), toxins; radionuclides; biological response modifiers (*e*.*g*., interferons (e.g., IFN-α) and interleukins (*e.g.*, IL-2)); adoptive immunotherapy agents; hematopoietic growth factors; agents that induce tumor cell differentiation (*e*.*g*., all-trans-retinoic acid); gene therapy reagents (*e*.*g*., antisense therapy reagents and nucleotides); tumor vaccines; angiogenesis inhibitors; proteosome inhibitors: NF-KB modulators; anti-CDK compounds; HDAC inhibitors; and the like. Numerous other examples of chemotherapeutic compounds and anticancer therapies suitable for co-administration with the disclosed compounds are known to those skilled in the art.

In certain examples, anticancer agents comprise agents that induce or stimulate apoptosis. Agents that induce apoptosis include, but are not limited to, radiation (*e.g.*, X-rays, gamma rays, UV); tumor necrosis factor (TNF)-related factors (*e*.*g*., TNF family receptor proteins, TNF family ligands, TRAIL, antibodies to TRAIL-R1 or TRAIL-R2); kinase inhibitors (*e*.*g*., epidermal growth factor receptor (EGFR) kinase inhibitor, vascular growth factor receptor (VGFR) kinase inhibitor, fibroblast growth factor receptor (FGFR) kinase inhibitor, platelet-derived growth factor receptor (PDGFR) kinase inhibitor, and Bcr-Abl kinase inhibitors (such as GLEEVEC)); antisense molecules; antibodies (*e.g.*, HERCEPTIN, RITUXAN, ZEVALIN, and AVASTIN); anti-estrogens (*e.g.*, raloxifene and tamoxifen); anti-androgens (e.g., flutamide, bicalutamide, finasteride, aminoglutethamide, ketoconazole, and corticosteroids); cyclooxygenase 2 (COX-2) inhibitors (e.g., celecoxib, meloxicam, NS-398, and non-steroidal anti-inflammatory drugs (NSAIDs)); anti-inflammatory drugs (*e*.*g*., butazolidin, DECADRON, DELTASONE, dexamethasone, dexamethasone intensol, DEXONE, HEXADROL, hydroxychloroquine, METICORTEN, ORADEXON, ORASONE, oxyphenbutazone, PEDIAPRED, phenylbutazone, PLAQUENIL, prednisolone, prednisone, PRELONE, and TANDEARIL); and cancer chemotherapeutic drugs (*e.g.*, irinotecan (CAMPTOSAR), CPT-11, fludarabine (FLUDARA), dacarbazine (DTIC), dexamethasone, mitoxantrone, MYLOTARG, VP-16, cisplatin, carboplatin, oxaliplatin, 5-FU, doxorubicin, gemcitabine, bortezomib, gefitinib, bevacizumab, TAXOTERE or TAXOL); cellular signaling molecules; ceramides and cytokines; staurosporine and the like.

In still other examples, the compositions and methods relating to the present invention provide a compound of Formula I and at least one anti-hyperproliferative or antineoplastic agent selected from alkylating agents, antimetabolites, and natural products (*e.g.*, herbs and other plant and/or animal derived compounds).

Alkylating agents suitable for use in the compositions and methods relating to the invention include, but are not limited to: 1) nitrogen mustards (*e*.*g*., mechlorethamine, cyclophosphamide, ifosfamide, melphalan (L-sarcolysin); and chlorambucil); 2) ethylenimines and methylmelamines (*e*.*g*., hexamethylmelamine and thiotepa); 3) alkyl sulfonates (*e*.*g*., busulfan); 4) nitrosoureas (*e*.*g*., carmustine (BCNU); lomustine (CCNU); semustine (methyl-CCNU); and streptozocin (streptozotocin)); and 5) triazenes (*e*.*g*., dacarbazine (DTIC; dimethyltriazenoimid-azolecarboxamide).

In some examples, antimetabolites suitable for use in the compositions and methods relating to the invention include, but are not limited to: 1) folic acid analogs (e.g., methotrexate (amethopterin)); 2) pyrimidine analogs (e.g., fluorouracil (5-fluorouracil; 5-FU), floxuridine (fluorode-oxyuridine; FudR), and cytarabine (cytosine arabinoside)); and 3) purine analogs (e.g., mercaptopurine (6-mercaptopurine; 6-MP), thioguanine (6-thioguanine; TG), and pentostatin (2'-deoxycoformycin)).

In still further examples, chemotherapeutic agents suitable for use in the compositions and methods relating to the present invention include, but are not limited to: 1) vinca alkaloids (*e*.*g*., vinblastine (VLB), vincristine); 2) epipodophyllotoxins (*e*.*g*., etoposide and teniposide); 3) antibiotics (*e*.*g*., dactinomycin (actinomycin D), daunorubicin (daunomycin; rubidomycin), doxorubicin, bleomycin, plicamycin (mithramycin), and mitomycin (mitomycin C)); 4) enzymes (*e.g*., L-asparaginase); 5) biological response modifiers (*e*.*g*., interferon-alfa); 6) platinum coordinating complexes (*e.g*., cisplatin (cis-DDP) and carboplatin); 7) anthracenediones (*e.g*., mitoxantrone); 8) substituted ureas (*e.g*., hydroxyurea); 9) methylhydrazine derivatives (*e.g.*, procarbazine (N-methylhydrazine; MIH)); 10) adrenocortical suppressants (*e.g.*, mitotane (o,p'-DDD) and aminoglutethimide); 11) adrenocorticosteroids (*e.g.*, prednisone); 12) progestins (e.g., hydroxyprogesterone caproate, medroxyprogesterone acetate, and megestrol acetate); 13) estrogens (*e.g.*, diethylstilbestrol and ethinyl estradiol); 14) antiestrogens (e.g., tamoxifen); 15) androgens (e.g., testosterone propionate and fluoxymesterone); 16) antiandrogens (e.g., flutamide): and 17) gonadotropin-releasing hormone analogs (e.g., leuprolide).

Any oncolytic agent that is routinely used in a cancer therapy context finds use in the compositions and methods relating to the present invention. For example, the U.S. Food and Drug Administration maintains a formulary of oncolytic agents approved for use in the United States. International counterpart agencies to the U.S.F.D.A. maintain similar formularies. Table 1 provides a list of exemplary antineoplastic agents approved for use in the U.S. Those skilled in the art will appreciate that the "product labels" required on all U.S. approved chemotherapeutics describe approved indications, dosing information, toxicity data, and the like, for the exemplary agents.

**Table 1.**

| | | |
|---|---|---|
| Aldesleukin | Proleukin | Chiron Corp., Emeryville, CA |
| (des-alanyl-1, serine-125 human interleukin-2) | | |
| Alemtuzumab | Campath | Millennium and ILEX Partners, LP, Cambridge, MA |
| (IgG1κ anti CD52 antibody) | | |
| Alitretinoin | Panretin | Ligand Pharmaceuticals, Inc., San Diego CA |
| (9-cis-retinoic acid) | | |
| Allopurinol | Zyloprim | GlaxoSmithKline, Research Triangle Park, NC |
| (1,5-dihydro-4 H -pyrazolo[3,4-d]pyrimidin-4-one monosodium salt) | | |
| Altretamine | Hexalen | US Bioscience, West Conshohocken, PA |
| (N,N,N',N,N",N",- hexamethyl-1,3,5-triazine-2, 4, 6-triamine) | | |
| Amifostine | Ethyol | US Bioscience |
| (ethanethiol, 2-[(3-aminopropyl)amino]-, dihydrogen phosphate (ester)) | | |
| Anastrozole | Arimidex | AstraZeneca Pharmaceuticals, LP, Wilmington, DE |
| (1,3-Benzenediacetonitrile, a, a, a', a'-tetramethyl-5-(1H-1,2,4-triazol-1-ylmethyl)) | | |
| Arsenic trioxide | Trisenox | Cell Therapeutic, Inc., Seattle, WA |
| Asparaginase | Elspar | Merck & Co., Inc., Whitehouse Station, NJ |
| (L-asparagine amidohydrolase, type EC-2) | | |
| BCG Live | TICE BCG | Organon Teknika, Corp., Durham, NC |
| (lyophilized preparation of an attenuated strain of *Mycobacterium bovis (Bacillus Calmette-Gukin* [BCG], substrain Montreal) | | |
| bexarotene capsules | Targretin | Ligand Pharmaceuticals |
| (4-[1-(5,6,7,8-tetrahydro-3,5,5,8,8-pentamethyl-2-napthalenyl) ethenyl] benzoic acid) | | |
| bexarotene gel | Targretin | Ligand Pharmaceuticals |
| Bleomycin | Blenoxane | Bristol-Myers Squibb Co., NY, NY |
| (cytotoxic glycopeptide antibiotics produced by *Streptomyces verticillus;* bleomycin A₂ and bleomycin B₂) | | |
| Capecitabine | Xeloda | Roche |
| (5'-deoxy-5-fluoro-N-[(pentyloxy)carbonyl]-cytidine) | | |
| Carboplatin | Paraplatin | Bristol-Myers Squibb |
| (platinum, diammine [1,1-cyclobutanedicarboxylato(2-)-0, 0']-,(SP-4-2)) | | |
| Carmustine | BCNU, BiCNU | Bristol-Myers Squibb |
| (1,3-bis(2-chloroethyl)-1-nitrosourea) | | |
| Carmustine with Polifeprosan 20 Implant | Gliadel Wafer | Guilford Pharmaceuticals, Inc., Baltimore, MD |
| Celecoxib | Celebrex | Searle Pharmaceuticals, England |
| (as 4-[5-(4-methylphenyl)-3- (trifluoromethyl)-1H-pyrazol-1-yl] benzenesulfonamide) | | |
| Chlorambucil | Leukeran | GlaxoSmithKline |
| (4-[bis(2chlorethyl)amino]benzenebutanoic acid) | | |
| Cisplatin | Platinol | Bristol-Myers Squibb |
| (PtCl₂,H₆N₂) | | |
| Cladribine | Leustatin, 2-CdA | R.W. Johnson Pharmaceutical Research Institute, Raritan, NJ |
| (2-chloro-2'-deoxy-b-D-adenosine) | | |
| Cyclophosphamide | Cytoxan, Neosar | Bristol-Myers Squibb |
| (2-[bis(2-chloroethyl)amino] tetrahydro-2H-13,2-oxazaphosphorine 2-oxide monohydrate) | | |
| Cytarabine | Cytosar-U | Pharmacia & Upjohn Company |
| (1-b-D-Arabinofuranosylcytosine, C₉H₁₃N₃O₅) | | |
| cytarabine liposomal | DepoCyt | Skye Pharmaceuticals, Inc., San Diego, CA |
| Dacarbazine | DTIC-Dome | Bayer AG, Leverkusen, Germany |
| (5-(3,3-dimethyl-1-triazeno)-imidazole-4-carboxamide (DTIC)) | | |
| Dactinomycin, actinomycin D | Cosmegen | Merck |
| (actinomycin produced by *Streptomyces parvullits,* C₆₂H₈₆N₁₂O₁₆) | | |
| Darbepoetin alfa | Aranesp | Amgen, Inc., Thousand Oaks, CA |
| (recombinant peptide) | | |
| daunorubicin liposomal | DanuoXom | Nexstar Pharmaceuticals, Inc., Boulder, CO |
| ((8S-cis)-8-acetyl-10-[(3-amino-2,3,6-trideoxy-á-L-lyxo-hexopyranosyl)oxy]-7,8,9,10-tetrahydro-6,8,11-trihydroxy-1-methoxy-5,12-naphthacenedione hydrochloride) | e | |
| Daunorubicin HCl, daunomycin | Cerubidine | Wyeth Ayerst, Madison, NJ |
| ((1 *S* ,3 *S* )-3-Acetyl-1,2,3,4,6,11-hexahydro-3,5,12-trihydroxy-10-methoxy-6,11-dioxo-1-naphthacenyl 3-amino-2,3,6-trideoxy-(alpha)-L*- lyxo* -hexopyranoside hydrochloride) | | |
| Denileukin diftitox | Ontak | Seragen, Inc., Hopkinton, MA |
| (recombinant peptide) | | |
| Dexrazoxane | Zinecard | Pharmacia & Upjohn Company |
| ((S)-4,4'-(1-methyl-1,2-ethanediyl)bis-2,6-piperazinedione) | | |
| Docetaxel | Taxotere | Aventis Pharmaceuticals, Inc., Bridgewater, NJ |
| ((2R,3S)-N-carboxy-3-phenylisoserine, N-tert-butyl ester, 13-ester with 5b-20-epoxy-12a,4,7b,10b,13a-hexahydroxytax-11-en-9-one 4-acetate 2-benzoate, trihydrate) | | |
| Doxorubicin HCl | Adriamycin , Rubex | Pharmacia & Upjohn Company |
| (85,10S)-10-[(3-amino-2,3,6-trideoxy-a-L-lyxo-hexopyranosyl)oxy] -8-glycolyl-7,8,9,10-tetrahydro-6,8,11- trihydroxy-1-methoxy-5,12-naphthacenedione hydrochloride) | | |
| doxorubicin | Adriamycin PFS Intravenous injection | Pharmacia & Upjohn Company |
| doxorubicin liposomal | Doxil | Sequus Pharmaceuticals, Inc., Menlo park, CA |
| dromostanolone propionate | Dromostano lone | Eli Lilly & Company, Indianapolis, IN |
| (17b-Hydroxy-2a-methyl-5a-androstan-3-one propionate) | | |
| dromostanolone propionate | Masterone injection | Syntex, Corp., Palo Alto, CA |
| Elliott's B Solution | Elliott's B Solution | Orphan Medical, Inc |
| Epirubicin | Ellence | Pharmacia & Upjohn Company |
| ((8S-cis)-10-[(3-amino-2,3,6-trideoxy-a-L-arabino- hexopyranosyl)oxy]-7,8,9,10-tetrahydro-6,8,11-trihydroxy-8-(hydroxyacetyl)-1-methoxy-5,12-naphthacenedione hydrochloride) | | |
| Epoetin alfa | Epogen | Amgen, Inc |
| (recombinant peptide) | | |
| Estramustine | Emcyt | Pharmacia & Upjohn Company |
| (estra-1,3,5(10)-triene-3,17-diol(17(beta))-, 3-[bis(2-chloroethyl)carbamate] 17-(dihydrogen phosphate), disodium salt, monohydrate, or estradiol 3-[bis(2-chloroethyl)carbamate] 17-(dihydrogen phosphate), disodium salt, monohydrate) | | |
| Etoposide phosphate | Etopophos | Bristol-Myers Squibb |
| (4'-Demethylepipodophyllotoxin 9-[4,6-O-(R)-ethylidene-(beta)-D-glucopyranoside], 4'-(dihydrogen phosphate)) | | |
| etoposide, VP-16 | Vepesid | Bristol-Myers Squibb |
| (4'-demethylepipodophyllotoxin 9-[4,6-0-(R)-ethylidene-(beta)-D-glucopyranoside]) | | |
| Exemestane | Aromasin | Pharmacia & Upjohn Company |
| (6-methylenandrosta-1,4-diene-3, 17-dione) | | |
| Filgrastim | Neupogen | Amgen, Inc |
| (r-metHuG-CSF) | | |
| floxuridine (intraarterial) | FUDR | Roche |
| (2'-deoxy-5-fluorouridine) | | |
| Fludarabine | Fludara | Berlex Laboratories, Inc., Cedar Knolls, NJ |
| (fluorinated nucleotide analog of the antiviral agent vidarabine, 9-b -D-arabinofuranosyladenine (ara-A)) | | |
| Fluorouracil, 5-FU | Adrucil | ICN Pharmaceuticals, Inc., Humacao, Puerto Rico |
| (5-fluoro-2,4(1H,3H)-pyrimidinedione) | | |
| Fulvestrant | Faslodex | IPR Pharmaceuticals, Guayama, Puerto Rico |
| (7-alpha-[9-(4,4,5,5,5-penta fluoropentylsulphinyl) nonyl]estra-1,3,5-(10)-triene-3,17-beta-diol) | | |
| Gemcitabine | Gemzar | Eli Lilly |
| (2'-deoxy-2', 2'-difluorocytidine monohydrochloride (b-isomer)) | | |
| Gemtuzumab Ozogamicin (anti-CD33 hP67.6) | Mylotarg | Wyeth Ayerst |
| Goserelin acetate | Zoladex Implant | AstraZeneca Pharmaceuticals |
| (acetate salt of [D-Ser(But)⁶,Azgly¹⁰]LHRH; pyro-Glu-His-Trp-Ser-Tyr-D-Ser(But)-Leu-Arg-Pro-Azgly-NH2 acetate [C₅₉H₈₄N₁₈O₁₄ •(C₂H₄O₂)ₓ | | |
| Hydroxyurea | Hvdrea | Bristol-Myers Squibb |
| Ibritumomab Tiuxetan | Zevalin | Biogen IDEC, Inc., Cambridge MA |
| (immunoconjugate resulting from a thiourea covalent bond between the monoclonal antibody Ibritumomab and the linker-chelator tiuxetan [N-[2-bis(carboxymethyl)amino]-3-(p-isothiocyanatophenyl)- propyl]-[N-[2-bis(carboxymethyl)amino]-2-(methyl) - ethyl]glycine) | | |
| Idarubicin | Idamycin | Pharmacia & Upjohn Company |
| (5, 12-Naphthacenedione, 9-acetyl-7-[(3-amino-2,3,6-trideoxy-(alpha)-L-lyxo-hexopyranosyl)oxy]-7,8,9,10-tetrahydro-6,9,11-trihydroxyhydrochloride, (7S- cis)) | | |
| Ifosfamide | IFEX | Bristol-Myers Squibb |
| (3-(2-chloroethyl)-2-[(2-chloroethyl)amino]tetrahydro-2H-1,3,2-oxazaphosphorine 2-oxide) | | |
| Imatinib Mesilate | Gleevec | Novartis AG, Basel, Switzerland |
| (4-[(4-Methyl-1-piperazinyl)methyl]-N-[4-methyl-3-[[4-(3-pyridinyl)-2-pyrimidinyl]amino]-phenyl]benzamide methanesulfonate) | | |
| Interferon alfa-2a | Roferon-A | Hoffmann-La Roche, Inc., Nutley, NJ |
| (recombinant peptide) | | |
| Interferon alfa-2b | Intron A (Lyophilize d Betaseron) | Schering AG, Berlin, Germany |
| (recombinant peptide) | | |
| Irinotecan HCl | Camptosar | Pharmacia & Upjohn Company |
| ((4S)-4,11-diethyl-4-hydroxy-9-[(4-piperidinopiperidino)carbonyloxy]-1H-pyrano[3', 4': 6,7] indolizino[1,2-b] quinoline-3,14(4H, 12H) dione hydrochloride trihydrate) | | |
| Letrozole | Femara | Novartis |
| (4,4'-(1H-1,2,4 -Triazol-1-ylmethylene) dibenzonitrile) | | |
| Leucovorin | Wellcovori n, Leucovorin | Immunex, Corp., Seattle, WA |
| (L-Glutamic acid, N(4[[(2amino-5-formyl-1,4,5,6,7,8 hexahydro4oxo6-pteridinyl)methyl]amino]benzoyl], calcium salt (1:1)) | | |
| Levamisole HCl | Ergamisol | Janssen Research Foundation, Titusville, NJ |
| ((-)-(S)-2,3,5, 6-tetrahydro-6-phenylimidazo [2,1-b] thiazole monohydrochloride C₁₁H₁₂N₂S·HCl) | | |
| Lomustine | CeeNU | Bristol-Myers Squibb |
| (1-(2-chloro-ethyl)-3-cyclohexyl-1-nitrosourea) | | |
| Meclorethamine, nitrogen mustard | Mustargen | Merck |
| (2-chloro-N-(2-chloroethyl)-N-methylethanamine hydrochloride) | | |
| Megestrol acetate | Megace | Bristol-Myers Squibb |
| 17α( acetyloxy)- 6- methylpregna- 4,6- diene-3,20- dione | | |
| Melphalan, L-PAM | Alkeran | GlaxoSmithKline |
| (4-[bis(2-chloroethyl)amino]-L-phenylalanine) | | |
| Mercaptopurine, 6-MP | Purinethol | GlaxoSmithKline |
| (1,7-dihydro-6 H -purine-6-thione monohydrate) | | |
| Mesna | Mesnex | Asta Medica |
| (sodium 2-mercaptoethane sulfonate) | | |
| Methotrexate | Methotrexat | Lederle Laboratories |
| (N-[4-[[(2,4-diamino-6-pteridinyl)methyl]methylamino]benzoyl]-L-glutamic acid) | e | |
| Methoxsalen | Uvadex | Therakos, Inc., Way Exton, Pa |
| (9-methoxy-7H-furo[3,2-g][1]-benzopyran-7-one) | | |
| Mitomycin C | Mutamycin | Bristol-Myers Squibb |
| mitomycin C | Mitozvtrex | SuperGen, Inc., Dublin, CA |
| Mitotane | Lysodren | Bristol-Myers Squibb |
| (1,1-dichloro-2-(o-chlorophenyl)-2-(p-chlorophenyl) ethane) | | |
| Mitoxantrone | Novantrone | Immunex Corporation |
| (1,4-dihydroxy-5,8-bis[[2- [(2-hydroxyethyl)amino]ethyl]amino]-9,10-anthracenedione dihydrochloride) | | |
| Nandrolone phenpropionate | Durabolin-50 | Organon, Inc., West Orange, NJ |
| Nofetumomab | Verluma | Boehringer Ingelheim Pharma KG, Germany |
| Oprelvekin | Neumega | Genetics Institute, Inc., Alexandria, VA |
| (IL-11) | | |
| Oxaliplatin | Eloxatin | Sanofi Synthelabo, Inc., NY, NY |
| (cis-[(1R,2R)-1,2-cyclohexanediamine-N,N'] [oxalato(2-)-O,O'] platinum) | | |
| Paclitaxel | TAXOL | Bristol-Myers Squibb |
| (5ß, 20-Epoxy-1,2a, 4,7ß, 10ß, 13a-hexahydroxytax-11-en-9-one 4,10-diacetate 2-benzoate 13-ester with (2R, 3 S)- N-benzoyl-3-phenylisoserine) | | |
| Pamidronate | Aredia | Novartis |
| (phosphonic acid (3-amino-1-hydroxypropylidene) bis-, disodium salt, pentahydrate, (APD)) | | |
| Pegademase | Adagen (Pegademas e Bovine) | Enzon Pharmaceuticals, Inc., Bridgewater, NJ |
| ((monomethoxypolyethylene glycol succinimidyl) 11 - 17 -adenosine deaminase) | | |
| Pegaspargase | Oncaspar | Enzon |
| (monomethoxypolyethylene glycol succinimidyl L-asparaginase) | | |
| Pegfilgrastim | Neulasta | Amgen, Inc |
| (covalent conjugate of recombinant methionyl human G-CSF (Filgrastim) and monomethoxypolyethylene glycol) | | |
| Pentostatin | Nipent | Parke-Davis Pharmaceutical Co., Rockville, MD |
| Pipobroman | Vercyte | Abbott Laboratories, Abbott Park, IL |
| Plicamycin, Mithramycin | Mithracin | Pfizer, Inc., NY, NY |
| (antibiotic produced by *Streptomyces plicatus)* | | |
| Porfimer sodium | Photofrin | QLT Phototherapeutics, Inc., Vancouver, Canada |
| Procarbazine | Matulane | Sigma Tau Pharmaceuticals, Inc., Gaithersburg, MD |
| (N-isopropyl-µ-(2-methylhydrazino)-p-toluamide monohydrochloride) | | |
| Quinacrine | Atabrine | Abbott Labs |
| (6-chloro-9-(1-methyl-4-diethyl-amine) butylamino-2-methoxyacridine) | | |
| Rasburicase | Elitek | Sanofi-Synthelabo, Inc., |
| (recombinant peptide) | | |
| Rituximab | Rituxan | Genentech, Inc., South San Francisco, CA |
| (recombinant anti-CD20 antibody) | | |
| Sargramostim (recombinant peptide) | Prokine | Immunex Corp |
| Streptozocin | Zanosar | Pharmacia & Upjohn Company |
| (streptozocin 2 -deoxy - 2 - [[(methylnitrosoamino)carbonyl]amino] - a(and b) - D - glucopyranose and 220 mg citric acid anhydrous) | | |
| Talc | Sclerosol | Bryan, Corp., Woburn, MA |
| (Mg₃Si₄O₁₀ (OH)₂) | | |
| Tamoxifen | Nolvadex | AstraZeneca Pharmaceuticals |
| ((Z)2-[4-(1,2-diphenyl-1-butenyl) phenoxy]-N, N-dimethylethanamine 2-hydroxy-1,2,3-propanetricarboxylate (1:1)) | | |
| Temozolomide | Temodar | Schering |
| (3,4-dihydro-3-methyl-4-oxoimidazo[5,1-d]-as-tetrazine-8-carboxamide) | | |
| teniposide, VM-26 | Vumon | Bristol-Myers Squibb |
| (4'-demethylepipodophyllotoxin 9-[4,6-0-(R)-2- thenylidene-(beta)-D-glucopyranoside]) | | |
| Testolactone | Teslac | Bristol-Myers Squibb |
| (13-hydroxy-3-oxo-13,17-secoandrosta-1,4-dien-17-oic acid [dgr]-lactone) | | |
| Thioguanine, 6-TG | Thioguanin e | GlaxoSmithKline |
| (2-amino-1,7-dihydro-6 H - purine-6-thione) | | |
| Thiotepa | Thioplex | Immunex Corporation |
| (Aziridine, 1,1',1"-phosphinothioylidynetris-, or Tris (1-aziridinyl) phosphine sulfide) | | |
| Topotecan HCl | Hycamtin | GlaxoSmithKline |
| ((S)-10-[(dimethylamino) methyl]-4-ethyl-4,9-dihydroxy-1H-pyrano[3', 4': 6,7] indolizino [1,2-b] quinoline-3,14-(4H,12H)-dione monohydrochloride) | | |
| Toremifene | Fareston | Roberts Pharmaceutical Corp., Eatontown, NJ |
| (2-(p-[(Z)-4-chloro-1,2-diphenyl-1-butenyl]-phenoxy)-N,N-dimethylethylamine citrate (1:1)) | | |
| Tositumomab, I 131 Tositumomab | Bexxar | Corixa Corp., Seattle, WA |
| (recombinant murine immunotherapeutic monoclonal IgG₂ₐ lambda anti-CD20 antibody (I 131 is a radioimmunotherapeutic antibody)) | | |
| Trastuzumab | Herceptin | Genentech, Inc |
| (recombinant monoclonal IgG₁ kappa anti-HER2 antibody) | | |
| Tretinoin, ATRA | Vesanoid | Roche |
| (all-trans retinoic acid) | | |
| Uracil Mustard | Uracil | Roberts Labs |
| | Mustard | |
| | Capsules | |
| Valrubicin, N-trifluoroacetyladriamycin-14-valerate | Valstar | Anthra --> Medeva |
| ((2S-cis)-2- [1,2,3,4,6,11-hexahydro-2,5,12-trihydroxy-7 methoxy-6,11-dioxo-[[4 2,3,6-trideoxy-3- [(trifluoroacetyl)-amino-α-L-*lyxo-*hexopyranosyl]oxyl]-2-naphthacenyl]-2-oxoethyl pentanoate) | | |
| Vinblastine, Leurocristine | Velban | Eli Lilly |
| (C₄₆H₅₆N₄O₁₀•H₂SO₄) | | |
| Vincristine | Oncovin | Eli Lilly |
| (C₄₆H₅₆N₄O₁₀•H₂SO₄) | | |
| Vinorelbine | Navelbine | GlaxoSmithKline |
| (3' ,4'-didehydro-4'-deoxy-C'-norvincaleukoblastine [R-(R*,R*)-2,3-dihydroxybutanedioate (1:2)(salt)]) | | |
| Zoledronate, Zoledronic acid | Zometa | Novartis |
| ((1-Hydroxy-2-imidazol-1-yl-phosphonoethyl) phosphonic acid monohydrate) | | |

Anticancer agents further include compounds which have been identified to have anticancer activity but are not currently approved by the U.S. Food and Drug Administration or other counterpart agencies or are undergoing evaluation for new uses. Examples include, but are not limited to, 3-AP, 12-O-tetradecanoylphorbol-13-acetate, 17AAG, 852A, ABI-007, ABR-217620, ABT-751, ADI-PEG 20, AE-941, AG-013736, AGRO100, alanosine, AMG 706, antibody G250, antineoplastons, AP23573, apaziquone, APC8015, atiprimod, ATN-161, atrasenten, azacitidine, BB-10901, BCX-1777, bevacizumab, BG00001, bicalutamide, BMS 247550, bortezomib, bryostatin-1, buserelin, calcitriol, CCI-779, CDB-2914, cefixime, cetuximab, CG0070, cilengitide, clofarabine, combretastatin A4 phosphate, CP-675,206, CP-724,714, CpG 7909, curcumin, decitabine, DENSPM, doxercalciferol, E7070, E7389, ecteinascidin 743, efaproxiral, eflornithine, EKB-569, enzastaurin, erlotinib, exisulind, fenretinide, flavopiridol, fludarabine, flutamide, fotemustine, FR901228, G17DT, galiximab, gefitinib, genistein, glufosfamide, GTI-2040, histrelin, HKI-272, homoharringtonine, HSPPC-96, hu14.18-interleukin-2 fusion protein, HuMax-CD4, iloprost, imiquimod, infliximab, interleukin-12, IPI-504, irofulven, ixabepilone, lapatinib, lenalidomide, lestaurtinib, leuprolide, LMB-9 immunotoxin, lonafarnib, luniliximab, mafosfamide, MB07133, MDX-010, MLN2704, monoclonal antibody 3F8, monoclonal antibody J591, motexafin, MS-275, MVA-MUC1-IL2, nilutamide, nitrocamptothecin, nolatrexed dihydrochloride, nolvadex, NS-9, O6-benzylguanine, oblimersen sodium, ONYX-015, oregovomab, OSI-774, panitumumab, paraplatin, PD-0325901, pemetrexed, PHY906, pioglitazone, pirfenidone, pixantrone, PS-341, PSC 833, PXD101, pyrazoloacridine, R115777, RAD001, ranpimase, rebeccamycin analogue, rhuAngiostatin protein, rhuMab 2C4, rosiglitazone, rubitecan, S-1, S-8184, satraplatin, SB-, 15992, SGN-0010, SGN-40, sorafenib, SR31747A, ST1571, SU011248, suberoylanilide hydroxamic acid, suramin, talabostat, talampanel, tariquidar, temsirolimus, TGFa-PE38 immunotoxin, thalidomide, thymalfasin, tipifarnib, tirapazamine, TLK286, trabectedin, trimetrexate glucuronate, TroVax, UCN-1, valproic acid, vinflunine, VNP40101M, volociximab, vorinostat, VX-680, ZD1839, ZD6474, zileuton, and zosuquidar trihydrochloride.

For a more detailed description of anticancer agents and other therapeutic agents, those skilled in the art are referred to any number of instructive manuals including, but not limited to, the Physician's Desk Reference and to Goodman and Gilman's "Pharmaceutical Basis of Therapeutics" tenth edition, Eds. Hardman et al., 2002.

The present invention relates to methods for administering a compound of Formula I with radiation therapy. The example is not limited by the types, amounts, or delivery and administration systems used to deliver the therapeutic dose of radiation to an animal. For example, the animal may receive photon radiotherapy, particle beam radiation therapy, other types of radiotherapies, and combinations thereof. In some examples, the radiation is delivered to the animal using a linear accelerator. In still other examples, the radiation is delivered using a gamma knife.

The source of radiation can be external or internal to the animal. External radiation therapy is most common and involves directing a beam of high-energy radiation to a tumor site through the skin using, for instance, a linear accelerator. While the beam of radiation is localized to the tumor site, it is nearly impossible to avoid exposure of normal, healthy tissue. However, external radiation is usually well tolerated by animals. Internal radiation therapy involves implanting a radiation-emitting source, such as beads, wires, pellets, capsules, particles, and the like, inside the body at or near the tumor site including the use of delivery systems that specifically target cancer cells (*e.g*., using particles attached to cancer cell binding ligands). Such implants can be removed following treatment, or left in the body inactive. Types of internal radiation therapy include, but are not limited to, brachytherapy, interstitial irradiation, intracavity irradiation, radioimmunotherapy, and the like.

The animal may optionally receive radiosensitizers (*e.g*., metronidazole, misonidazole, intra-arterial Budr, intravenous iododeoxyuridine (IudR), nitroimidazole, 5-substituted-4-nitroimidazoles, 2H-isoindolediones, [[(2-bromoethyl)-amino]methyl]-nitro-1H-imidazole-1-ethanol, nitroaniline derivatives, DNA-affinic hypoxia selective cytotoxins, halogenated DNA ligand, 1,2,4 benzotriazine oxides, 2-nitroimidazole derivatives, fluorine-containing nitroazole derivatives, benzamide, nicotinamide, acridine-intercalator, 5-thiotretrazole derivative, 3-nitro-1,2,4-triazole, 4,5-dinitroimidazole derivative, hydroxylated texaphrins, cisplatin, mitomycin, tiripazamine, nitrosourea, mercaptopurine, methotrexate, fluorouracil, bleomycin, vincristine, carboplatin, epirubicin, doxorubicin, cyclophosphamide, vindesine, etoposide, paclitaxel, heat (hyperthermia), and the like), radioprotectors (*e.g.*, cysteamine, aminoalkyl dihydrogen phosphorothioates, amifostine (WR 2721), IL-1, IL-6, and the like). Radiosensitizers enhance the killing of tumor cells. Radioprotectors protect healthy tissue from the harmful effects of radiation.

Any type of radiation can be administered to an animal, so long as the dose of radiation is tolerated by the animal without unacceptable negative side-effects. Suitable types of radiotherapy include, for example, ionizing (electromagnetic) radiotherapy (*e.g.,* X-rays or gamma rays) or particle beam radiation therapy (*e.g*., high linear energy radiation). Ionizing radiation is defined as radiation comprising particles or photons that have sufficient energy to produce ionization, *i.e.,* gain or loss of electrons (as described in, for example, U.S. 5,770,581 incorporated herein by reference in its entirety). The effects of radiation can be at least partially controlled by the clinician. In one example, the dose of radiation is fractionated for maximal target cell exposure and reduced toxicity.

In one example, the total dose of radiation administered to an animal is about .01 Gray (Gy) to about 100 Gy. In another example about 10 Gy to about 65 Gy *(e.g.,* about 15 Gy, 20 Gy, 25 Gy, 30 Gy, 35 Gy, 40 Gy, 45 Gy, 50 Gy, 55 Gy, or 60 Gy) are administered over the course of treatment. While in some examples a complete dose of radiation can be administered over the course of one day, the total dose is ideally fractionated and administered over several days. Desirably, radiotherapy is administered over the course of at least about 3 days, *e.g.,* at least 5, 7, 10, 14, 17, 21, 25, 28, 32, 35, 38, 42, 46, 52, or 56 days (about 1-8 weeks). Accordingly, a daily dose of radiation will comprise approximately 1-5 Gy (*e.g.,* about 1 Gy, 1.5 Gy, 1.8 Gy, 2 Gy, 2.5 Gy, 2.8 Gy, 3 Gy, 3.2 Gy, 3.5 Gy, 3.8 Gy, 4 Gy, 4.2 Gy, or 4.5 Gy), or 1-2 Gy *(e.g.,* 1.5-2 Gy). The daily dose of radiation should be sufficient to induce destruction of the targeted cells. If stretched over a period, in one example, radiation is not administered every day, thereby allowing the animal to rest and the effects of the therapy to be realized. For example, radiation desirably is administered on 5 consecutive days, and not administered on 2 days, for each week of treatment, thereby allowing 2 days of rest per week. However, radiation can be administered 1 day/week, 2 days/week, 3 days/week, 4 days/week, 5 days/week, 6 days/week, or all 7 days/week, depending on the animal's responsiveness and any potential side effects. Radiation therapy can be initiated at any time in the therapeutic period. In one example, radiation is initiated in week 1 or week 2, and is administered for the remaining duration of the therapeutic period. For example, radiation is administered in weeks 1-6 or in weeks 2-6 of a therapeutic period comprising 6 weeks for treating, for instance, a solid tumor. Alternatively, radiation is administered in weeks 1-5 or weeks 2-5 of a therapeutic period comprising 5 weeks. These exemplary radiotherapy administration schedules are not intended, however, to limit the present invention.

Antimicrobial therapeutic agents may also be used as therapeutic agents in the present invention. Any agent that can kill, inhibit, or otherwise attenuate the function of microbial organisms may be used, as well as any agent contemplated to have such activities. Antimicrobial agents include, but are not limited to, natural and synthetic antibiotics, antibodies, inhibitory proteins (*e.g.,* defensins), antisense nucleic acids, membrane disruptive agents and the like, used alone or in combination. Indeed, any type of antibiotic may be used including, but not limited to, antibacterial agents, antiviral agents, antifungal agents, and the like.

In some examples relating to present invention, a compound of Formula I and one or more therapeutic agents or anticancer agents are administered to an animal under one or more of the following conditions: at different periodicities, at different durations, at different concentrations, by different administration routes, *etc.* In some examples, the compound is administered prior to the therapeutic or anticancer agent, *e.g.,* 0.5, 1, 2, 3, 4, 5, 10, 12, or 18 hours, 1, 2, 3, 4, 5, or 6 days, or 1, 2, 3, or 4 weeks prior to the administration of the therapeutic or anticancer agent. In some examples, the compound is administered after the therapeutic or anticancer agent, *e.g.,* 0.5, 1, 2, 3, 4, 5, 10, 12, or 18 hours, 1, 2, 3, 4, 5, or 6 days, or 1, 2, 3, or 4 weeks after the administration of the anticancer agent. In some examples, the compound and the therapeutic or anticancer agent are administered concurrently but on different schedules, *e.g.,* the compound is administered daily while the therapeutic or anticancer agent is administered once a week, once every two weeks, once every three weeks, or once every four weeks. In other examples, the compound is administered once a week while the therapeutic or anticancer agent is administered daily, once a week, once every two weeks, once every three weeks, or once every four weeks.

Compositions within the scope of this invention include all compositions wherein the compounds of the present invention are contained in an amount which is effective to achieve its intended purpose. While individual needs vary, determination of optimal ranges of effective amounts of each component is within the skill of the art. Typically, the compounds may be administered to mammals, *e.g.* humans, orally at a dose of 0.0025 to 50 mg/kg, or an equivalent amount of the pharmaceutically acceptable salt thereof, per day of the body weight of the mammal being treated for disorders responsive to induction of apoptosis. In one example, about 0.01 to about 25 mg/kg is orally administered to treat, ameliorate, or prevent such disorders. For intramuscular injection, the dose is generally about one-half of the oral dose. For example, a suitable intramuscular dose would be about 0.0025 to about 25 mg/kg, or from about 0.01 to about 5 mg/kg.

The unit oral dose may comprise from about 0.01 to about 1000 mg, for example, about 0.1 to about 100 mg of the compound. The unit dose may be administered one or more times daily as one or more tablets or capsules each containing from about 0.1 to about 10 mg, conveniently about 0.25 to 50 mg of the compound or its solvates.

In a topical formulation, the compound may be present at a concentration of about 0.01 to 100 mg per gram of carrier. In a one example, the compound is present at a concentration of about 0.07-1.0 mg/ml, for example, about 0.1-0.5 mg/ml, and in one example, about 0.4 mg/ml.

In addition to administering the compound as a raw chemical, the compounds of the invention may be administered as part of a pharmaceutical preparation containing suitable pharmaceutically acceptable carriers comprising excipients and auxiliaries which facilitate processing of the compounds into preparations which can be used pharmaceutically. The preparations, particularly those preparations which can be administered orally or topically and which can be used for one type of administration, such as tablets, dragees, slow release lozenges and capsules, mouth rinses and mouth washes, gels, liquid suspensions, hair rinses, hair gels, shampoos and also preparations which can be administered rectally, such as suppositories, as well as suitable solutions for administration by intravenous infusion, injection, topically or orally, contain from about 0.01 to 99 percent, in one example from about 0.25 to 75 percent of active compound(s), together with the excipient.

The pharmaceutical compositions of the invention may be administered to any animal which may experience the beneficial effects of the compounds of the invention. Foremost among such animals are mammals, *e.g.,* humans, although the invention is not intended to be so limited. Other animals include veterinary animals (cows, sheep, pigs, horses, dogs, cats and the like).

The compounds and pharmaceutical compositions thereof may be administered by any means that achieve their intended purpose. For example, administration may be by parenteral, subcutaneous, intravenous, intramuscular, intraperitoneal, transdermal, buccal, intrathecal, intracranial, intranasal or topical routes. Alternatively, or concurrently, administration may be by the oral route. The dosage administered will be dependent upon the age, health, and weight of the recipient, kind of concurrent treatment, if any, frequency of treatment, and the nature of the effect desired.

The pharmaceutical preparations of the present invention are manufactured in a manner which is itself known, for example, by means of conventional mixing, granulating, dragee-making, dissolving, or lyophilizing processes. Thus, pharmaceutical preparations for oral use can be obtained by combining the active compounds with solid excipients, optionally grinding the resulting mixture and processing the mixture of granules, after adding suitable auxiliaries, if desired or necessary, to obtain tablets or dragee cores.

Suitable excipients are, in particular, fillers such as saccharides, for example lactose or sucrose, mannitol or sorbitol, cellulose preparations and/or calcium phosphates, for example tricalcium phosphate or calcium hydrogen phosphate, as well as binders such as starch paste, using, for example, maize starch, wheat starch, rice starch, potato starch, gelatin, tragacanth, methyl cellulose, hydroxypropylmethylcellulose, sodium carboxymethylcellulose, and/or polyvinyl pyrrolidone. If desired, disintegrating agents may be added such as the above-mentioned starches and also carboxymethyl-starch, cross-linked polyvinyl pyrrolidone, agar, or alginic acid or a salt thereof, such as sodium alginate. Auxiliaries are, above all, flow-regulating agents and lubricants, for example, silica, talc, stearic acid or salts thereof, such as magnesium stearate or calcium stearate, and/or polyethylene glycol. Dragee cores are provided with suitable coatings which, if desired, are resistant to gastric juices. For this purpose, concentrated saccharide solutions may be used, which may optionally contain gum arabic, talc, polyvinyl pyrrolidone, polyethylene glycol and/or titanium dioxide, lacquer solutions and suitable organic solvents or solvent mixtures. In order to produce coatings resistant to gastric juices, solutions of suitable cellulose preparations such as acetylcellulose phthalate or hydroxypropylmethyl-cellulose phthalate, are used. Dye stuffs or pigments may be added to the tablets or dragee coatings, for example, for identification or in order to characterize combinations of active compound doses.

Other pharmaceutical preparations which can be used orally include push-fit capsules made of gelatin, as well as soft, sealed capsules made of gelatin and a plasticizer such as glycerol or sorbitol. The push-fit capsules can contain the active compounds in the form of granules which may be mixed with fillers such as lactose, binders such as starches, and/or lubricants such as talc or magnesium stearate and, optionally, stabilizers. In soft capsules, the active compounds are in one example dissolved or suspended in suitable liquids, such as fatty oils, or liquid paraffin. In addition, stabilizers may be added.

Possible pharmaceutical preparations which can be used rectally include, for example, suppositories, which consist of a combination of one or more of the active compounds with a suppository base. Suitable suppository bases are, for example, natural or synthetic triglycerides, or paraffin hydrocarbons. In addition, it is also possible to use gelatin rectal capsules which consist of a combination of the active compounds with a base. Possible base materials include, for example, liquid triglycerides, polyethylene glycols, or paraffin hydrocarbons.

Suitable formulations for parenteral administration include aqueous solutions of the active compounds in water-soluble form, for example, water-soluble salts and alkaline solutions. In addition, suspensions of the active compounds as appropriate oily injection suspensions may be administered. Suitable lipophilic solvents or vehicles include fatty oils, for example, sesame oil, or synthetic fatty acid esters, for example, ethyl oleate or triglycerides or polyethylene glycol-400. Aqueous injection suspensions may contain substances which increase the viscosity of the suspension include, for example, sodium carboxymethyl cellulose, sorbitol, and/or dextran. Optionally, the suspension may also contain stabilizers.

The topical compositions relating to this invention are formulated in one example as oils, creams, lotions, ointments and the like by choice of appropriate carriers. Suitable carriers include vegetable or mineral oils, white petrolatum (white soft paraffin), branched chain fats or oils, animal fats and high molecular weight alcohol (greater than C₁₂). The carriers may be those in which the active ingredient is soluble. Emulsifiers, stabilizers, humectants and antioxidants may also be included as well as agents imparting color or fragrance, if desired. Additionally, transdermal penetration enhancers can be employed in these topical formulations. Examples of such enhancers can be found in U.S. Pat. Nos. 3,989,816 and 4,444,762.

Creams may be formulated from a mixture of mineral oil, self-emulsifying beeswax and water in which mixture the active ingredient, dissolved in a small amount of an oil such as almond oil, is admixed. A typical example of such a cream is one which includes about 40 parts water, about 20 parts beeswax, about 40 parts mineral oil and about I part almond oil.

Ointments may be formulated by mixing a solution of the active ingredient in a vegetable oil such as almond oil with warm soft paraffin and allowing the mixture to cool. A typical example of such an ointment is one which includes about 30% almond oil and about 70% white soft paraffin by weight.

Lotions may be conveniently prepared by dissolving the active ingredient, in a suitable high molecular weight alcohol such as propylene glycol or polyethylene glycol.

The following examples are illustrative, of the compositions of the present invention.

### EXAMPLE 1

### 6-CHLORO-4'-(3-CHLORO-PHENYL)-2'-(2,2-DIMETHYL-PROPYL)-5-FLUORO-2-OXO-1,2-DIHYDRO-SPIRO[INDOLE-3,3'-PYRROLIDINE]-5'-CARBOXYLIC ACID (3,4-DIHYDROXY-BUTYL)-AMIDE

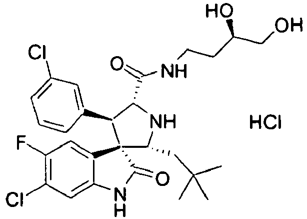

¹H NMR (300 MHz, CD₃OH), δ 7.70 (d, 1H, J = 8.4 Hz), 7.40-7.20 (m, 3H), 7.09 (m, 1H), 6.88 (d, 1H, J = 6.0 Hz), 5.22 (d, 1H, J = 11.4 Hz), 4.39 (m, 1H), 4.11 (d, 1H, J = 11.1 Hz), 3.65-3.32 (m, 9H), 1.85 (m, 1H), 1.60 (m, 1H), 1.41 (m, 1H), 1.19 (m, 1H), 0.84 (s, 9H).

### EXAMPLE 2

### 6-CHLORO-4'-(3-CHLORO-PHENYL)-2'-(2,2-DIMETHYL-PROPYL)-5-FLUORO-2-OXO-1,2-DIHYDRO-SPIRO[INDOLE-3,3'-PYRROLIDINE]-5'-CARBOXYLIC ACID (2,3-DIHYDROXY-PROPYL)-AMIDE

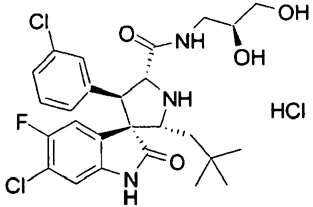

¹H NMR (300 MHz, CD₃OH), δ 7.74 (d, 1H, J = 7.5 Hz), 7.35-7.15 (m, 3H), 7.11 (m, 1H), 6.88 (d, 1H, J = 6.0 Hz), 5.22 (d, 1H, J = 11.4 Hz), 4.33 (m, 1 H), 4.28 (d, 1H, J = 10.5 Hz), 3.67 (m, 2H), 3.65-3.32 (m, 4H), 2.05 (m, 1H), 1.90 (m, 1H), 1.56 (m, 1H), 0.87 (s, 9H).

### EXAMPLE 3

### 6-CHLORO-4'-(3-CHLORO-PHENYL)-2'-(2,2-DIMETHYL-PROPYL)-5-FLUORO-2-OXO-1,2-DIHYDRO-SPIRO[INDOLE-3,3'-PYRROLIDINE]-5'-CARBOXYLIC ACID (3,5-DIHYDROXY-PENTYL)-AMIDE

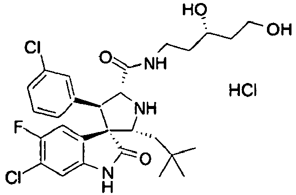

¹H NMR (300 MHz, CD₃OH), δ 7.72 (d, 1H, J = 8.4 Hz), 7.35-7.15 (m, 3H), 7.10 (m, 1H), 6.88 (d, 1H, J = 6.0 Hz), 5.23 (d, 1H, J = 11.1 Hz), 4.65 (m, 1H), 4.13 (d, 1H, J = 11.4 Hz), 3.61 (m, 2H), 3.65-3.32 (m, 5H), 2.02 (m, 1H), 1.93 (m, 1H), 1.56 (m, 3H), 1.15 (m, 1H), 0.92 (s, 9H).

### EXAMPLE 4

### 6-CHLORO-4'-(3-CHLORO-PHENYL)-2'-(2,2-DIMETHYL-PROPYL)-5-FLUORO-2-OXO-1,2-DIHYDRO-SPIRO[INDOLE-3,3'-PYRROLIDINE]-5'-CARBOXYLIC ACID [2-(3-HYDROXY-CYCLOPENTYL)-ETHYL]-AMIDE

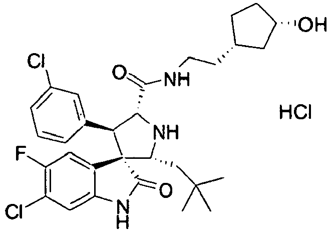

¹H NMR (300 MHz, CD₃OH), δ 7.69 (d, 1H, J = 8.4 Hz), 7.40-7.20 (m, 3H), 7.10 (m, 1H), 6.86 (m, 1H), 5.24 (d, 1H, J = 11.1 Hz), 4.35 (d, 1H, J = 8.4 Hz), 4.09 (m, 1H), 3.61-3.32 (m, 2H), 2.96 (m, 1H), 2.21-1.12 (m, 11H), 0.85 (s, 9H).

### EXAMPLE 5

### 6-CHLORO-4'-(3-CHLORO-PHENYL)-2'-(2,2-DIMETHYL-PROPYL)-5-FLUORO-2-OXO-1,2-DIHYDRO-SPIRO[INDOLE-3,3'-PYRROLIDINE]-5'-CARBOXYLIC ACID [2-(3-HYDROXY-CYCLOPENTYL)-ETHYL]-AMIDE

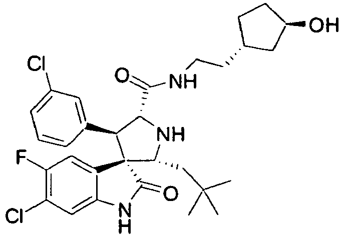

¹H NMR (300 MHz, CD₃OH), δ 7.69 (d, 1H, J = 8.4 Hz), 7.40-7.20 (m, 3H), 7.10 (m, 1H), 6.86 (m, 1H), 5.24 (d, 1H, J = 11.1 Hz), 4.48 (d, 1H, J = 8.4 Hz), 4.12 (m, 1H), 3.64-3.32 (m, 2H), 2.96 (m, 1H), 2.20-1.10 (m, 11H), 0.85 (s, 9H).

### EXAMPLE 6

### 6-CHLORO-4'-(3-CHLORO-PHENYL)-2'-(2,2-DIMETHYL-PROPYL)-5-FLUORO-2-OXO-1,2-DIHYDRO-SPIRO[INDOLE-3,3'-PYRROLIDINE]-5'-CARBOXYLIC ACID [2-(3-HYDROXY-CYCLOPENTYL)-ETHYL]-AMIDE

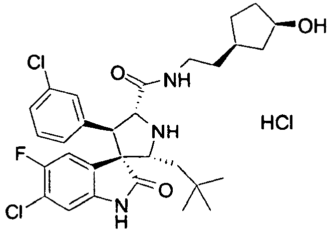

¹H NMR (300 MHz, CD₃OH), δ 7.69 (d, 1H, J = 8.4 Hz), 7.40-7.20 (m, 3H), 7.10 (m, 1H), 6.86 (m, 1H), 5.24 (d, 1H, J = 11.1 Hz), 4.47 (d, 1H, J = 8.4 Hz), 4.11 (m, 1H), 3.65-3.32 (m, 2H), 2.96 (m, 1H), 2.20-1.10 (m, 11H), 0.91 (s, 9H).

### EXAMPLE 7

### 6-CHLORO-4'-(3-CHLORO-PHENYL)-2'-(2,2-DIMETHYL-PROPYL)-5-FLUORO-2-OXO-1,2-DIHYDRO-SPIRO[INDOLE-3,3'-PYRROLIDINE]-5'-CARBOXYLIC ACID [2-(3-HYDROXY-CYCLOPENTYL)-ETHYL]-AMIDE

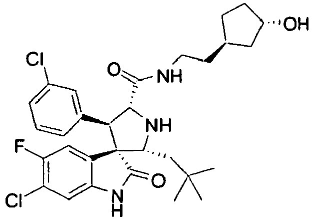

¹H NMR (300 MHz, CD₃OH), δ 7.72 (d, 1H, J = 8.4 Hz), 7.38-7.22 (m, 3H), 7.10 (m, 1H), 6.87 (m, 1H), 5.23 (d, 1H, J = 11.1 Hz), 4.48 (d, 1H, J = 8.4 Hz), 4.12 (m, 1H), 3.56-3.25 (m, 2H), 2.98 (m, 1H), 2.20-1.10 (m, 11H), 0.86 (s, 9H).

### EXAMPLE 8

### 6-CHLORO-4'-(3-CHLORO-4-FLUORO-PHENYL)-2'-(2,2-DIMETHYL-PROPYL)-5-FLUORO-2-OXO-1,2-DIHYDRO-SPIRO[INDOLE-3,3'-PYRROLIDINE]-5'-CARBOXYLIC ACID (4,5-DIHYDROXY-PENTYL)-AMIDE

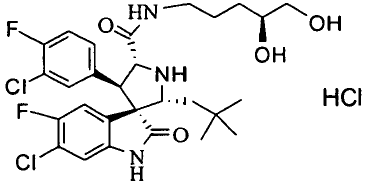

¹H NMR (CD₃OD, 300 MHz), δ 7.73 (d, J=8.4 Hz, 1H), 7.41 (d, J=7.0 Hz, 1H), 7.14 (d, J=6.7 Hz, 2H), 5.25 (d, J=11.3 Hz, 1H), 4.49 (d, J=6.6 Hz, 1H), 4.15 (d, J=6.4 Hz, 1H), 3.48-3.10 (m, 5H), 1.94 (dd, J=8.1, 15.4 Hz, 1H), 1.70-1.10 (m, 5H), 0.92 (s, 9H).

### EXAMPLE 9

### 6-CHLORO-4'-(3-CHLORO-4-FLUORO-PHENYL)-2'-(2,2-DIMETHYL-PROPYL)-5-FLUORO-2-OXO-1,2-DIHYDRO-SPIRO[INDOLE-3,3'-PYRROLIDINE]-5'-CARBOXYLIC ACID (3-MORPHOLIN-4-YL-PROPYL)-AMIDE

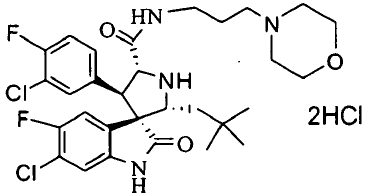

¹H NMR (CD₃OD, 300 MHz), δ 7.82-7.19 (m, 4H), 6.90 (d, J=5.56, 1H), 5.39 (d, J=9.9 Hz, 1H), 4.55 (d, J=6.9 Hz, 1H), 4.28 (d, J=9.3 Hz, 1H), 4.20-3.85 (m, 4H), 3.70-3.40 (m, 2H), 3.25-3.00 (m, 4H), 2.20-1.85 (m, 3H), 1.69 (d, J=15.3 Hz, 2H), 0.934 (s, 9H), 0.70 (t, J=11.3 Hz, 1H).

### EXAMPLE 10

### 6-CHLORO-4'-(3-CHLORO-2-FLUORO-PHENYL)-2'-(2,2-DIMETHYL-PROPYL)-5-FLUORO-2-OXO-1,2-DIHYDRO-SPIRO[INDOLE-3,3'-PYRROLIDINE]-5'-CARBOXYLIC ACID (4-HYDROXY-3-HYDROXYMETHYL-BUTYL)-AMIDE

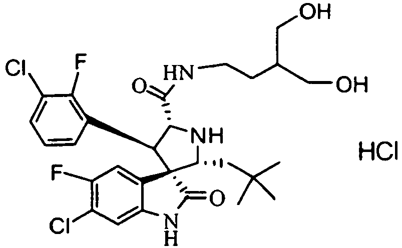

H¹ (300 MHz CD₃OD), δ 8.56 (s br, 1H), 7.68 (t, J = 6.6 Hz, 1H), 7.42 (t, J = 7.2 Hz, 1H), 7.25 (t, J = 7.8 Hz, 1H), 7.05 (d, J = 8.7 Hz, 1H), 6.95 (d, J = 6.0 Hz, 1H), 5.22 (d, J = 10.5 Hz, 1H), 4.54 (d, J = 10.5 Hz, 1H), 4.34-4.31 (m, 1H), 3.51-3.49 (m, 4H), 3.36-3.32 (m, 2H), 2.08 (dd, J = 15.3, 7.5 Hz, 1H), 1.53-1.49 (m, 2H), 1.33 (dd, J = 15.3, 2.7 Hz, 1H), 0.93-0.89 (m, 1H), 0.86 (s, 9H).

### EXAMPLE 11

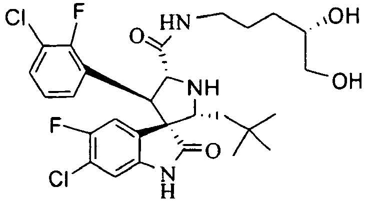

H¹ (300 MHz CD₃OD), δ 8.56 (s br, 1H), 7.65 (t, J = 6.6 Hz, 1H), 7.45 (t, J = 7.2 Hz, 1H), 7.26 (t, J = 7.8 Hz, 1H), 7.01 (d, J = 8.7 Hz, 1H), 6.95 (d, J = 6.0 Hz, 1H), 5.14 (d, J = 10.5 Hz, 1H), 4.51 (d, J = 10.5 Hz, 1H), 4.26 (m, 1H), 3.55-3.50 (m, 2H), 3.37-3.31 (m, 3H), 2.08-2.02 (m, 1H), 1.71-1.45 (m, 2H), 1.35-1.23 (m, 3H), 0.86 (s, 9H).

### EXAMPLE 12

### 6-CHLORO-4'-(3-CHLORO-2-FLUORO-PHENYL)-2'-(2,2-DIMETHYL-PROPYL)-5-FLUORO-2-OXO-1,2-DIHYDRO-SPIRO[INDOLE-3,3'-PYRROLIDINE]-5'-CARBOXYLIC ACID (3,4-DIHYDROXY-BUTYL)-AMIDE

### (MI-419)

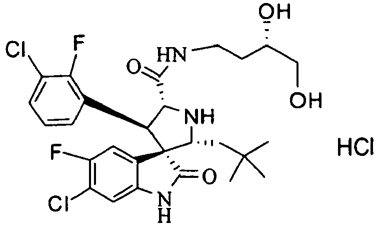

H¹ (300 MHz CD₃OD), δ 8.55 (s br, 1H), 7.66 (t, J = 6.6 Hz, 1H), 7.42 (t, J = 7.8 Hz, 1H), 7.28-7.23 (m, 2H), 7.01 (d, J = 8.7 Hz, 1H), 6.95 (d, J = 6.0 Hz, 1H), 5.20 (d, J = 10.5 Hz, 1H), 4.53 (d, J = 10.5 Hz, 1H), 4.26 (m, 1H), 3.52 (m, 1H), 3.40-3.33 (m, 4H), 2.03 (m, 1H), 1.71-1.45 (m, 2H), 1.35-1.28 (m, 1H), 0.86 (s, 9H).

### EXAMPLE 13

### 6-CHLORO-4'-(3-CHLORO-PHENYL)-2'-(2,2-DIMETHYL-PROPYL)-5-FLUORO-2-OXO-1,2-DIHYDRO-SPIRO[INDOLE-3,3'-PYRROLIDINE]-5'-CARBOXYLIC ACID (2-MORPHOLIN-4-YL-ETHYL)-AMIDE

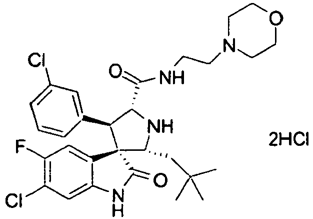

¹H NMR (300 MHz, CD₃OH), δ 7.74 (d, 1H, J = 7.5 Hz), 7.35-7.15 (m, 3H), 7.11 (m, 1H), 6.88 (d, 1H, J = 6.0 Hz), 5.22 (d, 1H, J = 11.4 Hz), 4.55 (d, 1H, J = 9.6 Hz), 4.24 (d, 1H, J = 6.3 Hz), 4.20-3.85 (m, 4H), 3.70-3.40 (m, 4H), 3.25-3.00 (m, 4H), 2.09 (m, 1H), 1.27 (m, I H), 0.934 (s, 9H).

### EXAMPLE 14

### 6-CHLORO-4'-(3-CHLORO-PHENYL)-2'-(2,2-DIMETHYL-PROPYL)-5-FLUORO-2-OXO-1,2-DIHYDRO-SPIRO[INDOLE-3,3'-PYRROLIDINE]-5'-CARBOXYLIC ACID (3-MORPHOLIN-4-YL-PROPYL)-AMIDE

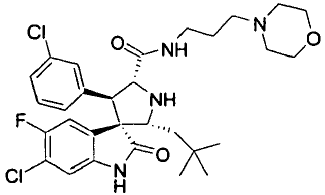

¹H NMR (CD₃OD, 300 MHz), δ 7.72 (d, 1H, J = 8.4 Hz), 7.38-7.22 (m, 3H), 7.10 (m, 1H), 6.87 (m, 1H), 5.23 (d, 1H, J = 11.1 Hz), 4.55 (d, J = 6.9 Hz, 1H), 4.28 (d, J = 9.3 Hz, 1H), 4.20-3.85 (m, 4H), 3.70-3.40(m, 2H), 3.25-3.00 (m, 4H), 2.20-1.85 (m, 4H), 1.69 (d, J = 15.3 Hz, 2H), 0.934 (s, 9H).

### EXAMPLE 15

### 6-CHLORO-4'-(3-CHLORO-PHENYL)-2'-(2,2-DIMETHYL-PROPYL)-5-FLUORO-2-OXO-1,2-DIHYDRO-SPIRO[INDOLE-3,3'-PYRROLIDINE]-5'-CARBOXYLIC ACID (4,5-DIHYDROXY-PENTYL)-AMIDE

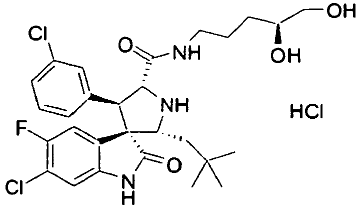

¹H NMR (CD₃OD, 300 MHz), δ 7.69 (d, 1H, J = 8.4 Hz), 7.40-7.20 (m, 3H), 7.10 (m, 1H), 6.86 (m, 1H), 5.24 (d, 1H, J = 11.1 Hz), 4.51 (d, J = 10.5 Hz, 1H), 4.26 (m, 1H), 3.55-3.50 (m, 2H), 3.37-3.31 (m, 3H), 2.08-2.02 (m, 2H), 1.71-1.45 (m, 2H), 1.35-1.23 (m, 3H), 0.86 (s, 9H).

### EXAMPLE 16

### 6-CHLORO-4'-(3-CHLORO-PHENYL)-2'-(2,2-DIMETHYL-PROPYL)-5-FLUORO-2-OXO-1,2-DIHYDRO-SPIRO[INDOLE-3,3'-PYRROLIDINE]-5'-CARBOXYLIC ACID (4-HYDROXY-3-HYDROXYMETHYL-BUTYL) -AMIDE

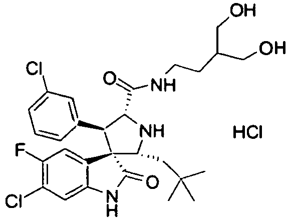

¹H NMR (300 MHz, CD₃OH), δ 7.69 (d, 1H, J = 8.4 Hz), 7.40-7.20 (m, 3H), 7.10 (m, 1H), 6.86 (m, 1H), 5.24 (d, 1H, J = 11.1 Hz), 4.54 (d, J = 10.5 Hz, 1H), 4.34-4.31 (m, 1H), 3.51-3.49 (m, 4H), 3.36-3.32 (m, 2H), 2.08 (dd, J = 15.3, 7.5 Hz, 1H), 1.53-1.49 (m, 2H), 1.33 (dd, J = 15.3, 2.7 Hz, 1H), 0.93-0.89 (m, 1H), 0.86 (s, 9H).

### EXAMPLE 17

### (2'R,3S,3"S,4'R,5'R) 6-CHLORO-4'-(3-CHLORO-4-FLUORO-PHENYL)-2'-(2,2-DIMETHYL-PROPYL)-5-FLUORO-2-OXO-1,2-DIHYDRO-SPIRO[INDOLE-3,3'-PYRROLIDINE]-5'-CARBOXYLIC ACID (3,4-DIHYDROXY-BUTYL)-AMIDE

### (MI-319)

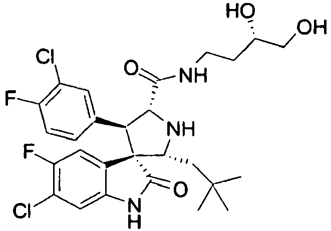

Formula: C₂₇H₃₁Cl₂F₂N₃O₄.HCl (HCl Salt) ¹H NMR (300 MHz, MeOD), δ 8.44 (m, 1H), 7.70 (d, 1H, J = 8.4 Hz), 7.41 (m, 1H), 7.14 (m, 2H), 6.90 (d, 1 H, J = 6.0 Hz), 5.20 (d, 1H, J = 11.1 Hz), 4.47 (d, 1H, J = 8.1 Hz), 4.14 (d, 1H, J = 10.8 Hz), 3.47 (m, 4H), 1.91 (dd, 1H, J = 15.6, 8.4 Hz), 1.57 (m, 1H), 1.43 (m, 1H), 1.21 (dd, 1H, J = 15.6, 1.8 Hz), 0.92 (s, 9H); ¹³C NMR (75 MHz, MeOD), δ 176.28, 166.52, 157.03, 153.19, 139.65, 130.67, 129.58, 129.48, 128.46, 128.40, 124.54, 124.44, 122.58, 122.33, 121.41, 121.17, 117.21, 116.93, 113.37, 113.03, 112.41, 69.69, 66.05, 64.36, 62.93, 61.91, 55.48, 42.25, 36.99, 32.59, 29.84, 28.40.

### EXAMPLE 18

### (MI-219)

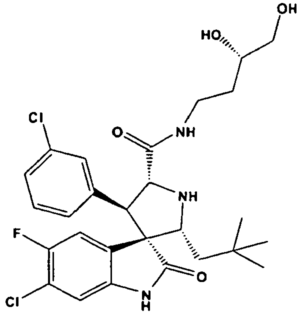

### EXAMPLE 19

### 6-CHLORO-4'-(3-CHLORO-4-FLUOROPHENYL)-2'-CYCLOPENTYLMETHYL-5-FLUORO-2-OXO-1,2-DIHYDRO-SPIRO[INDOLE-3,3'-PYRROLIDINE]-5'-CARBOXYLIC ACID (3,4-DIHYDROXY-BUTYL)-AMIDE

### (MDM2-319-1)

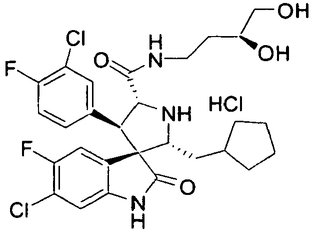

¹H NMR (300 MHz, CD₃OD), δ 7.72 (d, 1H, J = 8.5 Hz), 7.46-7.40 (m, 1H), 7.23-7.12 (m, 2H), 6.88 (d, 1H, J = 6.0 Hz), 5.12 (d, 1H, J = 11.3 Hz), 4.39-4.35 (m, 1H), 4.14 (d, 1H, J = 11.3 Hz), 3.41-3.28 (m, 5H), 1.92-1.78 (m, 2H), 1.62-1.42 (m, 8H), 1.28 (d, 1H, J = 15.8 Hz), 1.10-0.90 (m, 2H).

### EXAMPLE 20

### (MDM2-319-2)

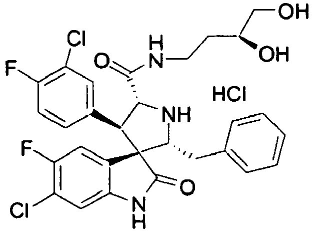

### EXAMPLE 21

### (MDM2-319-3)

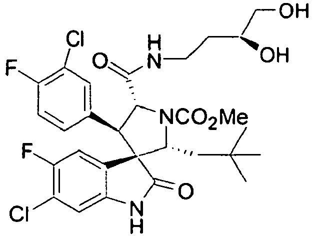

### EXAMPLE 22

### 6-CHLORO-4'-(3-CHLORO-4-FLUOROPHENYL)-2'-CYCLOHEXYL-5-FLUORO-2-OXO-1,2-DIHYDRO-SPIRO[INDOLE-3,3'-PYRROLIDINE]-5'-CARBOXYLIC ACID (3,4-DIHYDROXY-BUTYL)-AMIDE

### (MDM2-319-4)

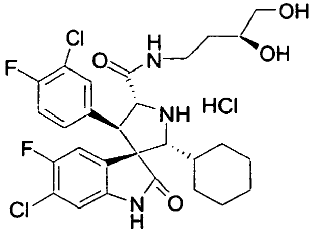

¹H NMR (300 MHz, CD₃OD), δ 8.54-8.40 (m, 2H), 7.56 (d, 1H, J = 8.4 Hz), 7.31-7.29 (m, 1H), 7.16-7.11 (m, 2H), 6.82 (d, 1H, J = 6.1 Hz), 4.99-4.95 (in, 1H), 4.21-4.01 (m, 2H), 3.39-3.19 (m, 5H), 2.50-2.40 (m, 1H), 2.00-1.83 (m, 2H), 1.63-1.54 (m, 4H), 1.44-1.09 (m, 6H).

### EXAMPLE 23

### (MDM2-319-5)

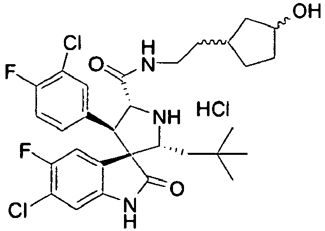

### EXAMPLE 24

### 6-CHLORO-4'-(3-CHLORO-4-FLUOROPHENYL)-2'-(2,2-DIMETHYL-PROPYL)-5-FLUORO-2-OXO-1,2-DIHYDRO-SPIRO[INDOLE-3,3'-PYRROLIDINE]-5'-CARBOXYLIC ACID [2-(3-HYDROXY-CYCLOPENTYL)-ETHYL)-AMIDE

### (MDM2-319-6)

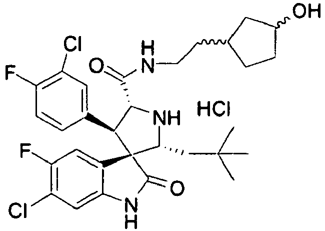

¹H NMR (300 MHz, CD₃OD), δ 8.52-8.49 (m, 1H), 7.72 (d, 1H, J = 8.5 Hz), 7.40 (d, 1H, J = 10.0 Hz), 7.16-7.15 (m, 2H), 6.90 (d, 1H, J = 6.0 Hz), 5.27-5.23 (m, 1H), 4.49 (d, 1H, J = 8.2 Hz), 4.14-4.07 (m, 2H), 3.24-3.22 (m, 1H), 3.10-3.02 (m, 1H), 1.93-1.83 (m, 2H), 1.75-1.18 (m, 7H), 1.05-0.93 (m, 1H), 0.92 (s, 9H), 0.85-0.81 (m, 1H).

### EXAMPLE 25

### (MI-319-7)

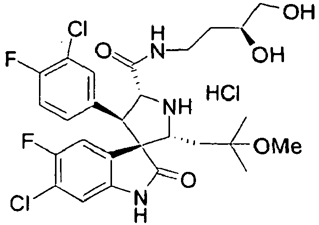

### EXAMPLE 26

### 6-CHLORO-4'-(3-CHLORO-4-FLUOROPHENYL)-2'-CYCLOPENTYL-5-FLUORO-2-OXO-1,2-DIHYDRO-SPIRO[INDOLE-3,3'-PYRROLIDINE]-5'-CARBOXYLIC ACID (3,4-DIHYDROXY-BUTYL)-AMIDE

### (MI-319-8)

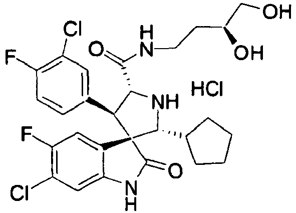

¹H NMR (300 MHz, CD₃OD), δ 7.68 (d, 1H, J = 8.5 Hz), 7.40-7.37 (m, 1H), 7.16-7.11 (m, 2H), 6.86 (d, 1H, J = 6.0 Hz), 5.15 (d, 1H, J = 11.6 Hz), 4.27 (d, 1H, J = 11.0 Hz), 4.15 (d, 1H, J = 11.6 Hz), 3.39-3.24 (m, 5H), 2.30-2.28 (m, 1H), 2.10-2.05 (m, 1H), 1.62-1.31 (m, 7H), 1.15-1.12 (m, 1H), 0.78-0.75 (m, 1H).

### EXAMPLE 27

### 6-CHLORO-4'-(3-CHLORO-4-FLUOROPHENYL)-2'-(2,2-DIMETHYL-PROPYL)-5-FLUORO-5'-(4-METHYL-PIPERAZINE-1-CARBONYL)-1H-SPIRO[INDOLE-3,3'-PYRROLIDIN]-2-ONE

### (MI-319-9)

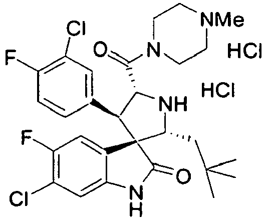

¹H NMR (300 MHz, CD₃OD), δ 7.73-7.70 (m, 1H), 7.58-7.51 (m, 1H), 7.29-7.19 (m, 2H), 6.93-6.89 (m, 1H), 5.83-5.80 (m, 1H), 4.70-4.68 (m, 1H), 4.44-4.25 (m, 2H), 3.98-3.52 (m, 3H), 3.36-3.22 (m, 2H), 2.92-2.85 (m, 3H), 2.40-1.89 (m, 2H), 1.20 (d, 1H, J = 15.3 Hz), 0.95-0.90 (m, 9H).

### EXAMPLE 28

### (MI-319-10)

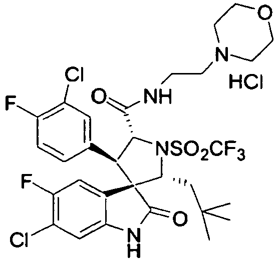

### EXAMPLE 29

### (MI-319-11)

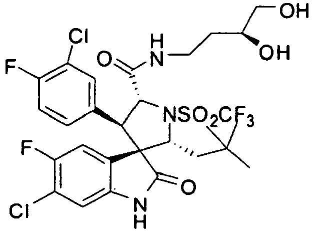

### EXAMPLE 30

### (MI-319-11-A)

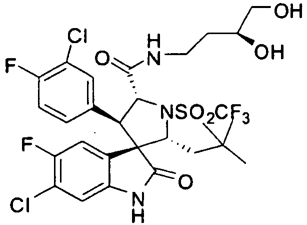

### EXAMPLE 31

### (MI-319-12)

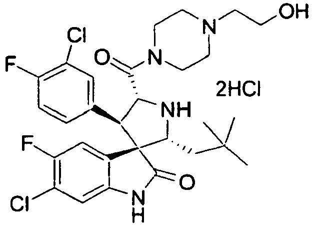

### EXAMPLE 32

### 1'-ACETYL-6-CHLORO-4'-(3-CHLORO-4-FLUOROPHENYL)-2'-(2,2-DIMETHYLPROPYL)-5-FLUORO-2-OXO-1,2-DIHYDRO-SPIRO[INDOLE-3,3'-PYRROLIDINE]-5'-CARBOXYLIC ACID (3,4-DIHYDROXY-BUTYL)-AMIDE

### (MI-319-13)

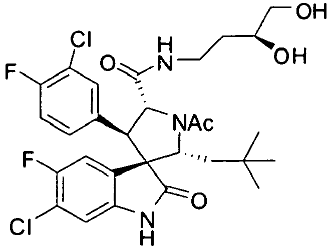

¹H NMR (300 MHz, CD₃Cl), δ 8.08 (s, 1H), 7.19-7.12 (m, 1H), 6.93-6.77 (m, 4H), 4.93 (d, 1H, J = 10.2 Hz), 4.75 (d, 1H, 10.2 Hz), 4.00-3.98 (m, 1H), 3.78-3.30 (m, 5H), 2.80-2.77 (m, 1H), 2.23 (s, 3H), 1.76-1.64 (m, 1H), 1.56-1.52 (m, 2H), 0.92 (s, 9H).

### EXAMPLE 33

### 6-CHLORO-4'-(3-CHLORO-4-FLUOROPHENYL)-2'-(2,2-DIMETHYL-PROPYL)-5-FLUORO-2-OXO-1'-OXY-1,2,4',5'-TETRAHYDRO-SPIRO[INDOLE-3,3'-PYRROLE]-5'-CARBOXYLIC ACID (3,4-DIHYDROXY-BUTYL)-AMIDE

### (MI-319-14)

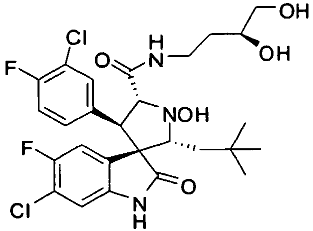

¹H NMR (300 MHz, CD₃Cl), δ 7.35-7.27 (m, 1H), 7.09-7.07 (m, 1H), 6.92-6.89 (m, . 1H), 6.79-6.75 (m, 2H), 5.55 (d, 1H, J = 11.0 Hz), 4.20 (d, 1H, J = 11.0 Hz), 3.67-3.65 (m, 2H), 3.44-3.32 (m, 3H), 2.80 (d, 1H, J = 13.1 Hz), 1.80 (d, 1H, J = 13.1 Hz), 1.70-1.60 (m, 2H), 0.84 (s, 9H).

### EXAMPLE 34

### {[6-CHLORO-4'-(3-CHLORO-4-FLUOROPHENYL)-2'-(2,2-DIMETHYL-PROPYL)-5-FLUORO-2-OXO-1,2-DIHYDRO-SPIRO[INDOLE-3,3'-PYRROLIDINE]-5'-CARBONYL]-AMINO}-ACETIC ACID

### (MI-319-15)

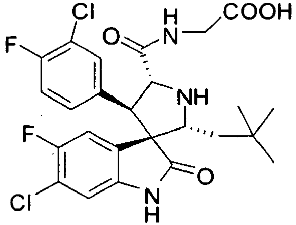

¹H NMR (300 MHz, CD₃OD), δ 7.64 (d, 1H, J = 8.4 Hz), 7.38 (d, 1H, J = 6.2 Hz), 7.27-7.21 (m, 1H), 7.10-7.07 (m, 1H), 6.90-6.84 (m, 1H), 5.08 (d, 1H, J = 9.9 Hz), 4.22-3.88 (m, 4H), 1.76-1.68 (m, 1H), 1.12-1.06 (m, 1H), 0.84 (s, 9H).

### EXAMPLE 35

### (MI-319-16)

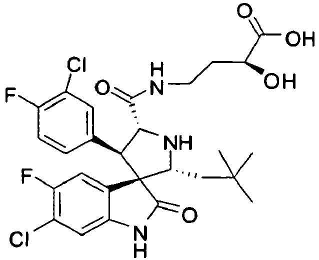

### EXAMPLE 36

### 6-CHLORO-4'-(3-CHLORO-4-FLUOROPHENYL)-2'-(2,2-DIMETHYL-PROPYL)-5-FLUORO-2-OXO-1,2-DIHYDRO-SPIRO[INDOLE-3,3'-PYRROLIDINE]-5'-CARBOXYLIC ACID (2-IMIDAZOL-1-YL-ETHYL)-AMIDE

### (MI-319-17)

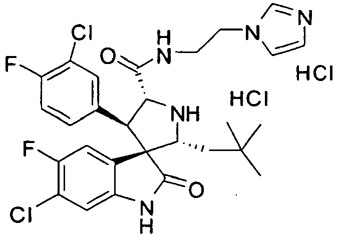

¹H NMR (300 MHz, CD₃OD), δ 8.99 (s, 1H), 7.78 (d, 1H, J = 8.4 Hz), 7.50-7.45 (m, 2H), 7.37-7.35 (m, 1H), 7.19-7.11 (m, 2H), 6.89 (d, 1H, J = 6.0 Hz), 5.32 (d, 1H, J = 11.4 Hz), 4.52 (d, 1H, J = 6.8 Hz), 4.41-4.36 (m, 2H), 4.19 (d, 1H, J = 11.4 Hz), 3.73-3.31 (m, 2H), 1.99-1.91 (m, 1H), 1.21-1.16 (m, 1H), 0.92 (s, 9H).

### EXAMPLE 37

### 7-{[6-CHLORO-4'-(3-CHLORO-4-FLUOROPHENYL)-2'-(2,2-DIMETHYL-PROPYL)-5-FLUORO-2-OXO-1,2-DIHYDRO-SPIRO[INDOLE-3,3'-PYRROLIDINE]-5'-CARBONYL]-AMINO}-3,5-DIHYDROXY-HEPTANOIC ACID

### (MI-319-18)

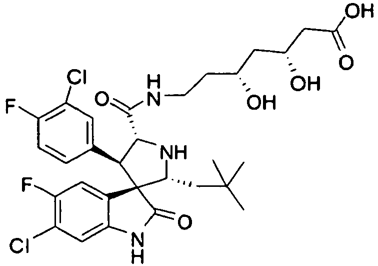

¹H NMR (300 MHz, CD₃OD), δ 7.60 (d, 1H, J = 8.4 Hz), 7.37 (d, 1H, J = 7.5 Hz), 7.08-7.06 (m, 2H), 6.87-6.84 (m, 1H), 4.84 (d, 1H, J = 10.2 Hz), 4.15-4.08 (m, 1H), 4.00-3.96 (m, 2H), 3.80-3.75 (m, 1H), 3.36-3.32 (m, 2H), 2.49-2.43 (m, 2H), 1.65-1.55 (m, 6H), 1.04 (m, 1H), 0.92 (s, 9H).

### EXAMPLE 38

### 6-CHLORO-4'-(3-CHLORO-4-FLUOROPHENYL)-2'-(2,2-DIMETHYL-PROPYL)-5-FLUORO-2-OXO-1,2-DIHYDRO-SPIRO[INDOLE-3,3'-PYRROLIDINE]-5'-CARBOXYLIC ACID (2,3,4-TRIHYDROXY-BUTYL)-AMIDE

### (MI-319-19)

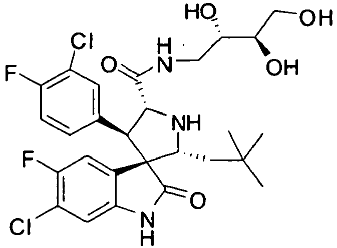

¹H NMR (300 MHz, CD₃OD), δ 7.83-7.66 (m, 1H), 7.30-7.10 (m, 3H), 6.92-6.88 (m, 1H), 5.30-5.11 (m, 1H), 4.48-4.21 (m, 1H), 4.15-3.95 (m, 1H), 3.64-3.60 (m, 2H), 3.51-3.47 (m, 2H), 3.32-3.24 (m, 2H), 1.95-1.88 (m, 1H), 1.23-1.13 (m, 1H), 0.92-0.82 (m, 9H).

### EXAMPLE 39

### 6-CHLORO-4'-(3-CHLORO-4-FLUOROPHENYL)-2'-(2,2-DIMETHYL-PROPYL)-5-FLUORO-2-OXO-1,2-DIHYDRO-SPIRO[INDOLE-3,3'-PYRROLIDINE]-5'-CARBOXYLIC ACID (2-GUANIDINO-ETHYL)-AMIDE

### (MI-319-20)

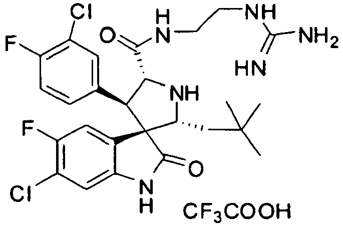

¹H NMR (300 MHz, CD₃OD), δ 7.69 (d, 1H, J = 8.4 Hz), 7.39 (d, 1H, J = 6.7 Hz), 7.14-7.04 (m, 2H), 6.89 (d, 1H, J = 5.8 Hz), 5.24 (d, 1H, J = 11.3 Hz), 4.44 (d, 1H, J = 8.0 Hz), 3.80-3.65 (m, 2H), 3.08-3.00 (m, 2H), 1.89-1.87 (m, 1H), 1.71-1.64 (m, 2H), 1.40-1.33 (m, 1H), 0.91 (s, 9H).

### EXAMPLE 40

### (MI-319-21)

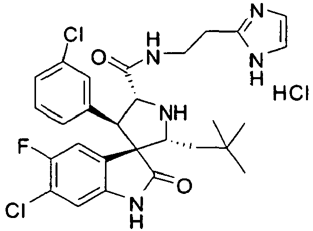

### EXAMPLE 41

### 6-CHLORO-4'-(3-CHLORO-4-FLUOROPHENYL)-5-FLUORO-2'-(2-METHYL-PROPENYL)-2-OXO-1,2-DIHYDRO-SPIRO[INDOLE-3,3'-PYRROLIDINE]-5'-CARBOXYLIC ACID (3,4-DIHYDROXY-BUTYL)-AMIDE

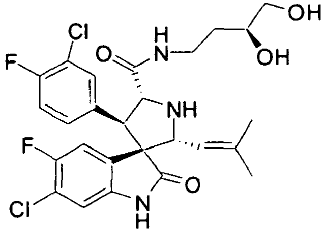

¹H NMR (300 MHz, CD₃Cl), δ 8.19-8.11 (m, 1H), 7.28-7.19 (m, 3H), 6.96-6.85 (m, 2H), 6.68 (d, 1H, J = 5.9 Hz), 4.97 (d, 1H, J = 8.9 Hz), 4.60-4.55 (m, 1H), 4.30-4.27 (m, 1H), 3.82 (d, 1H, J = 9.2 Hz), 3.71-3.23 (m, 4H), 1.58-1.54 (m, 5H), 1.46 (s, 3H).

### EXAMPLE 42

### 6-CHLORO-4'-(3-CHLORO-4-FLUOROPHENYL)-2'-(2,2-DIMETHYL-PROPYL)-5-FLUORO-2-OXO-1,2-DIHYDRO-SPIRO[INDOLE-3,3'-PYRROLIDINE]-5'-CARBOXYLIC ACID (2-UREIDO-ETHYL)-AMIDE

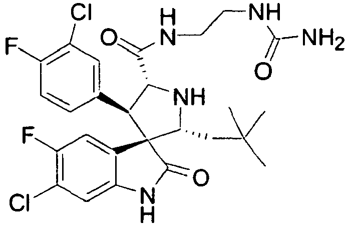

¹H NMR (300 MHz, CD₃OD), δ 7.71 (d, 1H, J = 8.4 Hz), 7.43 ( d, 1H , J = 6.0 Hz), 7.15-7.12 (m, 2H), 6.93-6.89 (m, 1H), 5.19 (d, 1H, J = 11.2 Hz), 4.48 (d, 1H, J = 11.2 Hz), 4.16-4.13 (m, 1H), 3.32-3.23 (m, 2H), 2.97-2.89 (m, 2H), 1.95-1.90 (m, 1H), 1.57-1.53 (m, 2H), 1.23-1.18 (m, 1H), 0.92 (s, 9H).

### EXAMPLE 43

### (2'R,3S,4'R,5'R)-6-CHLORO-N-((S)-3,4-DIHYDROXYBUTYL)-5-FLUORO-4'-(3-FLUOROPHENYL)-2'-NEOPENTYL-2-OXOSPIRO[INDOLINE-3,3'-PYRROLIDINE]-5'-CARBOXAMIDE

### (MI-519-1)

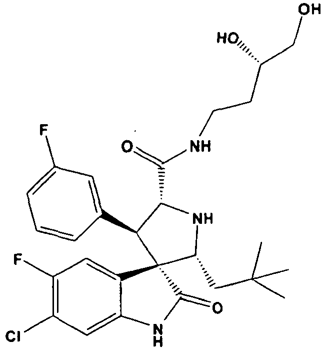

¹H NMR (300 MHz, CD₃OD), δ 8.43 (m, 1H), 7.72 (d, 1H, J = 8.4 Hz), 7.30-7.15 (m, 1H), 7.06 (d, 2H, J = 8.88 Hz), 6.95 (d, 1H, J = 7.8 Hz), 6.88 (d, 1H, J = 6.0 Hz), 5.28 (d, 1H, J = 11.3 Hz), 4.51-4.40 (m, 1H), 4.16 (d, J = 11.3 Hz), 3.50-3.20 (m, 5H), 1.93 (dd, 1H, J = 8.3, 15.5 Hz), 1.62-1.36 (m, 2H), 1.23-1.13 (m, 1H), 0.92 (s, 9H).

### EXAMPLE 44

### (2'R,3S,4'S,5'R)-6-CHLORO-4'-(2,3-DIFLUOROPHENYL)-N-((S)-3,4-DIHYDROXYBUTYL)-5-FLUORO-2'-NEOPENTYL-2-OXOSPIRO[INDOLINE-3,3'-PYRROLIDINE]-5'-CARBOXAMIDE

### (MI-519-2)

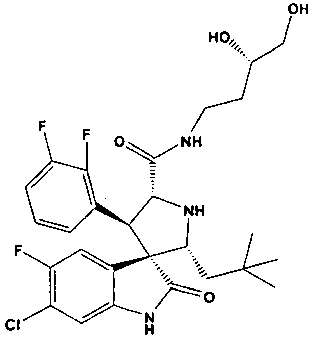

¹H NMR (300 MHz, CD₃OD), δ 8.47 (m, 1H), 7.73 (d, 1H, J = 7.2 Hz), 7.43-7.30 (m, 1H), 7.23-7.15 (m, 2H), 6.89 (d, 1H, J = 6.0 Hz), 5.29 (d, 1H, J = 11.1 Hz), 4.64 (d, 1H, J = 11.4 Hz), 4.58-4.50 (m, 1H), 3.50-3.20 (m, 5H), 1.93 (dd, 1H, J = 8.3, 15.5 Hz), 1.67-1.36 (m, 2H), 1.23-1.13 (m, 1H), 0.92 (s, 9H).

### EXAMPLE 45

### (2'R,3S,4'R,5'R)-6-CHLORO-4'-(3,4-DIFLUOROPHENYL)-N-((S)-3,4-DIHYDROXYBUTYL)-5-FLUORO-2'-NEOPENTYL-2-OXOSPIRO[INDOLINE-3,3'-PYRROLIDINE]-5'-CARBOXAMIDE

### (MI-519-3)

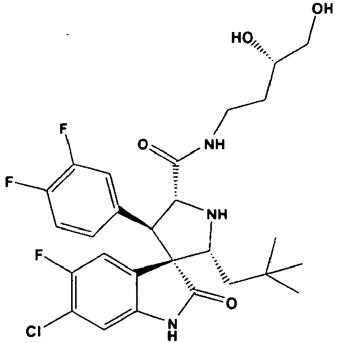

¹H NMR (300 MHz, CD₃OD), δ 8.43 (m, 1H), 7.73-7.69 (m, 1H), 7.27-1.10 (m, 2H), 6.97-6.94 (m, 1H), 6.90 (d, 1H, J = 6.0 Hz), 5.26-5.23 (m, 1H), 4.49 (d, 1H, J = 7.5 Hz), 4.16-4.13 (m, 1H), 3.50-3.15 (m, 5H), 1.96-1.88 (m, 1H), 1.62-1.40 (m, 2H), 1.23-1.13 (m, 1H), 0.92 (s, 9H).

### EXAMPLE 46

### (2'R,3S,4'S,5'R)-6-CHLORO-4'-(2,5-DIFLUOROPHENYL)-N-((S)-3,4-DIHYDROXYBUTYL)-5-FLUORO-2'-NEOPENTYL-2-OXOSPIRO[INDOLINE-3,3'-PYRROLIDINE]-5'-CARBOXAMIDE

### (MI-519-5)

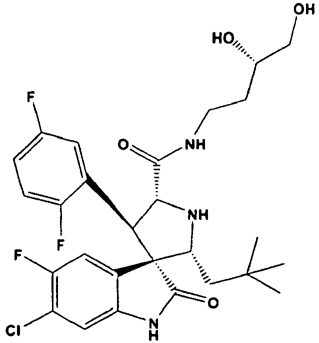

¹H NMR (300 MHz, CD₃OD), δ 8.49 (m, 1H), 7.71 (d, 1H, J = 7.2 Hz), 7.45-7.40 (m, 1H), 7.15-7.00 (m, 2H), 6.89 (d, 1H, J = 6.0 Hz), 5.23 (d, 1H, J = 11.1 Hz), 4.63 (d, 1H, J = 11.4 Hz), 4.58-4.50 (m, 1H), 3.50-3.20 (m, 5H), 1.93 (dd, 1H, J = 8.3, 15.5 Hz), 1.67-1.36 (m, 2H), 1.18 (dd, 1H, J = 1.9, 15.5 Hz), 0.92 (s, 9H).

### EXAMPLE 47

### (2'R,3S,4'S,5'R)-6-CHLORO-N-((S)-3,4-DIHYDROXYBUTYL)-5-FLUORO-4'-(2-FLUOROPHENYL)-2'-NEOPENTYL-2-OXOSPIRO[INDOLINE-3,3'-PYRROLIDINE]-5'-CARBOXAMIDE

### (MI-519-6)

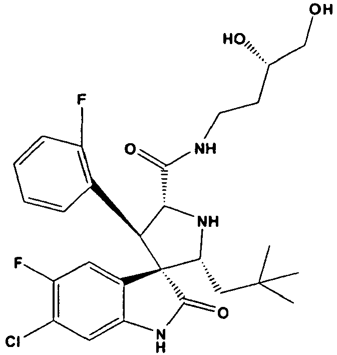

¹H NMR (300 MHz, CD₃OD), δ 8.43 (m, 1H), 7.70-7.64 (m, 2H), 7.40-7.18 (m, 2H), 7.01-6.94 (m, 1H), 6.85 (d, 1H, J = 6.0 Hz), 5.25 (d, 1H, J = 11.3 Hz), 4.62 (d, 1H, J = 12 Hz), 4.55-4.52 (m, 1H), 3.50-3.20 (m, 5H), 1.91 (dd, 1H, J = 8.3, 15.5 Hz), 1.62-1.32 (m, 2H), 1.18(dd, 1H, J = 1.8, 15.3 Hz), 0.92 (s, 9H).

### EXAMPLE 48

### (2'R,3S,4'R,5'R)-6-CHLORO-N-((S)-3,4-DIHYDROX-YBUTYL)-5-FLUORO-4'-(4-FLUOROPHENYL)-2'-NEOPENTYL-2-OXOSPIRO[INDOLINE-3,3'-PYRROLIDINE]-5'-CARBOXAMIDE

### (MI-519-7)

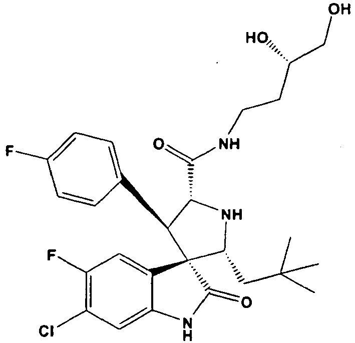

¹H NMR (300 MHz, CD₃OD), δ 8.39 (m, 1H), 7.70 (d, 1H, J = 8.5 Hz), 7.25-7.21 (m, 2H), 7.03-6.96 (m, 2H), 6.87 (d, 1H, J = 6.0 Hz), 5.26 (d, 1H, J = 11.3 Hz), 4.48 (m, 1H,), 4.12 (d, J = 11.4 Hz), 3.50-3.20 (m, 5H), 1.92 (dd, 1H, J = 8.3, 15.5 Hz), 1.62-1.32 (m, 2H), 1.20 (dd, 1H, J = 2.0, 15.3 Hz), 0.92 (s, 9H).

### EXAMPLE 49

### CELL GROWTH INHIBITION

One major advantage of non-peptide small-molecule inhibitors over peptide-based inhibitors is their superior cell permeability. It is predicted that potent, non-peptide inhibitors of the p53-MDM2 interaction will be effective in inhibition of cell growth and division in cancer cells with a wild-type form of p53 through stimulation of the activity of p53. Furthermore, they are predicted to have selectivity in cancer cells with either a loss of p53 or a mutated, non-functional form of p53. To test these predictions, a cell growth assay was developed using human prostate cancer LNCaP (p53 wild-type) and PC-3 (p53 null) cell lines. The toxic effects of compounds of the invention were tested.

Cells were seeded in 96-well flat bottom cell culture plates at a density of 3-4x 10³ cells/well and incubated in the presence of compounds for 4 days. The rate of cell growth inhibition after treatment with increasing concentrations of the compounds was determined using WST-8 (2-(2-methoxy-4-nitrophenyl)-3-(4-nitrophenyl)-5-(2,4disulfophenyl)-2H-tetrazolium monosodium salt (Dojindo Molecular Technologies Inc., Gaithersburg, Maryland). WST-8 was added at a final concentration of 10% to each well and the plates were incubated at 37°C for 2-3 hrs. The absorbance of the samples was measured at 450 nm in a plate reader (Molecular Device-TECAN ULTRA). The concentration of the compounds that inhibited cell growth by 50% (IC₅₀) was calculated by comparing absorbance in the untreated cells and the cells treated with the compounds. As shown in Table 2, the compounds of the invention were about 5-fold to about 50-fold more effective at inhibiting cell growth in p53-positive LNCaP cells as compared to p53-negative PC3 cells.

The compounds of the invention were similarly tested against HCT116 (wild-type p53), and HT-29 (mutant p53) colon cancer cell lines. As shown in Table 2, the majority of the tested compounds exhibited a higher potency for cell growth inhibition in p53-positive HCT116 cells as compared to p53-negative HCT116 cells, with the largest difference in potency being about 25-fold.

Since normal cells also have wild-type p53, a potential concern in development of inhibitors of the p53-MDM2 interaction as new anti-cancer drugs is that they may be nonselective and equally active in killing normal cells as they are in killing cancer cells. Compounds will be evaluated in normal human prostate epithelial cells (PrEC) with wild-type p53 to confirm whether they display a good selectivity for cancer cells.

**Table 2**

| Compound | IC₅₀ ± SD (µM)* (Ki ± SD) (µM) | LNCaP IC₅₀ (µM) | PC3 IC₅₀ (µM) | HCT116 p53WT IC₅₀ (µM) | HCT116 p53KO IC₅₀ (µM) |
|---|---|---|---|---|---|
| MDM2-319 | 0.076 | 0.2738 | 15.4 | 0.7174 | 11.83 |
| | | | | | |
| | (0.0063) | 0.1839 | 9.08 | 0.6145 | 12.2 |
| | 0.077 | 0.2291 | 1.35 | 0.2466 | 14.23 |
| | (0.006) | | | | |
| | 0.064 | 0.2289 ±0.04 | 11.61 ± 3.3 | 0.52 ± 0.24 | 12.75 ± 1.29 |
| | (0.005) | | | | |
| | 0.092 | | | | |
| | 0.115 | | | | |
| | 0.122 | | | | |
| | 0.13 | | | | |
| | 0.18 | | | | |
| | 0.17 | | | | |
| | 0.16 | | | | |
| | 0.10 | | | | |
| MDM2-319-1 | 0.194 | 0.2612 | HCT116 | 0.9085 | 12.66 |
| | 0.072 | 0.3602 | | 1.364 | 10.39 |
| MD2-319-2 | 175 | 3.474 | 14.39 | 15.46 | 22.24 |
| | (16.43) | 3.378 | 19.64 | 21.3 | 19.49 |
| | > 80 | 4.005 | 19.58 | 8.508 | 25.44 |
| | | | | | |
| | | 3.61 ± 0.3 | 17.87 ± 3.01 | 15.08 ± 6.4 | 22.39 ± 2.97 |
| MDM2-319-3 | 0.37 | 1.758 | 16.65 | 3.813 | 13.84 |
| | (0.034) | 1.241 | 17.45 | 4.109 | 24.78 |
| | 0.45 | 0.8472 | 22.16 | 2.826 | 16.72 |
| | 0.23 | | | | |
| | | 1.28 ± 0.4 | 18.75 ± 2.97 | 3.58 ± 0.67 | 18.44 ± 5.67 |
| MDM2-319-4 | 0.71 | 1.417 | 18.53 | 5.03 | 13.13 |
| | (0.066) | 1.294 | 23.38 | 5.801 | 26.48 |
| | 1.16 | 0.8902 | 25.6 | 2.413 | 13.39 |
| | 0.46 | | | | |
| | | 1.21 ± 0.29 | 22.5 ± 3.61 | 4.41 ± 1.77 | 17.6 ± 7.6 |
| MDM2-319-5 | 0.055 | 0.6020 | 9.47 | 0.9927 | 10.52 |
| | 0.040 | 0.4653 | 5.553 | 1.286 | 5.39 |
| | | 0.6341 | 8.711 | 1.502 | 8.403 |
| | | | | | |
| | | 0.56 ± 0.08 | 7.91 ± 2 | 1.23 ± 0.2 | 8.1 ± 2.5 |
| MDM2-319-6 | 0.59 | 2.583 | 21.4 | 5.452 | 14.29 |
| | 0.55 | 1.223 | 20.83 | 7.41 | 16.06 |
| | Flu | 1.931 | 14.15 | 5.957 | 14.58 |
| | | | | | |
| | | 1.91 ± 0.6 | 18.7 ± 4 | 6.26 ± 1.0 | 14.97 ± 0.9 |
| MI-319-7 | 39.33 | 2.17 | 39.74 | 8.22 | >30 |
| | | 1.917 | 34.29 | 7.954 | >30 |
| MI-319-8 | 0.34 | 0.9631 | 30.66 | 4.114 | 30.95 |
| | 0.53 | 0.8928 | 20.72 | 4.732 | 25.48 |
| | | 0.6625 | 18.43 | 2.738 | 22.69 |
| | | | | | |
| | | 1.46 ± 1.13 | 23.27 ± 6.5 | 3.86 ± 1.02 | 26.3 ± 4.2 |
| MI-319-9 | 3.58 | 2.866 | 20.91 | 7.48 | 9.711 |
| | | 2.7661.7 | 5.547 | 9.198 | 8.189 |
| | | 5 | 6.09 | 4.631 | 4.52 |
| | | | | | |
| | | 2.46 ± 0.61 | 11.12 ± 8.5 | 7.1 ± 2.3 | 7.47 ± 2.66 |
| MI-319-10 | 0.22 | 0.7764 | 10.47 | 1.411 | 11.58 |
| | 0.26 | 0.8283 | 10.36 | 1.947 | 9.84 |
| | | 0.4493 | 8.074 | 1.088 | 5.454 |
| | | | | | |
| | | 0.68 ± 0.2 | 9.63 ± 1.35 | 1.48 ± 0.4 | 8.9 ± 3.1 |
| MI-319-11 | 5.13 | 2.846 | 17.29 | 13.5 | 13.38 |
| | 5.39 | 3.078 | 14.71 | 15.82 | 18 |
| | 5.11 | 4.219 | 24.24 | 24.89 | 24.68 |
| | | | | | |
| | | 3.38 ± 0.73 | 18.7 ± 4.9 | 18.4 ± 5.9 | 18.85 ± 5.45 |
| MI-319-11-A | 2.24 | 0.3078 | 1.765 | 2.153 | 3.966 |
| | 1.64 | 0.5313 | 2.754 | 2.816 | 2.627 |
| | | 0.4624 | 4.024 | 0.9753 | 4.182 |
| | | | | | |
| | | 0.43 ± 0.11 | 2.84 ± 1.1 | 1.98 ± 0.9 | 3.59 ± 0.84 |
| MI-319-12 | 2.30 | 1.974 | 22.35 | 12.37 | 13.45 |
| | 2.96 | 2.571 | 20.89 | 13.2 | 17.84 |
| | | 1.931 | 13.31 | 4.365 | 14.13 |
| | | | | | |
| | | 2.15 ± 0.35 | 18.85 ± 4.85 | 9.97 ± 4.87 | 15.14 ± 2.36 |
| MI-319-13 | 5.87 | 3.663 | 17.2 | 3.227 | 32.64 |
| | 5.04 | 2.316 | 14.48 | 6.368 | 35.05 |
| | | 4.85 | 20.28 | 10.98 | 33.02 |
| | | | | | |
| | | 3.6 ± 1.26 | 17.32 ± 2.9 | 6.85 ± 3.8 | 33.57 ± 1.29 |
| MI-319-14 | 2.04 | 4.221 | 40.08 | 8.008 | >30 |
| | 1.66 | 2.63 | 40.2 | 9.266 | >30 |
| | | 1.979 | >30 | 5.738 | >30 |
| | | | | | |
| | | 2.94 ± 1.1 | | 7.67 ± 1.7 | |
| MI-319-15 | 0.35 | 4.522 | >30 | 12.13 | >30 |
| | 0.18 | 4.125 | >30 | 12.89 | >30 |
| | | 6.113 | >30 | 13.89 | >30 |
| | | | | | |
| | | 4.92 ± 1.05 | | 12.97 ± 0.88 | |
| MI-319-16 | 0.85 (Flu) | 24.07 | >30 | >30 | >30 |
| | 0.76 (Flu) | | | | |
| MI-319-17 | 0.39 | 1.098 | 14 | 2.096 | 25.13 |
| | 0.49 | 1.548 | 22.02 | 3.792 | 22.33 |
| | | 1.511 | 23.63 | 3.148 | 25.14 |
| MI-319-18 | 3.51 | >30 | >30 | >30 | >30 |
| | 3.57 | >30 | >30 | >30 | >30 |
| MI-319-19 | 0.50 | 1.235 | 30.68 | 2.68 | >30 |
| | 0.49 | 2.398 | >30 | 5.981 | >30 |
| | | 1.2 | 41.65 | 4.596 | 77.64 |
| MI-319-20 | | 27.44 | >30 | >30 | >30 |
| | | 31.59 | 66.12 | 93.54 | >100 |
| MI-319-21 | | 2.838 | 21.87 | 4.402 | 11.76 |
| | | 1.836 | 14.07 | 3.884 | 13.25 |

| | | | | | |
|---|---|---|---|---|---|
| Calculation of Kᵢ: MDM2 [10 nM]; F-6 [1 nM]; New Sample MDM2 protein: K_{d}: 0.88 ± 0.41 nM (Hyperbolic equation; using 1 nM 6F) K_{d}: 2.22 ± 0.93 nM (Klotz plot; using 1 nM 6F) | | | | | |

### EXAMPLE 50

### EFFECTS OF MDM2 INHIBITORS ON EXPRESSION OF P53 AND ITS TARGET GENE PRODUCTS MDM2 AND P21

Cancer cells are treated with test compounds or 0.1% DMSO for 24 hours. Cells are harvested by trypsinization and washed with cold phosphate buffer saline, pH 7.5 (Invitrogen, Carlsbad, CA). Cells are lysed for 30 minutes in ice-cold lysis buffer (50 mM Tris (pH 7.5), 150 mM NaCl, 1 mM EDTA (pH 8.0), 25 mM sodium fluoride, 1% NP-40 and 0.1% SDS) containing 2 mM sodium orthovanadate, 1 mM phenylmethylsulfonyl fluoride and protease inhibitor cocktail (Roche Applied Science, Indianapolis, IN). Next, cell extracts are centrifuged at 12,000×g at 4°C for 10 minutes to obtain clarified lysates. Protein is estimated by Bio-Rad dye. Cell lysates containing 35 µg protein are resolved on a 4-20% tris-glycine gel (Invitrogen, Carlsbad, CA) and transferred onto poly(vinylidene difluoride) membranes. Immunodetection of proteins on the transfer membrane is performed by using anti-p53 (Ab-6, Oncogene Research Products, Boston, MA), anti-MDM2 (SMP14, Santa Cruz Biotechnology, Santa Cruz, CA) and anti-p21 (BD Biosciences, San Diego, CA) mouse monoclonal antibodies. Antibody to β-actin (Sigma, St Louis, MO) is used to assess the protein loading. When RKO (wild-type p53) and HT-29 (mutant p53) colon cancer cell lines are treated for 24 hours with the compounds of the invention, it is expected that the compounds will induce accumulation of p53 and its target gene-products only in RKO cells expressing wild-type p53.

### EXAMPLE 51

### CELL DEATH AND APOPTOSIS INDUCED BY MDM2 INHIBITORS

RKO (wild-type p53) and HT-29 (mutant p53) colon cancer cell lines and CCD-18Co normal colon fibroblast cells are treated with increasing doses of the compounds of the invention for 4 days in 6-well Petri dishes. Trypan blue dye exclusion assays are performed to determine the ability of the inhibitors to induce cell death. After 4 days of treatment, floating and adherent cells are harvested and stained with 0.2% of trypan blue solution (Sigma, St Louis, MO). Each treatment is performed in triplicate and at least 100 cells are counted. Cells stained blue or morphologically unhealthy cells are scored as dead cells. To evaluate apoptosis, sub-diploid DNA content in cells treated with or without test inhibitors is analyzed by propidium iodide (PI) staining. After washing once with cold PBS, cells are fixed in 70% ethanol for 1 day at -20°C. Ethanol-fixed cells are then washed twice with PBS and stained with a staining solution containing propidium iodide (PI) at 50 µg/ml and RNAse A at 100 µg/ml in PBS, for 20 minutes in dark at room temperature. Acquisition of cells and analysis of sub-diploid DNA content is performed by flow cytometry using CellQuest software. It is expected that only cancer cells expressing wild-type p53 will undergo apoptosis in response to administration of the compounds.

### EXAMPLE 52

### EFFECT OF MDM2 INHIBITORS ON CELL CYCLE PROGRESSION OF COLON CANCER CELLS

Cell cycle progression is evaluated in RKO (wild-type p53) and HT-29 (mutant p53) colon cancer cell lines and CCD-18Co normal colon fibroblast cells by determining S-phase cells by incorporation of bromodeoxyuridine (BrdU) followed by staining with FITC-labeled anti-BrdU antibody and the total DNA-content by staining with 7-aminoactinomycin D (7-AAD) according to manufacturer's instructions (BD Biosciences, San Jose, CA). Briefly, cancer and normal cells, after overnight incubation, are treated with or without test compounds for 22 hours, followed by an additional 2 hours of incubation with 10 µM of BrdU. Cells are harvested, fixed and stained with FITC-labeled anti-BrdU and 7-AAD. Cell cycle distribution is analyzed by flow cytometry. Cells are acquired and data analyzed by using CellQuest software (BD Biosciences). It is expected that the compounds of the invention will induce a dose-dependent depletion of S-phase in RKO cancer cells and in CCD-18Co normal colon fibroblast cells, both of which express wild-type p53. It is also expected that the compounds will have no appreciable effect on cell cycle progression of HT-29 cells expressing mutant p53.

### EXAMPLE 53

### PROTECTION OF NORMAL CELLS FORM CHEMOTHERAPY WITH MDM2 INHIBITORS

PrEC normal prostate epithelial cells are seeded in 6 well plates and incubated with compounds of the invention for 24 hours, then 1 µM TAXOL (paclitaxel) is added for 2 days. Trypan blue is used to determine cell viability. The data is expected to show that when normal prostate epithelial cells are pretreated with the compounds of the invention, cells are protected from TAXOL.

## Claims

1. A compound having Formula I: 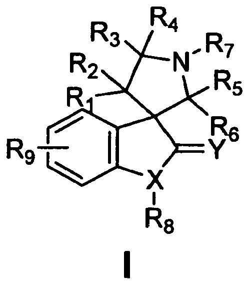 or a pharmaceutically acceptable salt thereof, wherein:
X is CH, O, N, or S, wherein R₈ is absent if X is O or S;
Y is O, S, or NR';
R₁, R₂, R₄, R₅, R₆, and R₇ are independently H or optionally substituted alkyl, cycloalkyl, alkenyl, cycloalkenyl, alkynyl, aryl, heterocyclic, CO₂R', OCOR', CONR'R", NR"COR', NR'SO₂R", SO₂NR'R", (C=NR')NR"R"', or NR'R"; or
R₇ forms an aryl, cycloalkyl, or heterocyclic group with one of R₅ or R₆;
R₈ is H or optionally substituted alkyl, cycloalkyl, alkenyl, cycloalkenyl, alkynyl, aryl, heterocyclic, CO₂R', OCOR', CONR'R", SO₂NR'R", or
(C=NR')NR"R"';
R₉ represents a 6-chloro and a 5-fluoro group; and
each R', R" and R"' is independently H or optionally substituted alkyl, cycloalkyl, alkenyl, cycloalkenyl, alkynyl, aryl, or heterocyclic; or
R' and R", or R" and R"', form a ring;
wherein R₃ is CONRR', and R is an optionally substituted cycloalkyl-alkyl or monocyclo-heterocycloalkyl group or a dihydroxyalkyl group not containing a hydroxyl group at the 3-position of the alkyl group.

2. The compound of claim 1, having Formula II or Formula III: 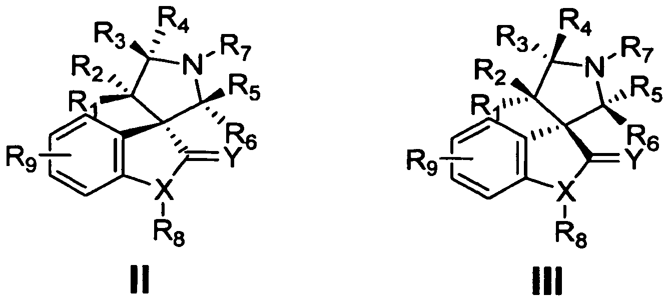 or a pharmaceutically acceptable salt thereof.

3. The compound of claim 1, having Formula VII: 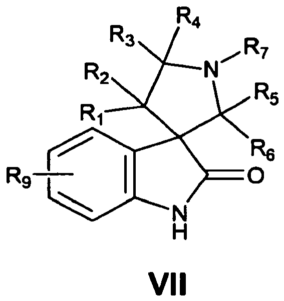 or a pharmaceutically acceptable salt thereof.

4. The compound of claim 3, having Formula VIII or Formula IX: 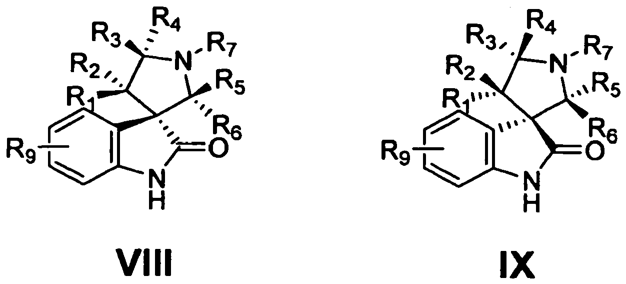 or a pharmaceutically acceptable salt thereof.

5. The compound of claim 1, wherein one of R₁ and R₂ is optionally substituted aryl or heteroaryl.

6. The compound of claim 1, wherein one of R₁ and R₂ is cycloalkyl, straight or branched alkyl, CONR'R" or CO₂R'.

7. The compound of claim 1, wherein one of R₅ and R₆ is C₃₋₁₈ alkyl, aryl, or heteroaryl.

8. A compound having Formula XXVII or Formula XXVIII: 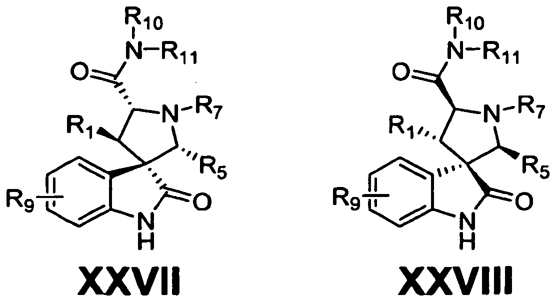 or a pharmaceutically acceptable salt thereof, wherein:
R₁, R₅, and R₇ are independently H or optionally substituted alkyl, cycloalkyl, alkenyl, cycloalkenyl, alkynyl, aryl, heterocyclic, CO₂R', OCOR', CONR'R", NR"COR', NR'SO₂R", SO₂NR'R", (C=NR')NR"R"', or NR'R"; or
R₇ forms an aryl, cycloalkyl, or heterocyclic group with R₅;
R₉ represents a 6-chloro and a 5-fluoro group;
each R', R" and R'" is independently H or optionally substituted alkyl, cycloalkyl, alkenyl, cycloalkenyl, alkynyl, aryl, or heterocyclic; or
R' and R", or R" and R"', form a ring;
R₁₀ is hydrogen, and
R₁₁ is an optionally substituted cycloalkyl-alkyl or monocyclo-heterocycloalkyl group or a dihydroxyalkyl group not containing a hydroxyl group at the 3-position of the alkyl group;
or R₁₀ and R₁₁ together form an optionally substituted monocyclo-heterocycloalkyl group.

9. A compound having Formulae LXVI or LXVII: 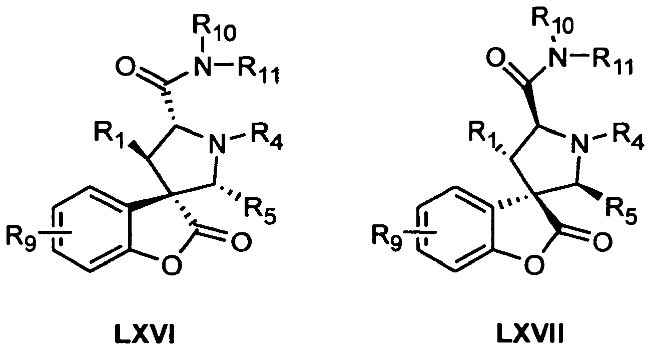 or a pharmaceutically acceptable salt thereof, wherein:
R₁, R₄, and R₅ are independently H or optionally substituted alkyl, cycloalkyl, alkenyl, cycloalkenyl, alkynyl, aryl, heterocyclic, CO₂R', OCOR', CONR'R", NR"COR', NR'SO₂R", SO₂NR'R", (C=NR')NR"R'", or NR'R"; or
R₄ forms an aryl, cycloalkyl, or heterocyclic group with R₅;
R₉ represents a 6-chloro and a 5-fluoro group;
each R', R" and R'" is independently H or optionally substituted alkyl, cycloalkyl, alkenyl, cycloalkenyl, alkynyl, aryl, or heterocyclic; or
R' and R", or R" and R"', form a ring;
R₁₀ is hydrogen, and
R₁₁ is an optionally substituted cycloalkyl-alkyl or monocyclo-heterocycloalkyl group or a dihydroxyalkyl group not containing a hydroxyl group at the 3-position of the alkyl group;
or R₁₀ and R₁₁ together form an optionally substituted monocyclo-heterocycloalkyl group.

10. A compound selected from the group consisting of:

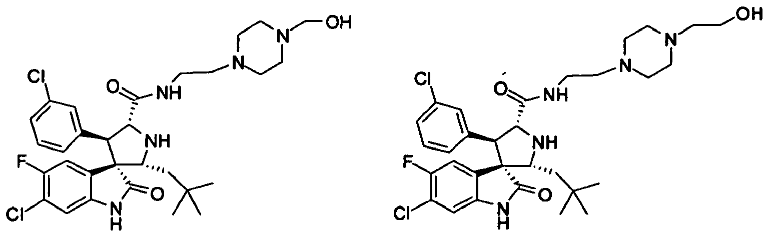

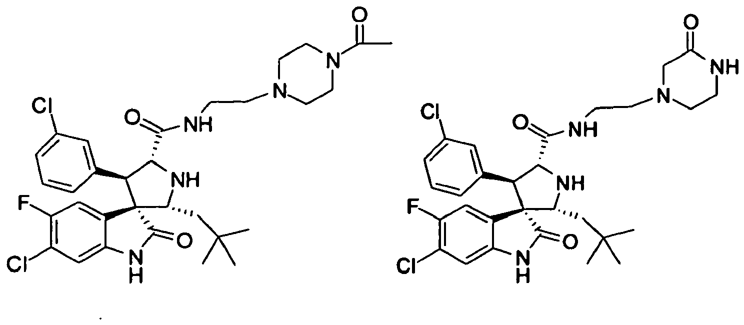

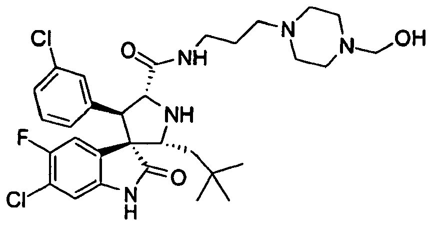

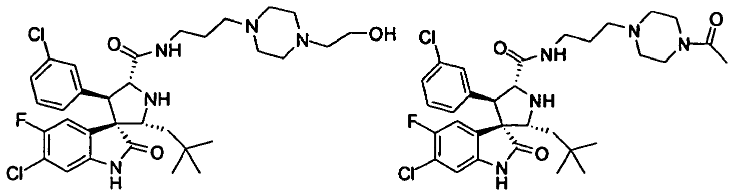

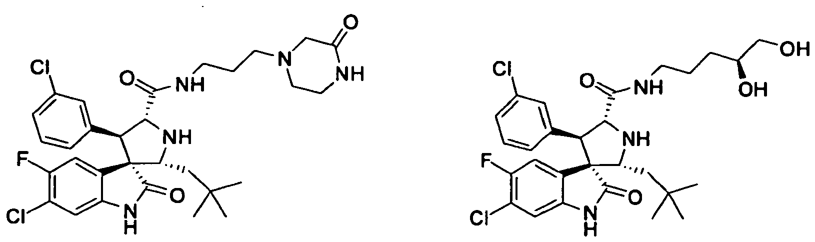

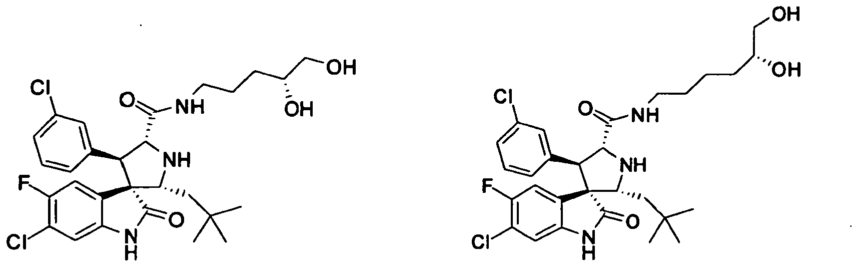

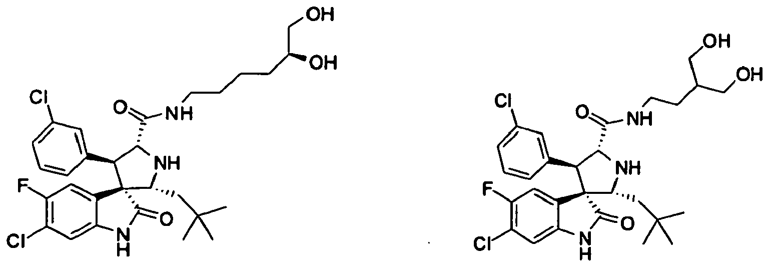

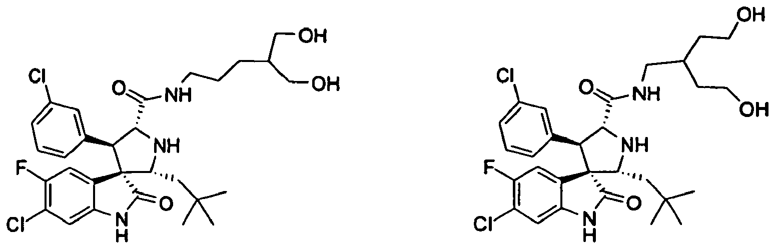

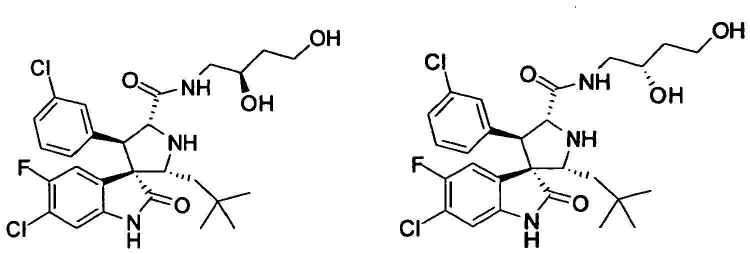

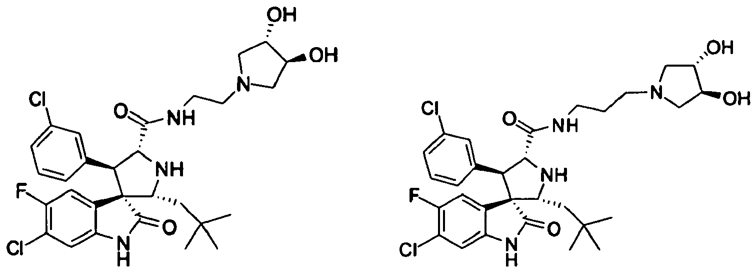

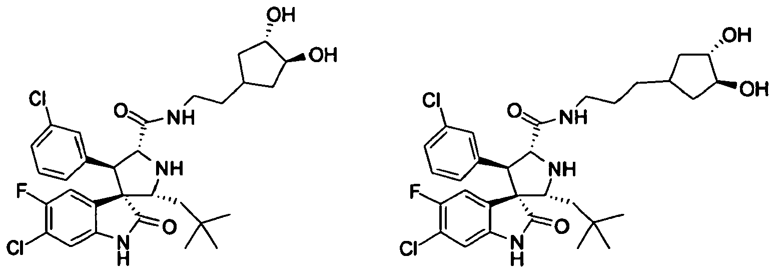

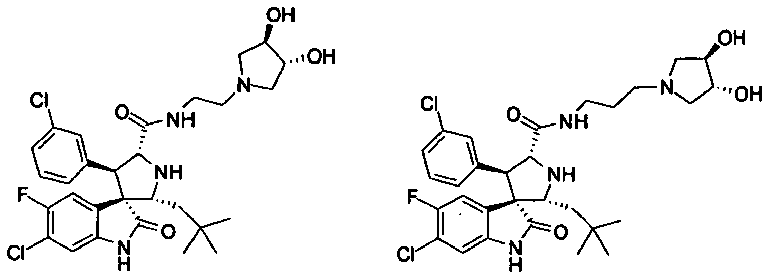

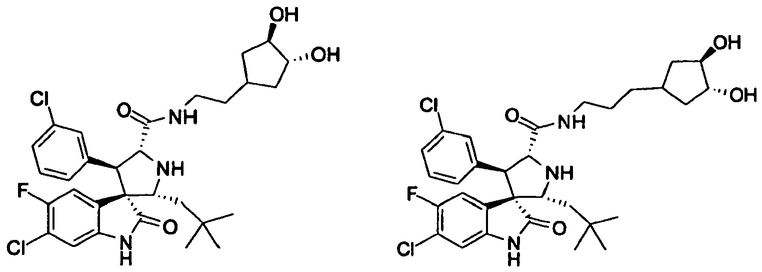

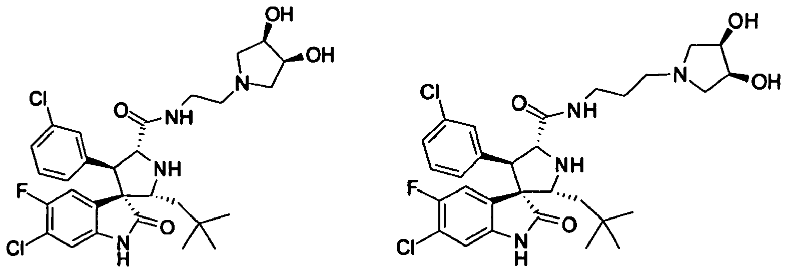

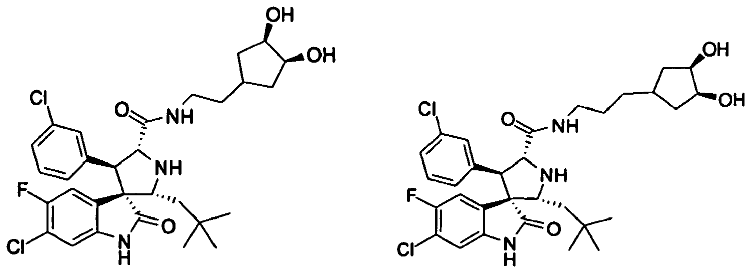

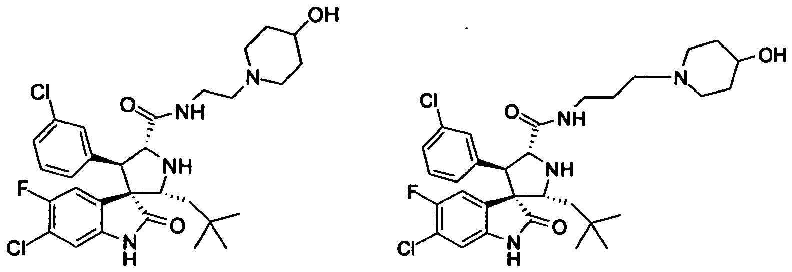

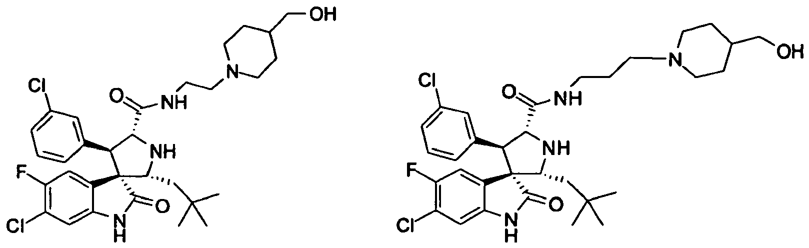

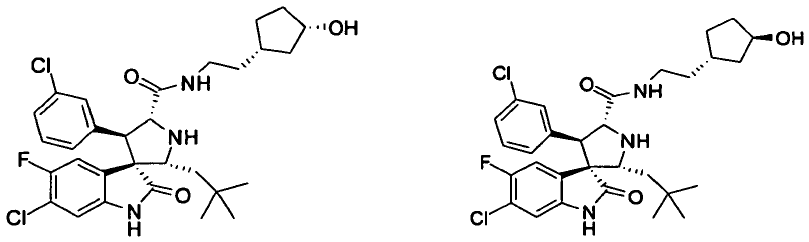

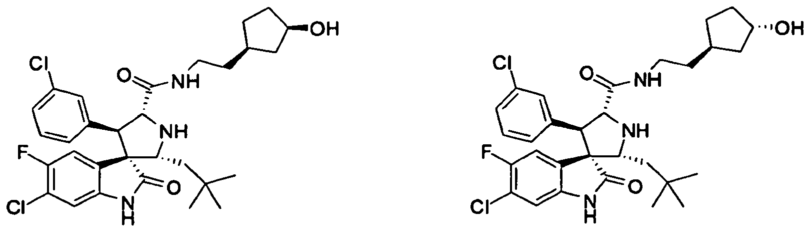

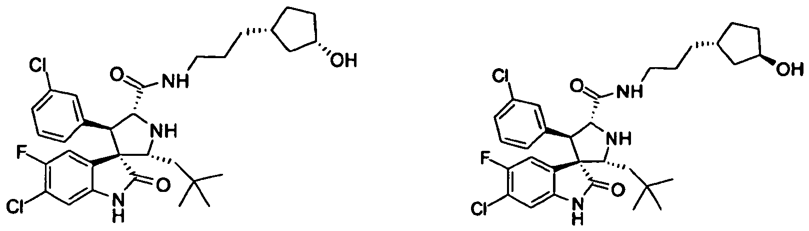

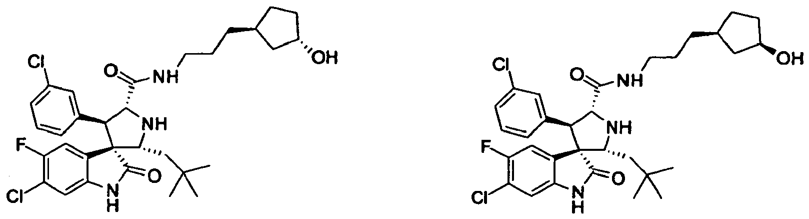

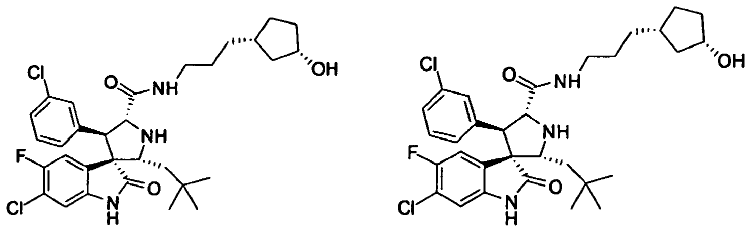

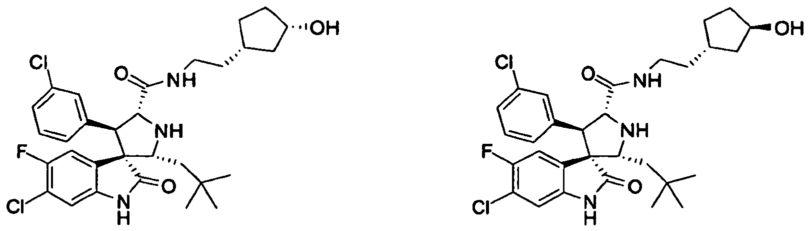

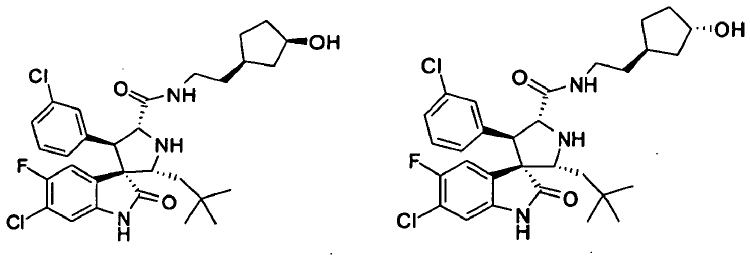

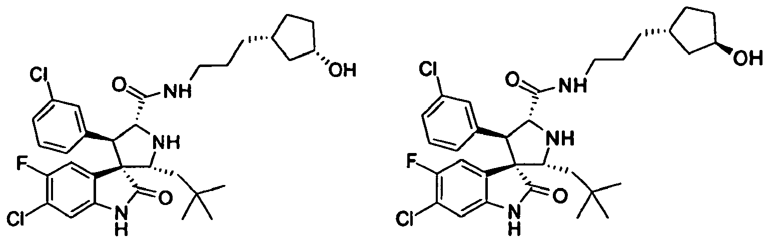

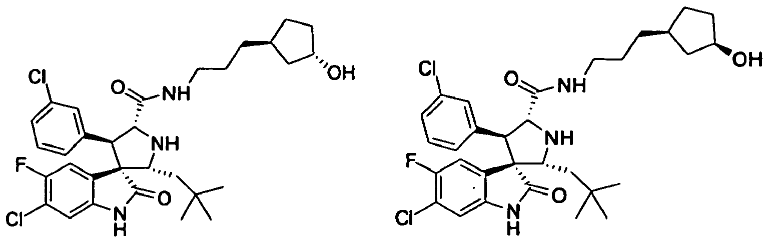

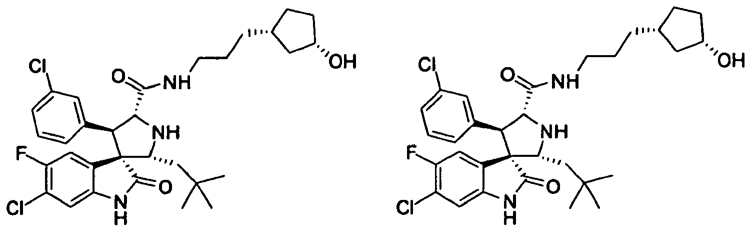

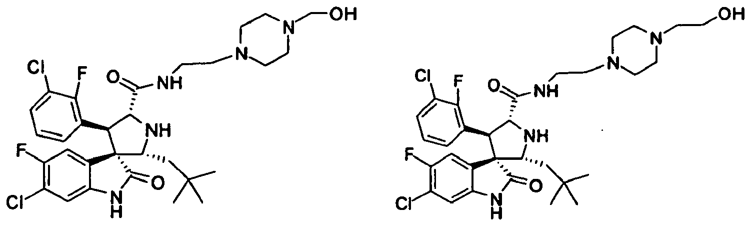

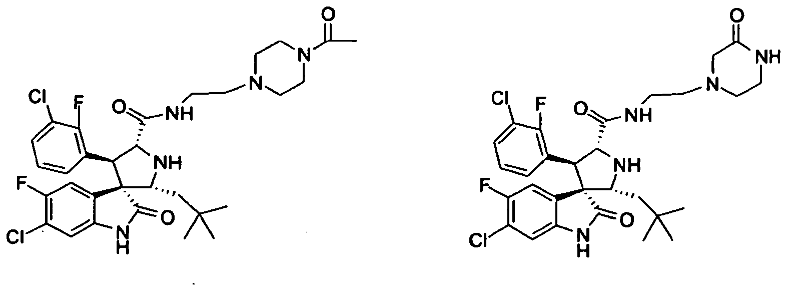

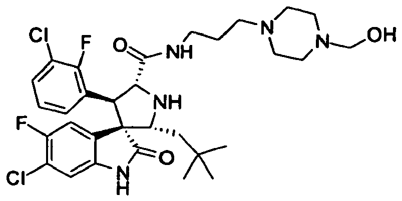

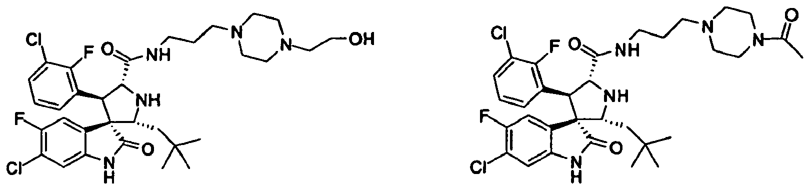

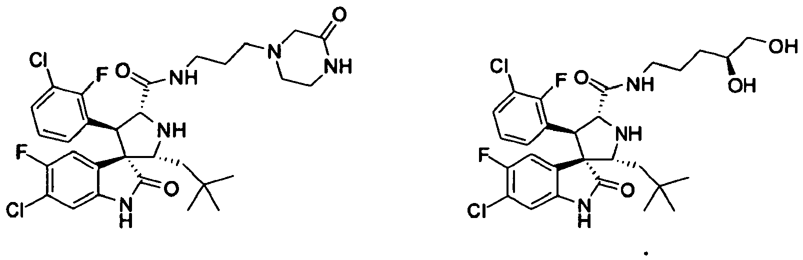

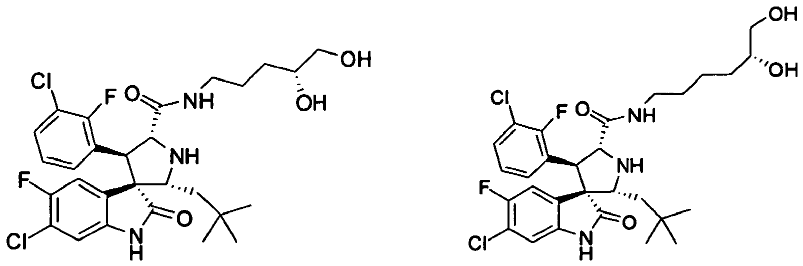

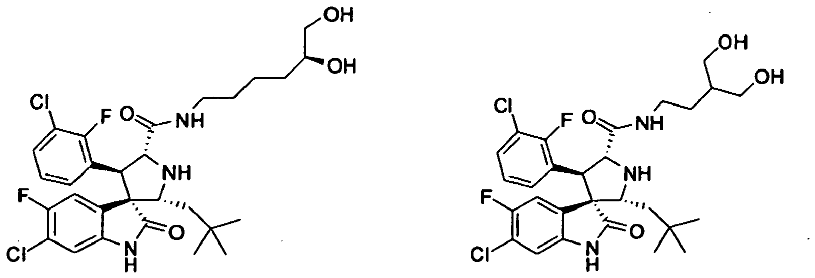

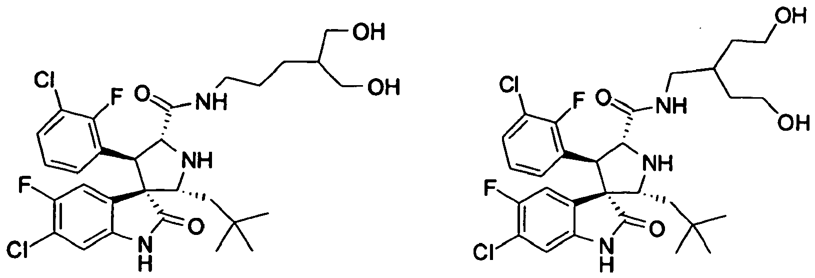

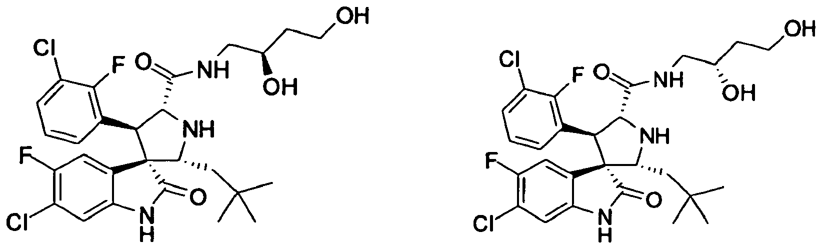

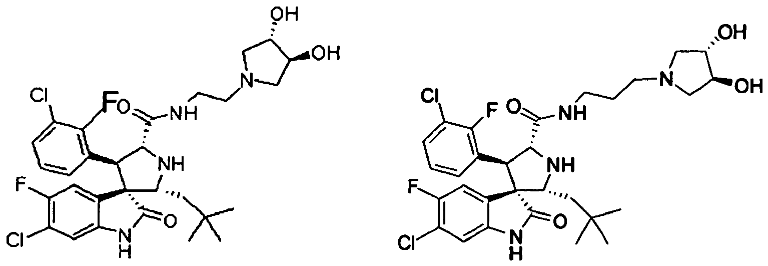

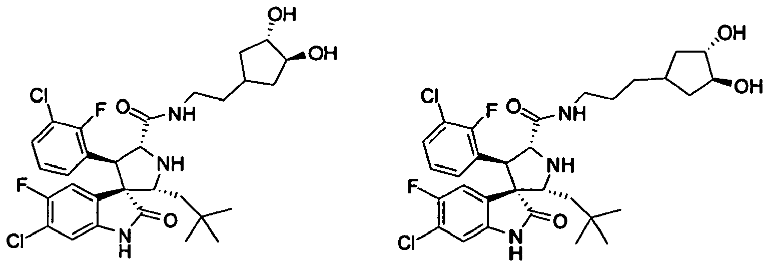

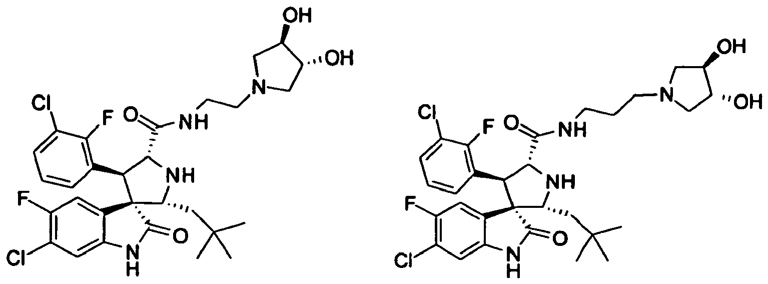

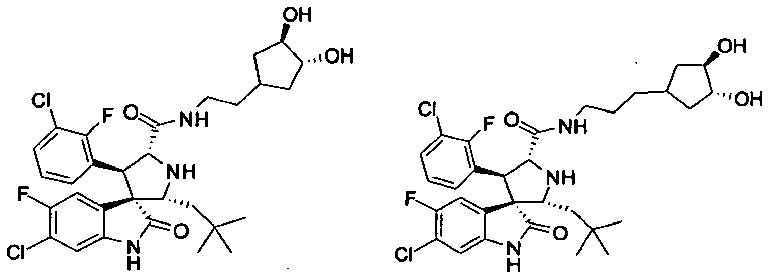

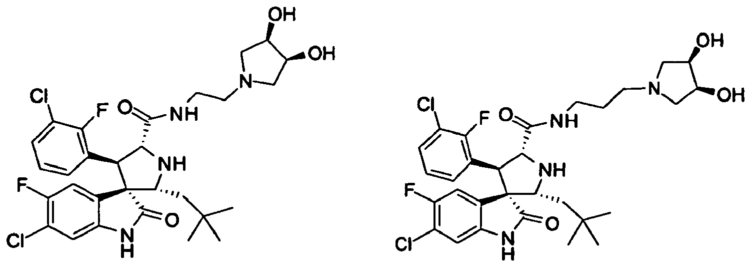

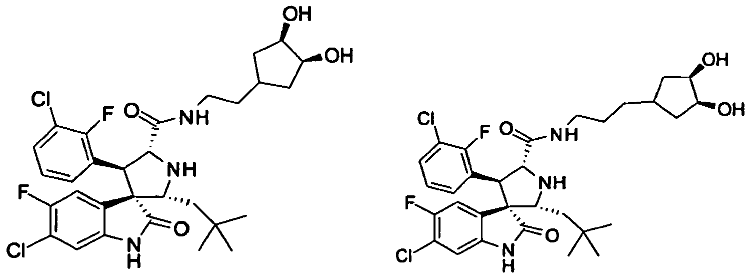

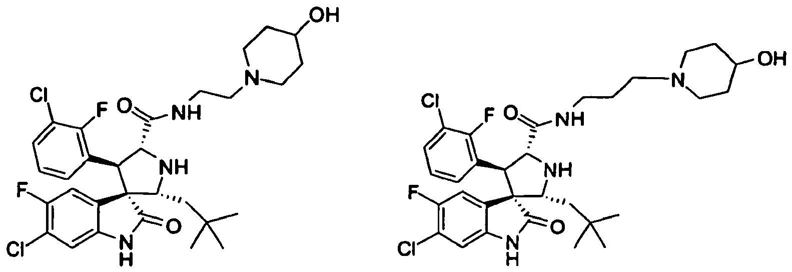

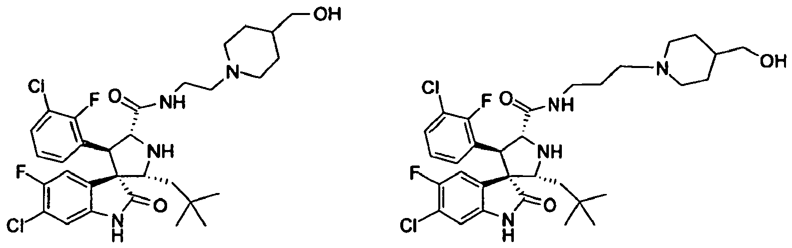

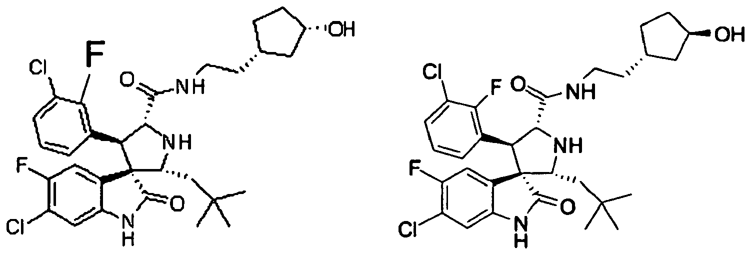

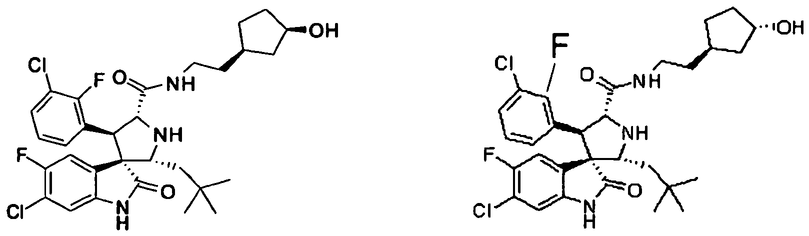

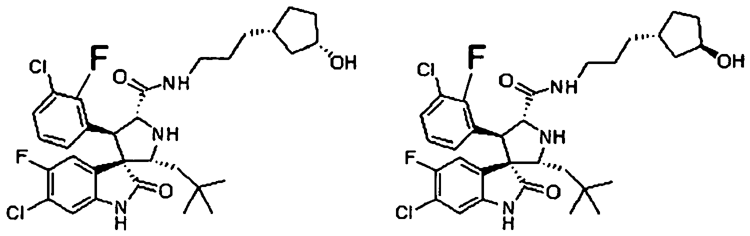

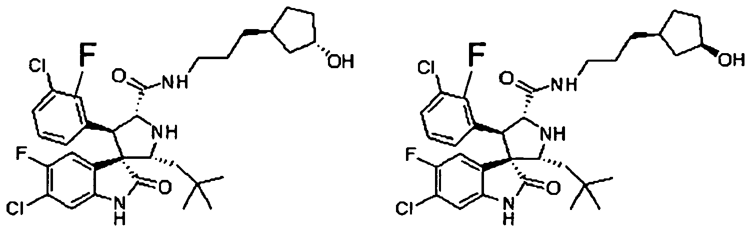

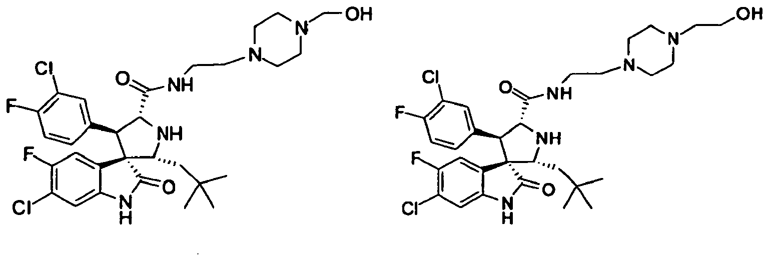

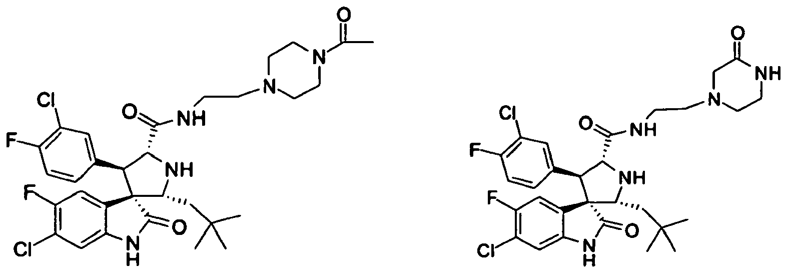

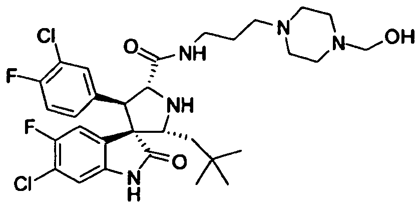

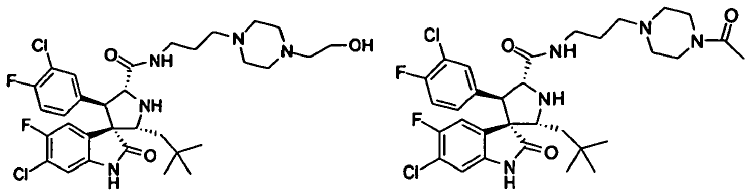

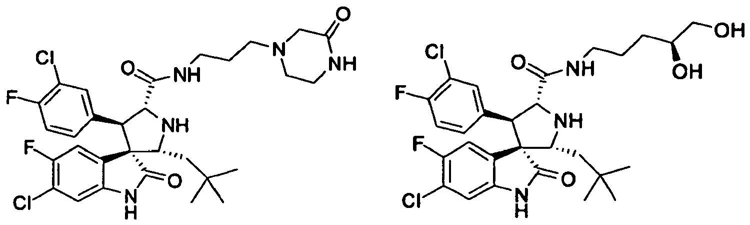

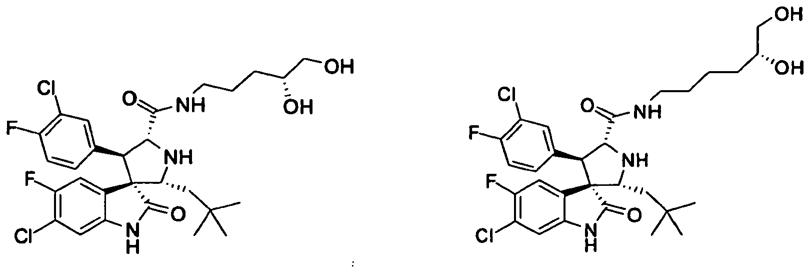

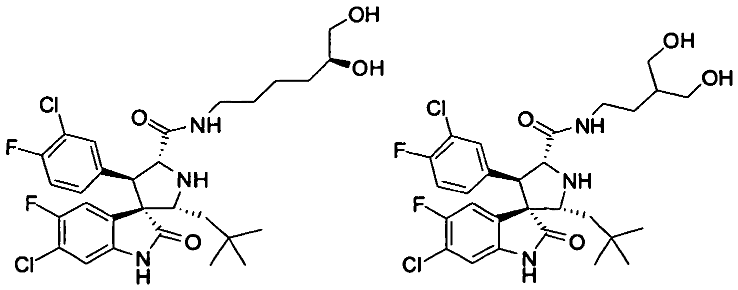

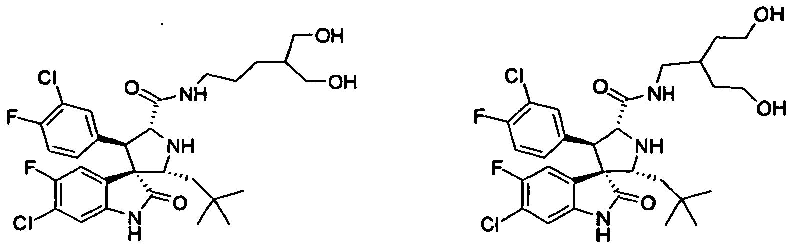

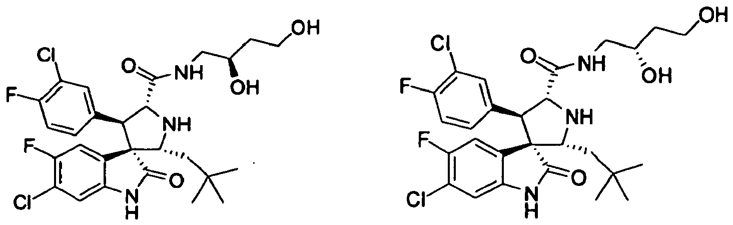

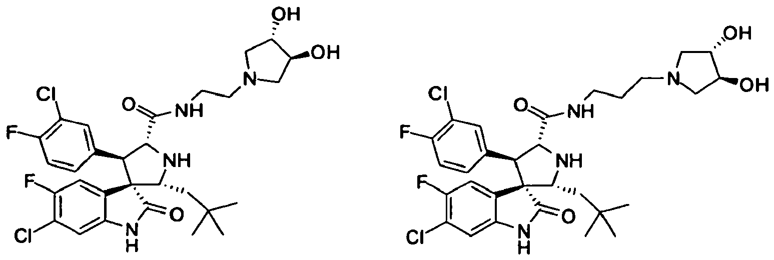

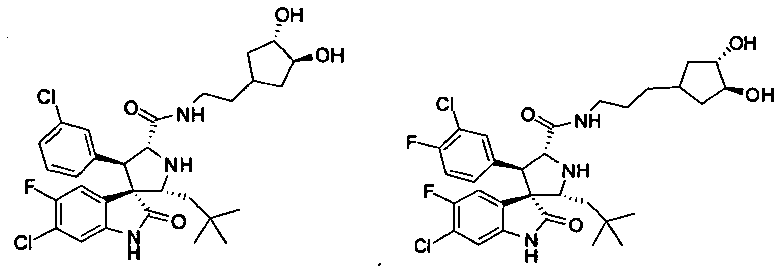

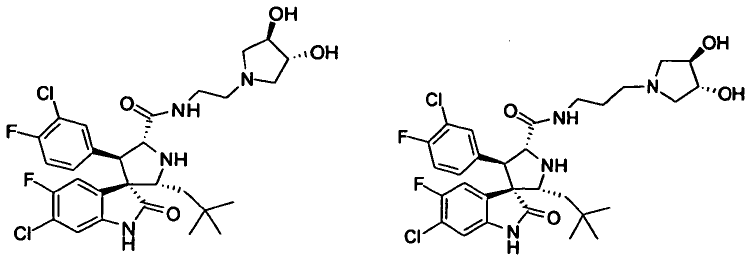

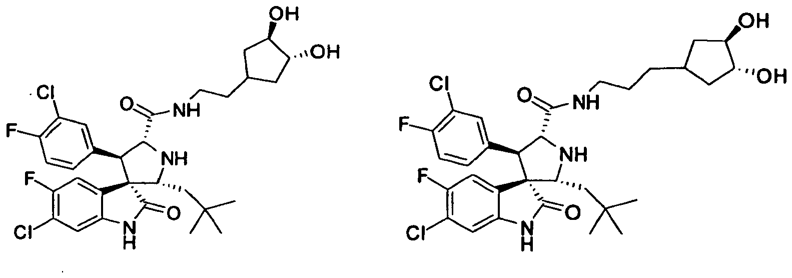

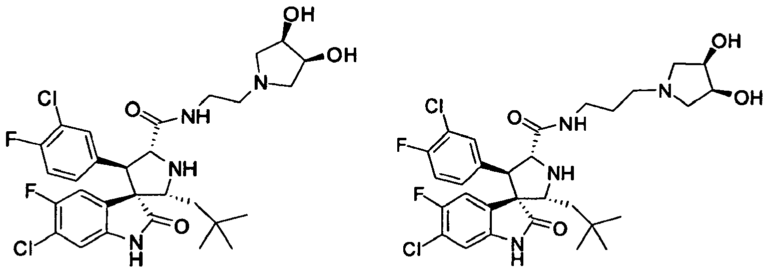

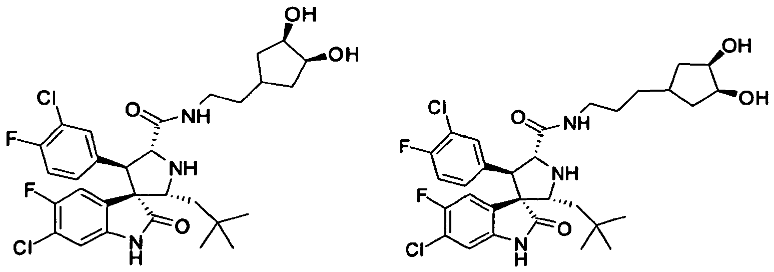

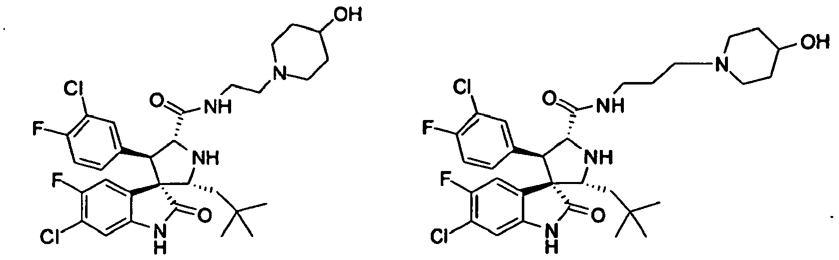

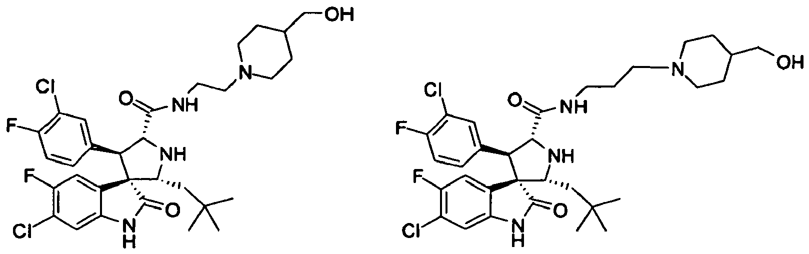

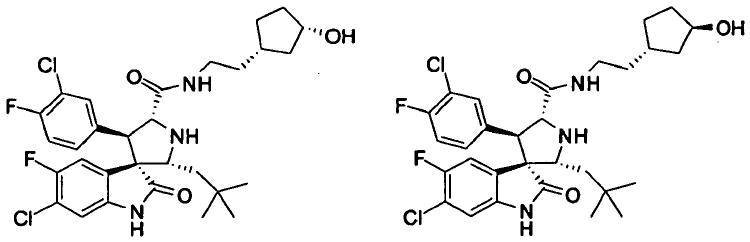

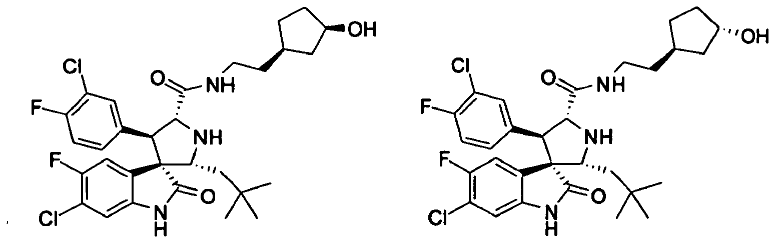

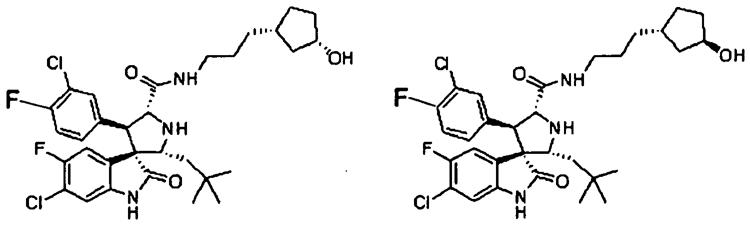

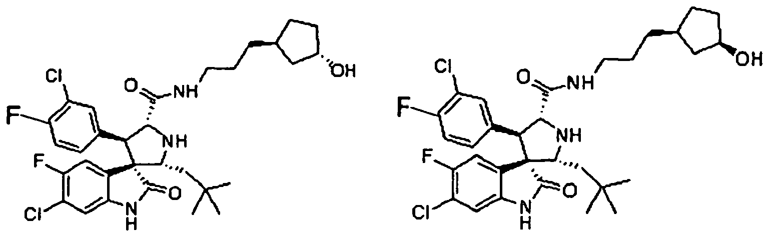

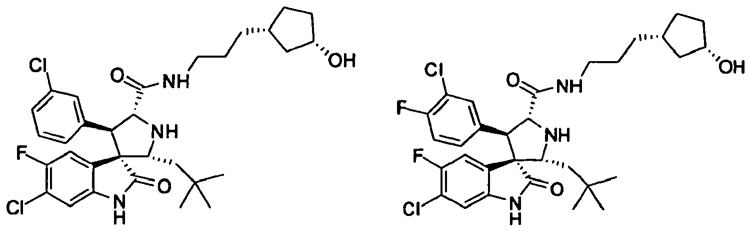

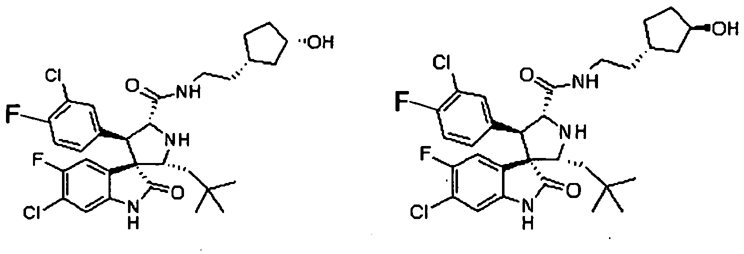

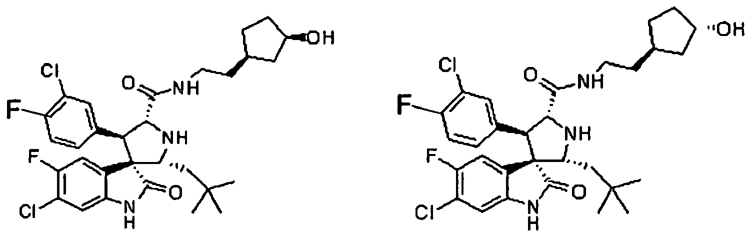

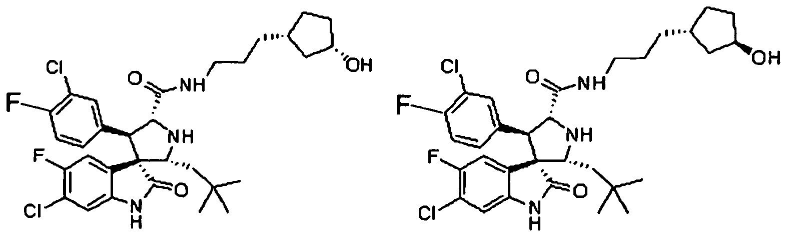

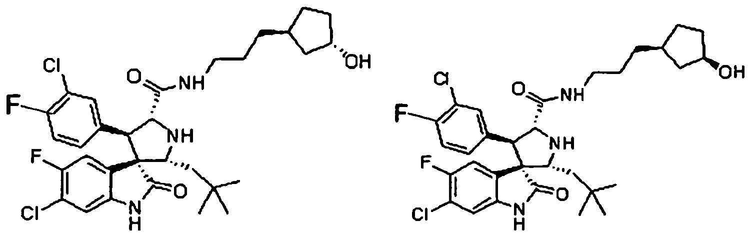

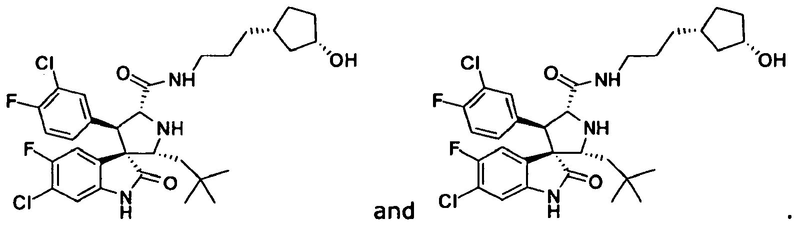

11. A compound selected from the group consisting of: 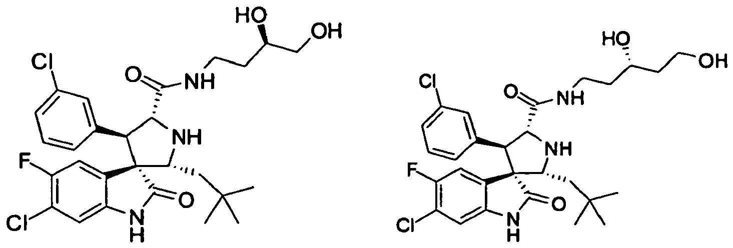 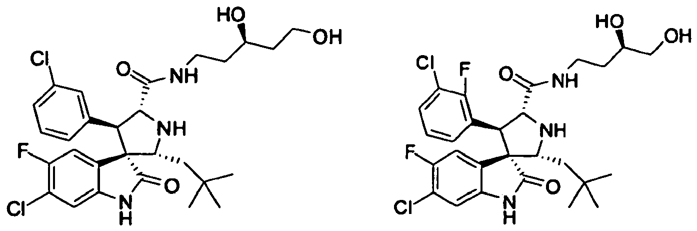 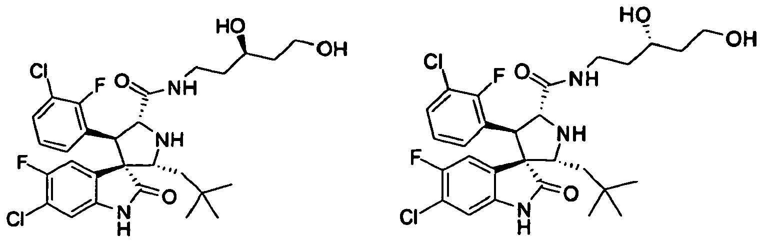 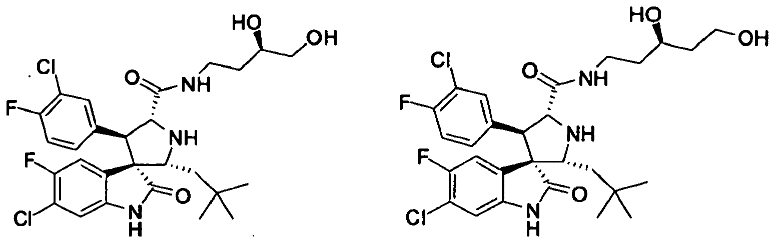 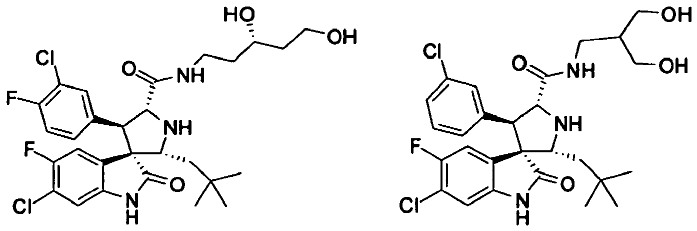 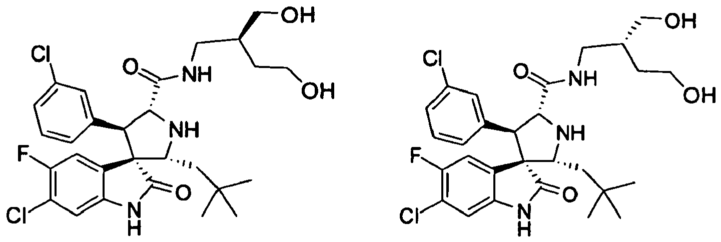 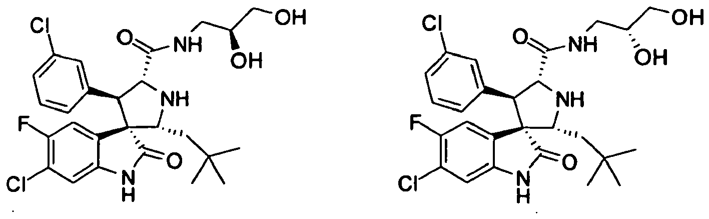 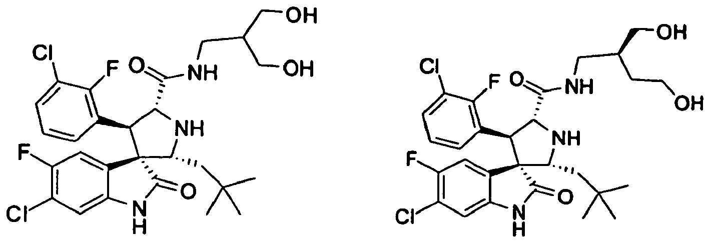 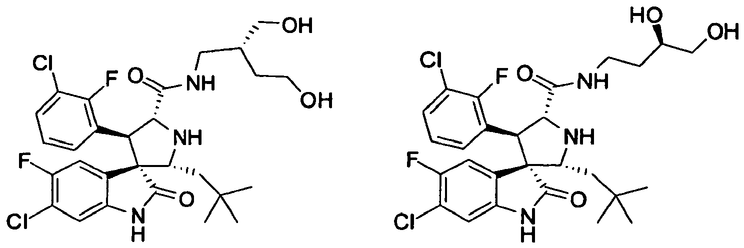 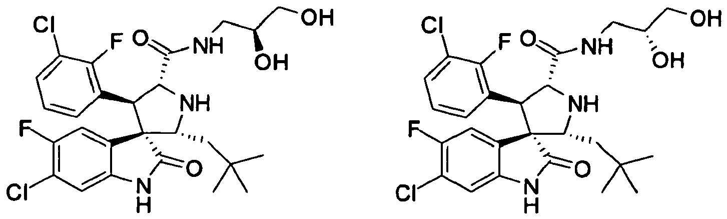 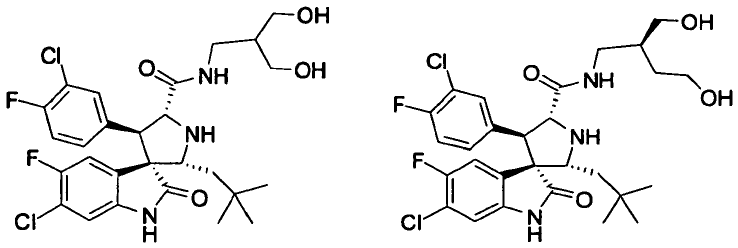 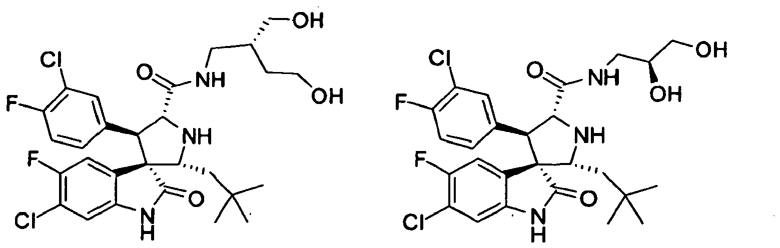 and 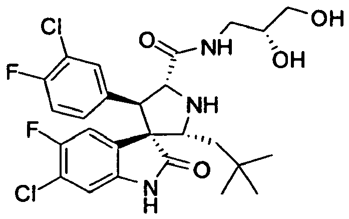 **or a pharmaceutically acceptable salt thereof.**

12. A compound which is 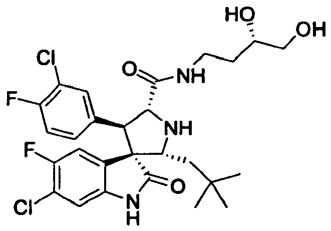 **or a pharmaceutically acceptable salt thereof.**

13. A compound selected from the group consisting of: 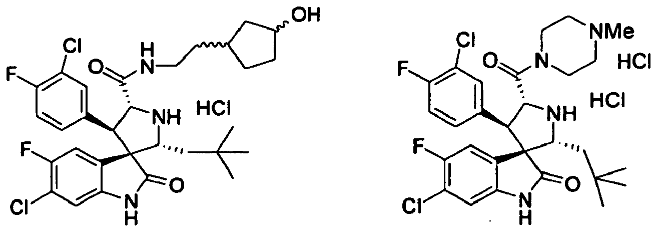 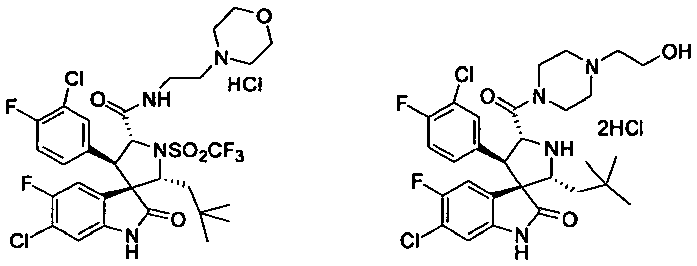 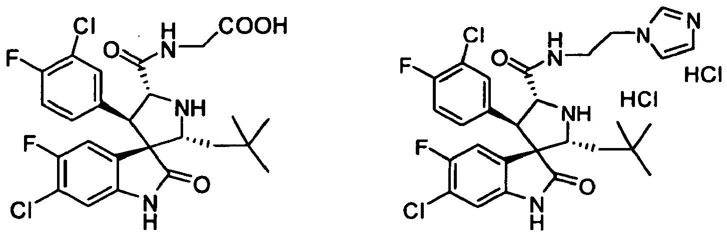 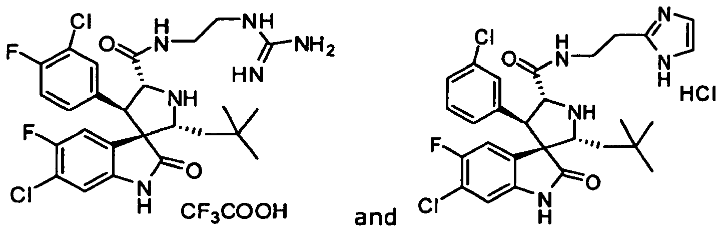 or a pharmaceutically acceptable salt thereof, or a different pharmaceutically acceptable salt thereof.

14. A compound selected from the group consisting of: 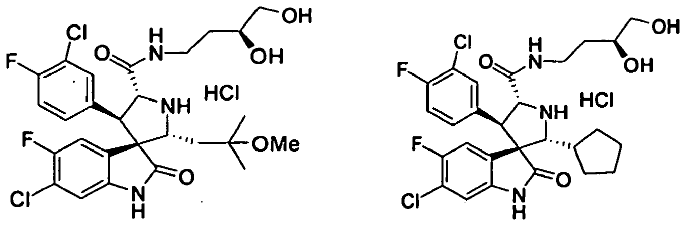 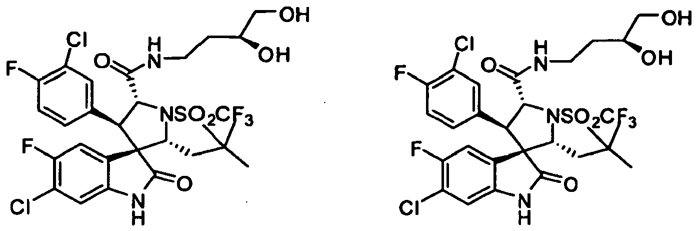 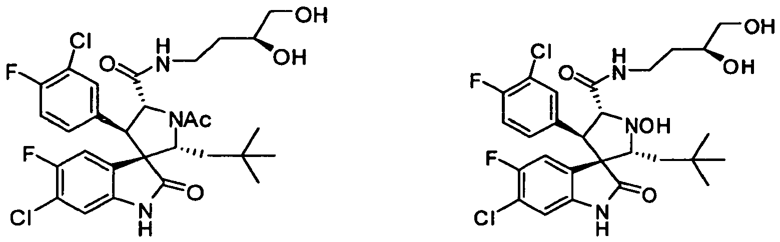 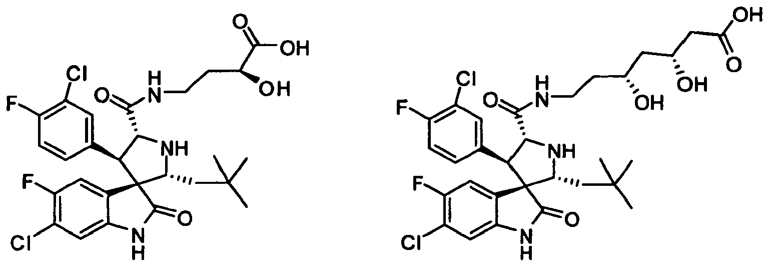 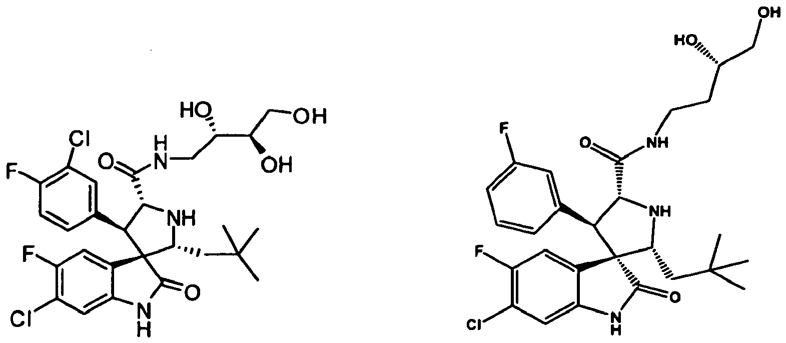 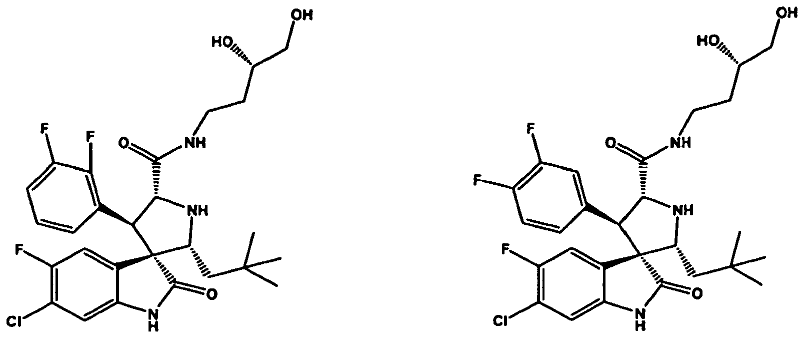 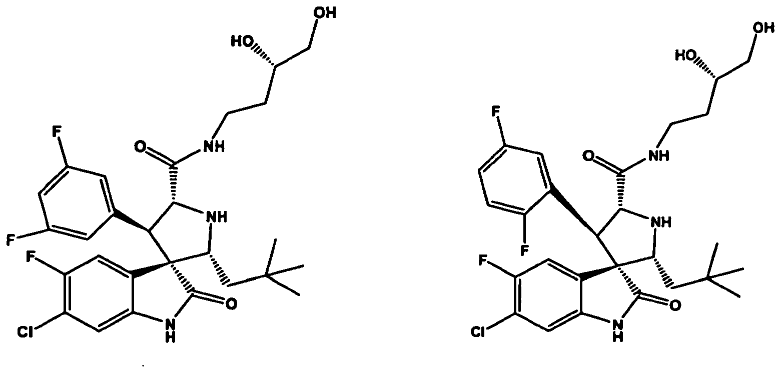 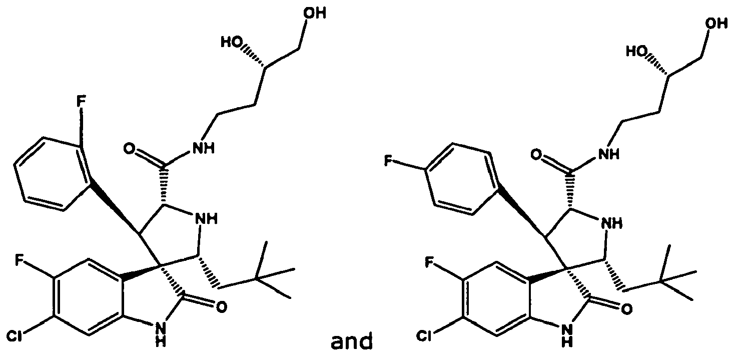 or a pharmaceutically acceptable salt thereof, or a different pharmaceutically acceptable salt thereof.

15. A pharmaceutical composition comprising a compound of any of the claims 1 to 14 and a pharmaceutically acceptable carrier.

16. A compound of any of the claims 1 to 14 for use in treating cancer.

17. The compound for use according to claim 16, wherein said cancer is selected from the group consisting of prostate cancer and colon cancer.

18. The compound for use according to claim 16, wherein said compound is to be administered with at least one additional therapeutic agent.

19. A compound of any of the claims 1 to 14 for use in treating mucositis, stomatitis, xerostomia, a gastrointestinal disorder, or alopecia.

## Patentansprüche

1. Stoff der Formel I: 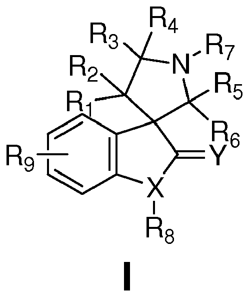 oder ein pharmazeutisch akzeptables Salz davon, wobei:
X CH, 0, N oder S ist, wobei R₈ fehlt wenn X 0 oder S ist;
Y 0, S oder NR' ist;
R₁, R₂, R₄, R₅, R₆ und R₇ unabhängig H oder optional substituiertes Alkyl, Cycloalkyl, Alkenyl, Cycloalkenyl, Alkynyl, Aryl, heterocyclisch, CO₂R', OCOR', CONR'R'', NR''COR', NR'SO₂R'', SO₂NR'R'', (C=NR')NR''R''' oder NR'R" sind; oder
R₇ ein Aryl, Cycloalkyl oder eine heterocyclische Gruppe mit R₅ oder R₆ bildet;
R₈ H oder optional substituiertes Alkyl, Cycloalkyl, Alkenyl, Cycloalkenyl, Alkynyl, Aryl, heterocyclisch, CO₂R', OCOR', CONR'R'', SO₂NR'R'' oder (C=NR')NR''R''' ist;
R₉ eine 6-Chlor- und eine 5-Fluorgruppe darstellt; und
R', R'' und R''' jeweils unabhängig H oder optional substitutiertes Alkyl, Cycloalkyl, Alkenyl, Cycloalkenyl, Alkynyl, Aryl oder heterocyclisch ist; oder R' und R'' oder R'' und R''' einen Ring bilden;
wobei R₃ CONRR' ist, und R eine optional substituierte Cycloalkyl-alkyl- oder Monocyclo-heterocycloalkyl-Gruppe oder eine Dihydroxyalkyl-Gruppe ist, die keine Hydroxylgruppe an der 3-Position der Alkylgruppe umfasst.

2. Stoff nach Anspruch 1 der Formel II oder der Formel III: 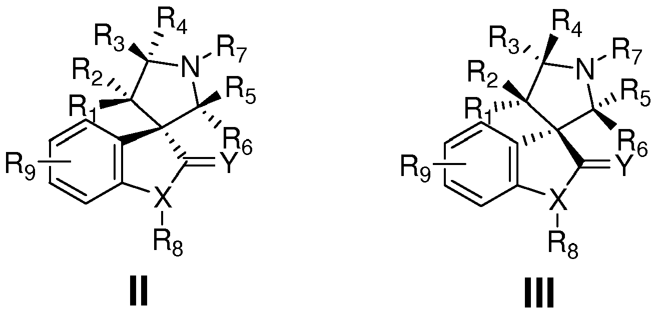 oder ein pharmazeutisch akzeptables Salz davon.

3. Stoff nach Anspruch 1 der Formel VII: 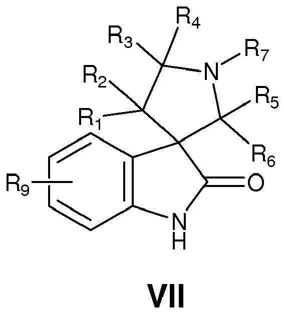 oder ein pharmazeutisch akzeptables Salz davon.

4. Stoff nach Anspruch 3 der Formel VIII oder der Formel IX: 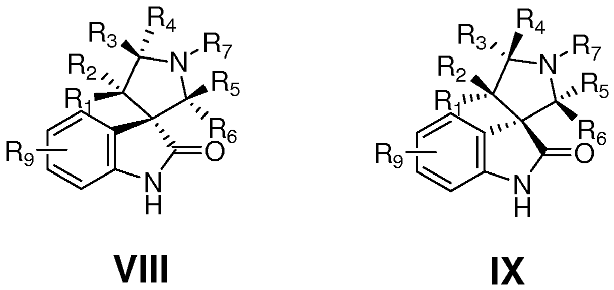 oder ein pharmazeutisch akzeptables Salz davon.

5. Stoff nach Anspruch 1, wobei eines der R₁ und R₂ optional substituiertes Aryl oder Heteroaryl ist.

6. Stoff nach Anspruch 1, wobei eines der R₁ und R₂ Cycloalkyl, geradekettiges oder verzweigtes Alkyl, CONR'R" oder CO₂R' ist.

7. Stoff nach Anspruch 1, wobei eines der R₅ und R₆ C₃₋₁₈ Alkyl, Aryl oder Heteroaryl ist.

8. Stoff der Formel XXVII oder der Formel XXVIII: 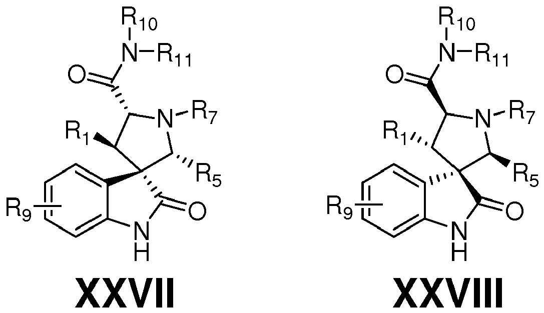 oder ein pharmazeutisch akzeptables Salz davon, wobei:
R₁, R₅ und R₇ unabhängig H oder optional substituiertes Alkyl, Cycloalkyl, Alkenyl, Cycloalkenyl, Alkynyl, Aryl, heterocyclisch, CO₂R', OCOR', CONR'R'', NR''COR', NR'SO₂R'', SO₂NR'R'', (C=NR')NR''R''' oder NR'R" sind; oder
R₇ ein Aryl, Cycloalkyl oder eine heterocyclische Gruppe mit R₅ bildet;
R₉ eine 6-Chlor- und eine 5-Fluorgruppe darstellt;
R', R'' und R''' jeweils unabhängig H oder optional substituiertes Alkyl, Cycloalkyl, Alkenyl, Cycloalkenyl, Alkynyl, Aryl oder heterocyclisch ist; oder
R' und R'' oder R'' und R''' einen Ring bilden;
R₁₀ Wasserstoff ist, und
R₁₁ eine optional substituierte Cycloalkyl-alkyl- oder Monocyclo-heterocycloalkyl-Gruppe oder eine Dihydroxyalkyl-Gruppe ist, die keine Hydroxylgruppe an der 3-Position der Alkylgruppe umfasst;
oder R₁₀ und R₁₁ gemeinsam eine optional substituierte Monocyclo-heterocycloalkylgruppe bilden.

9. Stoff der Formel LXVI oder LXVII: 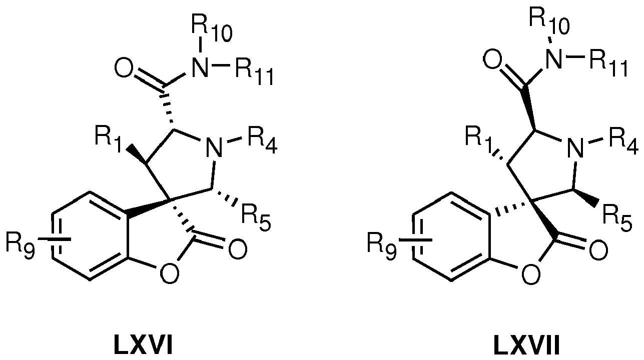 oder ein pharmazeutisch akzeptables Salz davon, wobei:
R₁, R₄ und R₅ unabhängig H oder optional substituiertes Alkyl, Cycloalkyl, Alkenyl, Cycloalkenyl, Alkynyl, Aryl, heterocyclisch, CO₂R', OCOR', CONR'R'', NR''COR', NR'SO₂R'', SO₂NR'R'', (C=NR')NR''R''' oder NR'R" sind; oder
R₄ ein Aryl, Cycloalkyl oder eine heterocyclische Gruppe mit R₅ bildet;
R₉ eine 6-Chlor- und eine 5-Fluorgruppe darstellt;
R', R'' und R''' jeweils unabhängig H oder optional substituiertes Alkyl, Cycloalkyl, Alkenyl, Cycloalkenyl, Alkynyl, Aryl oder heterocyclisch ist; oder
R' und R'' oder R'' und R''' einen Ring bilden;
R₁₀ Wasserstoff ist, und
R₁₁ eine optional substituierte Cycloalkyl-alkyl- oder Monocyclo-heterocycloalkyl-Gruppe oder eine Dihydroxyalkylgruppe ist, die keine Hydroxylgruppe an der 3-Position der Alkylgruppe umfasst;
oder R₁₀ und R₁₁ gemeinsam eine optional substituierte Monocyclo-heterocycloalkylgruppe bilden.

10. Stoff, ausgewählt aus der Gruppe bestehend aus:

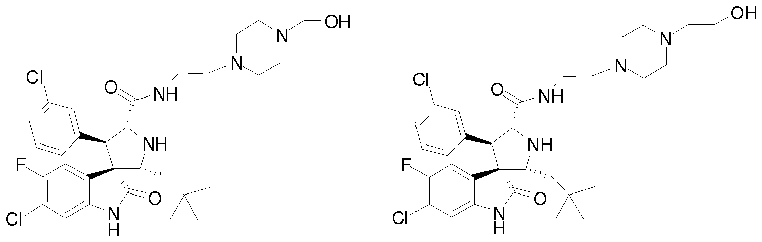

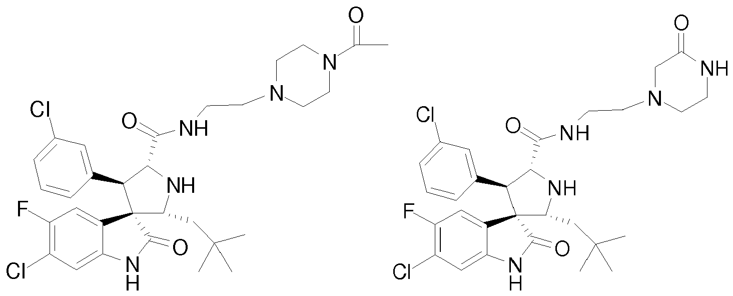

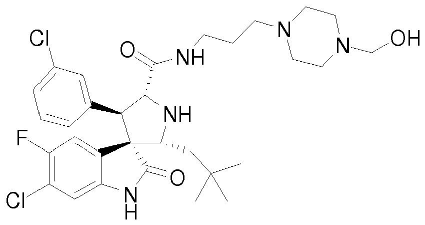

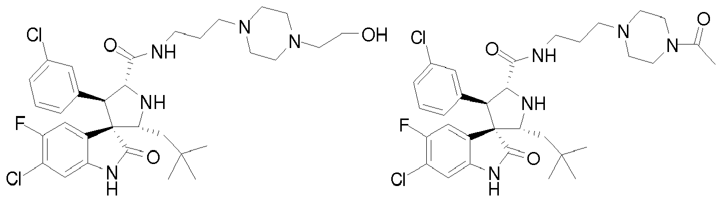

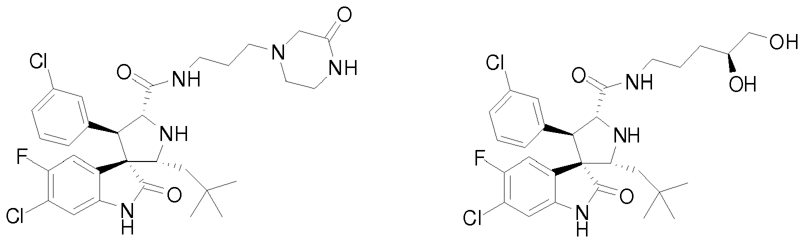

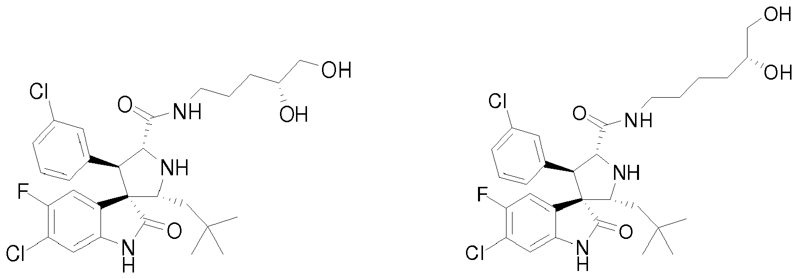

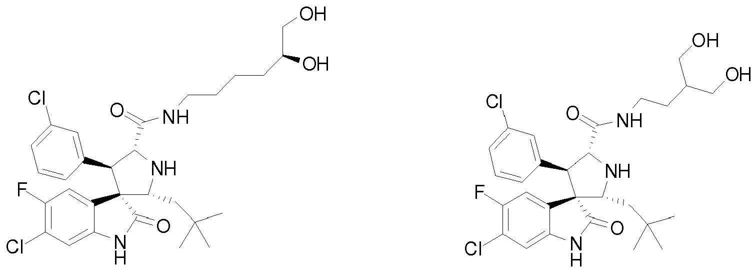

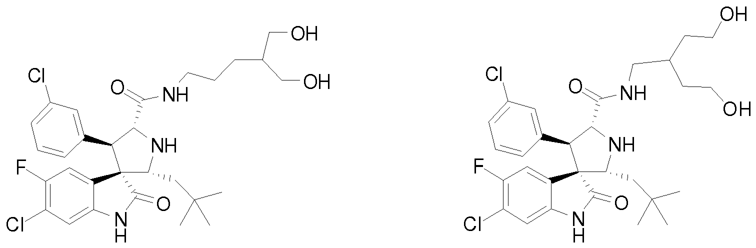

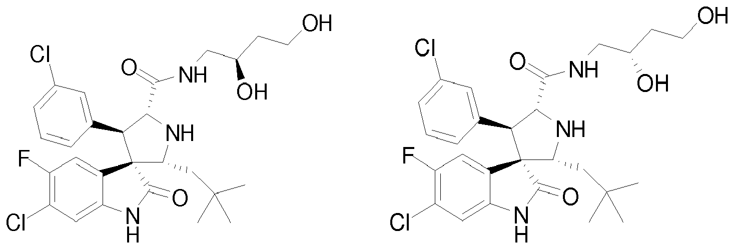

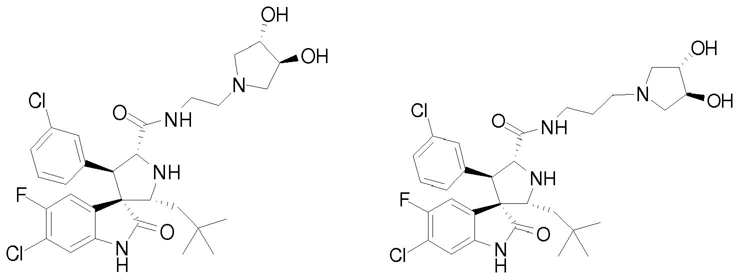

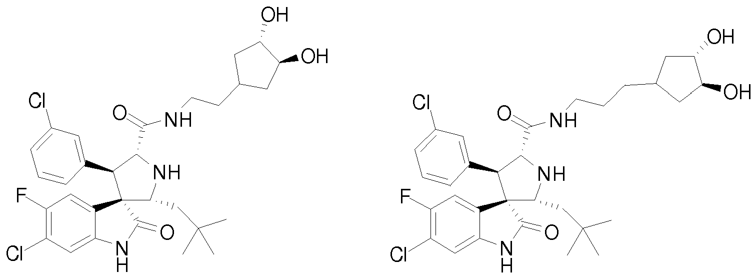

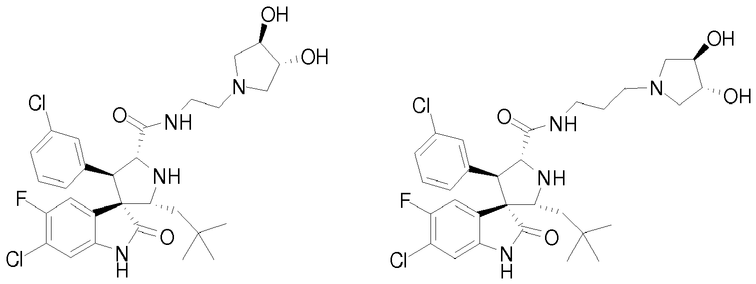

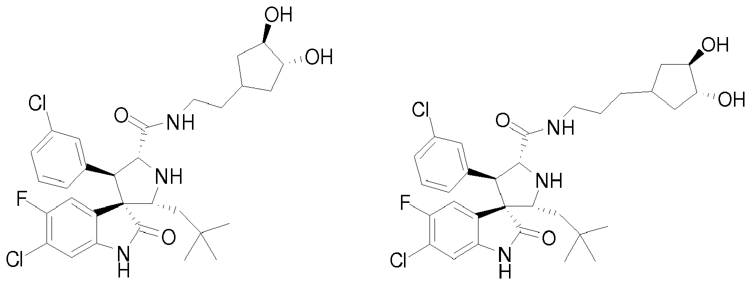

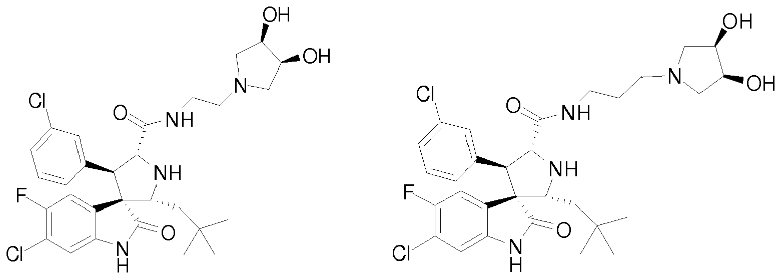

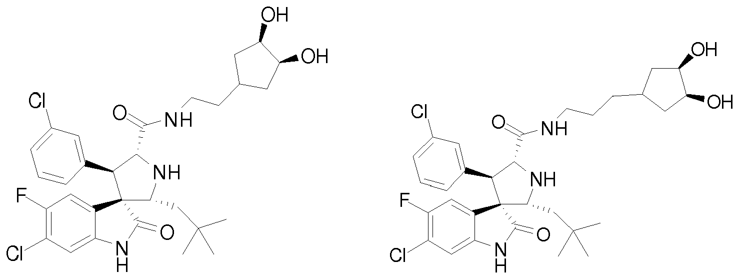

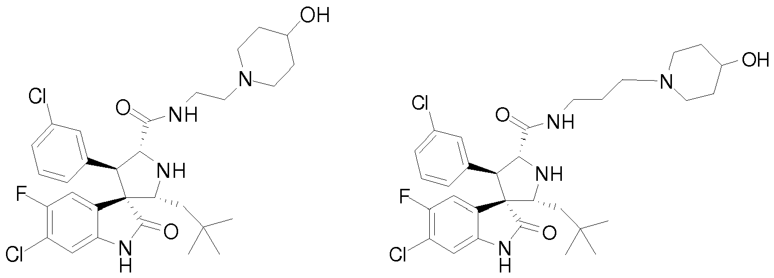

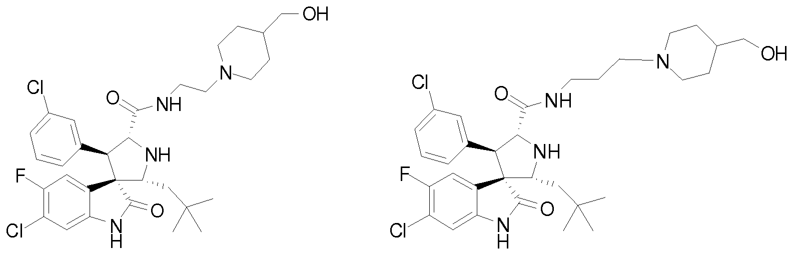

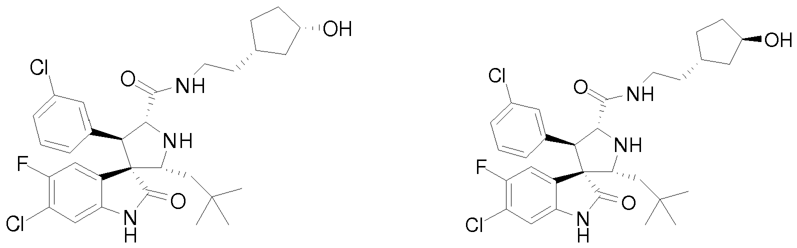

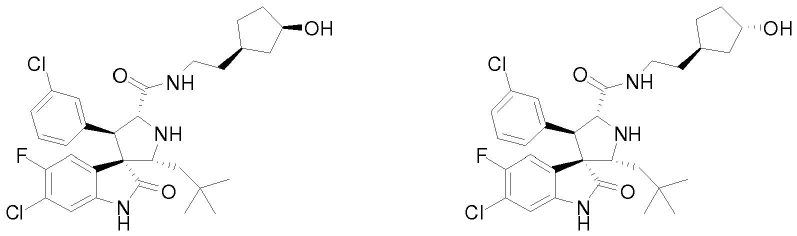

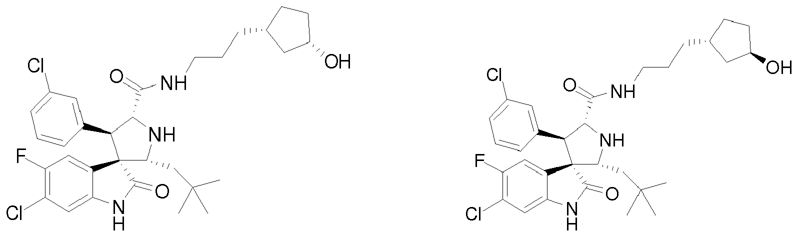

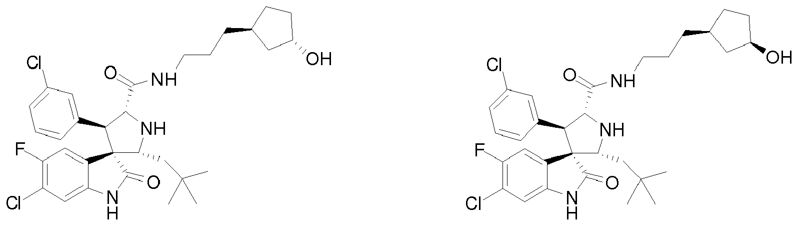

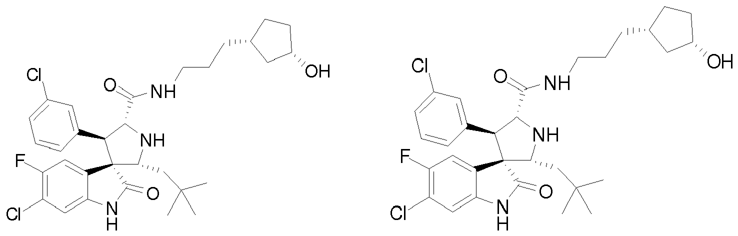

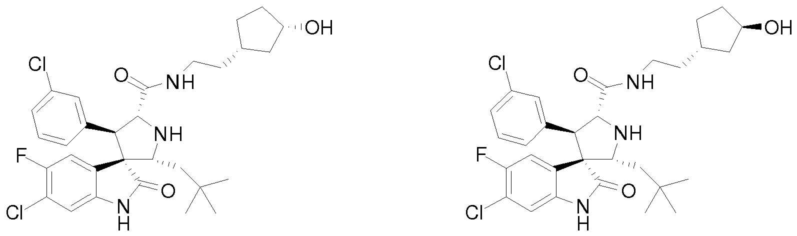

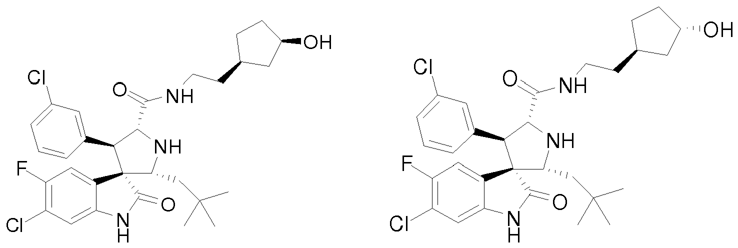

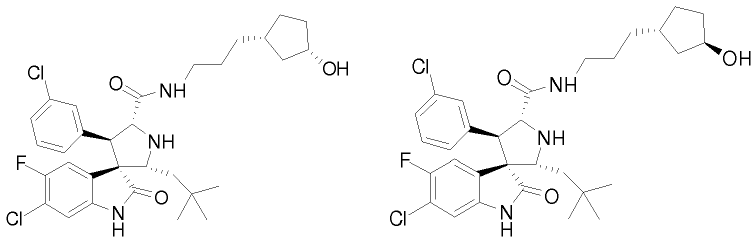

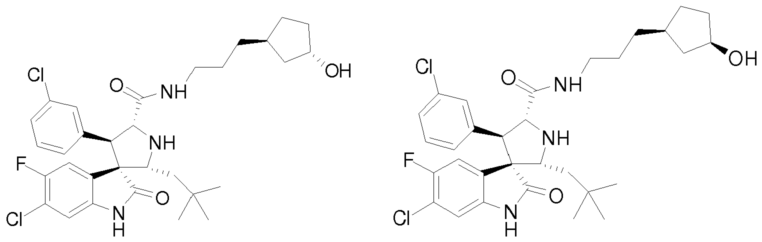

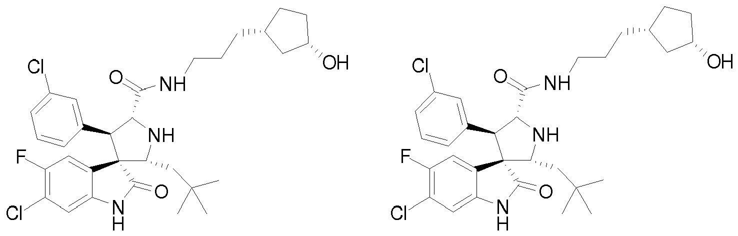

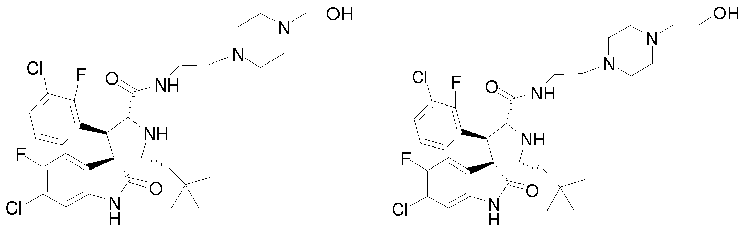

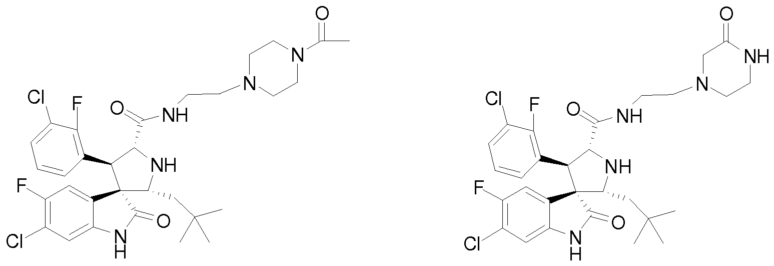

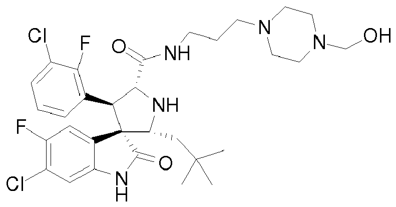

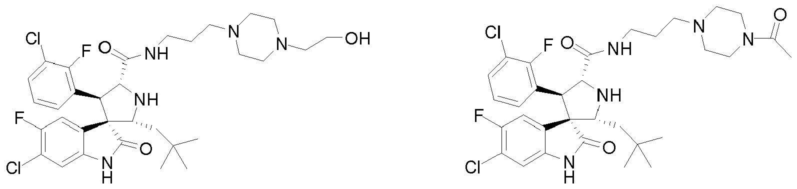

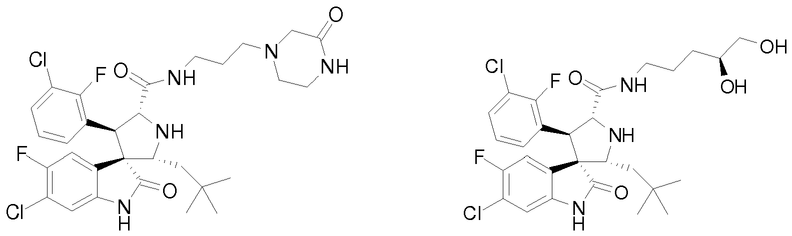

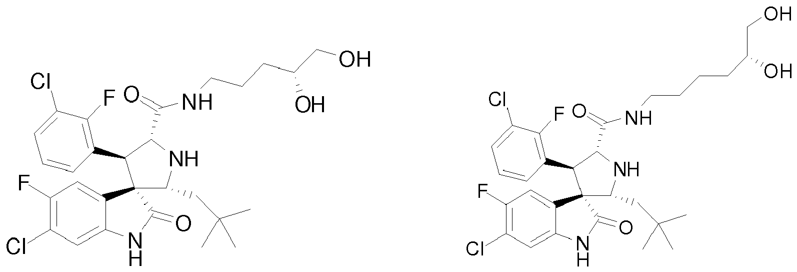

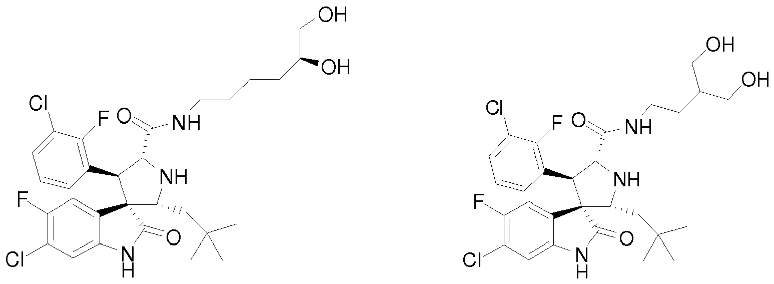

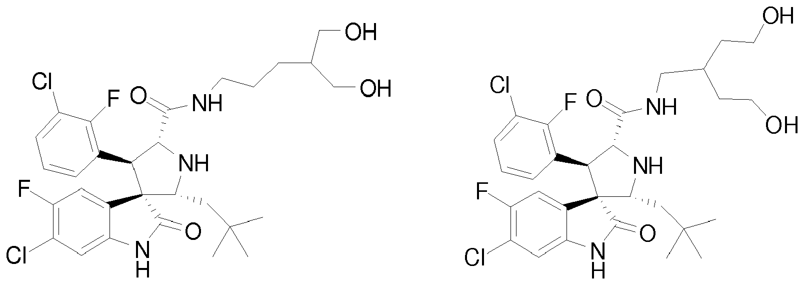

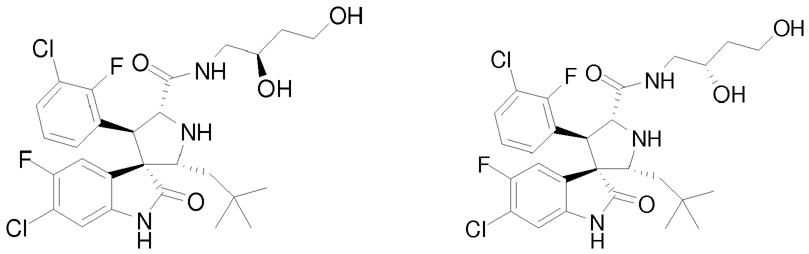

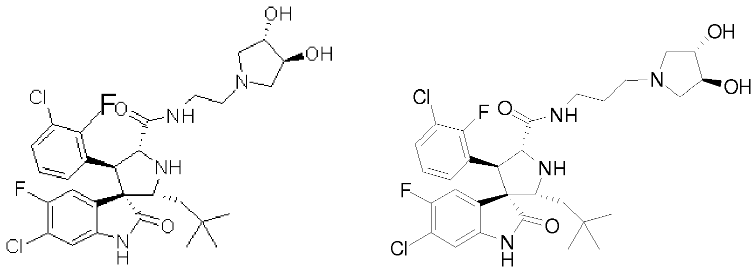

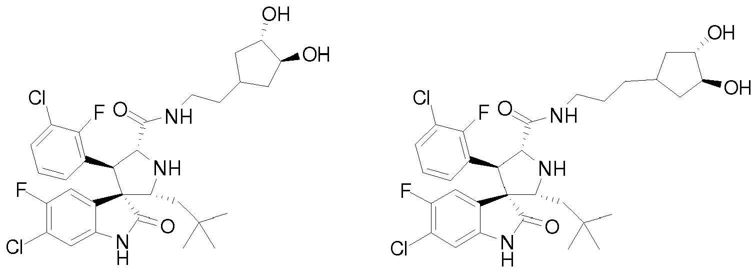

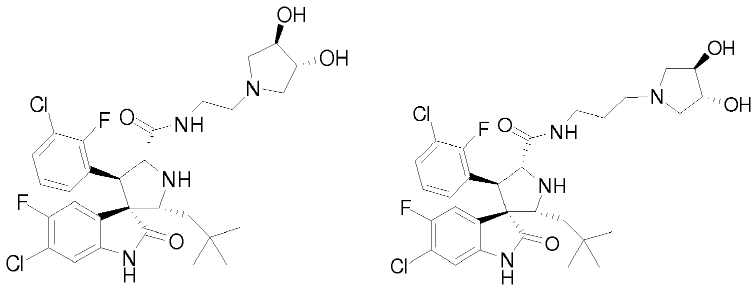

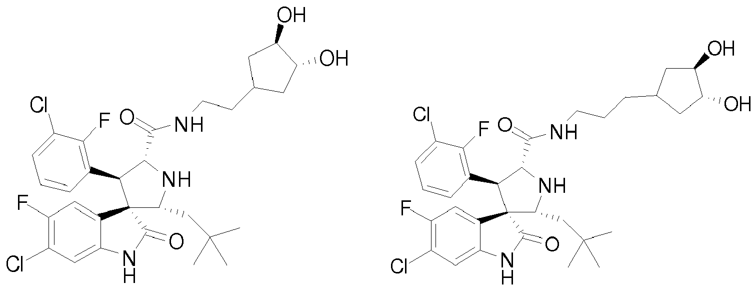

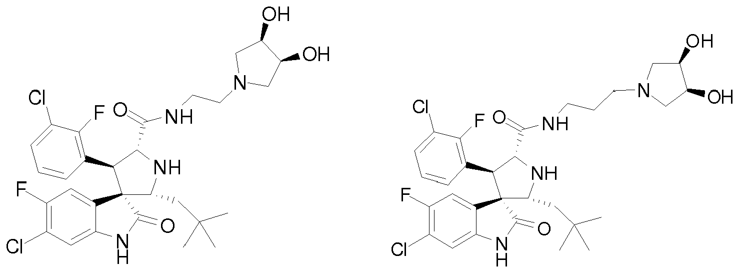

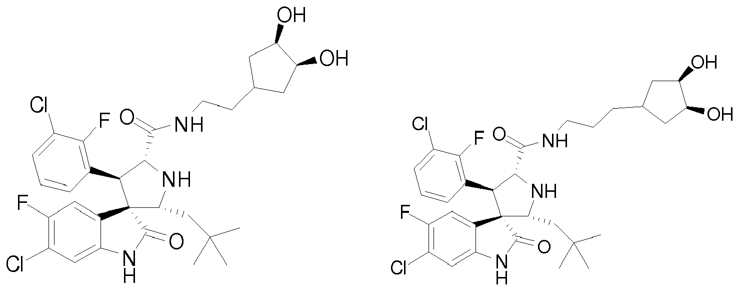

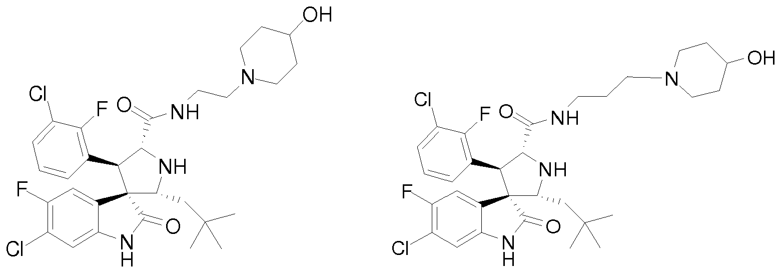

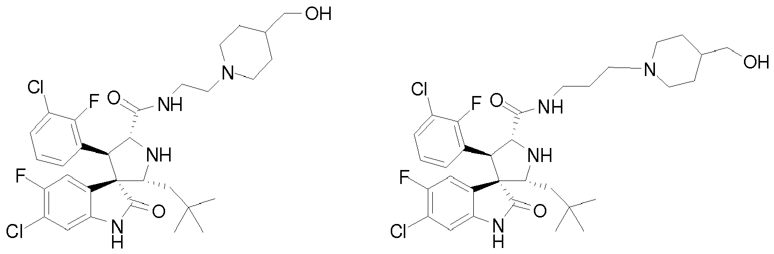

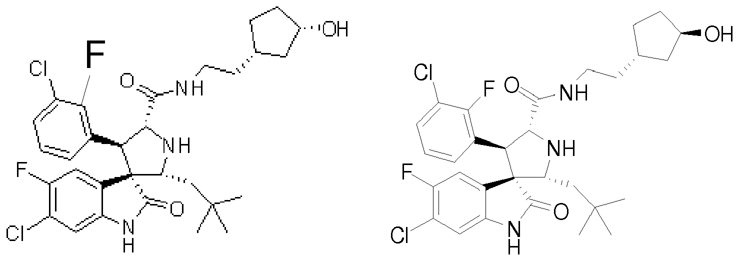

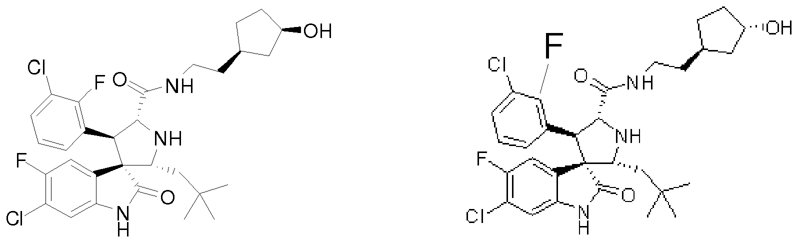

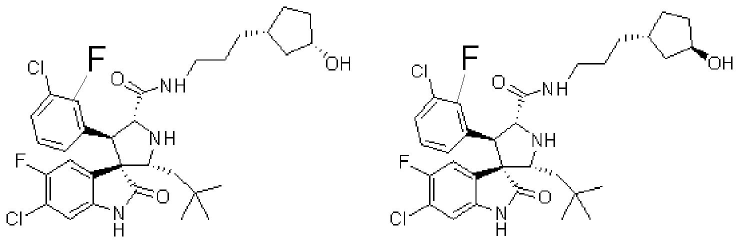

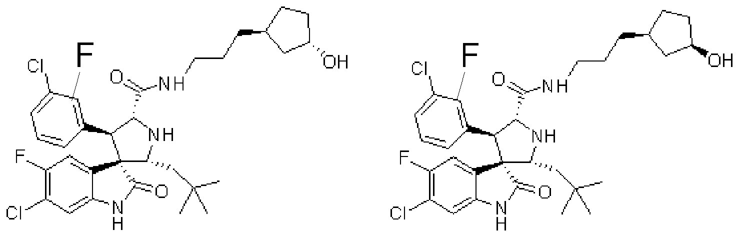

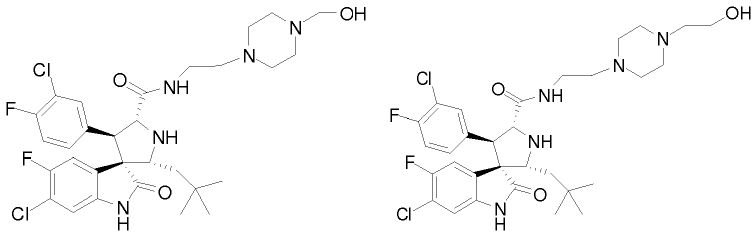

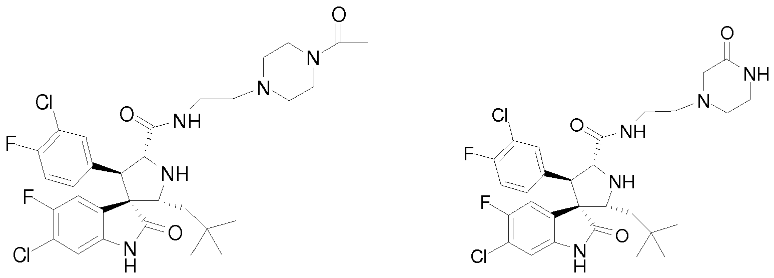

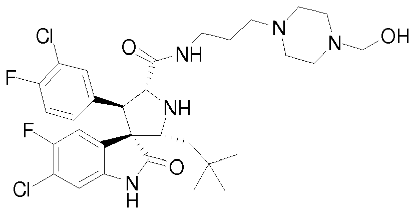

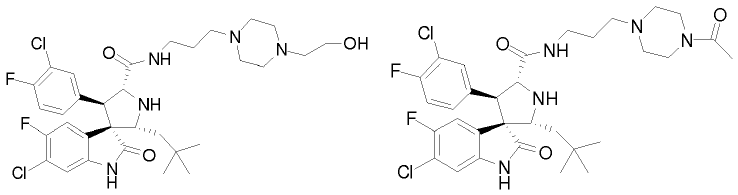

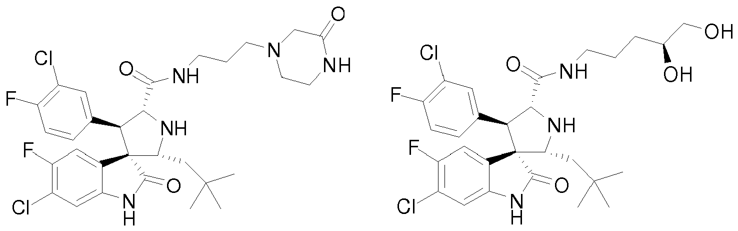

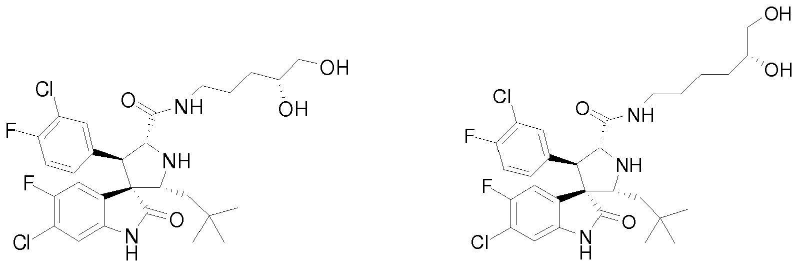

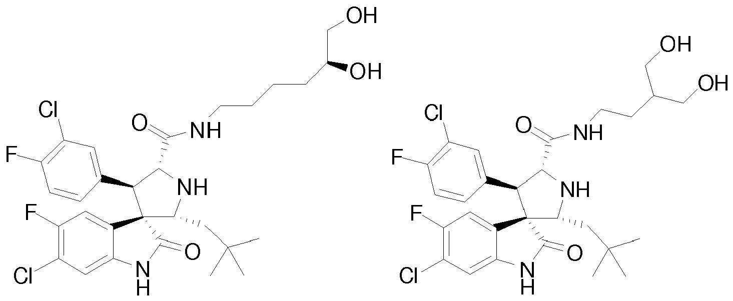

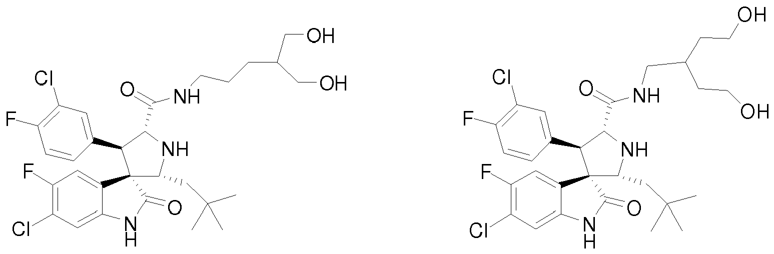

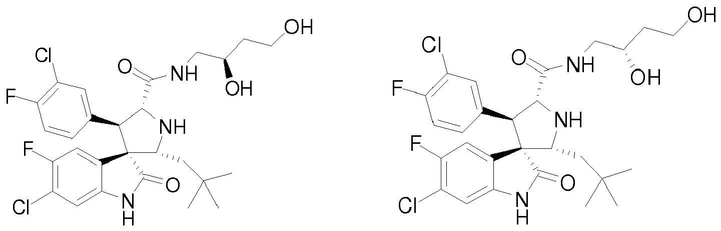

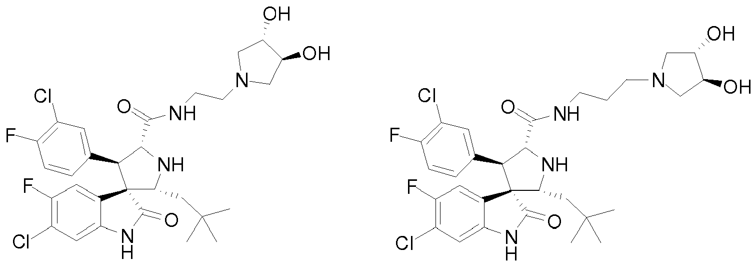

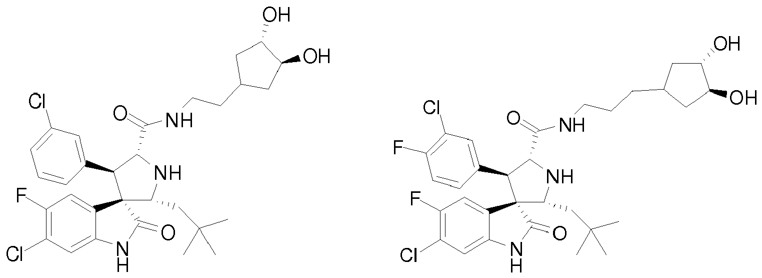

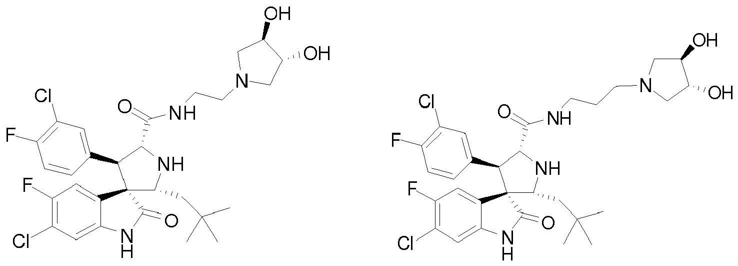

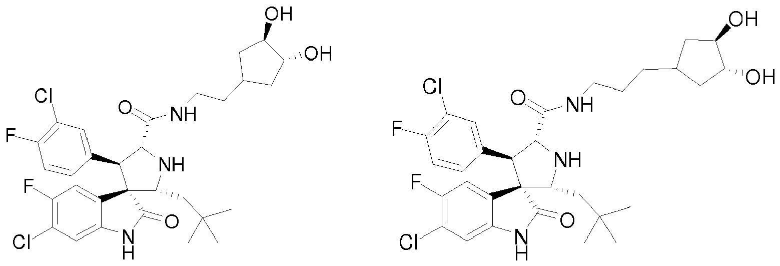

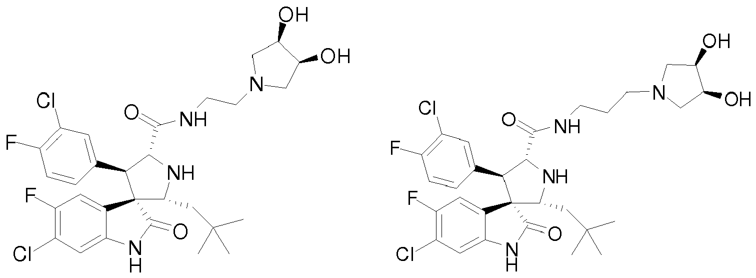

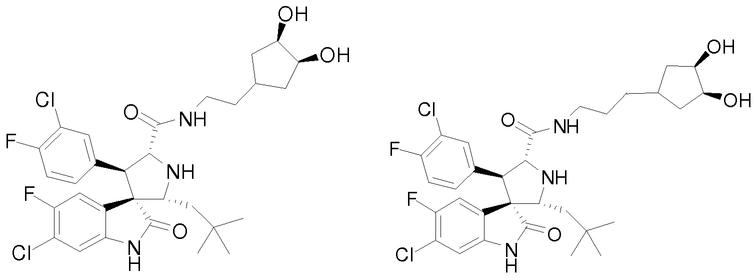

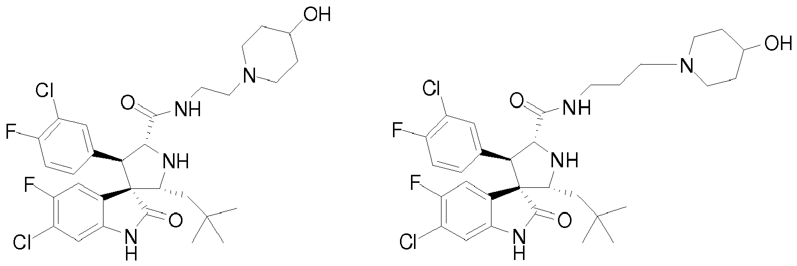

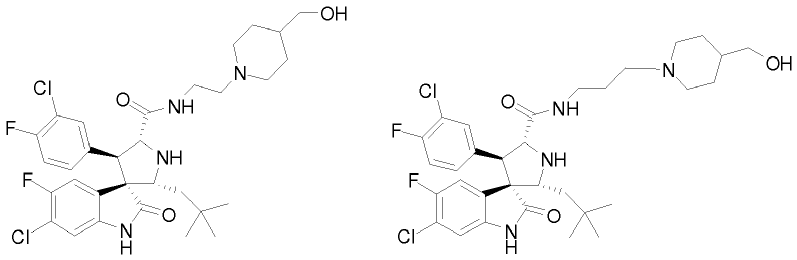

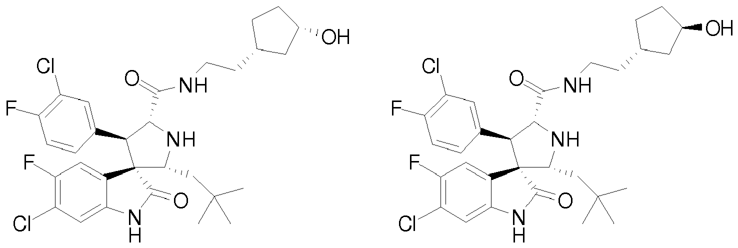

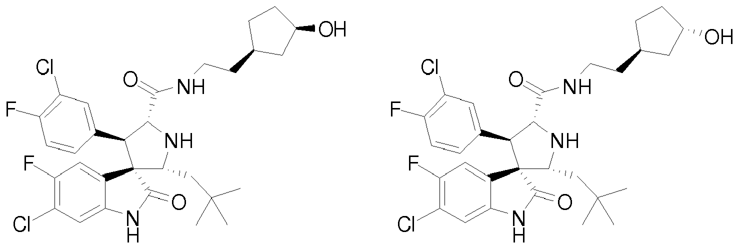

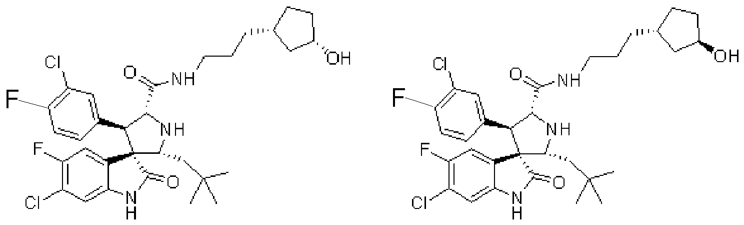

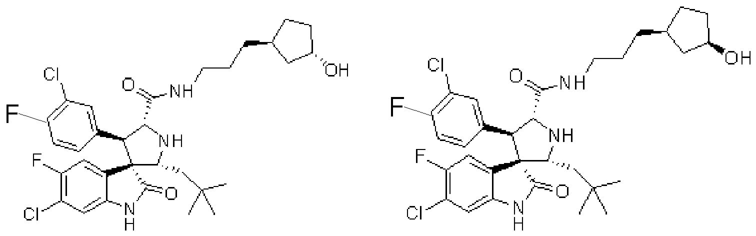

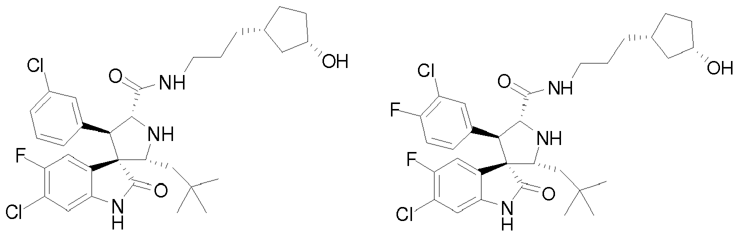

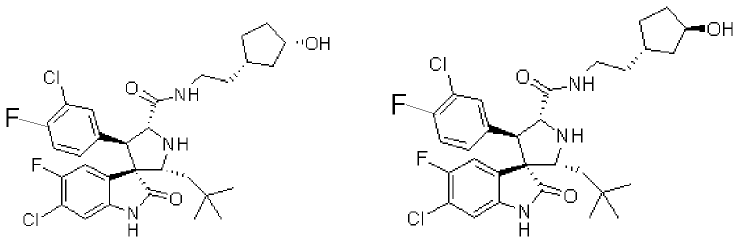

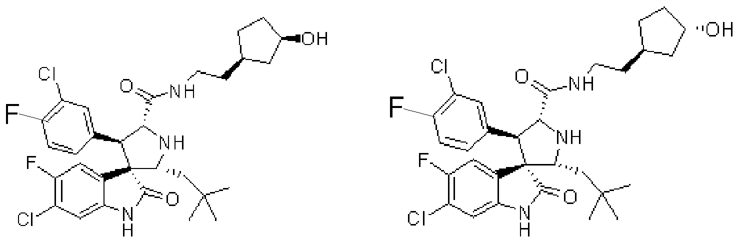

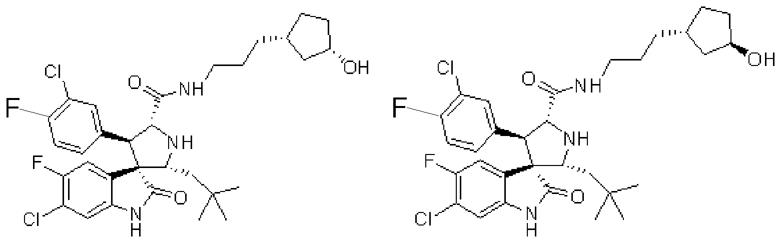

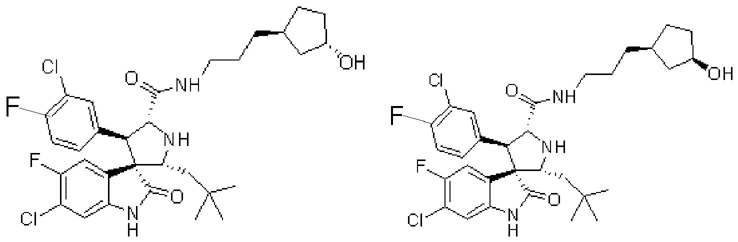

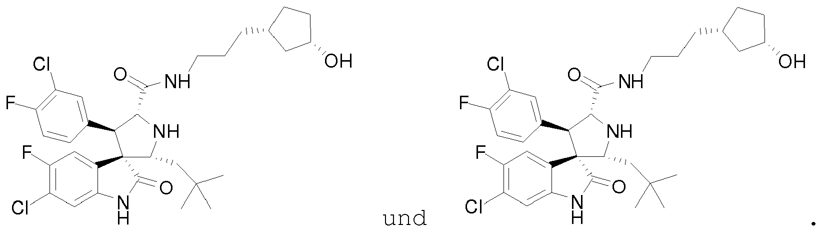

11. Stoff, ausgewählt aus der Gruppe bestehend aus: 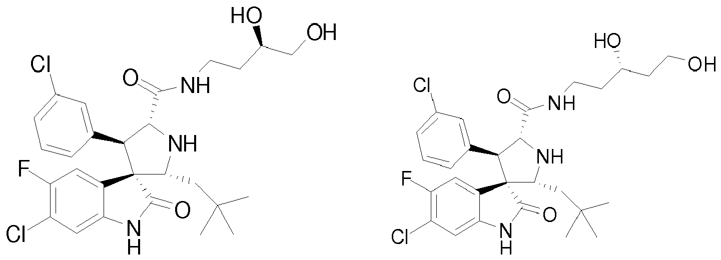 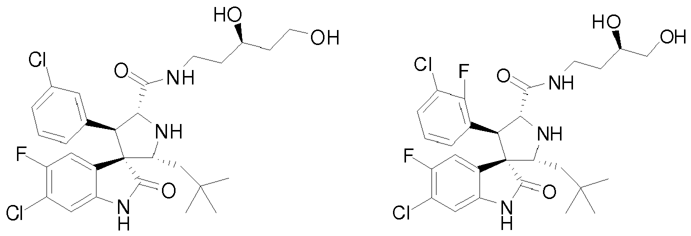 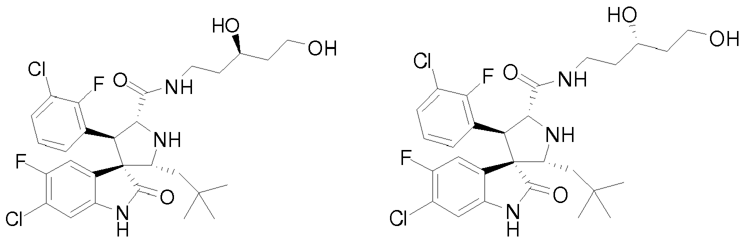 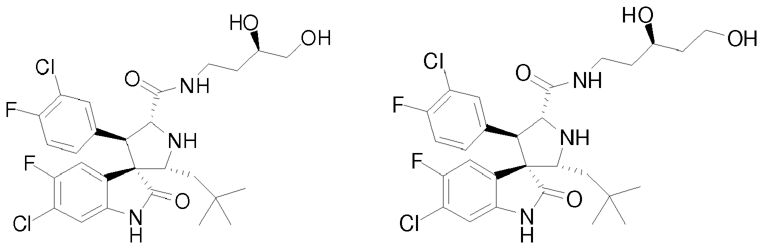 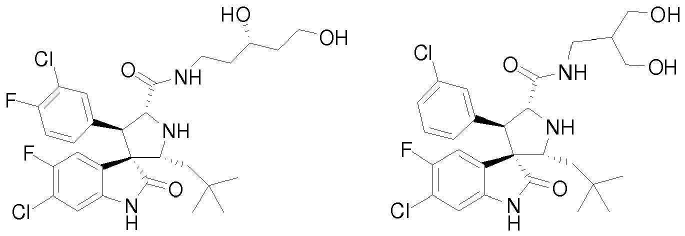 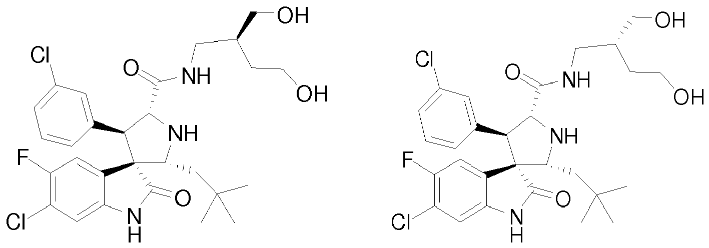 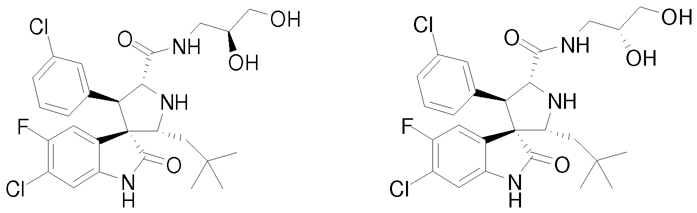 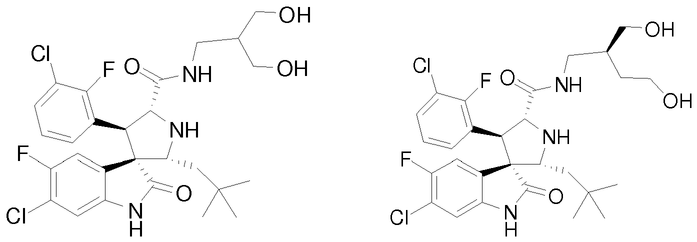 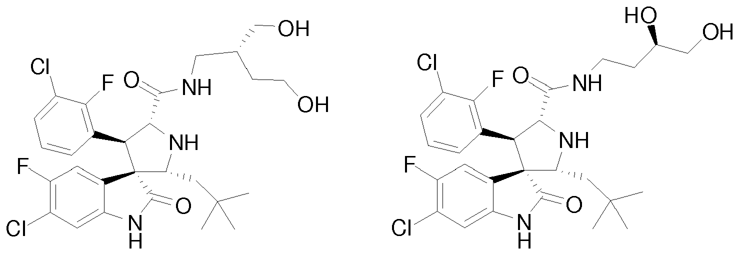 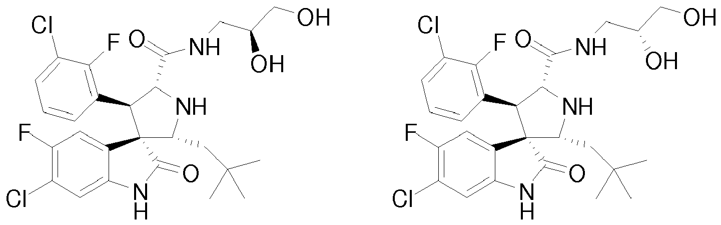 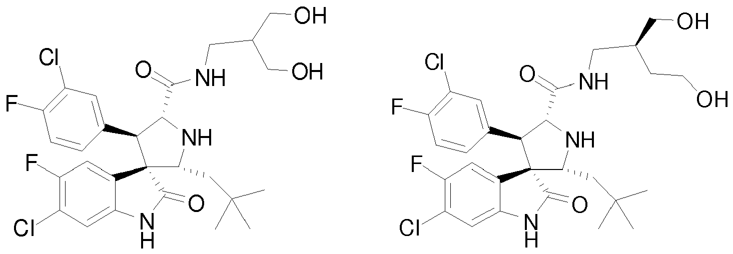 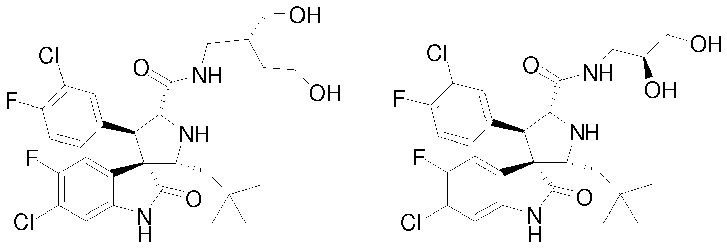 und 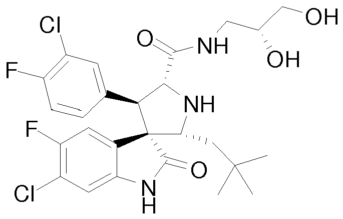 oder ein pharmazeutisch akzeptables Salz davon.

12. Stoff, der 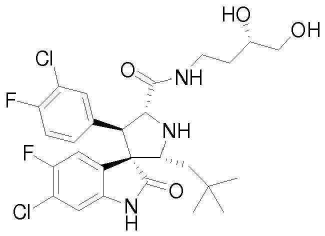 ist oder ein pharmazeutisch akzeptables Salz davon.

13. Stoff, ausgewählt aus der Gruppe bestehend aus: 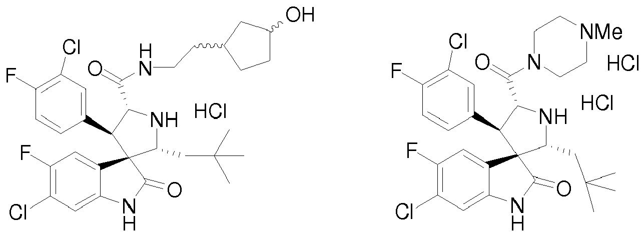 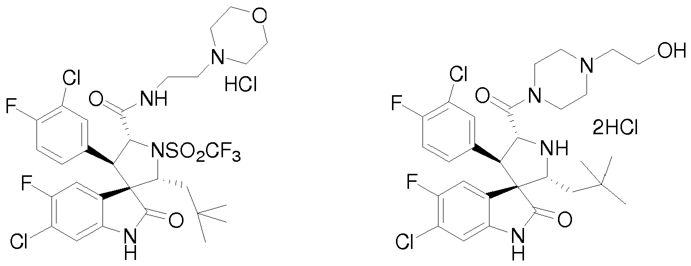 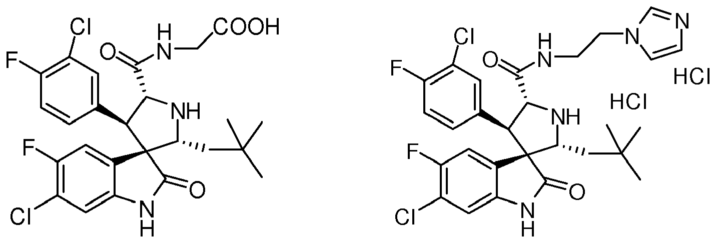 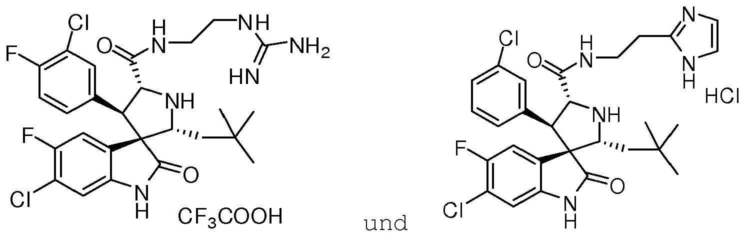 oder ein pharmazeutisch akzeptables Salz davon, oder ein anderes pharmazeutische akzeptables Salz davon.

14. Stoff, ausgewählt aus der Gruppe bestehend aus: 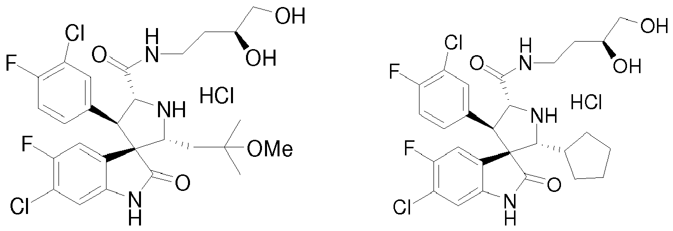 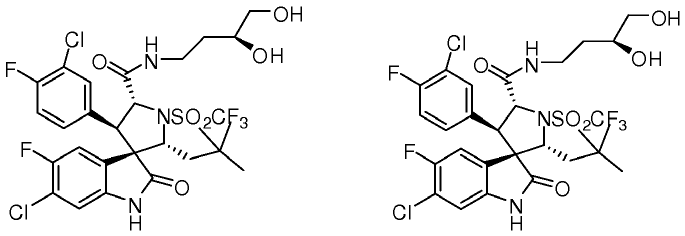 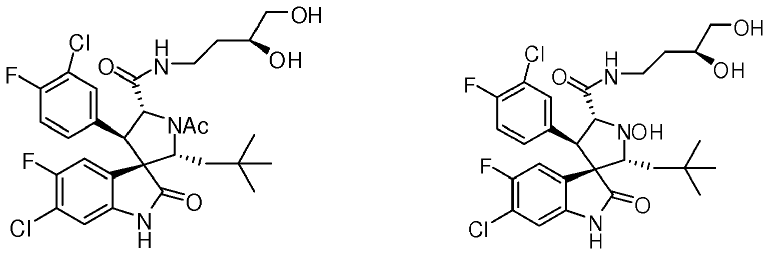 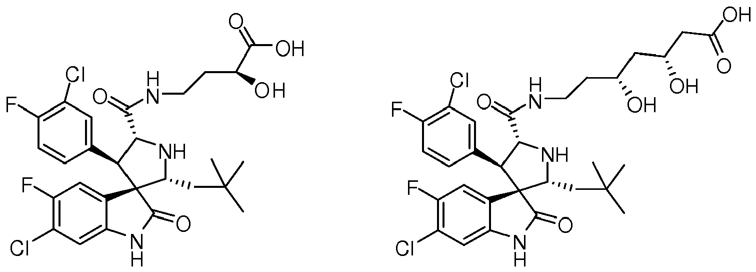 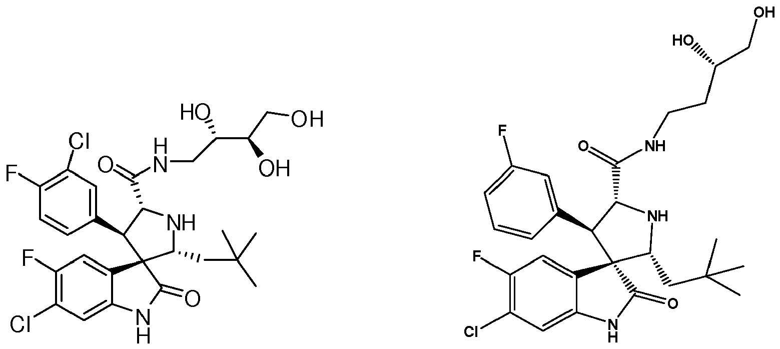 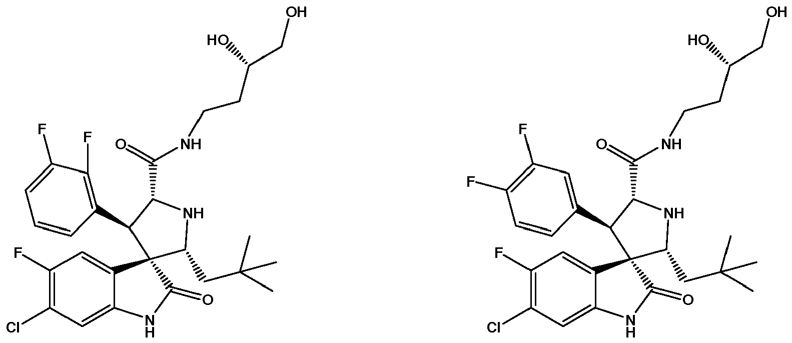 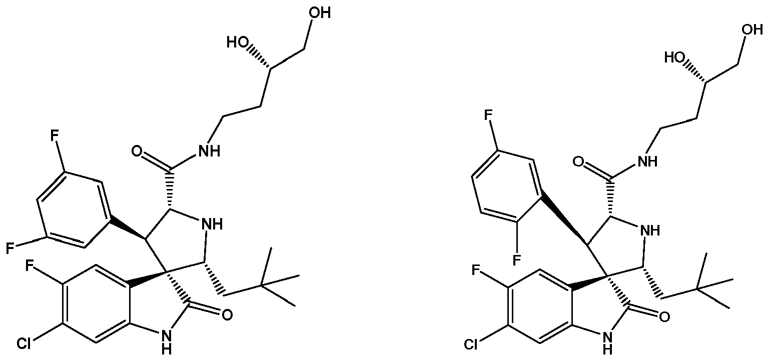 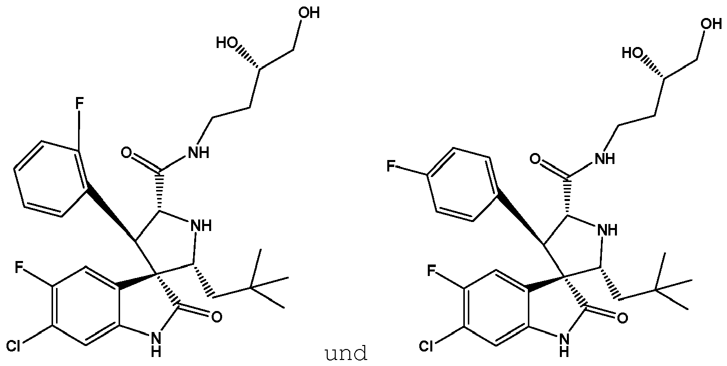 oder ein pharmazeutisch akzeptables Salz davon, oder ein anderes pharmazeutische akzeptables Salz davon.

15. Pharmazeutische Zusammensetzung umfassend einen Stoff nach einem der Ansprüche 1 bis 14 und einen pharmazeutisch akzeptablen Träger.

16. Stoff nach einem der Ansprüche 1-14 zur Verwendung in der Behandlung von Krebs.

17. Stoff zur Verwendung nach Anspruch 16, wobei der Krebs ausgewählt ist aus der Gruppe bestehend aus Prostatakrebs und Darmkrebs.

18. Stoff zur Verwendung nach Anspruch 16, wobei der Stoff mit mindestens einem weiteren therapeutischen Mittel verabreicht wird.

19. Stoff nach einem der Ansprüche 1 bis 14 zur Verwendung in der Behandlung von Mukositis, Stomatitis, Xerostomie, einer Margen-Darm-Erkrankung oder Alopezie.

## Revendications

1. Composé de formule I : 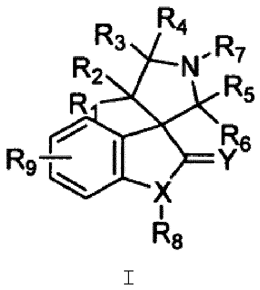 ou un sel pharmaceutiquement acceptable de celui-ci,
formule dans laquelle :
X est CH, 0, N, ou S, et R₈ est absent si X est 0 ou S ;
Y est 0, S, ou NR' ;
R₁, R₂, R₄, R₅, R₆ et R₇ sont indépendamment H ou un radical éventuellement substitué alkyle, cycloalkyle, alcényle, cycloalcényle, alcynyle, aryle, hétérocyclique, CO₂R', OCOR', CONR'R", NR"COR', NR'SO₂R", SO₂NR'R", (C=NR')NR"R"' ou NR'R" ; ou
R₇ forme un groupe aryle, cycloalkyle ou hétérocyclique avec l'un de R₅ et R₆ ;
R₈ est H ou un radical éventuellement substitué alkyle, cycloalkyle, alcényle, cycloalcényle, alcynyle, aryle, hétérocyclique, CO₂R', OCOR', CONR'R", SO₂NR'R", ou (C=NR')NR"R"' ;
R₉ représente un groupe 6-chloro ou 5-fluoro ; et
chaque R', R" et R"' est indépendamment H ou un radical éventuellement substitué alkyle, cycloalkyle, alcényle, cycloalcényle, alcynyle, aryle, ou hétérocyclique ; ou bien R' et R" ou R" et R"' forment un cycle ;
R₃ est CONRR', et R est un groupe cycloalkyl-alkyle ou monocyclo-hétérocycloalkyle éventuellement substitué ou un groupe dihydroxyalkyle ne contenant pas de groupe hydroxyle à la position 3 du groupe alkyle.

2. Composé selon la revendication 1 de formule II ou de formule III : 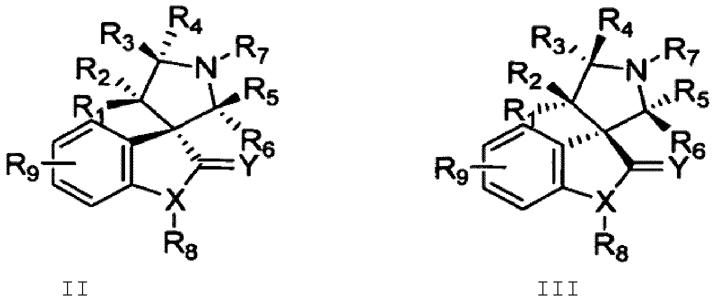 ou un sel pharmaceutiquement acceptable de celui-ci.

3. Composé selon la revendication 1 de formule VII : 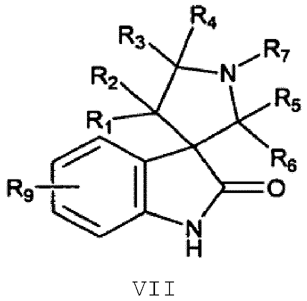 ou un sel pharmaceutiquement acceptable de celui-ci.

4. Composé selon la revendication 3 de formule VIII ou de formule IX : 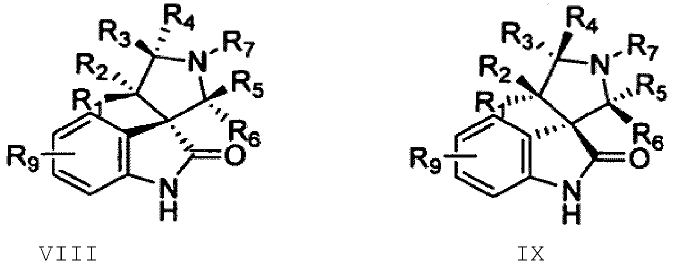 ou un sel pharmaceutiquement acceptable de celui-ci.

5. Composé selon la revendication 1, dans lequel l'un de R₁ et R₂ est un aryle ou hétéroaryle éventuellement substitué.

6. Composé selon la revendication 1, dans lequel l'un de R₁ et R₂ est un cycloalkyle, un alkyle linéaire ou ramifié, CONR'R" ou CO₂R'.

7. Composé selon la revendication 1, dans lequel l'un de R₅ et R₆ est un alkyle en C₃ à C₁₈, aryle, ou hétéroaryle.

8. Composé de formule XXVII ou de formule XXVIII : 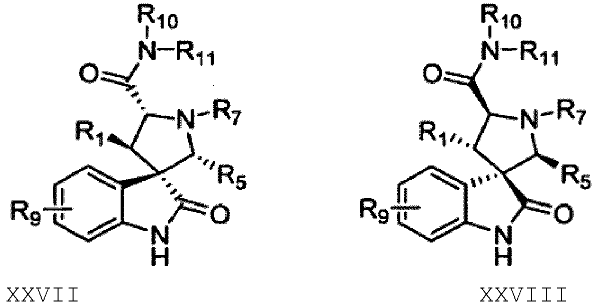 ou un sel pharmaceutiquement acceptable de celui-ci,
formule dans laquelle :
R₁, R₅ et R₇ sont indépendamment H ou un radical éventuellement substitué alkyle, cycloalkyle, alcényle, cycloalcényle, alcynyle, aryle, hétérocyclique, CO₂R', OCOR', CONR'R", NR"COR', NR'SO₂R", SO₂NR'R", (C=NR')NR"R"' ou NR'R" ; ou
R₇ forme un groupe aryle, cycloalkyle ou hétérocyclique avec R₅ ;
R₉ représente un groupe 6-chloro ou 5-fluoro ;
chaque R', R" et R"' est indépendamment H ou un radical éventuellement substitué alkyle, cycloalkyle, alcényle, cycloalcényle, alcynyle, aryle, ou hétérocyclique ; ou bien R' et R" ou R" et R"' forment un cycle ;
R₁₀ est l'hydrogène ; et
R₁₁ est un groupe cycloalkyl-alkyle ou monocyclo-hétérocycloalkyle éventuellement substitué ou un groupe dihydroxyalkyle ne contenant pas de groupe hydroxyle à la position 3 du groupe alkyle ;
ou bien R₁₀ et R₁₁ forment ensemble un groupe monocyclo-hétérocycloalkyle éventuellement substitué.

9. Composé de formule LXVI ou LXVII : 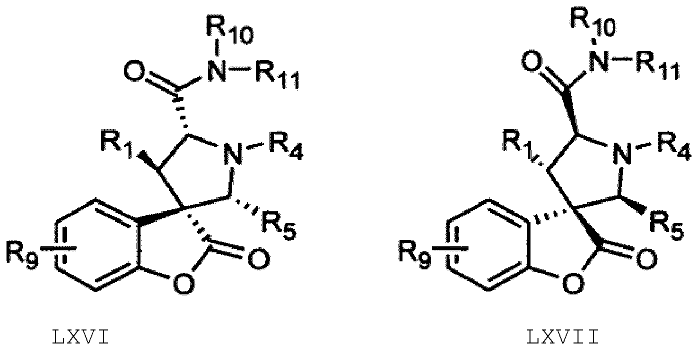 ou un sel pharmaceutiquement acceptable de celui-ci,
formule dans laquelle :
R₁, R₄ et R₅ sont indépendamment H ou un radical éventuellement substitué alkyle, cycloalkyle, alcényle, cycloalcényle, alcynyle, aryle, hétérocyclique, CO₂R', OCOR', CONR'R", NR"COR', NR'SO₂R", SO₂NR'R", (C=NR')NR"R"' ou NR'R" ; ou
R₄ forme un groupe aryle, cycloalkyle ou hétérocyclique avec R₅ ;
R₉ représente un groupe 6-chloro ou 5-fluoro ;
chaque R', R" et R"' est indépendamment H ou un radical éventuellement substitué alkyle, cycloalkyle, alcényle, cycloalcényle, alcynyle, aryle, ou hétérocyclique ; ou bien R' et R" ou R" et R"' forment un cycle ;
R₁₀ est l'hydrogène ; et
R₁₁ est un groupe cycloalkyl-alkyle ou monocyclo-hétérocycloalkyle éventuellement substitué ou un groupe dihydroxyalkyle ne contenant pas de groupe hydroxyle à la position 3 du groupe alkyle ;
ou bien R₁₀ et R₁₁ forment ensemble un groupe monocyclo-hétérocycloalkyle éventuellement substitué.

10. Composé choisi dans le groupe constitué par les suivants :

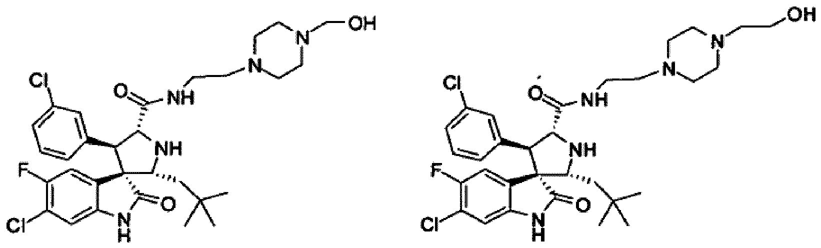

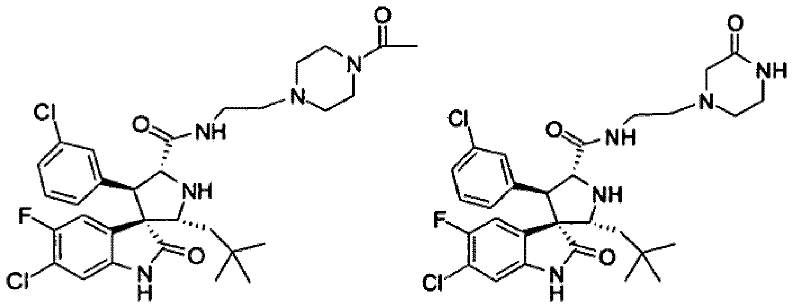

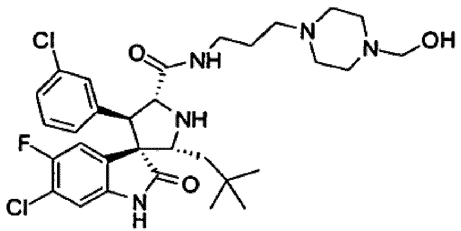

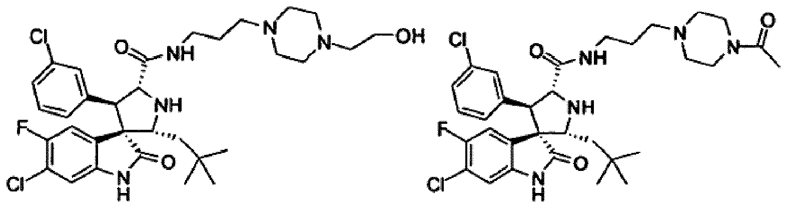

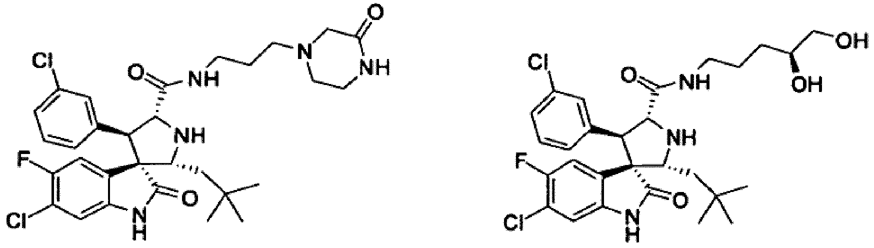

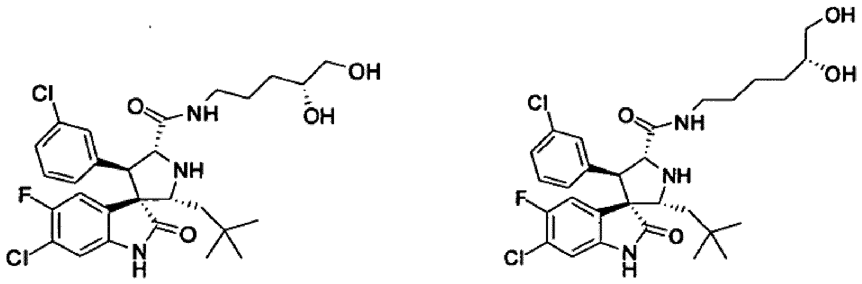

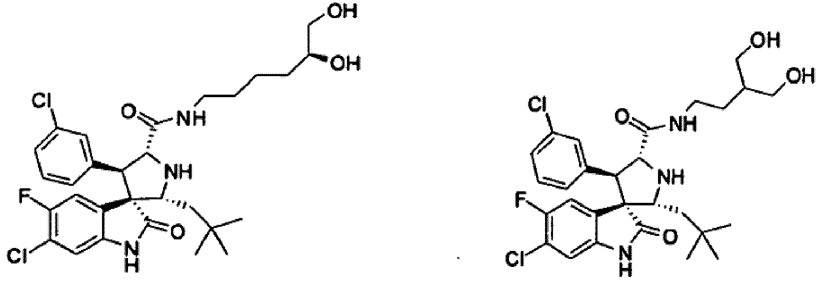

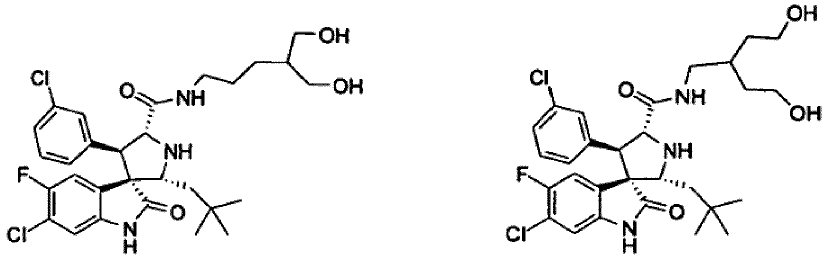

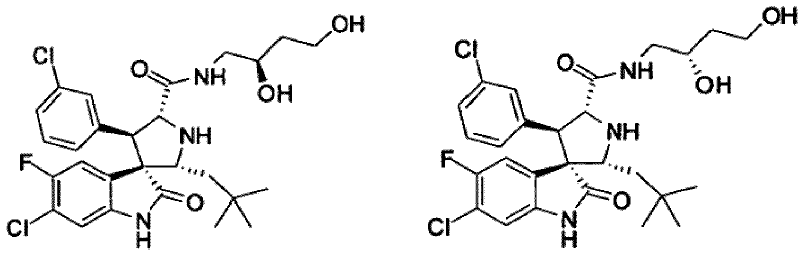

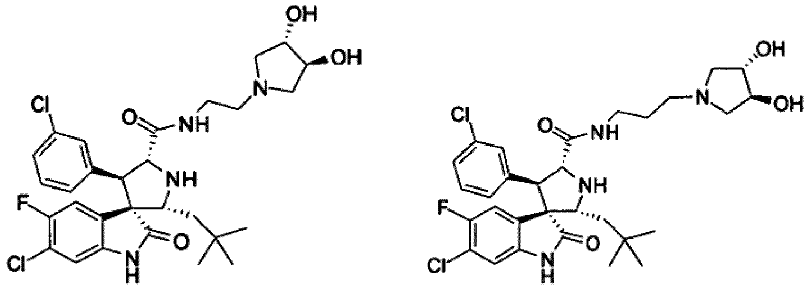

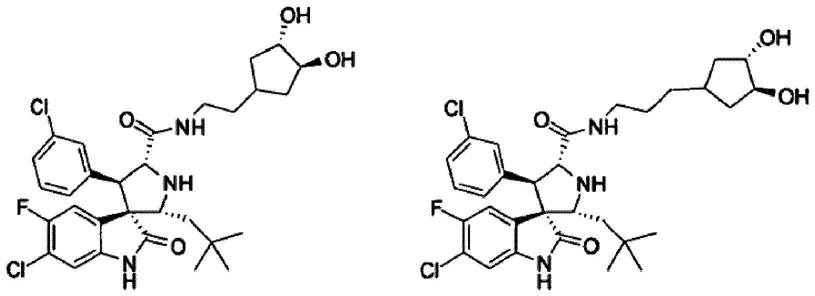

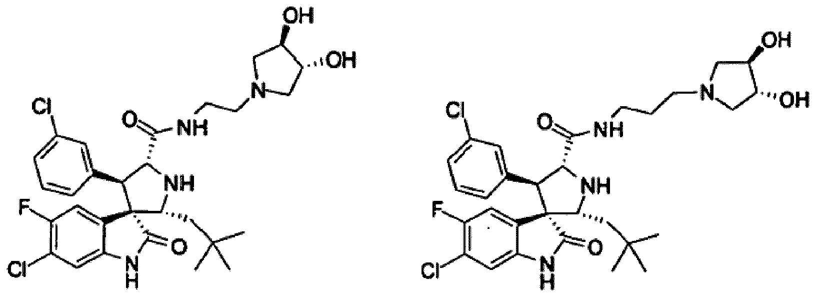

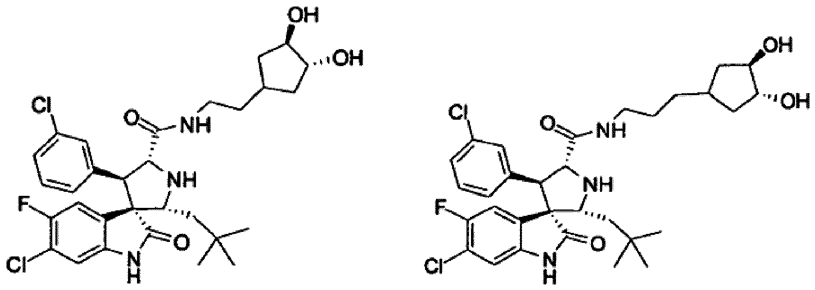

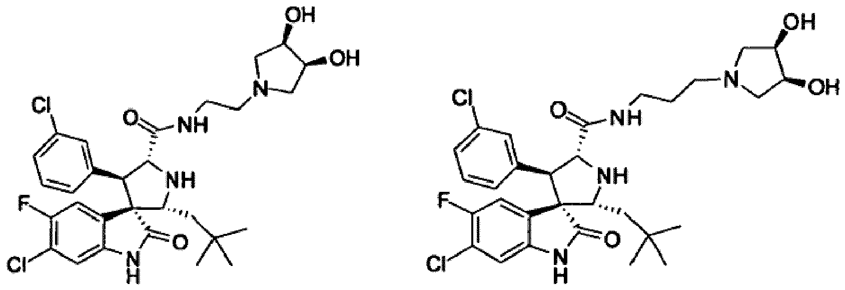

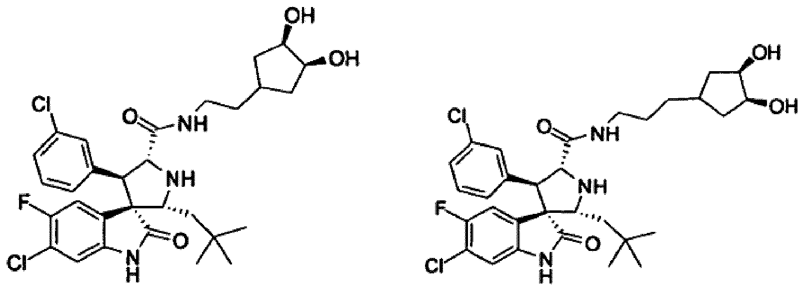

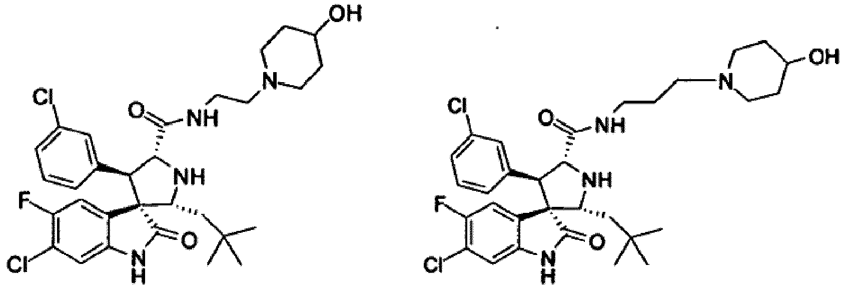

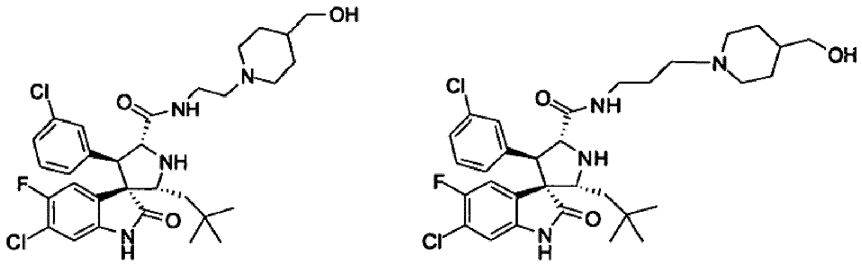

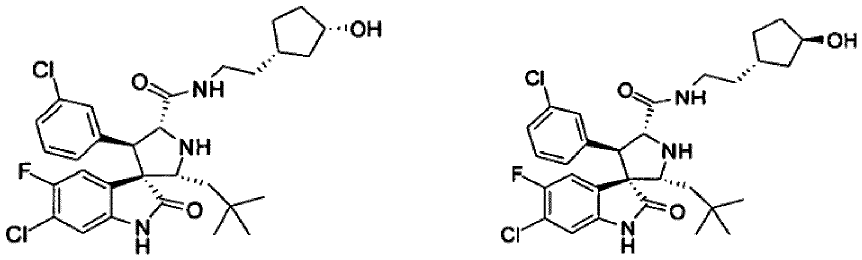

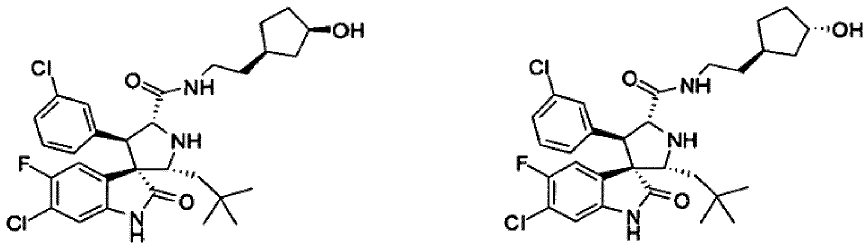

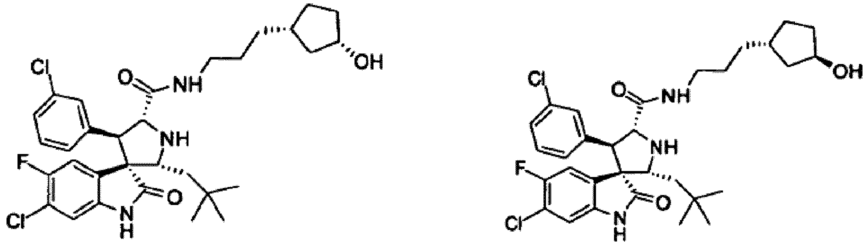

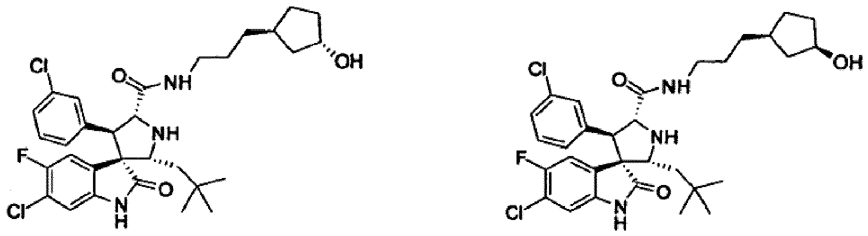

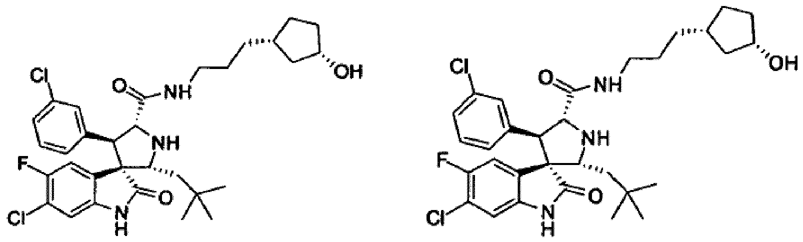

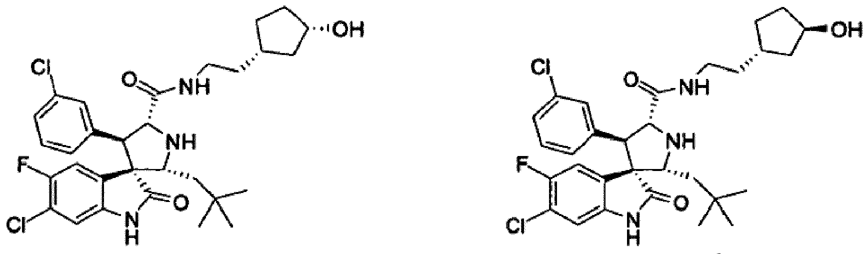

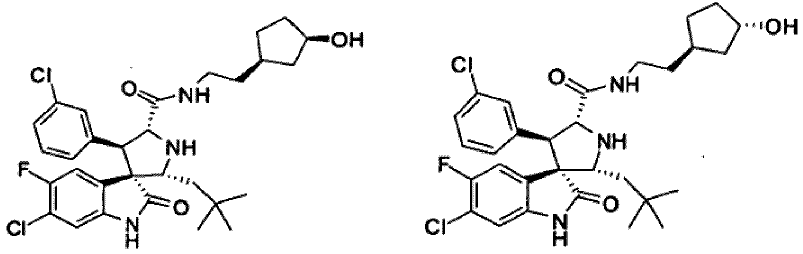

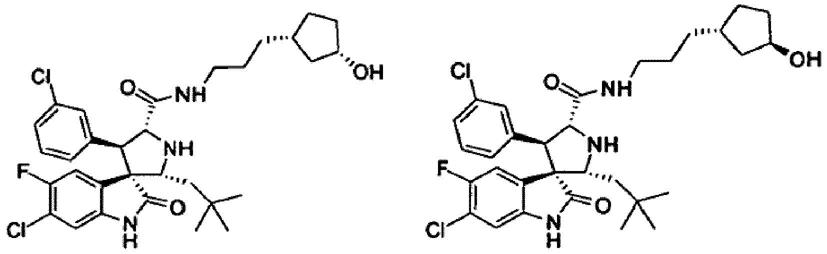

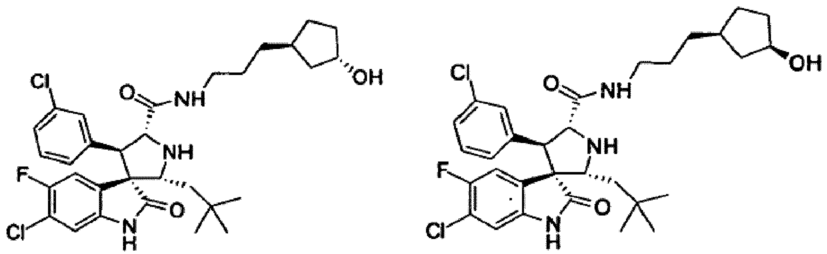

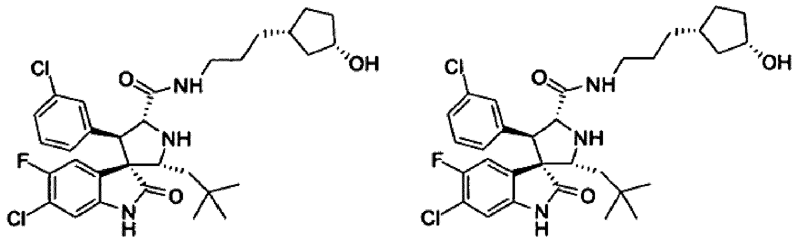

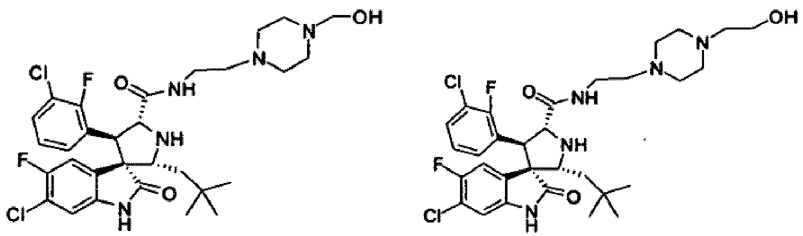

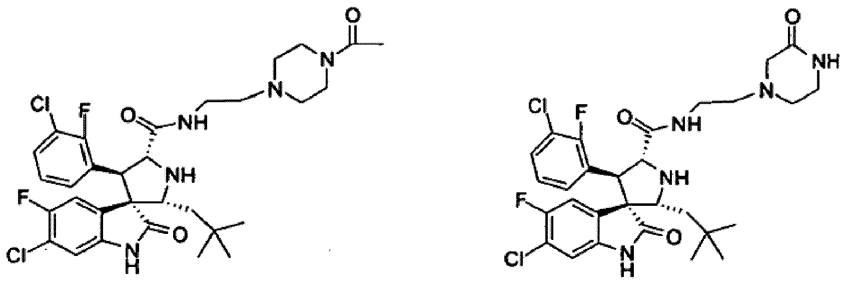

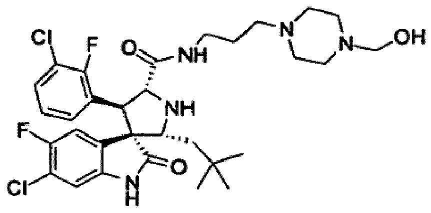

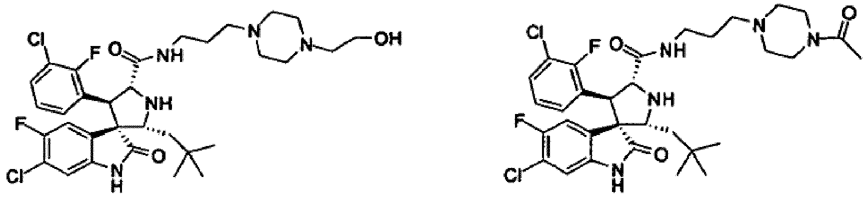

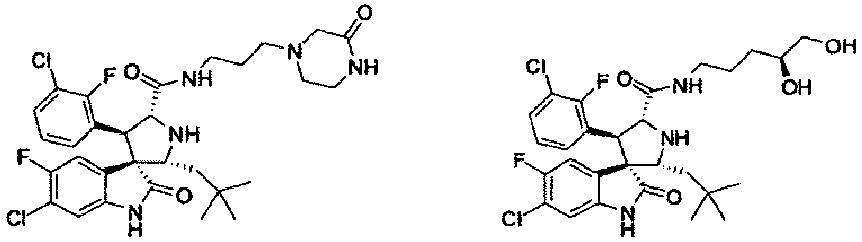

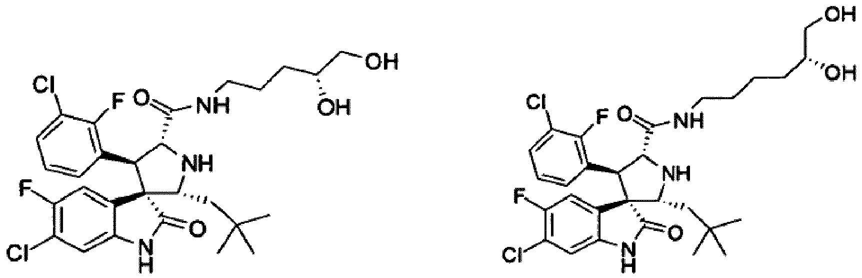

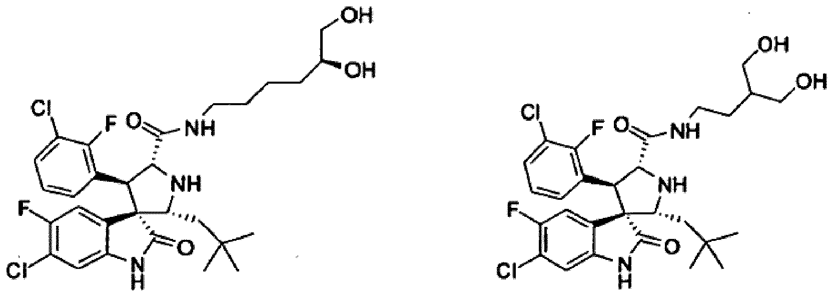

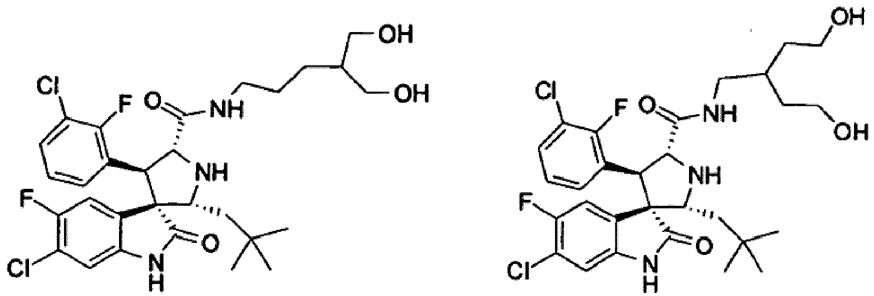

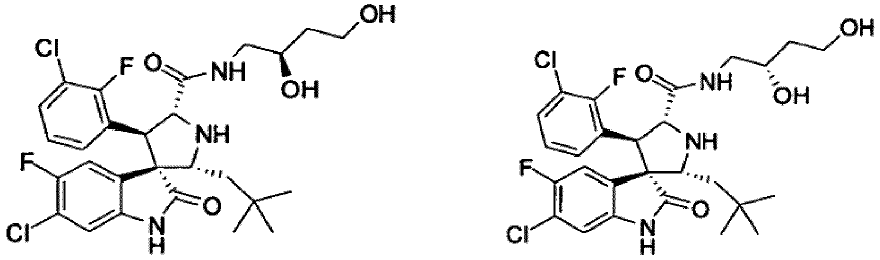

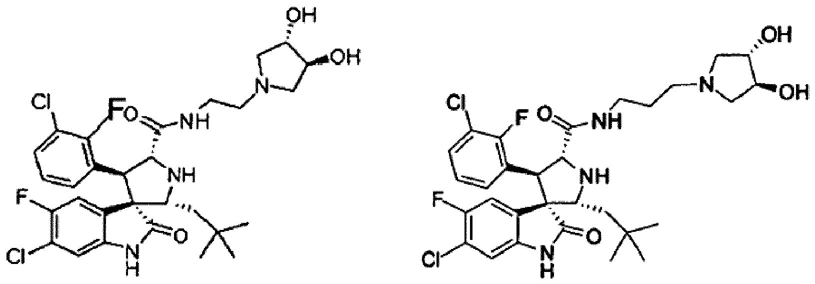

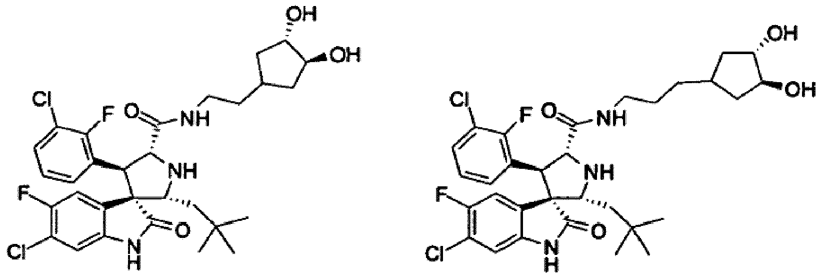

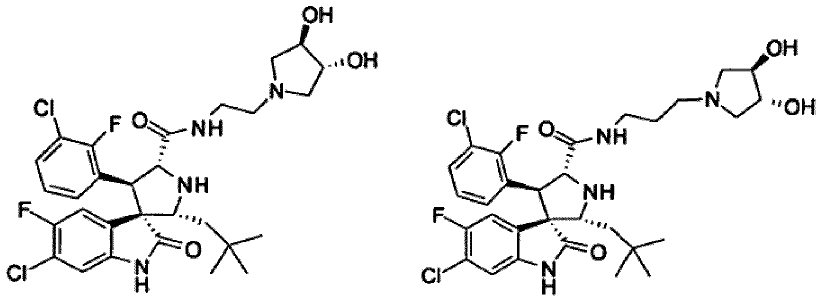

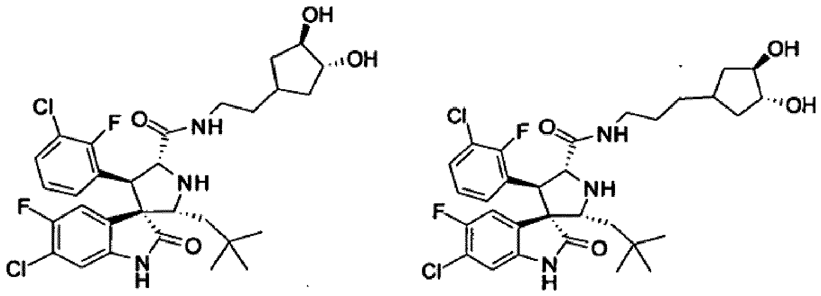

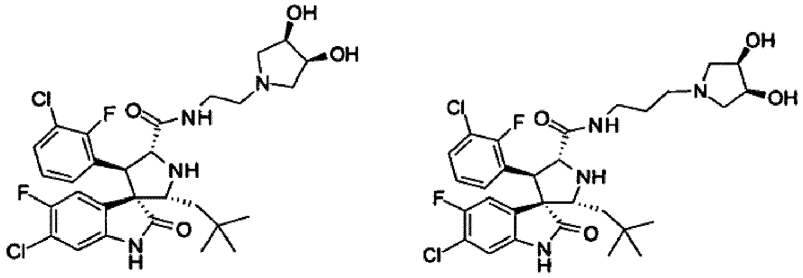

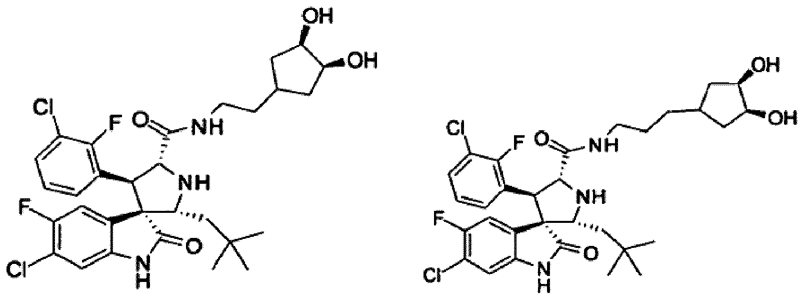

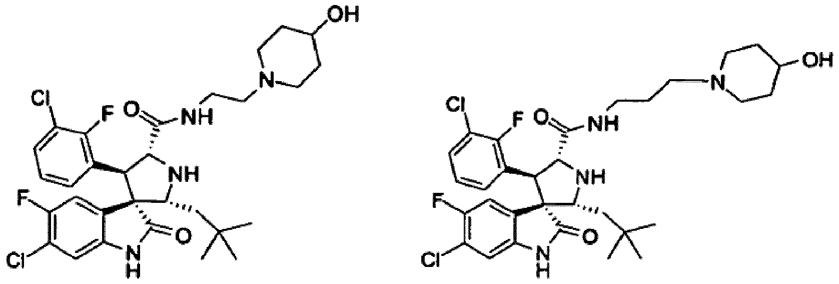

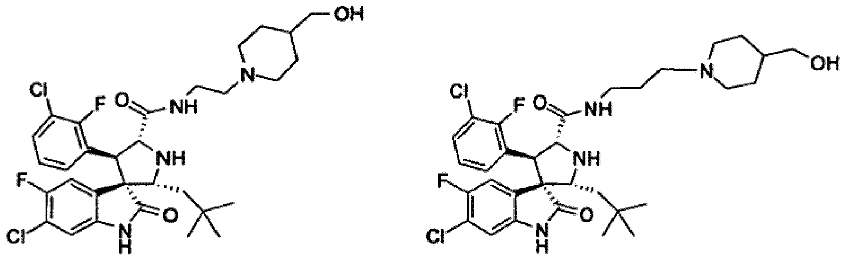

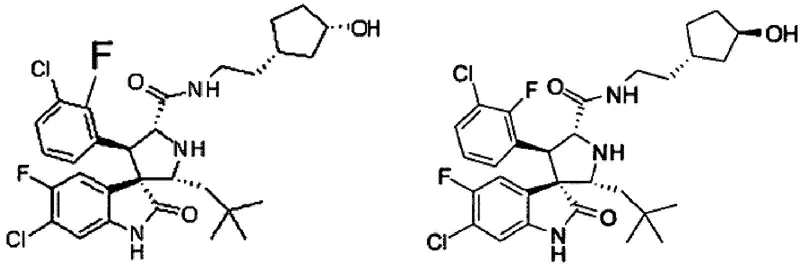

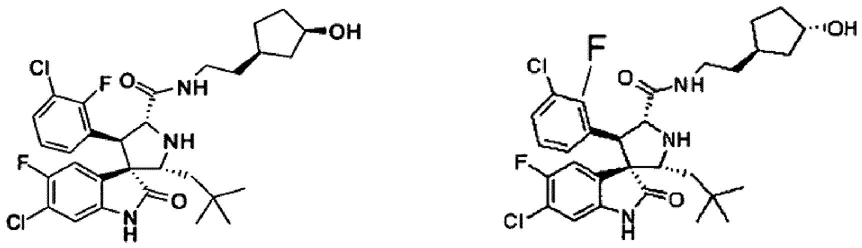

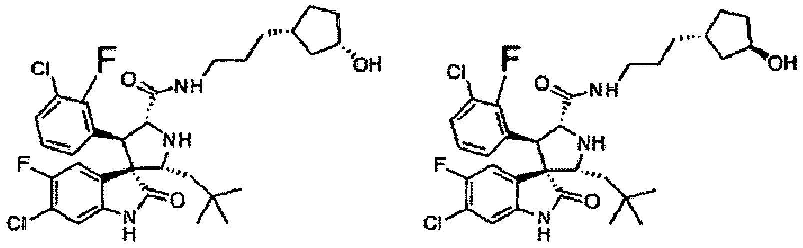

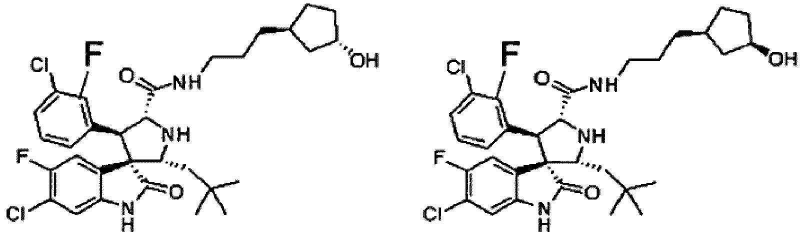

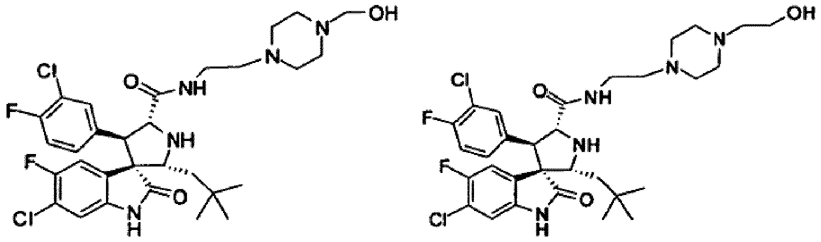

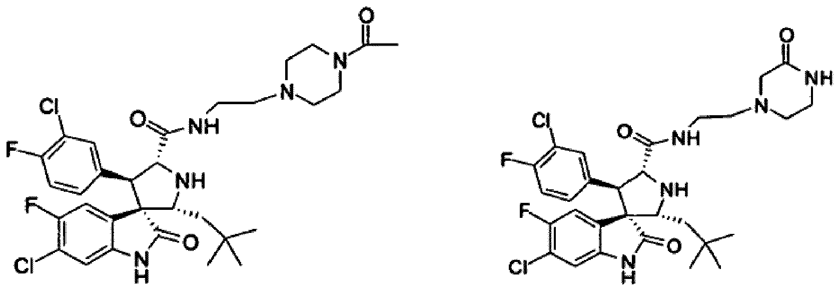

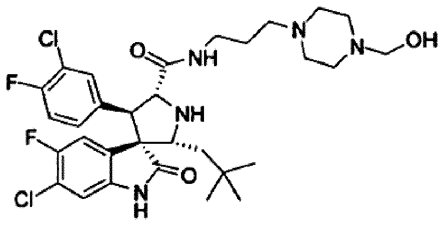

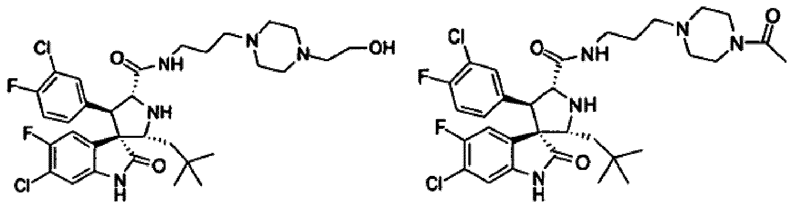

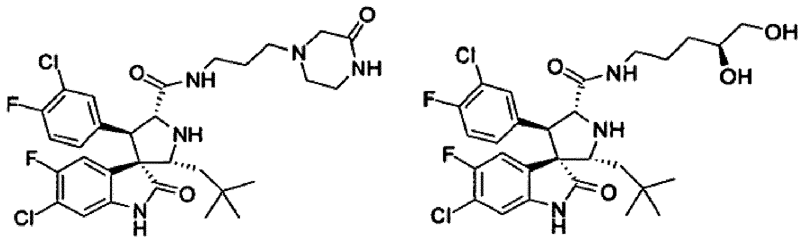

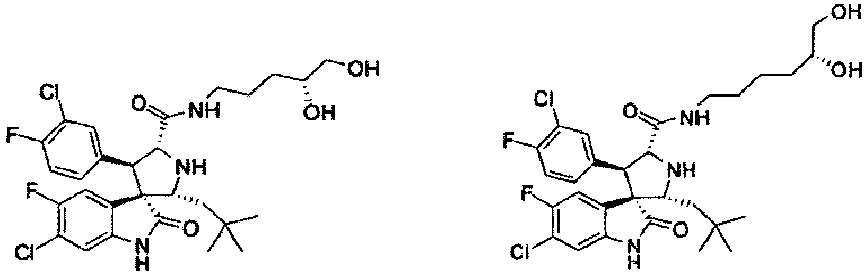

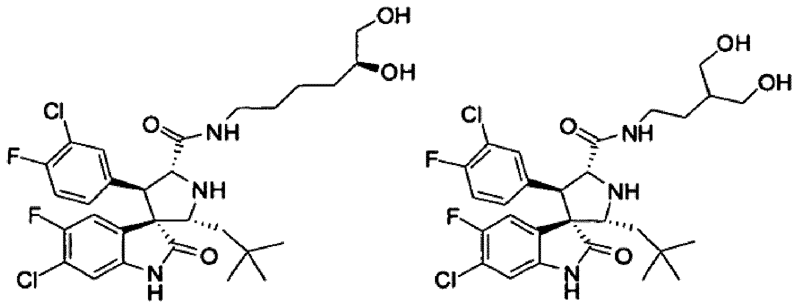

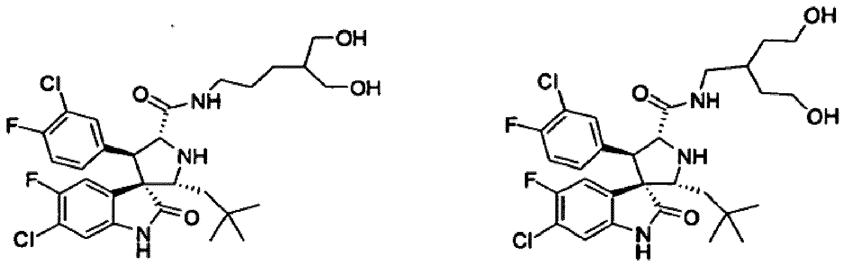

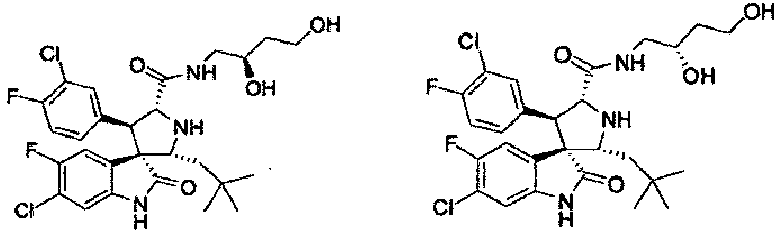

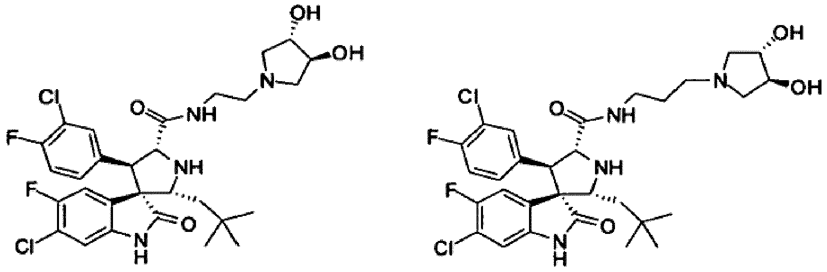

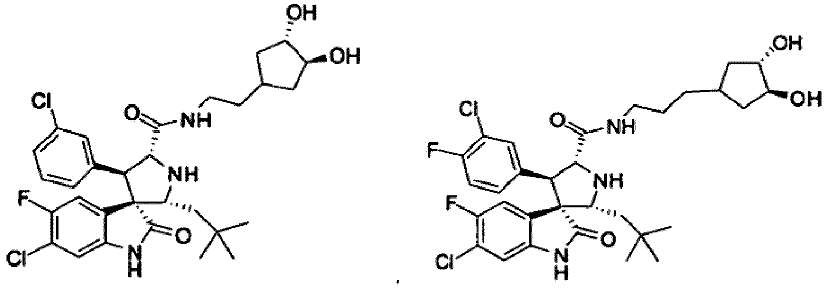

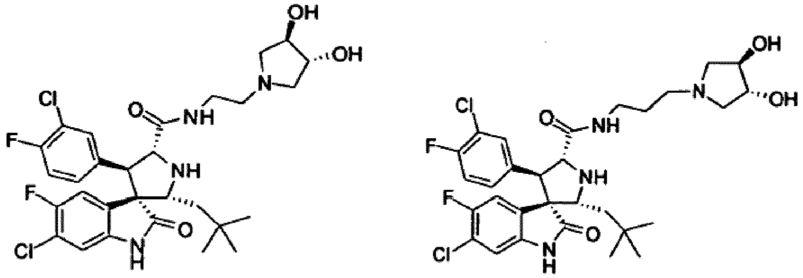

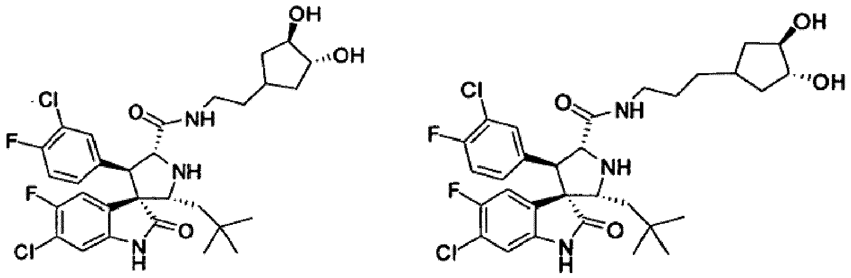

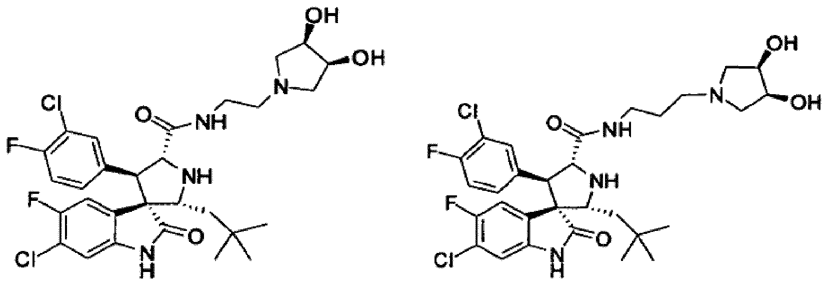

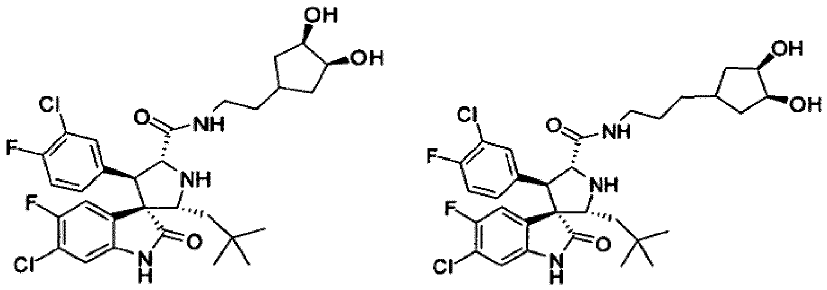

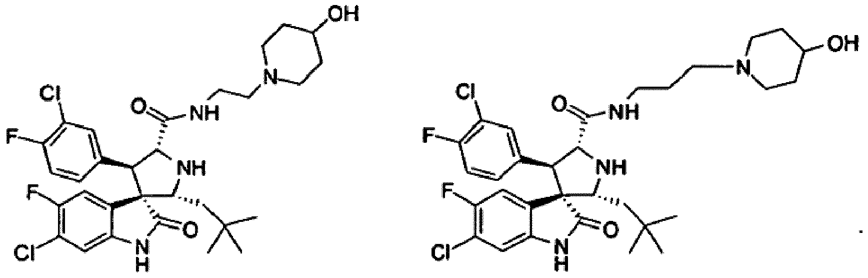

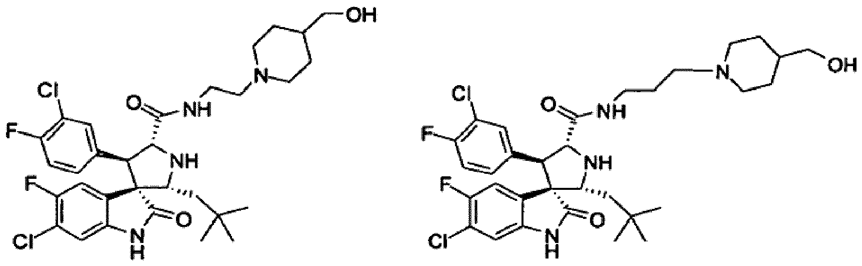

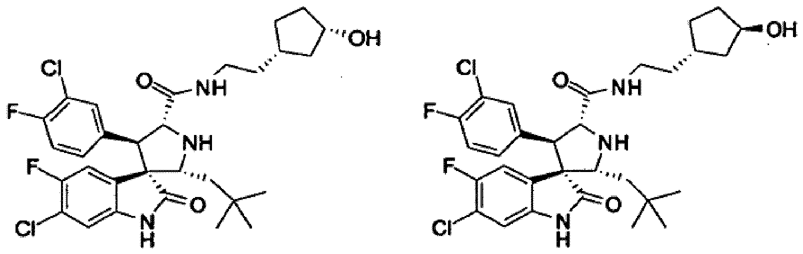

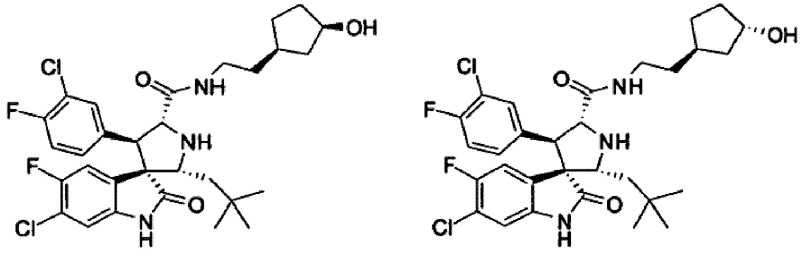

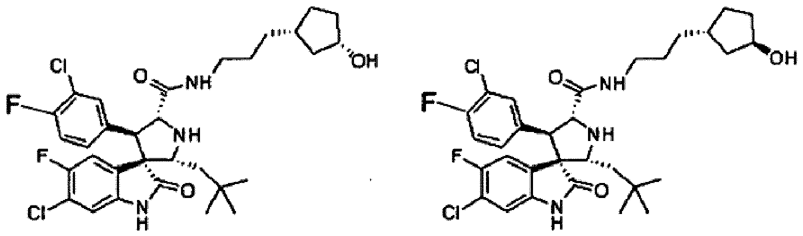

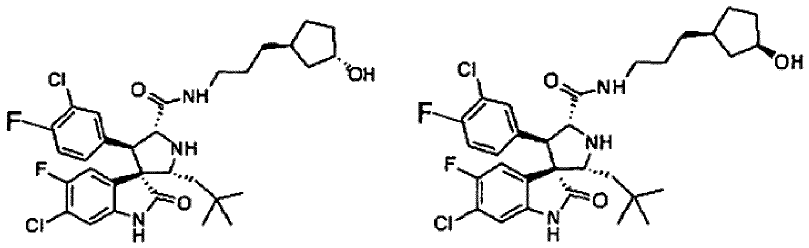

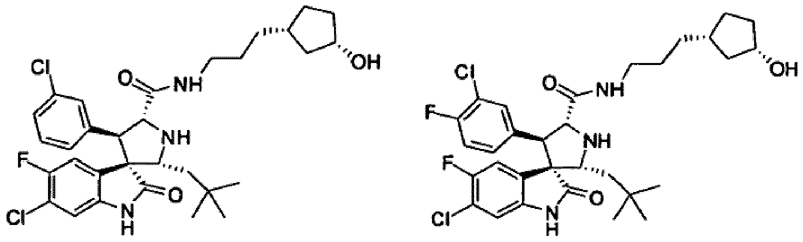

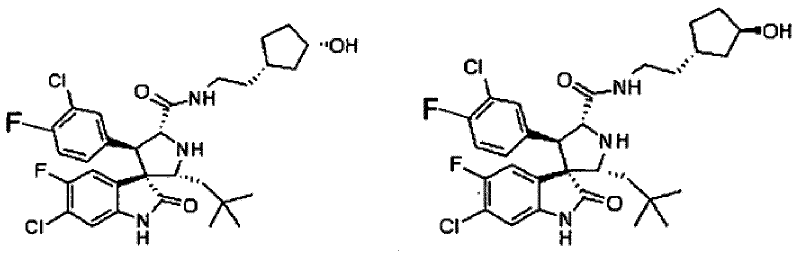

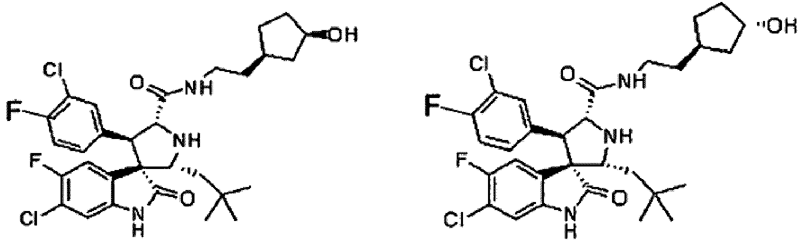

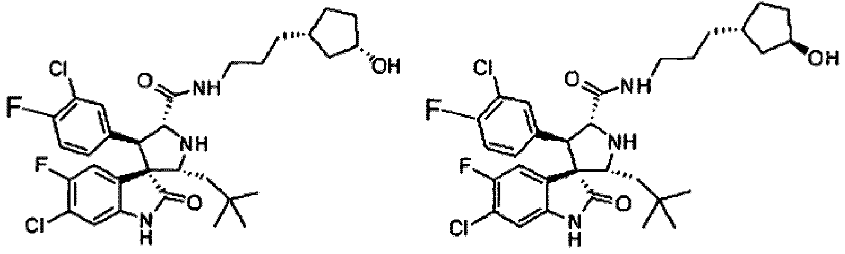

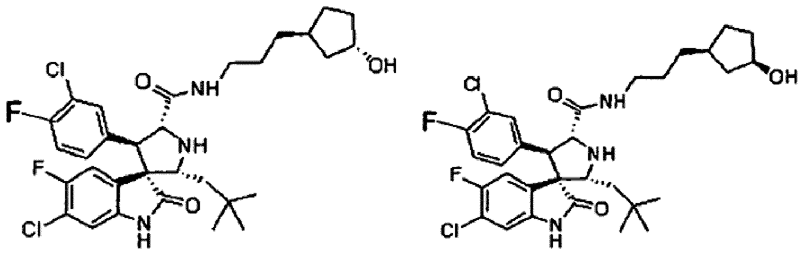

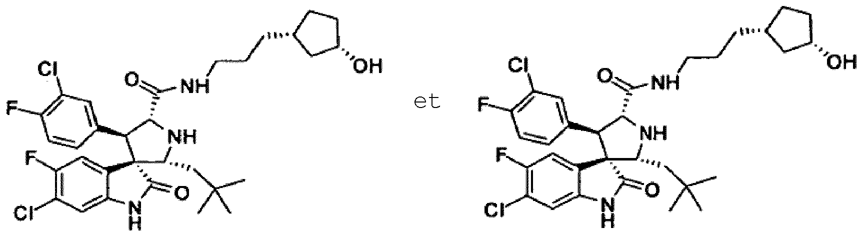

11. Composé choisi dans le groupe constitué par les suivants : 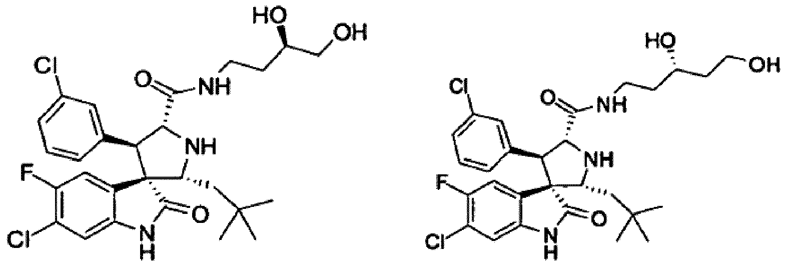 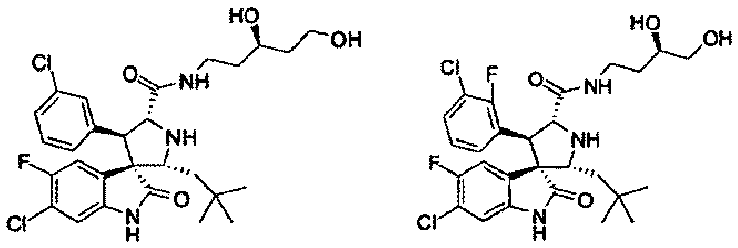 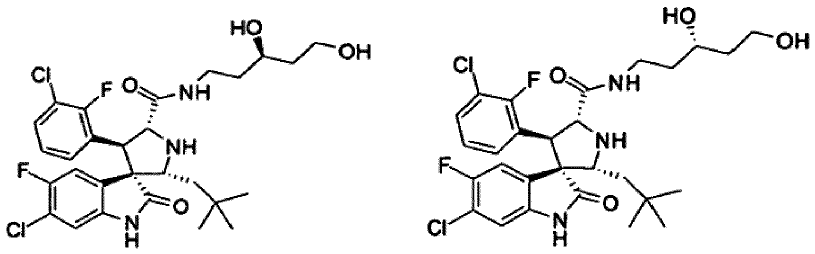 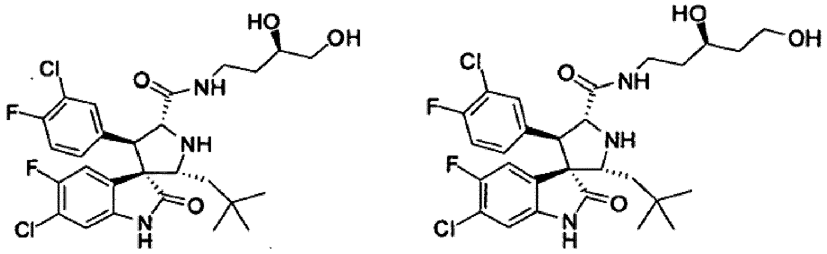 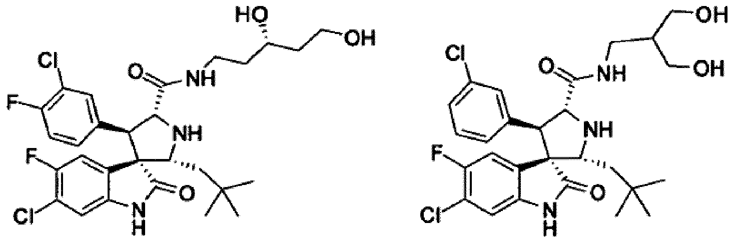 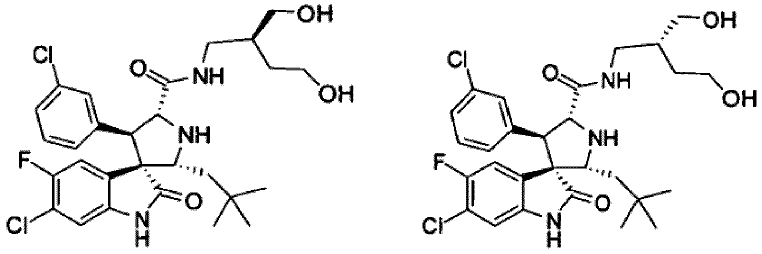 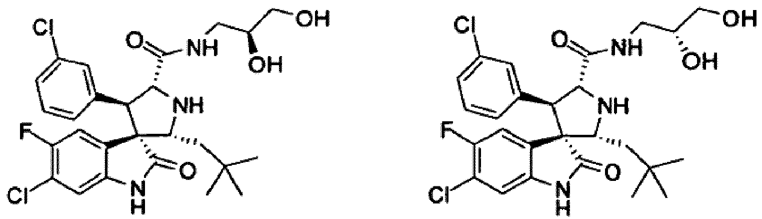 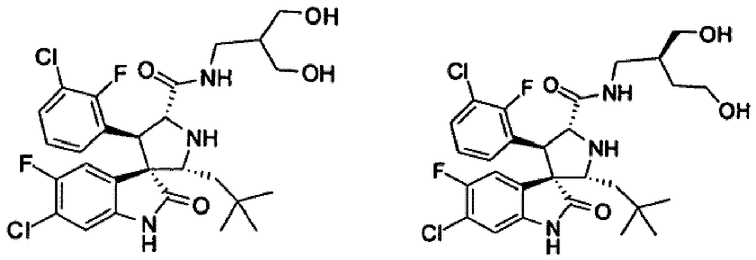 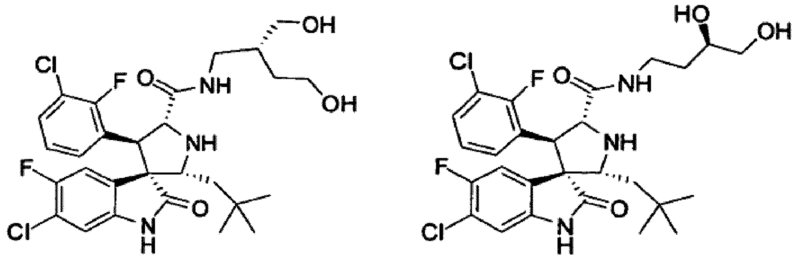 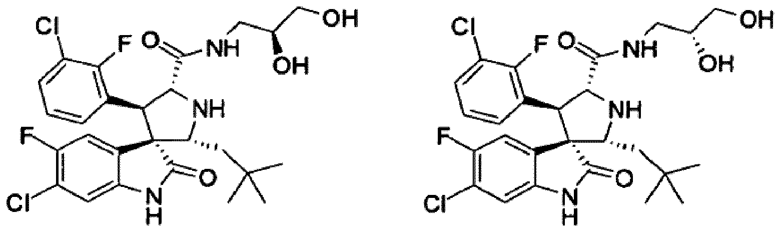 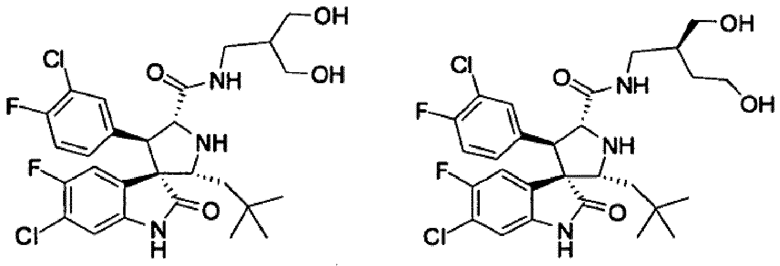 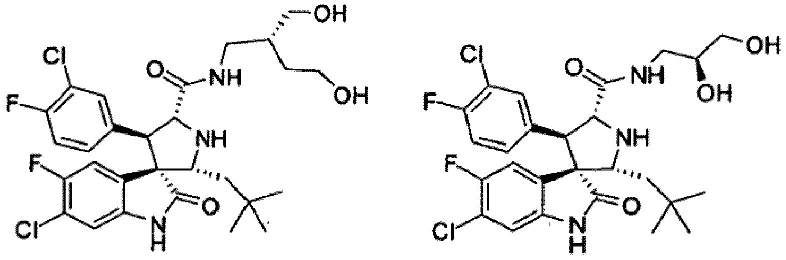 et 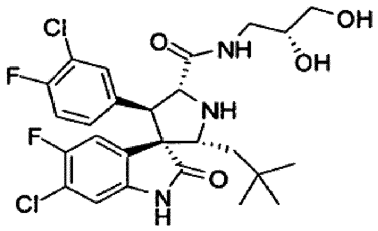 ou un sel pharmaceutiquement acceptable d'un tel composé.

12. Composé qui est le suivant : 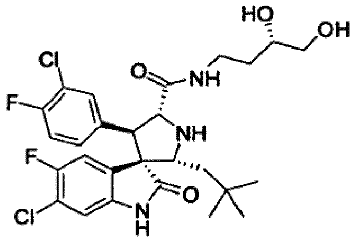 ou un sel pharmaceutiquement acceptable de celui-ci.

13. Composé choisi dans le groupe constitué par les suivants : 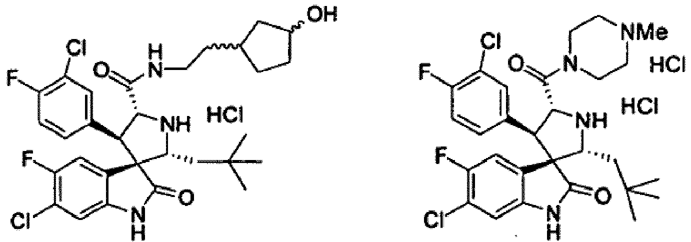 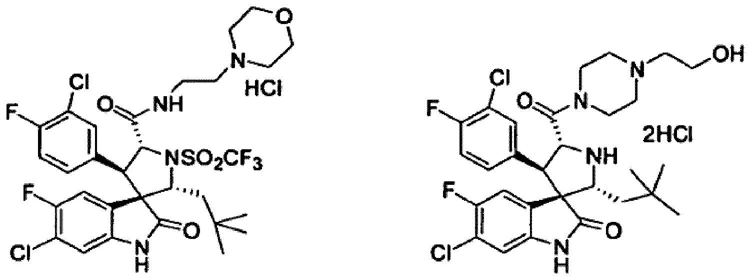 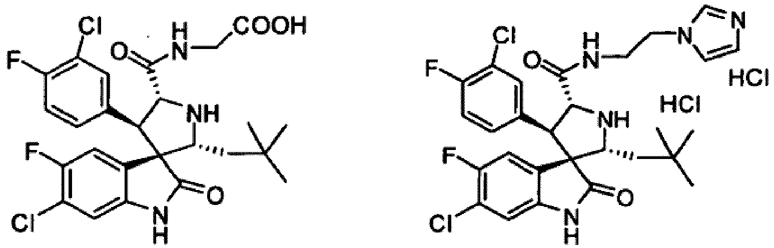 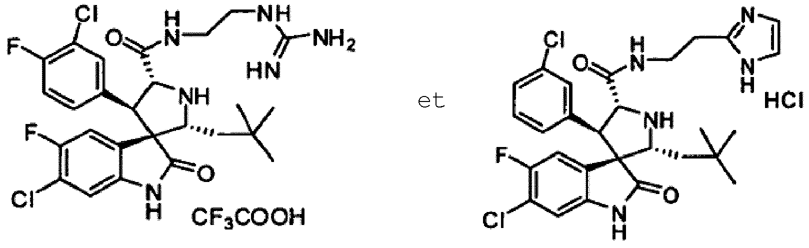 ou un sel pharmaceutiquement acceptable d'un tel composé, ou un sel pharmaceutiquement acceptable différent d'un tel composé.

14. Composé choisi dans le groupe constitué par les suivants : 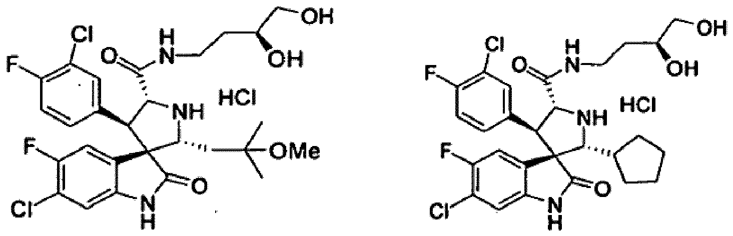 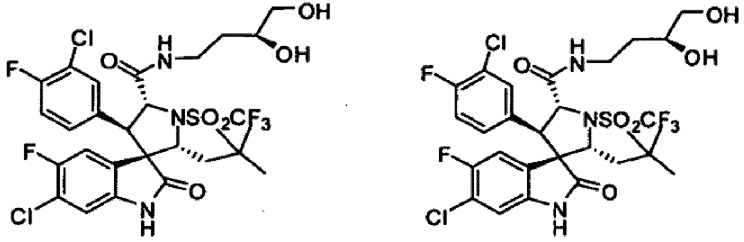 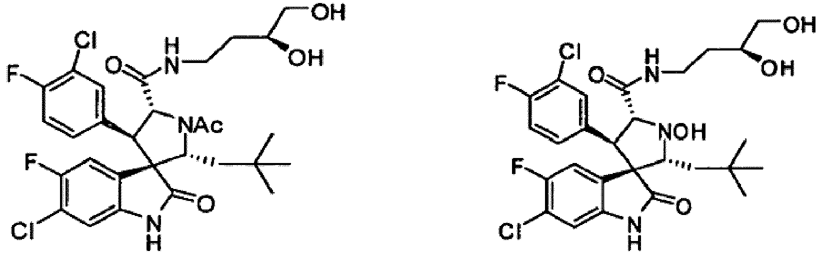 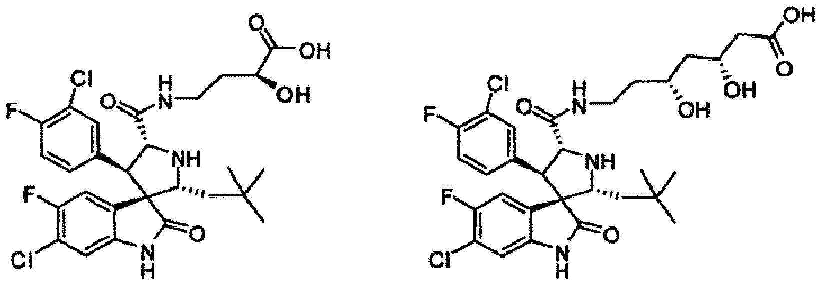 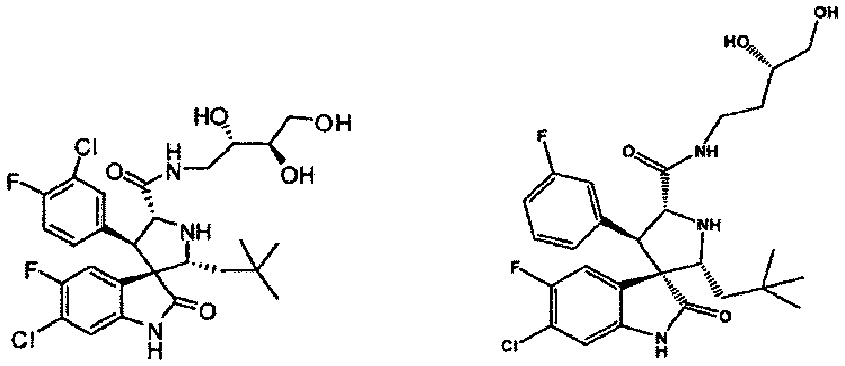 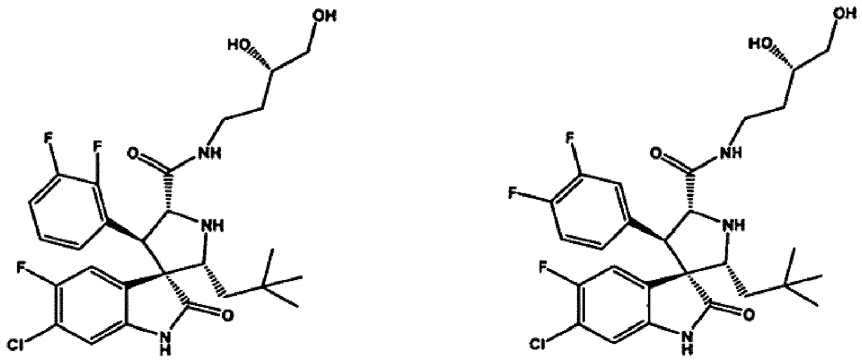 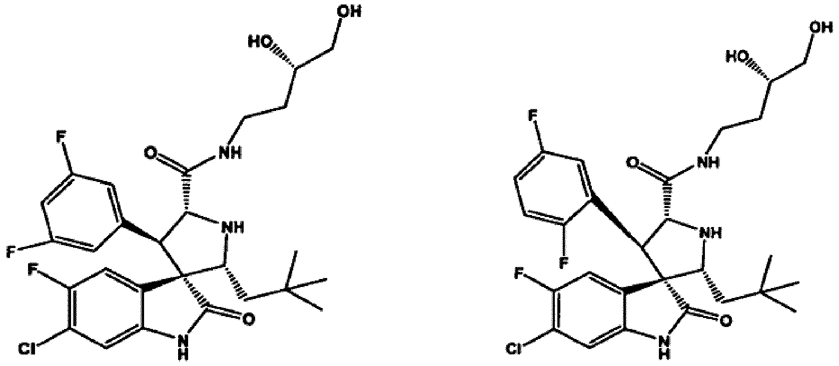 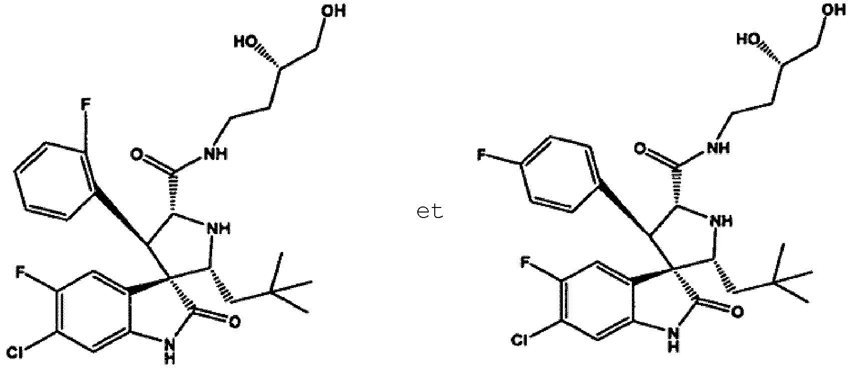 ou un sel pharmaceutiquement acceptable d'un tel composé, ou un sel pharmaceutiquement acceptable différent d'un tel composé.

15. Composition pharmaceutique comprenant un composé selon l'une quelconque des revendications 1 à 14 et un véhicule pharmaceutiquement acceptable.

16. Composé selon l'une quelconque des revendications 1 à 14, pour utilisation dans le traitement d'un cancer.

17. Composé pour utilisation selon la revendication 16, dans lequel ledit cancer est choisi dans le groupe constitué par un cancer de la prostate et un cancer du côlon.

18. Composé pour utilisation selon la revendication 16, lequel composé est destiné à être administré avec au moins un agent thérapeutique additionnel.

19. Composé selon l'une quelconque des revendications 1 à 14 pour utilisation dans le traitement d'une mucosite, d'une stomatite, d'une xérostomie, d'un trouble gastro-intestinal, ou d'une alopécie.
